(19) Europäisches Patentamt / European Patent Office / Office européen des brevets

(11) EP 3 543 362 B1

(12) **EUROPEAN PATENT SPECIFICATION**

(45) Date of publication and mention of the grant of the patent:
**08.09.2021 Bulletin 2021/36**

(51) Int Cl.:
***C12Q 1/689*** *(2018.01)*

(21) Application number: **19167951.3**

(22) Date of filing: **07.10.2014**

(54) **PCR PRIMER SET FOR BACTERIAL DNA AMPLIFICATION, KIT FOR DETECTING AND/OR IDENTIFYING BACTERIAL SPECIES, AND METHOD FOR DETECTING AND/OR IDENTIFYING BACTERIAL SPECIES**

PCR-PRIMER-SET FÜR BAKTERIELLE DNA-AMPLIFIKATION, KIT ZUM NACHWEIS UND/ODER ZUR IDENTIFIKATION BAKTERIELLER SPEZIES UND VERFAHREN ZUM NACHWEIS UND/ODER ZUR IDENTIFIKATION VON SPEZIES

ENSEMBLE D'AMORCES DE RÉACTION EN CHAÎNE PAR POLYMÉRASE POUR L'AMPLIFICATION D'ADN, TROUSSE POUR LA DÉTECTION ET/OU L'IDENTIFICATION D'ESPÈCES BACTÉRIENNES, ET PROCÉDÉ POUR LA DÉTECTION ET/OU L'IDENTIFICATION D'ESPÈCES BACTÉRIENNES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **07.10.2013 JP 2013210606**

(43) Date of publication of application:
**25.09.2019 Bulletin 2019/39**

(62) Document number(s) of the earlier application(s) in accordance with Art. 76 EPC:
**14853073.6 / 3 056 567**

(73) Proprietor: **Mitsui Chemicals, Inc.**
**Tokyo 105-7122 (JP)**

(72) Inventors:
- **AMANO, Koh**
  **Mobara-shi, Chiba 297-0017 (JP)**
- **SAKAI, Tomomi**
  **Mobara-shi, Chiba 297-0017 (JP)**
- **YANAI, Hisaaki**
  **Mobara-shi, Chiba 297-0017 (JP)**
- **TABATA, Homare**
  **Sunagawa-shi, Hokkaido 073-0138 (JP)**
- **MINAMI, Hiroshi**
  **Sunagawa-shi, Hokkaido 073-0138 (JP)**
- **KITAJIMA, Isao**
  **Toyama-shi, Toyama 930-0194 (JP)**
- **NIIMI, Hideki**
  **Toyama-shi, Toyama 930-0194 (JP)**

(74) Representative: **Mewburn Ellis LLP**
**Aurora Building**
**Counterslip**
**Bristol BS1 6BX (GB)**

(56) References cited:
**WO-A1-2007/097323**
**WO-A1-2010/082640**
**WO-A2-96/18731**
**CN-A- 103 184 270**
**JP-A- 2009 136 245**
**US-A1- 2010 196 888**

EP 3 543 362 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

Technical Field

The present invention, which is set out in the accompanying claims, relates to a primer set useful for detection and/or identification of a bacterial species by an examination method using PCR, particularly real time PCR, a kit containing this primer set for detection or identification of a bacterial species, and a method of detection and/or identification of bacterial species using them.

Background Art

Microorganism examinations are technologies required in research facilities, medical fronts, food manufacturing floors, etc. In medical fronts and food manufacturing floors, smearing, microscopy, cultivation, etc. are conventionally carried out as routine examination methods. In medical fronts, a drug susceptibility test is often carried out concurrently since some microorganisms may have antibiotic-resistant genes. However, the above-described general examination methods such as cultivation, etc. need fairly long times (days), thus, speeding up and higher sensitivity of examinations are recently required and studied eagerly. There are various examination methods developed owing to analysis of pathogenic genes, analysis of toxin genes, analysis of antibiotic-resistant genes, analysis of DNA sequences (rDNA) coding rRNA under phylogenetic viewpoint, etc., particularly due to accumulation of the published database based on gene analysis.

Now, gene examination methods are classified roughly into DNA probe methods and gene amplification methods (PCR). Especially, the PCR method is capable of amplifying a trace amount of DNA present in a sample in a short period of time and has high detection sensitivity as compared with the DNA probe method, and accordingly, its application range is wide.

Under such a background, there is an investigation on detection of unidentified bacteria using PCR, and there is a trial of amplifying a trace amount of DNA of a sepsis-derived microorganism by PCR, hybridizing the amplified causal microorganism DNA with a nucleotide probe specific to bacterial species targeting an empirically supposed microorganism, and detecting the causal microorganism and identifying the bacterial species (JP-A No. 6-90799).

Further, there is an investigation on development of sepsis examining technologies based on real time PCR using a hybridization probe, intending quickness of detection and identification of bacterial species (Journal of Analytical Bio-Science, Vol. 28, No. 5, (2005), 400 - 40). Furthermore, there is an investigation on quick detection of a causal bacterium and identification of the bacterial species, by conducting gene amplification by PCR utilizing microbial DNA as a template and using several specific primer sets and then analyzing a combination of melting temperatures (Tm value) according to the resultant several DNA fragments or a difference between the Tm values (International Publication WO 2007/097323).

However, it can be said that concomitant satisfaction of high sensitivity and high specificity is important in PCR, since precision should be ensured even in the result obtained in a short period of time. There prior technologies are a method limited to a supposed target microorganism though a gene amplification technology by PCR is applied.

In contrast, a method of producing a DNA polymerase preparation by a gene recombination technology is investigated, for providing a DNA polymerase used in PCR (JP-A No. 2006-180886). There is a suggestion on a method of suppressing amplification of a non-specific nucleic acid in conducting amplification of an extremely trace amount of sample bacterial DNA by a PCR method using a heat-resistant DNA preparation produced by using eukaryote as a host (International Publication WO 2010/082640). By using, in PCR, this heat-resistant DNA polymerase preparation produced by using eukaryote as a host, an analysis method which is suitable for novel examination of an extremely trace amount of a sample bacterium can be provided, and this technology is an extremely epoch-making method as a method of analyzing an extremely trace amount of a sample bacterium.

Further, a method is supposed in which curves are obtained by high resolution melting (HRM) curve analysis for three DNA fragments amplified from a bacterium, using a latest real time PCR apparatus, and the bacterial species is judged according to a combination of their shapes (Journal of Clinical Microbiology 2009, p.2252-2255: Journal of Clinical Microbiology 2010, p.3410-3413). However, these documents do not disclose clear criteria for judging conformity or inconformity of wave shapes. In the above-described documents, judgment of 60 bacteria is performed, however, it is guessed to be difficult to identify a wide range of bacterium species over 100 kinds by way of three curves by HRM from three DNA fragments. Additionally, a sample after hemoculture is used as a sample for extraction of DNA in this method, needing a time required for culture.

Further additionally, there is disclosed a method in which the Tm values of a amplified 16S rDNA fragment and several probes are measured using a real time PCR apparatus, and the bacterial species is specified based on the Tm values (Journal of Clinical Microbiology 2010, p.258-267). Also in this method, a sample after hemoculture is used as a sample for extracting DNA, needing a time necessary for culture.

Prior Art Document

Patent Document

Patent document 1: Japanese Patent Laid-Open No. 90799/1994 (JP H06-90799 A)
Patent document 2: International Publication WO 2007/097323
Patent document 3: Japanese Patent Laid-Open No. 2006-180886 (JP2006-180886 A)
Patent document 4: International Publication WO 2010/082640

Non-patent Document

Non-patent document 1: Journal of Analytical Bio-Science, Vol. 28, No. 5, (2005), 400-40
Non-patent document 2: Journal of Clinical Microbiology 2009, p.2252-2255
Non-patent document 3: Journal of Clinical Microbiology 2010, p.3410-3413
Non-patent document 4: Journal of Clinical Microbiology 2010, p.258-267

Summary of the Invention

Problems to be solved by the Invention

Based on the technology disclosed in Patent document 4, it is believed that if DNA can be amplified simply from an extremely trace amount of a sample bacterium and if analysis, particularly quantification or identification analysis can be effected in a short period of time, then, even a gene at extremely trace amount level unanalyzable to date can be analyzed, and additionally, quick and correct judgment is attained in various sample analysis fields such as the medical field, the veterinary field, the fields of daily water and food, etc. However, further improvement in detection sensitivity and quickness are needed from the clinical sites, food production floors, etc.

As described above, a detection method using a PCR method gives higher quickness as compared with a DNA probe method, and is suitable also for detection with high sensitivity, thus, is useful as a quick and highly sensitive detection method in the clinical sites, food production floors, etc.. However, conventional detection methods using a PCR method generate a case in which detection is impossible when the bacterial species contained in a sample is unclear and a case in which furthermore improvement in detection sensitivity and quickness cannot be sufficiently satisfied due to a longer time necessary for bacterial proliferation in a sample for ensuring the amount of nucleic acids necessary for detection, etc.

For example, in Non-patent document 4, a fair amount of bacterial bodies necessary for analysis after culture, etc. is understood also from a use amount of bacterial DNA subjected to PCR of 2-10 ng. When E. coli is exemplified as the bacterium, the genome size of the E. coli MG1655 strain is 4639675 bp, and if the average molecular weight of one base pair is calculated from this and further if the presence of one copy (1 molecule) of a genome per one bacterium cell is taken into consideration, then, it is understood that the amount of the genome DNA per 1 cell of E. coli is about 5 fg and the bacterial amount corresponding to the amount of DNA subjected to PCR is the fair amount, leading to a supposition that the detection sensitivity of this method is not sufficient.

In a case of detection and identification of a bacterium using a PCR method, selection of a primer set used in a PCR method is one important factor for enhancing sensitivity of detection and identification of an examination. For example, the amplification efficiency in PCR, the threshold cycle, the production ratio of the unintended amplification product, etc., vary depending on selection of a primer set, and for enhancing sensitivity of detection and identification of an examination, a primer set optimizing these factors is necessary. Depending on the kind of a sample, there is also a case where large amount of DNA derived from human cells other than the DNA as an analyte to be detected is present in the sample, such as a sample derived from blood, etc. For example, large amount of DNA derived from human cells such as leucocytes, desquamated epithelial cells, etc. is present in a blood specimen. According to investigations of the present inventors, when the content of the bacterial DNA as a target to be tested is small in such samples, selectivity by a primer set for PCR to bacterial DNA to be detected or productivity of the amplification product become low. As a result, there are cases where an amount of amplification product necessary for identification of species as a target to be tested cannot be obtained, or where rising of the background in identification occurs due to increase of undesired DNA amplification products. In Patent document 4, remarkable improvement in the identification sensitivity is attained by using a heat-resistant DNA polymerase preparation containing a suppressed amount of bacteria-derived DNA produced by using eukaryote as a host for identification of a bacterium as an analyte using a PCR method, in an identification method by a conventional technology which Patent document 4 utilizes, however, it is required to enable identification at further higher sensitivity also for a sample in which the amount of DNA of a bacterium as an analyte is further smaller for a bacterium other than the bacterium as an analyte present in large amount. Also from such a standpoint, design of a primer giving higher selectivity, not generating unintended amplification products, is important for a primer set to be used in a PCR method. However, a primer set designed noticing the technical problems in using samples such as blood, etc. as an analyte as described above has not been found in conventional technologies, under present circumstances.

Further, in analyzing a intended bacterium to be detected and identifying an unidentified bacterium using the Tm value of a amplification product, it is preferable to select a primer set giving a amplification product having a Tm value suitable for enhancing quickness of an examination and examination sensitivity.

In contrast, also the property of a heat-resistant DNA polymerase preparation used for amplification by a PCR method and the use condition thereof are one of important factors for enhancing the detection sensitivity of an examination using a PCR method.

Commercially available heat-resistant DNA polymerase preparations generally used in a PCR reaction include also highly purified preparations supplied in the market, and in a PCR reaction using these highly purified preparations, amplification products of unknown reasons and derivation are detected when a gene amplification reaction is required to be repeated in a frequency over usual 30 cycles, for attaining detection at high sensitivity, thus, use thereof is limited.

Patent document 4 describes mixing of host-derived bacterial DNA of a DNA polymerase, however, does not describe mixing of bacteria-derived DNA as the other contamination source from reagents and instruments. In contrast, it has been found by investigation of the present inventors that mixing of bacteria-derived DNA as a contamination source from reagents and instruments exerts an extremely critical influence on detection of an extremely trace amount of bacterial DNA in a sample and identification of the bacterial species.

Means for controlling sensitivity and specificity together at higher levels in PCR analysis for amplifying an extremely trace amount of bacteria-derived DNA in a sample have not been provided yet, and also identification of bacterial species at high sensitivity targeting bacteria-derived DNA in a sample has not been sufficiently attained.

By a detection method using a heat-resistant DNA polymerase preparation produced by using eukaryote as a host disclosed in Patent document 2, the detection limit of bacteria-derived DNA can be lowered to 10 fg/μL, and analysis of sample-derived DNA at higher sensitivity becomes possible as compared with a conventional detection method using PCR.

According to the method disclosed in Patent document 2, bacterial DNA of a concentration of 10 fg/μL or more can be detected when a PCR method is carried out using SEQ ID No. 51 and SEQ ID No. 52, however, DNA of a concentration of less than 10 fg/μL cannot be detected. It can be confirmed from this that if amplification of an intended product cannot be observed in performing PCR using SEQ ID No. 51 and SEQ ID No. 52 by the method disclosed in Patent document 2 using a certain solution as a template, then, the concentration of bacteria-derived DNA mixed in its solution is less than 10 fg/μL.

For conducting not only detection of bacterial species in a sample but also identification of bacterial species in a sample at high sensitivity with high precision, it is necessary that a sample-derived DNA amplification product is obtained in an amount sufficient for identification of bacterial species, in addition to lowering of the detection limit of the amount of bacterial-derived DNA. For example, for obtaining the highly reliable measurement result of the Tm value in performing identification of bacterial species in a sample by measuring the Tm value of the amplification product of sample-derived DNA, it is important that the amplification product in an amount sufficient for this is obtained by PCR. In other words, when sufficient amplification of DNA is not conducted, the Tm value cannot be measured correctly, and identification with high precision becomes difficult.

For example, in the case of a detection method in which the amount of genome DNA per one cell of E. coli is about 5 fg and the detection limit is 10 fg/μL, these amount of DNA per 1 μL of a sample provided to PCR corresponds theoretically to two cells of E. coli and, thus, detection of E. coli at high sensitivity in a sample becomes possible. In contrast, if the amount of E. coli-derived DNA in a sample which is necessary at minimum for measuring the reliable Tm value of a DNA amplification product, namely, the detection limit is assumed to 20 pg/μL, it is supposed that the bacterial amount corresponding to the amount of DNA provided to PCR is a fair amount.

Investigation of the present inventors obtained novel findings that it is effective to use a primer set giving further improved amplification efficiency of a amplification product and selectivity, for further lowering the detection limit and conducting identification of bacterial species at high sensitivity with good precision in a measurement system capable of detecting bacterial species at high sensitivity. For example, primers made of sequence regions common to 16SrRNA gene of all bacteria used in Patent document 2 and Patent document 4 have the same sequence, and if a primer set giving further improved amplification efficiency and selectivity of the amplification product than primer sets disclosed in these patent documents can be provided, it becomes possible to further improve not only the detection sensitivity of bacterial species but also the identification sensitivity of bacterial species.

Therefore, the present invention has an object of providing a primer set which is useful for further improvement of sensitivity in detection or identification of bacterial species in a sample by a PCR method, a set for PCR using this, and a method of detection or identification of bacterial species in a sample.

The present invention has another object of attaining further improvement of sensitivity in detection or identification of bacterial species in a sample, by conducting a PCR method using the above-described primer set while minimizing the mixing amount of bacteria-derived nucleic acids.

Means for Solving the Problem

The present inventors have intensively studied in view of the above-described problems and resultantly found that bacterial species identification kit capable of carrying out detection of an extremely trace amount of bacterial DNA in a sample and identification of the bacterial species at high sensitivity with high precision can be obtained by using a specific primer set and further minimizing mixing of bacteria-derived DNA as a contamination source of reagents and instruments to be used.

The present invention is set out in the claims. In a first embodiment, the invention provides a primer set for polymerase chain reaction (PCR) for amplification of bacterial DNA, which comprises one primer pair obtained by selecting from group S2: 2-16A: a primer pair, which consists of a combination of a forward primer consisting of a sequence of SEQ ID: No. 43 and a reverse primer consisting of any one sequence of SEQ ID: No. 44, 46 to 48 and 50;

The invention further provides a kit for detection of a bacterial species, which comprises one primer pair obtained by selecting from the following group K2: 2-16: a primer pair, which consists of a combination of a forward primer consisting of a sequence of SEQ ID: No. 43 and a reverse primer consisting of any one sequence of SEQ ID: No. 44 to 48 and 50.

The invention also provides a primer set as set out in the claims for detection and/or identification of a bacterial species.

The invention also provides a kit as set out in the claims for identification of a bacterial species.

The invention also provides a method of identification of a bacterial species as set out in the claims.

Further embodiments and aspects of the invention are set out in the claims and described in more detail below.

The kit may further comprise at least one of the regents and the parts selected from an enzyme for PCR, a pH buffer, fluorochrome, dNTP, $MgCl_2$ and a vessel for measurement, which may have, independently or as a whole, a contamination amount of bacterial nucleic acid of 10 fg or less than 10 fg.

A method of detection and/or identification of an bacterial species as a target in a sample according to the present invention is characterized in comprising:

a step of conducting PCR using bacterial genome DNA prepared from the sample, primers to obtain amplification product comprising a target gene specific to the bacterial species as a target, a thermostable DNA polymerase, and
a step of detecting and/or identifying the bacterial species as a target in the sample by detecting the target gene in the amplification product by PCR, or analyzing the amplification product by PCR,
wherein, as the primers, primer set according to the claims is used.

Also disclosed herein is a primer set for polymerase chain reaction (PCR) for amplification of bacterial DNA characterized in comprising at least one primer pair of seven primer pairs obtained by selecting one primer pair from each group of Groups S1 to S7 as described below.

Also disclosed herein is a kit for detection of a bacterial species characterized in comprising at least one primer pair of seven primer pairs obtained by selecting one primer pair from each of Groups K 1 to K7 as described below.

Effect of the Invention

The present invention can provide a primer set for bacterial DNA amplification PCR useful for detection and/or identification of bacterial species at high sensitivity and a kit for detection and/or identification of bacterial species. Further, sensitivity in detection and/or identification of bacterial species can be further improved by minimizing mixing of bacteria-derived DNA as a contamination source of reagents and instruments used in this kit. Therefore, according to the present invention, detection of an extremely trace amount of bacteria-derived DNA in a sample and detection and/or identification of bacterial species in the medical field, food field, etc. can be carried out at higher sensitivity and with higher precision than conventional examination kits.

Brief Explanation of Drawings

[Figure 1A] The figure is a view showing a part of the results of electrophoresis in Example 2 and Comparative Example 2.
[Figure 1B] The figure is a graph showing a part (amplification curve) of the results of real time PCR in Example 3 and Comparative Example 3.
[Figure 1C] The figure is a graph showing a part (melting curve) of the results of real time PCR of Example 3 and Comparative Example 3.
[Fig. 2] The figure is a view showing the result in Example 12.
[Fig. 3] The figure is a view showing the result in Example 13.
[Fig. 4] The figure is a view showing the result in Example 14. Lines represent the amplification results of the following primer pairs. (1) a combination of SEQ ID No. 15 and 16, (2) a combination of SEQ ID No. 43 and 50, (3) a combination of SEQ ID No. 51 and 52, (4) a combination of SEQ ID No. 53 and 57, (5) a combination of SEQ ID No. 62 and 70, (6) a combination of SEQ ID No. 81 and 103 and (7) a combination of SEQ ID No. 115 and 5.
[Fig. 5] The figure is a view showing the result in Example 16.

Modes for Carrying Out the Invention

<Primer>

The first point of the present invention resides in a specific primer set set out in the claims capable of obtaining an effect of improvement of detection sensitivity and an effect of improvement of identification precision based thereon.
Primers in primer sets described in Patent document 4 have DNA sequences hybridizing to bacterial-common conservation regions in DNA (16S rDNA) coding 16S rRNA of the bacteria (SEQ ID No. 1, SEQ ID No. 16, SEQ ID No. 28, SEQ ID No. 44, SEQ ID No. 51, SEQ ID No. 52, SEQ ID No. 53, SEQ ID No. 57, SEQ ID No. 62, SEQ ID No. 70, SEQ ID No. 76, SEQ ID No. 91, SEQ ID No. 104, SEQ ID No. 2). As shown in lane 2 in Fig. 1A, however, bands of other components other than the analyte to be detected appeared and the intended amplification product could not be detected in electrophoresis analysis by the primer sets described in Patent document 4. In addition, cases with low amplification efficiencies were observed occasionally in real time PCR (see dotted line in Fig. 1B). Furthermore, a peak (or shoulder) other than the main peak emerged in melting curve analysis in real time PCR (see dotted line in Fig. 1C). These may constitute an obstacle to further improvement of detection sensitivity in detection of an extremely trace amount of sample-derived DNA and identification of bacterial species.
The seven regions recognized by the primer sets of Patent document 2 were investigated, while there are commonly conserved bacterial regions on 16S rDNA. Specifically, regions were selected base on the primer sets of Patent Document 2, which shifted from several bases to several tens of bases in the directions to the 3' terminals and the 5' terminals of the commonly conserved regions, respectively, and, then, each of forward and reverse primers was designed in a length of a base number of ten to several tens. The effects were confirmed for the primer pairs corresponding to the seven designed conserved regions, and it was found that the primer pairs classified to the following groups S1 through S7 and the primer pairs classified to the following groups K1 through K7 were suitable.
Thus, also discosed herein is a primer set for PCR for amplification of bacterial DNA, which is applicable for detection and/or identification of a bacterial species,-is prepared by selecting one primer pair from every one of Groups S1 to S7 to obtain seven primer pairs, i. e., a first to a seventh primer pair, and using at least one primer pairs, preferably at least four primer pairs from the primer pair group thus selected.
A primer set for identification of a bacterial species may be prepared by using at least 4 primer pairs of the first to the seventh primer pairs thus selected.

(Primer Pairs for Primer Set)

Group S1:

1-1A: a primer pair, which consists of a combination of a forward primer consisting of a sequence of SEQ ID: No. 1 and a reverse primer consisting of any one sequence of SEQ ID: No. 22 and 27;
1-2A: a primer pair, which consists of a combination of a forward primer consisting of a sequence of SEQ ID: No. 10 and a reverse primer consisting of any one sequence of SEQ ID: No. 20 and 21;
1-3A: a primer pair, which consists of a combination of a forward primer consisting of a sequence of SEQ ID: No. 11 and a reverse primer consisting of any one sequence of SEQ ID: No. 16, 18 and 26;
1-4; a primer pair, which consists of a combination of a forward primer consisting of a sequence of SEQ ID: No. 12 and a reverse primer consisting of any one sequence of SEQ ID: No. 16 to 27;

1-5: a primer pair, which consists of a combination of a forward primer consisting of a sequence of SEQ ID: No. 13 and a reverse primer consisting of any one sequence of SEQ ID: No. 16 to 27;

1-7: a primer pair, which consists of a combination of a forward primer consisting of a sequence of SEQ ID: No. 15 and a reverse primer consisting of any one sequence of SEQ ID: No. 16 to 27;

1-8A: a primer pair, which consists of a combination of a forward primer consisting of a sequence of SEQ ID. No. 6 and a reverse primer consisting of any one sequence of SEQ ID: No. 16, 19 to 21, 23, 24, 26 and 27;

1-9A: a primer pair, which consists of a combination of a forward primer consisting of a sequence of SEQ ID: No. 7 and a reverse primer consisting of any one sequence of SEQ ID: No. 16 to 21, 23, 24, 26 and 27; or

1-10A: a primer pair, which consists of a combination of a forward primer consisting of a sequence of SEQ ID: No. 8 and a reverse primer consisting of any one sequence of SEQ ID: No. 16 to 21, 23, 24, 26 and 27;

Group S2:

2-1A: a primer pair, which consists of a combination of a forward primer consisting of a sequence of SEQ ID: No. 28 and a reverse primer consisting of any one sequence of SEQ ID: No. 45, and 47 to 50;

2-2: a primer pair, which consists of a combination of a forward primer consisting of a sequence of SEQ ID: No. 29 and a reverse primer consisting of any one sequence of SEQ ID: No. 44, 45, 47, 48 and 50;

2-3: a primer pair, which consists of a combination of a forward primer consisting of a sequence of SEQ ID: No. 30 and a reverse primer consisting of any one sequence of SEQ ID: No. 45, and 47 to 50;

2-4: a primer pair, which consists of a combination of a forward primer consisting of a sequence of SEQ ID: No. 31 and a reverse primer consisting of any one sequence of SEQ ID: No. 46 and 47;

2-5: a primer pair, which consists of a combination of a forward primer consisting of a sequence of SEQ ID: No. 32 and a reverse primer consisting of any one sequence of SEQ ID: No. 45, and 47 to 49;

2-6A: a primer pair, which consists of a combination of a forward primer consisting of a sequence of SEQ ID: No. 33 and a reverse primer consisting of any one sequence of SEQ ID: No. 44, 45 and 47 to 50;

2-7A: a primer pair, which consists of a combination of a forward primer consisting of a sequence of SEQ ID: No. 34 and a reverse primer consisting of any one sequence of SEQ ID: No. 45, 47 and 48;

2-8A: a primer pair, which consists of a combination of a forward primer consisting of a sequence of SEQ ID: No. 35 and a reverse primer consisting of any one sequence of SEQ ID: No. 44, 45, 48 and 49;

2-9A: a primer pair, which consists of a combination of a forward primer consisting of a sequence of SEQ ID: No. 36 and a reverse primer consisting of any one sequence of SEQ ID: No. 44, 45, 48 and 49;

2-10A: a primer pair, which consists of a combination of a forward primer consisting of a sequence of SEQ ID: No. 37 and a reverse primer consisting of any one sequence of SEQ ID: No. 45, and 47 to 50;

2-11: a primer pair, which consists of a combination of a forward primer consisting of a sequence of SEQ ID: No. 38 and a reverse primer consisting of any one sequence of SEQ ID: No. 44 to 50;

2-12A: a primer pair, which consists of a combination of a forward primer consisting of a sequence of SEQ ID: No. 39 and a reverse primer consisting of any one sequence of SEQ ID: No. 44 to 48 and 50;

2-13: a primer pair, which consists of a combination of a forward primer consisting of a sequence of SEQ ID: No. 40 and a reverse primer consisting of any one sequence of SEQ ID: No. 44 to 50;

2-14A: a primer pair, which consists of a combination of a forward primer consisting of a sequence of SEQ ID: No. 41 and a reverse primer consisting of any one sequence of SEQ ID: No. 45 and 46;

2-15A: a primer pair, which consists of a combination of a forward primer consisting of a sequence of SEQ ID: No. 42 and a reverse primer consisting of any one sequence of SEQ ID: No. 48 and 49; or

2-16A: a primer pair, which consists of a combination of a forward primer consisting of a sequence of SEQ ID: No. 43 and a reverse primer consisting of any one sequence of SEQ ID: No. 44, 46 to 48 and 50;

Group S3:
a primer pair, which consists of a combination of a forward primer consisting of a sequence of SEQ ID: No. 51 and a reverse primer consisting of a sequence of SEQ ID: No. 52;
Group S4:

4-1: a primer pair, which consists of a combination of a forward primer consisting of a sequence of SEQ ID: No. 53 and a reverse primer consisting of any one sequence of SEQ ID: No. 57 to 61;

4-2: a primer pair, which consists of a combination of a forward primer consisting of a sequence of SEQ ID: No. 54 and a reverse primer consisting of any one sequence of SEQ ID: No. 59 and 61;

4-3: a primer pair, which consists of a combination of a forward primer consisting of a sequence of SEQ ID: No. 55 and a reverse primer consisting of any one sequence of SEQ ID: No. 57 to 59 and 61; or

4-4: a primer pair, which consists of a combination of a forward primer consisting of a sequence of SEQ ID: No.

56 and a reverse primer consisting of any one sequence of SEQ ID: No. 57 to 59 and 61;

Group S5:

5-1A: a primer pair, which consists of a combination of a forward primer consisting of a sequence of SEQ ID: No. 62 and a reverse primer consisting of any one sequence of SEQ ID: No. 70, 73 to 75;
5-2: a primer pair, which consists of a combination of a forward primer consisting of a sequence of SEQ ID: No. 63 and a reverse primer consisting of any one sequence of SEQ ID: No. 70 to 75;
5-3: a primer pair, which consists of a combination of a forward primer consisting of a sequence of SEQ ID: No. 64 and a reverse primer consisting of any one sequence of SEQ ID: No. 70 to 75;
5-4: a primer pair, which consists of a combination of a forward primer consisting of a sequence of SEQ ID: No. 65 and a reverse primer consisting of any one sequence of SEQ ID: No. 70 to 75;
5-5A: a primer pair, which consists of a combination of a forward primer consisting of a sequence of SEQ ID: No. 66 and a reverse primer consisting of any one sequence of SEQ ID: No. 70, 72, 73 and 75;
5-6A: a primer pair, which consists of a combination of a forward primer consisting of a sequence of SEQ ID: No. 67 and a reverse primer consisting of any one sequence of SEQ ID: No. 70, 71 and 73 to 75;
5-7: a primer pair, which consists of a combination of a forward primer consisting of a sequence of SEQ ID: No. 68 and a reverse primer consisting of any one sequence of SEQ ID: No. 70 to 75; or
5-8: a primer pair, which consists of a combination of a forward primer consisting of a sequence of SEQ ID: No. 69 and a reverse primer consisting of any one sequence of SEQ ID: No. 70 to 75;

Group S6:

6-1A: a primer pair, which consists of a combination of a forward primer consisting of a sequence of SEQ ID: No. 76 and a reverse primer consisting of any one sequence of SEQ ID: No. 93, 96 and 101 to 103;
6-2: a primer pair, which consists of a combination of a forward primer consisting of a sequence of SEQ ID: No. 77 and a reverse primer consisting of any one sequence of SEQ ID: No. 91, 96, and 101 to 103;
6-3: a primer pair, which consists of a combination of a forward primer consisting of a sequence of SEQ ID: No. 78 and a reverse primer consisting of any one sequence of SEQ ID: No. 91, and 101 to 103;
6-4A: a primer pair, which consists of a combination of a forward primer consisting of a sequence of SEQ ID: No. 79 and a reverse primer consisting of any one sequence of SEQ ID: No. 91, and 101 to 103;
6-5: a primer pair, which consists of a combination of a forward primer consisting of a sequence of SEQ ID: No. 80 and a reverse primer consisting of any one sequence of SEQ ID: No. 91, 93, and 100 to 103;
6-6: a primer pair, which consists of a combination of a forward primer consisting of a sequence of SEQ ID: No. 81 and a reverse primer consisting of any one sequence of SEQ ID: No. 91, 93, and 101 to 103;
6-7: a primer pair, which consists of a combination of a forward primer consisting of a sequence of SEQ ID: No. 82 and a reverse primer consisting of any one sequence of SEQ ID: No. 91, 96, 97, and 101 to 103;
6-8: a primer pair, which consists of a combination of a forward primer consisting of a sequence of SEQ ID: No. 83 and a reverse primer consisting of any one sequence of SEQ ID: No. 91, 101 to 103;
6-9: a primer pair, which consists of a combination of a forward primer consisting of a sequence of SEQ ID: No. 84 and a reverse primer consisting of any one sequence of SEQ ID: No. 91, and 101 to 103;
6-10: a primer pair, which consists of a combination of a forward primer consisting of a sequence of SEQ ID: No. 85 and a reverse primer consisting of any one sequence of SEQ ID: No. 91, and 101 to 103;
6-11A: a primer pair, which consists of a combination of a forward primer consisting of a sequence of SEQ ID: No. 86 and a reverse primer consisting of any one sequence of SEQ ID: No. 91, 93, and 101 to 103;
6-12: a primer pair, which consists of a combination of a forward primer consisting of a sequence of SEQ ID: No. 87 and a reverse primer consisting of any one sequence of SEQ ID: No. 91, 96, and 101 to 103;
6-13: a primer pair, which consists of a combination of a forward primer consisting of a sequence of SEQ ID: No. 88 and a reverse primer consisting of any one sequence of SEQ ID: No. 91, and 101 to 103;
6-14: a primer pair, which consists of a combination of a forward primer consisting of a sequence of SEQ ID: No. 89 and a reverse primer consisting of any one sequence of SEQ ID: No. 91, and 101 to 103; or
6-15: a primer pair, which consists of a combination of a forward primer consisting of a sequence of SEQ ID: No. 90 and a reverse primer consisting of any one sequence of SEQ ID: No. 91, 92, 96, and 101 to 103;

Group S7:

7-1A: a primer pair, which consists of a combination of a forward primer consisting of a sequence of SEQ ID: No. 104 and a reverse primer consisting of any one sequence of SEQ ID: No. 5, and 118 to 125, 127, 128, 130 and 131;
7-2A: a primer pair, which consists of a combination of a forward primer consisting of a sequence of SEQ ID: No. 105 and a reverse primer consisting of any one sequence of SEQ ID: No. 5, and 118 to 125;
7-3A: a primer pair, which consists of a combination of a forward primer consisting of a sequence of SEQ ID: No. 106 and a reverse primer consisting of any one sequence of SEQ ID: No. 2, 5, 116, and 118 to 131;
7-4A: a primer pair, which consists of a combination of a forward primer consisting of a sequence of SEQ ID: No. 107 and a reverse primer consisting of any one sequence of SEQ ID: No. 120 and 121;
7-5A: a primer pair, which consists of a combination of a forward primer consisting of a sequence of SEQ ID: No. 108 and a reverse primer consisting of any one sequence of SEQ ID: No. 120 and 123;
7-6A: a primer pair, which consists of a combination of a forward primer consisting of a sequence of SEQ ID: No. 109 and a reverse primer consisting of any one sequence of SEQ ID: No. 2, 118 to 122, 124 and 126;
7-7A: a primer pair, which consists of a combination of a forward primer consisting of a sequence of SEQ ID: No. 110 and a reverse primer consisting of any one sequence of SEQ ID: No. 118, 120, 121, 124, and 126 to 130;
7-8A: a primer pair, which consists of a combination of a forward primer consisting of a sequence of SEQ ID: No. 111 and a reverse primer consisting of any one sequence of SEQ ID: No. 2, 5, and 118 to 131;
7-9A: a primer pair, which consists of a combination of a forward primer consisting of a sequence of SEQ ID: No. 112 and a reverse primer consisting of any one sequence of SEQ ID: No. 2, 5, and 116, and 118 to 125;
7-10A: a primer pair, which consists of a combination of a forward primer consisting of a sequence of SEQ ID: No. 113 and a reverse primer consisting of any one sequence of SEQ ID: No. 2, 5, 118 to 126, 130 and 131 ;
7-11A: a primer pair, which consists of a combination of a forward primer consisting of a sequence of SEQ ID: No. 114 and a reverse primer consisting of a sequence of SEQ ID: No. 5; or
7-12A: a primer pair, which consists of a combination of a forward primer consisting of a sequence of SEQ ID: No. 115 and a reverse primer consisting of any one sequence of SEQ ID: No. 2 and 5.

Furthermore, it has been revealed that the primer pairs divided into the following Groups K1 to K7 is suitable to compose a kit for detection of a bacterial species.

Thus, a kit for PCR for detection and/or identification of a bacterial species, is prepared by selecting one primer pair from every one of Groups K1 to K7 to obtain seven primer pairs, i. e., a first to a seventh primer pair, and using at least one primer pairs, preferably at least 4 primer pairs from the primer pair group thus selected.

A kit for identification of a bacterial species may be prepared by using at least 4 primer pairs of the first to the seventh primer pairs thus selected.

(Primer Pairs for Kit)

Group K1:

1-1: a primer pair, which consists of a combination of a forward primer consisting of a sequence of SEQ ID: No. 1 and a reverse primer consisting of any one sequence of SEQ ID: No. 16 to 27;
1-2: a primer pair, which consists of a combination of a forward primer consisting of a sequence of SEQ ID: No. 10 and a reverse primer consisting of any one sequence of SEQ ID: No. 16, 18, 20 and 21;
1-3: a primer pair, which consists of a combination of a forward primer consisting of a sequence of SEQ ID: No. 11 and a reverse primer consisting of any one sequence of SEQ ID: No. 16 to 21, and 23 to 26;
1-4: a primer pair, which consists of a combination of a forward primer consisting of a sequence of SEQ ID: No. 12 and a reverse primer consisting of any one sequence of SEQ ID: No. 16 to 27;
1-5: a primer pair, which consists of a combination of a forward primer consisting of a sequence of SEQ ID: No. 13 and a reverse primer consisting of any one sequence of SEQ ID: No. 16 to 27;
1-6: a primer pair, which consists of a combination of a forward primer consisting of a sequence of SEQ ID: No. 14 and a reverse primer consisting of any one sequence of SEQ ID: No. 16 to 27;
1-7: a primer pair, which consists of a combination of a forward primer consisting of a sequence of SEQ ID: No. 15 and a reverse primer consisting of any one sequence of SEQ ID: No. 16 to 27;
1-8: a primer pair, which consists of a combination of a forward primer consisting of a sequence of SEQ ID: No. 6 and a reverse primer consisting of any one sequence of SEQ ID: No. 16 to 27;
1-9: a primer pair, which consists of a combination of a forward primer consisting of a sequence of SEQ ID: No. 7 and a reverse primer consisting of any one sequence of SEQ ID: No. 16 to 27;
1-10: a primer pair, which consists of a combination of a forward primer consisting of a sequence of SEQ ID: No. 8 and a reverse primer consisting of any one sequence of SEQ ID: No. 16 to 27; or

1-11: a primer pair, which consists of a combination of a forward primer consisting of a sequence of SEQ ID: No. 9 and a reverse primer consisting of any one sequence of SEQ ID: No. 16, 17, 19 and 26;

Group K2:

2-1: a primer pair, which consists of a combination of a forward primer consisting of a sequence of SEQ ID: No. 28 and a reverse primer consisting of any one sequence of SEQ ID: No. 44, 45, and 47 to 50;
2-2: a primer pair, which consists of a combination of a forward primer consisting of a sequence of SEQ ID: No. 29 and a reverse primer consisting of any one sequence of SEQ ID: No. 44, 45, 47, 48 and 50;
2-3: a primer pair, which consists of a combination of a forward primer consisting of a sequence of SEQ ID: No. 30 and a reverse primer consisting of any one sequence of SEQ ID: No. 45, and 47 to 50;
2-4: a primer pair, which consists of a combination of a forward primer consisting of a sequence of SEQ ID: No. 31 and a reverse primer consisting of any one sequence of SEQ ID: No. 46 and 47;
2-5: a primer pair, which consists of a combination of a forward primer consisting of a sequence of SEQ ID: No. 32 and a reverse primer consisting of any one sequence of SEQ ID: No. 45, and 47 to 49;
2-6: a primer pair, which consists of a combination of a forward primer consisting of a sequence of SEQ ID: No. 33 and a reverse primer consisting of any one sequence of SEQ ID: No. 44 to 50;
2-7: a primer pair, which consists of a combination of a forward primer consisting of a sequence of SEQ ID: No. 34 and a reverse primer consisting of any one sequence of SEQ ID: No. 45 to 50;
2-8: a primer pair, which consists of a combination of a forward primer consisting of a sequence of SEQ ID: No. 35 and a reverse primer consisting of any one sequence of SEQ ID: No. 44 to 50;
2-9: a primer pair, which consists of a combination of a forward primer consisting of a sequence of SEQ ID: No. 36 and a reverse primer consisting of any one sequence of SEQ ID: No. 44 to 46, and 48 to 50;
2-10: a primer pair, which consists of a combination of a forward primer consisting of a sequence of SEQ ID: No. 37 and a reverse primer consisting of any one sequence of SEQ ID: No. 45 to 50;
2-11: a primer pair, which consists of a combination of a forward primer consisting of a sequence of SEQ ID: No. 38 and a reverse primer consisting of any one sequence of SEQ ID: No. 44 to 50;
2-12: a primer pair, which consists of a combination of a forward primer consisting of a sequence of SEQ ID: No. 39 and a reverse primer consisting of any one sequence of SEQ ID: No. 44 to 50;
2-13: a primer pair, which consists of a combination of a forward primer consisting of a sequence of SEQ ID: No. 40 and a reverse primer consisting of any one sequence of SEQ ID: No. 44 to 50;
2-14: a primer pair, which consists of a combination of a forward primer consisting of a sequence of SEQ ID: No. 41 and a reverse primer consisting of any one sequence of SEQ ID: No. 44 to 50;
2-15: a primer pair, which consists of a combination of a forward primer consisting of a sequence of SEQ ID: No. 42 and a reverse primer consisting of any one sequence of SEQ ID: No. 44 to 50; or
2-16: a primer pair, which consists of a combination of a forward primer consisting of a sequence of SEQ ID: No. 43 and a reverse primer consisting of any one sequence of SEQ ID: No. 44 to 48, and 50;

Group K3:
a primer pair, which consists of a combination of a forward primer consisting of a sequence of SEQ ID: No. 51 and a reverse primer consisting of a sequence of SEQ ID: No. 52;
Group K4:

4-1: a primer pair, which consists of a combination of a forward primer consisting of a sequence of SEQ ID: No. 53 and a reverse primer consisting of any one sequence of SEQ ID: No. 57 to 61;
4-2: a primer pair, which consists of a combination of a forward primer consisting of a sequence of SEQ ID: No. 54 and a reverse primer consisting of any one sequence of SEQ ID: No. 59 and 61;
4-3: a primer pair, which consists of a combination of a forward primer consisting of a sequence of SEQ ID: No. 55 and a reverse primer consisting of any one sequence of SEQ ID: No. 57 to 59, and 61; or
4-4: a primer pair, which consists of a combination of a forward primer consisting of a sequence of SEQ ID: No. 56 and a reverse primer consisting of any one sequence of SEQ ID: No. 57 to 59, and 61;

Group K5:

5-1: a primer pair, which consists of a combination of a forward primer consisting of a sequence of SEQ ID: No. 62 and a reverse primer consisting of any one sequence of SEQ ID: No. 70, 71, and 73 to 75;
5-2: a primer pair, which consists of a combination of a forward primer consisting of a sequence of SEQ ID: No. 63 and a reverse primer consisting of any one sequence of SEQ ID: No. 70 to 75;

5-3: a primer pair, which consists of a combination of a forward primer consisting of a sequence of SEQ ID: No. 64 and a reverse primer consisting of any one sequence of SEQ ID: No. 70 to 75;
5-4: a primer pair, which consists of a combination of a forward primer consisting of a sequence of SEQ ID: No. 65 and a reverse primer consisting of any one sequence of SEQ ID: No. 70 to 75;
5-5: a primer pair, which consists of a combination of a forward primer consisting of a sequence of SEQ ID: No. 66 and a reverse primer consisting of any one sequence of SEQ ID: No. 70 to 75;
5-6: a primer pair, which consists of a combination of a forward primer consisting of a sequence of SEQ ID: No. 67 and a reverse primer consisting of any one sequence of SEQ ID: No. 70 to 75;
5-7: a primer pair, which consists of a combination of a forward primer consisting of a sequence of SEQ ID: No. 68 and a reverse primer consisting of any one sequence of SEQ ID: No. 70 to 75; or
5-8: a primer pair, which consists of a combination of a forward primer consisting of a sequence of SEQ ID: No. 69 and a reverse primer consisting of any one sequence of SEQ ID: No. 70 to 75;

Group K6:

6-1: a primer pair, which consists of a combination of a forward primer consisting of a sequence of SEQ ID: No. 76 and a reverse primer consisting of any one sequence of SEQ ID: No. 91, 93, 96, and 101 to 103;
6-2: a primer pair, which consists of a combination of a forward primer consisting of a sequence of SEQ ID: No. 77 and a reverse primer consisting of any one sequence of SEQ ID: No. 91, 96, and 101 to 103;
6-3: a primer pair, which consists of a combination of a forward primer consisting of a sequence of SEQ ID: No. 78 and a reverse primer consisting of any one sequence of SEQ ID: No. 91, and 101 to 103;
6-4: a primer pair, which consists of a combination of a forward primer consisting of a sequence of SEQ ID: No. 79 and a reverse primer consisting of anyone sequence of SEQ ID: No. 91, 97, and 101 to 103;
6-5: a primer pair, which consists of a combination of a forward primer consisting of a sequence of SEQ ID: No. 80 and a reverse primer consisting of any one sequence of SEQ ID: No. 91, 93, and 100 to 103;
6-6: a primer pair, which consists of a combination of a forward primer consisting of a sequence of SEQ ID: No. 81 and a reverse primer consisting of anyone sequence of SEQ ID: No. 91, 93, and 101 to 103;
6-7: a primer pair, which consists of a combination of a forward primer consisting of a sequence of SEQ ID: No. 82 and a reverse primer consisting of any one sequence of SEQ ID: No. 91, 96, 97, and 101 to 103;
6-8: a primer pair, which consists of a combination of a forward primer consisting of a sequence of SEQ ID: No. 83 and a reverse primer consisting of any one sequence of SEQ ID: No. 91, 101 to 103;
6-9: a primer pair, which consists of a combination of a forward primer consisting of a sequence of SEQ ID: No. 84 and a reverse primer consisting of any one sequence of SEQ ID: No. 91, and 101 to 103;
6-10: a primer pair, which consists of a combination of a forward primer consisting of a sequence of SEQ ID: No. 85 and a reverse primer consisting of any one sequence of SEQ ID: No. 91, and 101 to 103;
6-11: a primer pair, which consists of a combination of a forward primer consisting of a sequence of SEQ ID: No. 86 and a reverse primer consisting of any one sequence of SEQ ID: No. 91, 93, 97, and 101 to 103;
6-12: a primer pair, which consists of a combination of a forward primer consisting of a sequence of SEQ ID: No. 87 and a reverse primer consisting of any one sequence of SEQ ID: No. 91, 96, and 101 to 103;
6-13: a primer pair, which consists of a combination of a forward primer consisting of a sequence of SEQ ID: No. 88 and a reverse primer consisting of any one sequence of SEQ ID: No. 91, and 101 to 103;
6-14: a primer pair, which consists of a combination of a forward primer consisting of a sequence of SEQ ID: No. 89 and a reverse primer consisting of any one sequence of SEQ ID: No. 91, and 101 to 103; or
6-15: a primer pair, which consists of a combination of a forward primer consisting of a sequence of SEQ ID: No. 90 and a reverse primer consisting of any one sequence of SEQ ID: No. 91, 92, 96, and 101 to 103;

Group K7:

7-1: a primer pair, which consists of a combination of a forward primer consisting of a sequence of SEQ ID: No. 104 and a reverse primer consisting of any one sequence of SEQ ID: No. 2, 5, and 116 to 131;
7-2: a primer pair, which consists of a combination of a forward primer consisting of a sequence of SEQ ID: No. 105 and a reverse primer consisting of any one sequence of SEQ ID: No. 2, 5, and 116 to 131;
7-3: a primer pair, which consists of a combination of a forward primer consisting of a sequence of SEQ ID: No. 106 and a reverse primer consisting of any one sequence of SEQ ID: No. 2, 5, and 116 to 131;
7-4: a primer pair, which consists of a combination of a forward primer consisting of a sequence of SEQ ID: No. 107 and a revers primer consisting of any one sequence of SEQ ID: No. 2, 5, and 116 to 131;
7-5: a primer pair, which consists of a combination of a forward primer consisting of a sequence of SEQ ID: No. 108 and a reverse primer consisting of any one sequence of SEQ ID: No. 2, 5, and 116 to 131;

7-6: a primer pair, which consists of a combination of a forward primer consisting of a sequence of SEQ ID: No. 109 and a reverse primer consisting of any one sequence of SEQ ID: No. 2, 5, and 116 to 131;
7-7: a primer pair, which consists of a combination of a forward primer consisting of a sequence of SEQ ID: No. 110 and a reverse primer consisting of any one sequence of SEQ ID: No. 2, 5, and 116 to 131;
7-8: a primer pair, which consists of a combination of a forward primer consisting of a sequence of SEQ ID: No. 111 and a reverse primer consisting of any one sequence of SEQ ID: No. 2, 5, and 116 to 131;
7-9: a primer pair, which consists of a combination of a forward primer consisting of a sequence of SEQ ID: No. 112 and a reverse primer consisting of any one sequence of SEQ ID: No. 2, 5, and 116 to 131;
7-10: a primer pair, which consists of a combination of a forward primer consisting of a sequence of SEQ ID: No. 113 and a reverse primer consisting of any one sequence of SEQ ID: No. 2, 5, and 116 to 131;
7-11: a primer pair, which consists of a combination of a forward primer consisting of a sequence of SEQ ID: No. 114 and a reverse primer consisting of any one sequence of SEQ ID: No. 2, 5 and 125; or
7-12: a primer pair, which consists of a combination of a forward primer consisting of a sequence of SEQ ID: No. 115 and a reverse primer consisting of any one sequence of SEQ ID: No. 2, 5, 116, 118, 123, and 125 to 128.

The above primer set, which comprises a primer pair selected from Groups S1 to S7, preferably comprises at least one primer pair from the following seven primer pairs of S-1) to S-7).

S-1) as the primer set of Group S1, a primer pair, which consists of a combination of a forward primer consisting of a sequence of SEQ ID: No. 15 and a reverse primer consisting of a sequence of SEQ ID: No. 16;
S-2) as the primer set of Group S2, a primer pair, which consists of a combination of a forward primer consisting of a sequence of SEQ ID: No. 43 and a reverse primer consisting of a sequence of SEQ ID: No. 50;
S-3) as the primer set of Group S3, a primer pair, which consists of a combination of a forward primer consisting of a sequence of SEQ ID: No. 51 and a reverse primer consisting of a sequence of SEQ ID: No. 52;
S-4) as the primer set of Group S4, a primer pair, which consists of a combination of a forward primer consisting of a sequence of SEQ ID: No. 53 and a reverse primer consisting of a sequence of SEQ ID: No. 57;
S-5) as the primer set of Group S5, a primer pair, which consists of a combination of a forward primer consisting of a sequence of SEQ ID: No. 62 and a reverse primer consisting of a sequence of SEQ ID: No. 70;
S-6) as the primer set of Group S6, a primer pair, which consists of a combination of a forward primer consisting of a sequence of SEQ ID: No. 81 and a reverse primer consisting of a sequence of SEQ ID: No. 1 03; and
S-7) as the primer set of Group S7, a primer pair, which consists of a combination of a forward primer consisting of a sequence of SEQ ID: No. 115 and a reverse primer consisting of a sequence of SEQ ID: No. 5.

In addition, the above primer set comprises preferably any one of the primer pairs of the above S-1) to S-7) as an essential component for detection of bacterial species, and comprises preferably any one combination of the following A to G for detection and/or identification of bacterial species.

(A) when the primer set essentially comprises primer set S-1), the primer set further comprises at least 3 primer pairs selected form S-2) to S-7);
(B) when the primer set essentially comprises primer set S-2), the primer set further comprises at least 3 primer pairs selected form S-1) and S-3) to S-7);
(C) when the primer set essentially comprises primer pair S-3), the primer set further comprises at least 3 primer pairs selected form S-1), S-2) and S-4) to S-7);
(D) when the primer set essentially comprises primer pair S-4), the primer set further comprises at least 3 primer pairs selected form S-1) to S-3) and S-5) to S-7);
(E) when the primer set essentially comprises primer pair S-5), the primer set further comprises at least 3 primer pairs selected form S-1) to S-4) and S-6) to S-7);
(F) when the primer set essentially comprises primer pair S-6), the primer set further comprises at least 3 primer pairs selected form S-1) to S-5) and S-7);
(G) when the primer set essentially comprises primer pair S-7), the primer set further comprises at least 3 primer pairs selected form S-1) to S-6).

The above primer set, which comprises a primer pair selected from the above Groups K1 to K7, preferably comprises at least one primer pair from the seven primer pairs of the following K-1) to K-7).

K-1) as the primer set of Group K1, a primer pair, which consists of a combination of a forward primer consisting of a sequence of SEQ ID: No. 15 and a reverse primer consisting of a sequence of SEQ ID: No. 16;
K-2) as the primer set of Group K2, a primer pair, which consists of a combination of a forward primer consisting of a sequence of SEQ ID: No. 43 and a reverse primer consisting of a sequence of SEQ ID: No. 50;
K-3) as the primer set of Group K3, a primer pair, which consists of a combination of a forward primer consisting of a sequence of SEQ ID: No. 51 and a reverse primer consisting of a sequence of SEQ ID: No. 52;
K-4) as the primer set of Group K4, a primer pair, which consists of a combination of a forward primer consisting of a sequence of SEQ ID: No. 53 and a reverse primer consisting of a sequence of SEQ ID: No. 57;
K-5) as the primer set of Group K5, a primer pair, which consists of a combination of a forward primer consisting of a sequence of SEQ ID: No. 62 and a reverse primer consisting of a sequence of SEQ ID: No. 70;
K-6) as the primer set of Group K6, a primer pair, which consists of a combination of a forward primer consisting of a sequence of SEQ ID: No. 81 and a reverse primer consisting of a sequence of SEQ ID: No. 103; and
K-7) as the primer set of Group K7, a primer pair, which consists of a combination of a forward primer consisting of a sequence of SEQ ID: No. 115 and a reverse primer consisting of a sequence of SEQ ID: No. 5.

In addition, the above primer set for a kit comprises preferably any one of the primer pairs in the above K-1) to K-7) as an essential component for detection of bacterial species, and comprises preferably any one combination of the following A to G for detection and/or identification of bacterial species.

(A) when the primer set essentially comprises primer set K-1), the primer set further comprises at least 3 primer pairs selected form K-2) to K-7);
(B) when the primer set essentially comprises primer set K-2), the primer set further comprises at least 3 primer pairs selected form K-1) and K-3) to K-7);
(C) when the primer set essentially comprises primer pair K-3), the primer set further comprises at least 3 primer pairs selected form K-1), K-2) and K-4) to K-7);
(D) when the primer set essentially comprises primer pair K-4), the primer set further comprises at least 3 primer pairs selected form K-1) to K-3) and K-5) to K-7);
(E) when the primer set essentially comprises primer pair K-5), the primer set further comprises at least 3 primer pairs selected form K-1) to K-4) and K-6) to K-7);
(F) when the primer set essentially comprises primer pair K-6), the primer set further comprises at least 3 primer pairs selected form K-1) to K-5) and K-7);
(G) when the primer set essentially comprises primer pair K-7), the primer set further comprises at least 3 primer pairs selected form K-1) to K-6).

By combining four from the primer pairs S-1 to S-7 in the above-described primer sets, identification of bacterial species can be conducted, and it is preferable that the number of pairs selected from these 7 primer pairs is increased to 5 or more, and it is more preferable that all of the seven primer pairs S-1 to S-7 are combined. Identification precision can be further improved by thus increasing the number of pairs to be selected.
Likewise, by combining four out of primer pairs K-1 to K-7 in the above-described primer sets, identification of bacterial species can be conducted, and it is preferable that the number of pairs selected from these 7 primer pairs is increased to 5 or more, and it is more preferable that all of the seven primer pairs K-1 to K-7 are combined. Identification precision can be further improved by thus increasing the number of pairs to be selected.
Those, in which some bases in a primer sequence are converted, can also be applied, provided that the effect of the present invention can be obtained. Basically, those hybridizable with complementary strands of DNA fragments having SEQ ID Numbers described in the claims of the present application are regarded as equivalent to the primer of the present invention. The primer sequences obtained by addition, deletion or substitution of 1 to 2 bases of primer sequences are regarded also as equivalent to the primer of the present invention. Such equivalents are not covered by the claimed invention.
It is noted that the primer sets of the present invention which are set out in the claims have been found for the first time after confirmation of their effects based on a large amount of combinations and could not be attained by simple confirmation of the effects, and that the primer sets have been confirmed as the novel combinations as the primer sets for detection and/or identification of bacterial species, as a result of sequence search.
The present invention does not prevent concomitant use of several known primers for fungi, specific microorganisms or viruses, in addition to the primer set of the present claimed invention.
The primer set of the present claimed invention is suitably used for the purpose of amplifying the intended DNA fragment by PCR from bacterial DNA extracted from a sample, measuring Tm of the amplified DNA fragment, measuring the molecular weight thereof, and decoding its sequence. Specifically, it is suitably used for identifying bacterial species by the Tm value of a DNA fragment composed of a part of 16S rDNA sequence of a bacterium to be amplified by the primer set of the present claimed invention.
In amplification of DNA by PCR and in measurement of Tm, DNA containing the intended sequence may be previously amplified by a pair of forward and reverse primers having a base number of 15 to 30 b containing a DNA sequence hybridizing to bacterial-common conservation region on 16S rDNA before using at least one primer pair out of the above-described first to seventh primer pairs for a primer set or for a kit, and PCR amplification may be further performed on the amplified sample by the above-described primer set (so-called, Nested PCR method and Semi-nested PCR method).

The pair of forward and reverse primers having a base number of 15 to 30 b used for first amplification of DNA by PCR is not particularly restricted providing it contains a DNA sequence hybridizing to a bacterial-common conservation region on 16S rDNA sequence, and it is preferable that the DNA chain length of the PCR product using this primer pair is 500 bp to 1500 bp. In particular, primers including those which generate amplification product, to which a primer set for the second amplification hybridizes, concretely such the primer pair described in Groups K 1 to K7, and, thus, from which at least four DNA fragments are obtained, are preferable.

The primer set used in the present claimed invention is suitably used for detection of an extremely trace amount of sample bacterial DNA and/or identification of bacterial species. Therefore, it is necessary that contamination of bacteria-derived DNA as a contamination source into each primer pair is suppressed to minimum. The amount of contamination of bacteria-derived DNA as a contamination source is less than 10 fg, preferably less than 5 fg, further preferably less than 1 fg with respect to a primer pair preparation solution.

The primer concentration in PCR is not particularly restricted and in general, it is appropriately set depending on investigation in a range of 0.05 μM to 1.0 μM.

<Reagent>

As the reagents other than the primer set used for PCR analysis used in the present invention, any known reagents can be used in any known combinations. The reagents necessary for measurement include an enzyme for PCR, a pH buffer solution, dNTP, a $Mg^{2+}$ source, and sterile water purified by a filtration treatment, etc. In real time PCR analysis, a fluorochrome is required in addition to the above-described materials.

<Enzyme for PCR>

As the enzyme for PCR used in the present invention, a heat-resistant DNA polymerase in which the amount of contamination of bacteria-derived DNA is suppressed to minimum is preferably used. Specific examples thereof include, but not limited to, those produced by eukaryotes, those highly purified, or those treated with EMA (ethidium monoazide) or PMA (propidium monoazide) as a selectively membrane-permeable dye.

In the present invention, the amount of mixing of bacteria-derived DNA from a host or contamination source is less than 10 fg, preferably less than 5 fg, further preferably less than 1 fg with respect to an enzyme preparation solution. In the case of use of a heat-resistant DNA polymerase preparation (preparation solution) produced by an eukaryote utilizing a bacteria-derived heat-resistant DNA polymerase gene in which DNA from a bacteria-derived heat-resistant DNA polymerase gene is mixed, the amount of mixing of bacteria-derived DNA other than a gene coding the heat-resistant DNA polymerase is not higher than the above-described upper limit. Taq DNA Polymerase having a bacteria-derived DNA content less than 10 fg/μL is preferable as the enzyme for PCR.

The gene coding the enzyme for PCR used in the present invention may be any gene such as cDNA coding a heat-resistant DNA polymerase, genome DNA, synthetic DNA, etc., and may be a single-stranded chain or a double-stranded chain having its complementary strand, and may contain a natural or artificial nucleotide derivative. Further, when the heat-resistant DNA polymerase is derived from an organism, the derivation of the heat-resistant DNA polymerase is not particularly restricted.

Specific examples of the heat-resistant, i.e., thermostable DNA polymerase used in the present invention include heat-resistant DNA polymerases derived from Thermus aquaticus, Thermus thermophilus, Bacillus stearothermophilus, Thermococcus gorgonarius, Thermococcus kodakaraensis KOD1, Pyrococcus woesei, Pyrococcus furiosus, Aeropyrum pernix, Aquifex aeolicus, Sulfolobus tokodaii, Pyrolobus fumarii or Methanopyrus kandleri.

Further, the heat-resistant DNA polymerase may be a biogenetically artificially synthesized heat-resistant DNA polymerase.

The enzyme for PCR used in the present invention is preferably an organism-derived heat-resistant DNA polymerase having heat resistance, more preferably one derived from a prokaryote such as methane bacteria, thermoacidophiles, thermophiles, hyperthermophiles, etc.

(Production by eukaryote)

The enzyme for PCR used in the present invention is preferably an enzyme produced by using a eukaryote as a host.

The eukaryotic cell includes fungi, animal cells, plant cells, insect cells, etc., and the host cell is not particularly restricted and may be a cell derived from a eukaryote.

The fungi include ascomycetes such as yeasts, fungi, etc., filamentous bacteria, basidiomycetes, zygomycetes, etc., and of them, yeasts or filamentous bacteria are preferable. Specific examples thereof include Saccharomyces, Schizosaccharomyces, Candida, Pichia, Hansenula, Kluyveromyces, Zygosaccharomyces, Yarrowia, Trichosporon, Rhodosporidi, Aspergillus, Fusarium, Trichoderma, etc.

Specifically, Saccharomyces cerevisiae, Schizosaccharomyces pombe, Candida utilis, Candida boidini, Pichia metanolica, Pichia angusta, Pichia pastoris, Pichia anomala, Hansenula polymorpha, Kluyveromyces lactis, Zygosaccharomyces rouxii, Yarrowia lipolytica, Trichosporon pullulans, Rhodosporidium toruloides, Aspergillus niger, Aspergillus nidulans, Aspergillus awamori, Aspergillus oryzae, Trichoderma reesei, etc. are mentioned.

The animal cells include human-derived cultured cells, mouse-derived cultured cells, etc., and specific examples thereof include CHO cells, Hela cells, etc. The plant cells may be cells derived from plants, are desirably established cultured cells and include Nicotiana cells, Arabidopsis cells, Ipomoea cells, Daucus cells, Oryza cells, etc. Specific examples thereof include Nicotiana tabacum BY-2 cultured cells, Arabidopsis thaliana cultured cells, Ipomoea batatas cultured cells, Daucus carota cultured cells, Oryza sativa cultured cells, etc. The insect cells may be cells derived from insects, are desirably established cultured cells and include Spodoptera frugiperda ovary cell-derived cultured cell sf9 strain and sf21 strain, Bombix mori cultured cell Bm-N strain, etc. The host cells are preferably microorganisms or eukaryotes of quick multiplication such as yeasts, etc., and examples thereof include yeasts typically including Saccharomyces such as Saccharomyces cerevisiae, etc., plant cells typically including cultured cells of Nicotiana plants such as Nicotiana tabacum, etc., and filamentous bacteria typically including Aspergillus such as Aspergillus oryzae, etc.

The production method is not particularly restricted, and production can be carried out appropriately according to, for example, a method described in Patent document 4.

(High purification treatment)

Even general enzymes, which was produced by using bacteria such as E. coli, etc. as a host, can be used in the present invention provided that they were treated by a high purification treatment to physically remove host bacterium-derived DNA using a DNA degrading enzyme, an ion exchange resin, etc. and in which the amount of contamination of bacteria-derived DNA as a contamination source is less than 10 fg with respect to the enzyme preparation.

(Treatment with selectively membrane-permeable dye)

By mixing the enzyme for PCR with EMA (ethidium monoazide) or PMA (propidium monoazide) as a selectively membrane-permeable dye and irradiating it with light, bacteria-derived DNA as a contamination source, particularly, killed bacteria-derived DNA can be inactivated. Since the inactivated DNA does not present amplification by PCR, only DNA derived from an intended bacterium to be detected present in a sample can be detected and the bacterial species can be identified. The treatment conditions comply with conditions recommended by a company of manufacturing and distributing the reagent and the reagent treating agent.

<Other reagent, instrument, etc.>

The pH buffer solution is used for adjusting pH of a sample provided to PCR analysis to 7.0 to 10.0 (more preferably, pH 8.0 to 9.0). Specific examples thereof include a Tris-HCl buffer solution, etc. For example, the Tris-HCl buffer solution is used at a concentration of 5 mM to 100 mM.

dNTP is a nucleoside source for amplification of DNA by PCR, and four kinds: dATP, dGTP, dCTP and dTTP are necessary. As the dNTP, one chemically modified for application in a hot start method, for example, CleanAmp™ dNTP manufactured by TriLink BioTechnologies, Inc. may be used. Regarding the use amount, dATP, dGTP, dCTP and dTTP are used each at a concentration of around 0.2 mM.

In amplification of DNA by PCR, $Mg^{2+}$ is necessary. The $Mg^{2+}$ source includes $MgCl_2$, $MgSO_4$, etc. $MgCl_2$ is preferable and used appropriately in a use amount of 0.5 mM to 5.0 mM depending on investigation.

The fluorochrome is used for the purpose of detecting a DNA amplification product by real time PCR, and further, for the purpose of measuring Tm of an amplified DNA fragment, and various known fluorochromes can be used. For example, a method of using an intercalator having a labeling function, a method of using a probe obtained by connecting a fluorescent substance to a nucleotide hybridizing specifically to a DNA sequence to be amplified, etc., are mentioned. The intercalator includes ethidium bromide and SYBR Green I as unsaturated fluorochromes; and Resolight (manufactured by Roche), EvaGreen (manufactured by Biotim), etc., as saturated fluorochromes, etc. Preferable intercalators are SYBR Green I as an unsaturated fluorochrome and EvaGreen and Resolight as a saturated fluorochrome, more preferable intercalators are EvaGreen and Resolight as a saturated fluorochrome. The use amount complies with recommendation of a company of producing and distributing the fluorochrome to be used.

Additionally, genome DNA of a bacterium which is used as a positive control of PCR and sterile water which is used as a negative control may be contained in the kit.

As the vessel for PCR analysis and measurement used in the present invention (for example, tube for analysis and measurement), those having a shape and a structure appropriate to a measuring machine can be selected and used. Those recommended by a measuring machine distributing source can be used for measurement without particular problems.

Other necessary instruments in carrying out the present invention include instruments widely used in molecular biological experiments such as pipettes, pipette tips, 1.5 ml microtubes, etc., and apparatuses thereof include means widely used in molecular biological experiments such as PCR, a clean bench, a centrifugal machine for a tube, etc.

<Contamination level>

The second point of the present invention is reduction of the level of contamination of bacteria-derived DNA as a contamination source in the whole sample solution prepared for PCR measurement including a primer used for detection and/or identification having the reduced level thereof, which does not disturb detection of an extremely trace amount of a sample bacterium and identification of bacterial species.

Patent document 4 describes that the amount of contamination of bacteria-derived DNA in a heat-resistant polymerase preparation solution is preferably 10 fg or less. As a result of investigation by the present inventors, however, it was found that also bacterial contamination in reagents and instruments is an extremely important factor for detection of an extremely trace amount of a sample bacterium and identification of bacterial species. When commercially available reagents and instruments were used directly to analysis, as they are, PCR amplifications was occasionally observed (Comparative Example 11).

In the present invention, suppression of contamination of bacteria-derived DNA as a contamination source is necessary, not only for reagents to be used, but also for instruments and filtered water used for preparation of solutions for measurement. Regarding the instruments, the instruments are washed with water for washing capable of dissolving DNA, which has no DNA contamination in an operation space closing off DNA contamination from the environment, such as a clean room, etc. DNA contamination can be checked by analyzing the washing water after washing by a PCR method. When appropriate, contamination of DNA may be checked by using this method.

In the present invention, the amount of contamination of bacteria-derived DNA as a contamination source is 10 fg or less, preferably 5 fg or less, further preferably 1 fg or less with respect to the whole kit for identification. For respective reagents and instruments, the amount is lower than 10 fg, preferably lower than 5 fg, further preferably lower than 1 fg with respect to an individual preparation solution, depending on the embodiment of a kit described later. By suppressing the amount of mixing of bacteria-derived DNA as a contamination source to such a level, there are no emergence of unnecessary peaks and no deviation of the Tm value even in execution of amplification of 30 cycles or more in PCR analysis necessary for detection of an extremely trace amount of a sample bacterium and identification of bacterial species, thus, detection of bacterial species and identification of bacterial species can be effected at high sensitivity with high precision.

< Method of removing contaminant >

The method of suppressing contamination of bacteria-derived DNA as a contamination source in reagents and instruments used in the present invention includes an UV irradiation treatment, a gamma ray sterilization treatment, an ultrafiltration treatment, an EMA treatment, etc. When it is evident that the amount of mixing of bacteria-derived DNA as a contamination source is 10 fg or less by a receiving inspection, etc., the following treatments are not necessarily required.

(Gamma ray sterilization treatment)

Reagents and instruments used in the present invention can be directly irradiated with a gamma ray to break bacteria-derived DNA as a contamination source. Gamma ray irradiation conditions vary depending on the kind of the material to be irradiated. In general, 5 kGy to 30 kGy, preferably 10 kGy to 25 kGy are applied.

For specific fluorochromes such as Resolight, EvaGreen, SYBR Green I, etc., primers and instruments made of specific materials such as polypropylene, etc., however, a gamma ray sterilization treatment cannot be applied depending on conditions since they deteriorate by gamma ray irradiation. Example 15 described later shows the effect of a gamma ray sterilization treatment.

(Ultrafiltration treatment)

Bacteria-derived DNA as a contamination source can be separated and removed, by treating reagents used in the present invention with ultrafiltration. Concretely, when bacteria-derived DNA is bacterial cells per se or genome, their molecular weights are so large. Thus, ultrafiltration of the reagents can be carried out by selecting an ultrafiltration membrane through which the reagents, etc., pass, but genomic DNA does not pass. As the ultrafiltration membrane, a membrane having a molecular weight cut off of 1 kDa to 1000 kDa, preferably 10 kDa to 100 kDa, more preferably 30 kDa to 50 kDa is preferably used. An ultrafiltration membrane having a molecular weight cut off of 10 kDa to 1000 kDa, preferably 30 kDa to 100 kDa, more preferably 30 kDa to 50 kDa is used for filtration of a primer(s). The material of the ultrafiltration membrane is not particularly restricted and includes regenerated cellulose, cellulose acetate, polyether sulfone, etc. As the pressure source for ultrafiltration, any of a vapor pressurization method, a centrifugal method, etc. may be used, and a preferable method is selected depending on the treating amount.

When the treating amount is small, a centrifugal membrane filtration treatment is preferable. In the case of the centrifugal membrane filtration treatment, the amount of a treating solution and the centrifugal force (G) in a centrifugal operation comply with recommendation by a company of producing and distributing a filtration apparatus to be used.

Example 13, Example 14, Comparative Example 10 and Comparative Example 11 described later show the effect of a centrifugal membrane filtration treatment.

(Treatment with selectively membrane-permeable dye)

Reagents and instruments can be treated with EMA (ethidium monoazide) or PMA (propidium monoazide) as a selectively membrane-permeable dye and irradiated with light to inactivate bacteria-derived DNA as a contamination source, particularly, killed bacteria-derived DNA. Since inactivated DNA does not manifest multiplication by PCR, only DNA derived from an intended bacterium to be detected present in a sample can be detected and identification of bacterial species can be conducted.

Treatment conditions comply with conditions recommended by a company of producing and distributing the reagents and the reagent treating agents. For example, it is disclosed for "Viable Bacteria Selection Kit for PCR" manufactured by TAKARA BIO Inc. that appended Solution A-gn (10 μl) and Solution B-gn (5 μl) are added to 40 μl of a sample solution to be treated, the solution is allowed to stand still and reacted under light shielding for 5 to 15 minutes, then, irradiated with light for about 5 minutes. Example 16 described later shows the effect of an EMA treatment.

<Embodiment of kit>

The bacterial species identification kit of the present claimed invention is a kit for identifying the bacterial species by analyzing a DNA fragment composed of a part of 16S rDNA sequence of a bacterium to be amplified by a primer set of the present claimed invention. The analysis of a DNA fragment includes analysis of the molecular weight of a DNA fragment, measurement of the Tm value, etc. Measurement of the Tm value is preferable, and real time PCR is suitably used for this purpose. The molecular weight can be analyzed by electrophoresis, a mass spectrometer, etc.

As the primer pair composing a kit for detection of bacterial species, the primer pairs classified into the following groups K1 to K7 were found to be suitable. Then, each one primer pair was selected from respective groups K1 to K7, and at least one, preferably at least four of thus obtained seven primer pairs were used to constitute bacterial species detection kit.

(Primer pair for kit)

Group K1:

1-1: a primer pair consisting of a combination of a forward primer consisting of a sequence of SEQ ID No. 1 and a reverse primer consisting of any one of SEQ ID No. 16 to 27,

1-2: a primer pair consisting of a combination of a forward primer consisting of a sequence of SEQ ID No. 10 and a reverse primer consisting of any one of SEQ ID No. 16, 18, 20 and 21,

1-3: a primer pair consisting of a combination of a forward primer consisting of a sequence of SEQ ID No. 11 and a reverse primer consisting of any one of SEQ ID No. 16 to 21, and 23 to 26,

1-4: a primer pair consisting of a combination of a forward primer consisting of a sequence of SEQ ID No. 12 and a reverse primer consisting of any one of SEQ ID No. 16 to 27,

1-5: a primer pair consisting of a combination of a forward primer consisting of a sequence of SEQ ID No. 13 and a reverse primer consisting of any one of SEQ ID No. 16 to 27,

1-6: a primer pair consisting of a combination of a forward primer consisting of a sequence of SEQ ID No. 14 and a reverse primer consisting of any one of SEQ ID No. 16 to 27,

1-7: a primer pair consisting of a combination of a forward primer consisting of a sequence of SEQ ID No. 15 and a reverse primer consisting of any one of SEQ ID No. 16 to 27,

1-8: a primer pair consisting of a combination of a forward primer consisting of a sequence of SEQ ID No. 6 and a reverse primer consisting of any one of SEQ ID No. 16 to 27,

1-9: a primer pair consisting of a combination of a forward primer consisting of a sequence of SEQ ID No. 7 and a reverse primer consisting of any one of SEQ ID No. 16 to 27,

1-10: a primer pair consisting of a combination of a forward primer consisting of a sequence of SEQ ID No. 8 and a reverse primer consisting of any one of SEQ ID No. 16 to 27, or

1-11: a primer pair consisting of a combination of a forward primer consisting of a sequence of SEQ ID No. 9 and a reverse primer consisting of any one of SEQ ID No. 16, 17, 19 and 26,

Group K2:

2-1: a primer pair consisting of a combination of a forward primer consisting of a sequence of SEQ ID No. 28 and a reverse primer consisting of any one of SEQ ID No. 44, 45, and 47 to 50,

2-2: a primer pair consisting of a combination of a forward primer consisting of a sequence of SEQ ID No. 29 and a reverse primer consisting of any one of SEQ ID No. 44, 45, 47, 48 and 50,

2-3: a primer pair consisting of a combination of a forward primer consisting of a sequence of SEQ ID No. 30 and a reverse primer consisting of any one of SEQ ID No. 45, and 47 to 50,

2-4: a primer pair consisting of a combination of a forward primer consisting of a sequence of SEQ ID No. 31 and a reverse primer consisting of any one of SEQ ID No. 46 and 47,

2-5: a primer pair consisting of a combination of a forward primer consisting of a sequence of SEQ ID No. 32 and a reverse primer consisting of any one of SEQ ID No. 45, and 47 to 49,

2-6: a primer pair consisting of a combination of a forward primer consisting of a sequence of SEQ ID No. 33 and a reverse primer consisting of any one of SEQ ID No. 44 to 50,

2-7: a primer pair consisting of a combination of a forward primer consisting of a sequence of SEQ ID No. 34 and a reverse primer consisting of any one of SEQ ID No. 45 to 50,

2-8: a primer pair consisting of a combination of a forward primer consisting of a sequence of SEQ ID No. 35 and a reverse primer consisting of any one of SEQ ID No. 44 to 50,

2-9: a primer pair consisting of a combination of a forward primer consisting of a sequence of SEQ ID No. 36 and a reverse primer consisting of any one of SEQ ID No. 44 to 46, and 48 to 50,

2-10: a primer pair consisting of a combination of a forward primer consisting of a sequence of SEQ ID No. 37 and a reverse primer consisting of any one of SEQ ID No. 45 to 50,

2-11: a primer pair consisting of a combination of a forward primer consisting of a sequence of SEQ ID No. 38 and a reverse primer consisting of any one of SEQ ID No. 44 to 50,

2-12: a primer pair consisting of a combination of a forward primer consisting of a sequence of SEQ ID No. 39 and a reverse primer consisting of any one of SEQ ID No. 44 to 50,

2-13: a primer pair consisting of a combination of a forward primer consisting of a sequence of SEQ ID No. 40 and a reverse primer consisting of any one of SEQ ID No. 44 to 50,

2-14: a primer pair consisting of a combination of a forward primer consisting of a sequence of SEQ ID No. 41 and a reverse primer consisting of any one of SEQ ID No. 44 to 50,

2-15: a primer pair consisting of a combination of a forward primer consisting of a sequence of SEQ ID No. 42 and a reverse primer consisting of any one of SEQ ID No. 44 to 50, or

2-16: a primer pair consisting of a combination of a forward primer consisting of a sequence of SEQ ID No. 43 and a reverse primer consisting of any one of SEQ ID No. 44 to 48, and 50,

Group K3:
a primer pair consisting of a combination of a forward primer consisting of a sequence of SEQ ID No. 51 and a reverse primer consisting of a sequence of SEQ ID No. 52,
Group K4:

4-1: a primer pair consisting of a combination of a forward primer consisting of a sequence of SEQ ID No. 53 and a reverse primer consisting of any one of SEQ ID No. 57 to 61,

4-2: a primer pair consisting of a combination of a forward primer consisting of a sequence of SEQ ID No. 54 and a reverse primer consisting of any one of SEQ ID No. 59 and 61,

4-3: a primer pair consisting of a combination of a forward primer consisting of a sequence of SEQ ID No. 55 and a reverse primer consisting of any one of SEQ ID No. 57 to 59 and 61, or

4-4: a primer pair consisting of a combination of a forward primer consisting of a sequence of SEQ ID No. 56 and a reverse primer consisting of any one of SEQ ID No. 57 to 59, and 61,

Group K5:

5-1: a primer pair consisting of a combination of a forward primer consisting of a sequence of SEQ ID No. 62 and a reverse primer consisting of any one of SEQ ID No. 70, 71, and 73 to 75,
5-2: a primer pair consisting of a combination of a forward primer consisting of a sequence of SEQ ID No. 63 and a reverse primer consisting of any one of SEQ ID No. 70 to 75,
5-3: a primer pair consisting of a combination of a forward primer consisting of a sequence of SEQ ID No. 64 and a reverse primer consisting of any one of SEQ ID No. 70 to 75,
5-4: a primer pair consisting of a combination of a forward primer consisting of a sequence of SEQ ID No. 65 and a reverse primer consisting of any one of SEQ ID No. 70 to 75,
5-5: a primer pair consisting of a combination of a forward primer consisting of a sequence of SEQ ID No. 66 and a reverse primer consisting of any one of SEQ ID No. 70 to 75,
5-6: a primer pair consisting of a combination of a forward primer consisting of a sequence of SEQ ID No. 67 and a reverse primer consisting of any one of SEQ ID No. 70 to 75,
5-7: a primer pair consisting of a combination of a forward primer consisting of a sequence of SEQ ID No. 68 and a reverse primer consisting of any one of SEQ ID No. 70 to 75, or
5-8: a primer pair consisting of a combination of a forward primer consisting of a sequence of SEQ ID No. 69 and a reverse primer consisting of any one of SEQ ID No. 70 to 75,

Group K6:

6-1: a primer pair consisting of a combination of a forward primer consisting of a sequence of SEQ ID No. 76 and a reverse primer consisting of any one of SEQ ID No. 91, 93, 96, and 101 to 103,
6-2: a primer pair consisting of a combination of a forward primer consisting of a sequence of SEQ ID No. 77 and a reverse primer consisting of any one of SEQ ID No. 91, 96, and 101 to 103,
6-3: a primer pair consisting of a combination of a forward primer consisting of a sequence of SEQ ID No. 78 and a reverse primer consisting of any one of SEQ ID No. 91, and 101 to 103,
6-4: a primer pair consisting of a combination of a forward primer consisting of a sequence of SEQ ID No. 79 and a reverse primer consisting of any one of SEQ ID No. 91, 97, and 101 to 103,
6-5: a primer pair consisting of a combination of a forward primer consisting of a sequence of SEQ ID No. 80 and a reverse primer consisting of any one of SEQ ID No. 91, 93, and 100 to 103,
6-6: a primer pair consisting of a combination of a forward primer consisting of a sequence of SEQ ID No. 81 and a reverse primer consisting of any one of SEQ ID No. 91, 93, and 101 to 103,
6-7: a primer pair consisting of a combination of a forward primer consisting of a sequence of SEQ ID No. 82 and a reverse primer consisting of any one of SEQ ID No. 91, 96, 97, and 101 to 103,
6-8: a primer pair consisting of a combination of a forward primer consisting of a sequence of SEQ ID No. 83 and a reverse primer consisting of any one of SEQ ID No. 91, and 101 to 103,
6-9: a primer pair consisting of a combination of a forward primer consisting of a sequence of SEQ ID No. 84 and a reverse primer consisting of any one of SEQ ID No. 91, and 101 to 103,
6-10: a primer pair consisting of a combination of a forward primer consisting of a sequence of SEQ ID No. 85 and a reverse primer consisting of any one of SEQ ID No. 91, and 101 to 103,
6-11: a primer pair consisting of a combination of a forward primer consisting of a sequence of SEQ ID No. 86 and a reverse primer consisting of any one of SEQ ID No. 91, 93, 97, and 101 to 103,
6-12: a primer pair consisting of a combination of a forward primer consisting of a sequence of SEQ ID No. 87 and a reverse primer consisting of any one of SEQ ID No. 91, 96, and 101 to 103,
6-13: a primer pair consisting of a combination of a forward primer consisting of a sequence of SEQ ID No. 88 and a reverse primer consisting of any one of SEQ ID No. 91, and 101 to 103,
6-14: a primer pair consisting of a combination of a forward primer consisting of a sequence of SEQ ID No. 89 and a reverse primer consisting of any one of SEQ ID No. 91, and 101 to 103, or
6-15: a primer pair consisting of a combination of a forward primer consisting of a sequence of SEQ ID No. 90 and a reverse primer consisting of any one of SEQ ID No. 91, 92, 96, and 101 to 103,

Group K7:

7-1: a primer pair consisting of a combination of a forward primer consisting of a sequence of SEQ ID No. 104 and a reverse primer consisting of any one of SEQ ID No. 2, 5, and 116 to 131,

7-2: a primer pair consisting of a combination of a forward primer consisting of a sequence of SEQ ID No. 105 and a reverse primer consisting of any one of SEQ ID No. 2, 5, and 116 to 131,
7-3: a primer pair consisting of a combination of a forward primer consisting of a sequence of SEQ ID No. 106 and a reverse primer consisting of any one of SEQ ID No. 2, 5, and 116 to 131,
7-4: a primer pair consisting of a combination of a forward primer consisting of a sequence of SEQ ID No. 107 and a reverse primer consisting of any one of SEQ ID No. 2, 5, and 116 to 131,
7-5: a primer pair consisting of a combination of a forward primer consisting of a sequence of SEQ ID No. 108 and a reverse primer consisting of any one of SEQ ID No. 2, 5, and 116 to 131,
7-6: a primer pair consisting of a combination of a forward primer consisting of a sequence of SEQ ID No. 109 and a reverse primer consisting of any one of SEQ ID No. 2, 5, and 116 to 131,
7-7: a primer pair consisting of a combination of a forward primer consisting of a sequence of SEQ ID No. 110 and a reverse primer consisting of any one of SEQ ID No. 2, 5, and 116 to 131,
7-8: a primer pair consisting of a combination of a forward primer consisting of a sequence of SEQ ID No. 111 and a reverse primer consisting of any one of SEQ ID No. 2, 5, and 116 to 131 ,
7-9: a primer pair consisting of a combination of a forward primer consisting of a sequence of SEQ ID No. 112 and a reverse primer consisting of any one of SEQ ID No. 2, 5, and 116 to 131,
7-10: a primer pair consisting of a combination of a forward primer consisting of a sequence of SEQ ID No. 113 and a reverse primer consisting of any one of SEQ ID No. 2, 5, and 116 to 131,
7-11: a primer pair consisting of a combination of a forward primer consisting of a sequence of SEQ ID No. 114 and a reverse primer consisting of any one of SEQ ID No. 2, 5 and 125, or
7-12: a primer pair consisting of a combination of a forward primer consisting of a sequence of SEQ ID No. 115 and a reverse primer consisting of any one of SEQ ID No. 2, 5, 116, 118, 123, and 125 to 128.

It is preferable that the above-described primer sets and kits contain at least one primer pair from the following seven primer pairs 1) to 7).

1) a primer pair consisting of a combination of a forward primer consisting of a sequence of SEQ ID No. 15 and a reverse primer consisting of a sequence of SEQ ID No. 16,
2) a primer pair consisting of a combination of a forward primer consisting of a sequence of SEQ ID No. 43 and a reverse primer consisting of a sequence of SEQ ID No. 50,
3) a primer pair consisting of a combination of a forward primer consisting of a sequence of SEQ ID No. 51 and a reverse primer consisting of a sequence of SEQ ID No. 52,
4) a primer pair consisting of a combination of a forward primer consisting of a sequence of SEQ ID No. 53 and a reverse primer consisting of a sequence of SEQ ID No. 57,
5) a primer pair consisting of a combination of a forward primer consisting of a sequence of SEQ ID No. 62 and a reverse primer consisting of a sequence of SEQ ID No. 70,
6) a primer pair consisting of a combination of a forward primer consisting of a sequence of SEQ ID No. 81 and a reverse primer consisting of a sequence of SEQ ID No. 103, and
7) a primer pair consisting of a combination of a forward primer consisting of a sequence of SEQ ID No. 115 and a reverse primer consisting of a sequence of SEQ ID No. 5.

Further, the above-described kit containing as an essential component at least one of the above-described primer pairs 1) to 7) are preferable, and those containing at least one of the following combinations A to G are preferable for identification of bacterial species.

(A) A combination essentially containing the above-described primer set 1) and further containing at least three primer pairs selected from the above-described 2) to 7).
(B) A combination essentially containing the above-described primer set 2) and further containing at least three primer pairs selected from the above-described 1) and 3) to 7).
(C) A combination essentially containing the above-described primer pair 3) and further containing at least three primer pairs selected from the above-described 1), 2) and 4) to 7).
(D) A combination essentially containing the above-described primer pair 4) and further containing at least three primer pairs selected from the above-described 1) to 3) and 5) to 7).
(E) A combination essentially containing the above-described primer pair 5) and further containing at least three primer pairs selected from the above-described 1) to 4) and 6) to 7).
(F) A combination essentially containing the above-described primer pair 6) and further containing at least three primer pairs selected from the above-described 1) to 5) and 7).
(G) A combination essentially containing the above-described primer pair 7) and further containing at least three primer pairs selected from the above-described 1) to 6).

The kit for detection and/or identification of bacterial species of the present invention has a constitution containing one or more out of the above-described primer pairs as set out in the claims. In addition to this primer, if necessary, at least one reagent and/or part selected from reagents such as an enzyme for PCR, a pH buffer solution, $MgCl_2$, dNTP (or CleanAmp-dNTP), sterile water, etc. and parts such as vessels for PCR analysis and measurement (for example, tube for analysis and measurement), other necessary instruments, etc. can be contained in the kit. Further, in analysis in real time PCR, a fluorochrome can be added as a component. Depending on various embodiments of a PCR method, components necessary for a PCR method, in addition to these components, may be added to a kit. These reagents or parts may be packed in any form such as an individual form, a partial form, an integrated form, etc. Specifically, the following forms are exemplified.

1) Reagents to be used are divided individually.
2) Reagents are divided into three components: a mixture obtained by previously mixing a pH buffer solution, $MgCl_2$, dNTP (or CleanAmp-dNTP), and a fluorochrome in analysis by real time PCR; a primer pair; and an enzyme.
3) Reagents are divided into two components: a mixture obtained by previously mixing a pH buffer solution, $MgCl_2$, dNTP (or CleanAmp-dNTP), a fluorochrome in analysis by real time PCR and a primer pair; and an enzyme.
4) A pH buffer solution, $MgCl_2$, dNTP (or CleanAmp-dNTP), a fluorochrome in analysis by real time PCR, a primer and an enzyme are all previously mixed.

In the above-described kit, the amount of mixing of bacteria-derived DNA as a contamination source is 10 fg or less on the whole, the enzyme to be used is one having undergone production by an eukaryote, a high purification treatment or a treatment with a selectively membrane-permeable dye such as EMA or the like, and the reagents and instruments to be used (including tube for PCR analysis) are those individually or wholly having undergone a gamma ray sterilization treatment, a centrifugal membrane filtration treatment or a treatment with a selectively membrane-permeable dye, if needed. In packing reagents and parts, various treatments are so conducted that the amount of mixing of bacteria-derived DNA in a package after packing and sealing is 10 fg or less.

<Application field, Sample>

The kit for detection and/or identification of bacterial species of the present claimed invention can be used for detection of a bacterium and identification of bacterial species in various fields such as the medical field, food field, environmental analysis, etc. Particularly, in the medical field, the kit is useful since it can detect an infecting bacterium in an infectious disease and/or identify bacterium species.

The sample analyzed by the bacteria test is not particularly restricted, and a wide variety of samples can be applied. Specifically, in the medical field, the sample includes blood, spinal fluid, lacrimal fluid, amnion fluid, other body fluids, materials adhered to medical devices such as catheters, etc. Blood, amnion fluid and spinal fluid are particularly useful for detection of a pathogenic bacterium of a serious infectious disease. In the food field, the sample includes foods themselves, liquid or solid samples in the course of production, materials adhered to a machine in the production process, etc.

<DNA extraction treatment>

In execution of detection of a bacterium in a sample and/or identification of bacterium species using the identification kit of the present claimed invention, it is necessary that DNA is previously extracted from a bacterium possibly present in a sample. As the DNA extraction method, an alkali melting method, a boiling method, a phenol extraction method, etc. are currently known, and dedicated DNA extraction kits are marketed from various makers.

The DNA extraction method which can be used in the present invention is not particularly restricted, and it is desirable to select a method proper for a subject sample since the suitable method varies depending on a sample. The suitably applicable means for DNA extraction includes QIAamp DNA Mini Kit manufactured by Qiagen and High pure PCR template preparation kit manufactured by Roche used in the examples of the present application. As described in the manual of QIAamp DNA Mini Kit manufactured by Qiagen, the elution amount can be changed in a range of 50 $\mu$l to 200 $\mu$l according to necessity.

<PCR analysis>

The primer set or kit of the present invention as claimed is used in a PCR method. As the PCR method, various PCR methods for amplification of a target gene for detecting intended bacteria can be used. Real time PCR is a preferable analysis method, and a combination of real time PCR and analysis of a melting curve for Tm measurement is more preferable. In this case, it is preferable that the performance of controlling temperature is ±0.1°C as the specification of a machine of real time PCR to be used. Specific examples of PCR and real time PCR and measurement of Tm using them are listed below.

In PCR and real time PCR, apparatuses, methods, etc. which are usually known can be used.

Conditions of thermal cycles in PCR (temperature, time, temperature elevating and dropping rate, cycle number) are not particularly restricted and may be appropriately set depending on the properties of a primer, an enzyme, a template, etc. to be used and the sensitivity of a DNA detection method after PCR. For setting such conditions, a lot of documents are already known.

In PCR, a template double-stranded DNA thermal denaturation step, a primer annealing step and a DNA elongation step with an enzyme are repeated, in general. The thermal denaturation step may include temperature and time at which a template double-stranded DNA dissociates into single strands, and for example, a step of heating at 90°C to 98°C for several seconds to several minutes is set. In initiation of PCR, a process of thermal denaturation of several minutes to 10 minutes is often added only in the first cycle. The primer annealing step is set according to the base sequence of a primer and the number of bases, and a step of heating at 40°C to 72°C for several seconds to dozens of seconds is often set. In the DNA elongation step, the temperature complies with the property of the enzyme such as its optimum temperature, etc., and, for example, the range of 58°C to 76°C is general. Regarding the time of the DNA elongation step, the necessary time is roughly estimated and set from the chain length of DNA to be amplified and the DNA synthesis speed of an enzyme. The intended DNA is amplified by repeating steps of thermal denaturation, annealing and elongation, and the number of this repetition may be appropriately altered depending on the amount of template DNA, the amount of an enzyme and the sensitivity of a DNA detection method after PCR, and in general, 10 to 50 times are exemplified. When the annealing temperature and the DNA elongation temperature are approximately the same, it is also possible to carry out the both steps simultaneously.

Also for real time PCR, conditions for thermal denaturation, annealing and DNA elongation necessary for DNA amplification, and further, the numbers of repetitions of these steps, are the same as those for the above-described PCR. In real time PCR, the amount of amplified DNA can be quantified and estimated by measuring the fluorescent intensity derived from an intercalator and a probe, before and after the step of DNA elongation. The temperature for measuring fluorescent intensity can be appropriately altered depending on the kind of a probe, etc. The temperature for measuring fluorescent intensity may be, for example, the temperature in DNA elongation as it stands in the case of execution using an intercalator, and when the chain length of intended DNA to be amplified is relatively long and its Tm value is relatively high, the temperature for measuring fluorescent intensity may be set at temperatures between the Tm value of intended DNA and the Tm value of non-specifically amplified DNA typically including the intermediate value therebetween, by utilizing a fact that non-specifically amplified unintended DNA having relatively short chain length such as a primer dimer, etc. (also referred to as non-specifically amplified DNA) has relatively low Tm value. By this, it is possible that, particularly in execution using an intercalator, only non-specifically amplified DNA such as a primer dimer, etc. dissociates into single strands from a double-stranded structure, and the amount of DNA quantified or estimated from fluorescent intensity is a value for intended DNA.

Further, in real time PCR, Tm of amplified DNA can be measured by analysis of a melting curve after completion of the DNA amplification step. In a melting curve analysis, dissociation of DNA from a double-stranded structure into single strands according to temperature change is observed, however, its temperature and detection conditions are not particularly restricted. In general, stages of thermal denaturation (Denaturation) (90°C to 98°C), double strand formation (Annealing) (40°C to 80°C) and melting (Melting) (gradually rising from temperature of double strand formation up to around 98°C) are implemented and the change of fluorescent intensity in the melting step is monitored to obtain a melting curve, and the Tm value can be obtained from this. Such measurement is possible in a lot of models of the real time PCR apparatus, and can be carried out according to device usage instructions.

<Method of detection and/or identification>

As a method of detection or identification of bacterial species in a sample according to the present invention, the method comprising the following steps can be used.

(1) A step of conducting PCR using genome DNA of bacterium prepared from the above-described sample, a primer for obtaining an amplification product containing an intended gene specific to the intended bacterial species to be detected as a target, and a heat-resistant DNA polymerase.

(2) A step of conducting detection or identification of the intended bacterial species to be detected in the above-described sample, by detection of the above-described intended gene in the amplification product in the above-described PCR or analysis of the amplification product.

By using a primer set according to the present claims as the primer, identification of bacterial species at high sensitivity can be performed. Further, by using reagents and instruments having controlled contamination level described above, further higher sensitivity and higher treating speed can be attained.

In this detection and/or identification method, the amplification step is preferably conducted under suppression of amplification of genes other than the intended gene (unintended gene). For this suppression of amplification of unintended genes, PCR by a hot start method can be used. As an example, a hot start method using an anti-DNA polymerase antibody is mentioned. In this case, it is preferable to use an anti-DNA polymerase antibody in an amount surplus to heat-resistant DNA polymerase 1 U. Also, a hot start method in which reversible chemical modification is performed on a DNA polymerase as disclosed in Japanese Patent Laid-Open No. 2000-4847 (JP 2000 4847 A) and Japanese Patent Laid-Open No. 276776/1998 (JP H10 276776 A) can be suitably used. Further, a hot start method in which chemically modified dNTP is used or a chemically modified primer is used as described in US Patent Publication No. 20070281308 can also be suitably used. Furthermore, a hot start method in which a DNA polymerase and constituent components (for example, primer, dNTP, $Mg^{2+}$ salt) indispensable for DNA amplification by a DNA polymerase are physically isolated using a wax melting by heating can also be suitably used.

The step of detection and/or identification of a amplification product can be carried out by measuring Tm by real time PCR using an intercalator or a probe having a fluorescent label for detection. Preferable is real time PCR using an intercalator, and the intercalator includes, preferably, unsaturated fluorochromes: SYBR Green I, saturated fluorochromes: EvaGreen and Resolight; more preferably, saturated fluorochromes: EvaGreen and Resolight.

In the step of detection and/or identification by measurement of Tm using real time PCR, the amplification product of the above-described intended gene can be made detectable and the amplification products of unintended genes other than this can be made undetectable. For this purpose, a method is preferable, which detects the amplification product of the intended gene by setting the conditions that detection of the amplification product of the intended gene can be detected, while the amplification products of unintended genes other than the intended gene can be not detected, by:

(1) designing the above-described primer so that the melting temperature ($Tm^A$) of the amplification product of an intended gene is higher than the melting temperature ($Tm^B$) of the amplification products of unintended genes, and
(2) performing detection of the amplification product at temperatures between $Tm^A$ and $Tm^B$,.

Further, a method can be used in which the amplification step and the step of detection and/or identification are conducted by real time PCR using a display indicating the amount of the amplification product, and the amplification products of unintended genes are hidden on the above-described display.

The step of detection and/or identification can also be conducted by analysis of the amplification product in which the amplification product is developed by electrophoresis on a gel, etc. and visualized. The step of detection and/or identification can also be conducted by analysis of the amplification product in which the base sequence of the amplification product is read. Further, this step can also be conducted by a method in which the molecular weight of the amplification product is measured and analyzed by a mass spectrometer.

<Identification method>

For identifying the detected bacterium, the Tm value of a DNA fragment obtained from the detected bacterium using a primer set of the present claimed invention can be utilized. In the algorithm for identification, a process can be added in which an influence of measurement errors such as, for example, a measuring error in every trial run of an apparatus is minimized by effecting identification utilizing not only a combination of the Tm values themselves described above but also a combination of differences between the Tm values.

As the method for correcting the measuring error in every trial run of an apparatus described above, "the average value of a combination of Tm values is calculated, a combination of the relative values of respective Tm values from its average value" can be utilized. Namely, it is a method in which the configuration of a combination of Tm values is identified as "shape". "Shape" shown in two dimensions of the configuration of a combination of Tm values is not influenced by a measuring error. For example, a combination of Tm values (n (n is an integer of 4 or more and 7 or less)) specific to a detected bacterium are represented as T1db to Tndb (db is database), and the relative values from its average value are represented as d1db to dndb, respectively. In the same way, a combination of Tm values (n (n is an integer of 4 or more and 7 or less)) of unknown intended organisms to be detected obtained from a sample are represented as T1ref to Tnref (ref is reference), and the relative values from its average value are represented as d1ref to dnref, respectively. After comparison with the database, "one having a near combination of relative values = one in which "shape" of the configuration of a combination of Tm values is close" is utilized as the identification algorithm.

Specific calculation methods include, but not limited to, for example, a method of calculating the distance between two points on the Euclidean space (formula 1).

[Formula: 1]

$$Dist.=\sqrt{[(D1_{db}-D1_{ref})^2+(D2_{db}-D2_{ref})^2+\ldots(Dn_{db}-Dn_{ref})^2]}$$

In the calculation method by Formula 1, one in which the D value obtained by this calculation formula is nearest to 0 is identified as a required bacterium species to be detected. However, in view of the measuring error of a PCR apparatus to be used, the allowable range of the D value is 0 to 0.37, preferably 0 to 0.30 depending on the temperature control specification of the apparatus and the number of primers.

The above-described algorithm can be used as a data base type identification software on a computer.

<Identifiable bacterial species>

Regarding the identifiable microorganisms, any microorganisms corresponding to bacteria according to the taxonomic designation can be detected and identified according to the system.

Concrete bacterial species include: Achromobacter denitrificans, Achromobacter xylosoxidans, Acinetobacter baumannii, Acinetobacter calcoaceticus, Actinomyces israelii, Aerococcus christensenii, Aeromonas hydrophila, Aeromonas sobria, Aggregatibacter actinomycetemcomitans, Alcaligenes faecalis, Alistipes onderdonkii, Anaerococcus vaginalis, Anaeroglobus geminatus, Arcanobacterium haemolyticum, Arcanobacterium pyogenes, Arthrobacter cumminsii, Atopobium vaginae, Bacillus anthracis, Bacillus cereus, Bacillus coagulans, Bacillus licheniformis, Bacillus megaterium, Bacillus pumilus, Bacillus sphaericus, Bacillus subtilis, Bacteroides dorei, Bacteroides finegoldii, Bacteroides fragilis, Bacteroides nordii, Bacteroides salyersiae, Bacteroides thetaiotaomicron, Bacteroides uniformis, Bacteroides vulgatus, Bartonella henselae, Bartonella quintana, Bifidobacterium bifidum, Bifidobacterium breve, Bilophila wadsworthia, Bordetella pertussis, Borrelia burgdorferi, Borrelia recurrentis, Brevibacillus laterosporus, Brucella abortus, Brucella melitensis, Brucella suis, Burkholderia cepacia, Burkholderia mallei, Burkholderia pseudomallei, Campylobacter coli, Campylobacter curvus, Campylobacter jejuni, Campylobacter rectus, Capnocytophaga gingivalis, Capnocytophaga granulosa, Capnocytophaga haemolytica, Capnocytophaga sputigena, Cardiobacterium hominis, Chryseobacterium meningosepticum, Citrobacter amalonaticus, Citrobacter freundii, Citrobacter koseri, Clostridium butyricum, Clostridium difficile, Clostridium histolyticum, Clostridium hylemonae, Clostridium paraputrificum, Clostridium perfringens, Clostridium septicum, Clostridium sporogenes, Clostridium subterminale, Clostridium tertium, Clostridium tetani, Corynebacterium amycolatum, Corynebacterium confusum, Corynebacterium diphtheriae, Corynebacterium glucuronolyticum, Corynebacterium jeikeium, Corynebacterium kroppenstedtii, Corynebacterium macginleyi, Corynebacterium minutissimum, Corynebacterium pseudodiphtheriticum, Corynebacterium pseudotuberculosis, Corynebacterium riegelii, Corynebacterium tuberculostearicum, Corynebacterium ulcerans, Corynebacterium xerosis, Edwardsiella tarda, Eggerthella lenta, Eikenella corrodens, Elizabethkingia meningoseptica, Empedobacter brevis, Enterobacter aerogenes, Enterobacter aerogenes, Enterobacter cloacae, Enterobacter sakazakii, Enterococcus avium, Enterococcus bovis, Enterococcus casseliflavus, Enterococcus cecorum, Enterococcus dispar, Enterococcus durans, Enterococcus faecium, Enterococcus flavescens, Enterococcus gallinarum, Enterococcus gilvus, Enterococcus hirae, Enterococcus italicus, Enterococcus malodoratus, Enterococcus mundtii, Enterococcus pallens, Enterococcus pseudoavium, Enterococcus raffinosus, Enterococcus sanguinicola, Erysipelothrix rhusiopathiae, Escherichia albertii, Escherichia coli, Eubacterium lentum, Eubacterium limosum, Finegoldia magna, Francisella tularensis, Fusobacterium necrophorum, Fusobacterium nucleatum, Fusobacterium periodonticum, Fusobacterium varium, Gardnerella vaginalis, Gemella morbillorum, Geobacillus stearothermophilus, Granulicatella adiacens, Haemophilus ducreyi, Haemophilus influenzae, Haemophilus parainfluenzae, Hafnia alvei, Halomonas venusta, Helicobacter cinaedi, Helicobacter pylori, Kingella kingae, Klebsiella granulomatis, Klebsiella oxytoca, Klebsiella pneumoniae, Lactobacillus acidophilus, Lactobacillus crispatus, Lactobacillus delbrueckii, Lactobacillus jensenii, Lactococcus garvieae, Legionella pneumophila, Leptospira interrogans, Listeria monocytogenes, Micrococcus luteus, Moraxella catarrhalis, Morganella morganii, Mycoplasma genitalium, Mycoplasma hominis, Neisseria gonorrhoeae, Neisseria meningitidis, Nocardia cyriacigeorgica, Odoribacter splanchnicus, Pantoea agglomerans, Parabacteroides distasonis, Parvimonas micra, Pasteurella multocida, Pediococcus damnosus, Peptoniphilus asaccharolyticus, Peptoniphilus gorbachii, Peptostreptococcus anaerobius, Plesiomonas shigelloides, Porphyromonas asaccharolytica, Porphyromonas gingivalis, Prevotella bivia, Prevotella bivia, Prevotella corporis, Prevotella intermedia, Prevotella melaninogenica, Prevotella nigrescens, Prevotelia timonensis, Prevotella veroralis, Propionibacterium acnes, Propionibacterium avidum, Propionibacterium granulosum, Proteus mirabilis, Proteus vulgaris, Providencia rettgeri, Providencia stuartii, Pseudomonas aeruginosa, Pseudomonas fluorescens, Pseudomonas putida, Rothia dentocariosa, Rothia mucilaginosa, Salmonella enterica, Serratia marcescens, Serratia plymuthica, Shigella boydii, Shigella dysenteriae, Shigella flexneri, Shigella sonnei, Spirillum minus, Staphylococcus aureus, Staphylococcus auricularis, Staphylococcus capitis, Staphylococcus caprae, Staphylococcus carnosus, Staphylococcus cohnii, Staphylococcus epidermidis, Staphylococcus haemolyticus, Staphylococcus hominis, Staphylococcus lugdunensis, Staphylococcus pasteuri, Staphylococcus pettenkoferi, Staphylococcus pulvereri, Staphylococcus saccharolyticus. Staphylococcus saprophyticus, Staphylococcus schleiferi, Staphylococcus simulans, Staphylococcus warneri, Staphylococcus xylosus, Stenotrophomonas maltophilia, Streptobacillus moniliformis, Streptococcus agalactiae, Streptococcus anginosus, Streptococcus bovis, Streptococcus canis, Streptococcus constellatus, Streptococcus dysgalactiae, Streptococcus equi, Streptococcus gallolyticus, Streptococcus gordonii, Streptococcus infantarius, Streptococcus iniae, Streptococcus intermedius, Streptococcus lutetiensis, Streptococcus mitis, Streptococcus mutans, Streptococcus oralis, Streptococcus pasteurianus, Streptococcus pneumoniae, Streptococcus porcinus, Streptococcus pyogenes, Streptococcus salivarius, Streptococcus salivarius, Streptococcus sanguinis, Streptococcus sobrinus, Streptococcus suis, Streptococcus vestibularis, Sutterella wadsworthensis, Treponema pallidum, Ureaplasma parvum, Vagococcus fluvialis, Veillonella atypica, Veillonella parvula, Vibrio alginolyticus, Vibrio cholerae, Vibrio fluvialis, Vibrio parahaemolyticus, Vibrio vulnificus, Yersinia enterocolitica, Yersinia pestis, Yersinia pseudotuberculosis. The identifiable microorganisms are not limited to the above bacteria.

## EXAMPLES

The present invention will be illustrated specifically by examples and test examples below, but the present invention is not limited to them.

### (Production Example 1) DNAP preparation method

e-DNAP was prepared by the following method according to paragraphs 0195 to 0201 of Patent document 4, and used in examples below.

#### (1) Synthesis of DNA

The entire DNA sequence of a heat-resistant DNA polymerase derived from T.aquaticus was synthesized by GenScript. In this procedure, the codon sequence was optimized to a yeast host, S.cerevisiae. The synthesized DNA was incorporated into a plasmid pUC57 and supplied from GenScript, and a vector pUC-TA01 was obtained. The gene coding the heat-resistant DNA polymerase was so designed that Hind III added into the 5' end sequence and an EcoRI restriction enzyme site added into the 3' end sequence.

#### (2) Construction of vector for expression of T. aquaticus-derived heat-resistant DNA polymerase

The gene coding the synthesized T. aquaticus-derived heat-resistant DNA polymerase was inserted into a plasmid pYES2 (Invitrogen), to construct a vector pYES-TA01. For the gene coding the heat-resistant DNA polymerase, pUC-TA01 was digested by restriction enzymes HindIII, EcoRI (TaKaRa Bio), electrophoresed on 1 % agarose gel (Wako), and the gene coding the heat-resistant DNA polymerase was recovered by QIAquick gel extraction kit (Qiagen). The plasmid pYES2 was digested by EcoRI, NotI (TaKaRa Bio), and the gene coding the heat-resistant DNA polymerase and pYES2 were connected by DNA Ligation Kit Ver. 2.1 (TaKaRa Bio).

#### (3) Transformation of S.cerevisiae

PCR was conducted using the genome of Saccharomyces cerevisiae X2180 as a template and using a forward primer of SEQ ID No. 134 and a reverse primer of SEQ ID No. 135, to obtain a fragment A of about 550 bp. Likewise, PCR was conducted using the genome of Saccharomyces cerevisiae X2180 as a template and using a forward primer of SEQ ID No. 136 and a reverse primer of SEQ ID No. 137, to obtain a fragment B of about 550 bp. Next, PCR was conducted using the fragment A and the fragment B as a template and using a forward primer of SEQ ID No. 134 and a reverse primer of SEQ ID No. 137, to obtain a fragment AB. Next, Saccharomyces cerevisiae X2180 was cultured, then, a competent cell was made, and the fragment AB was transformed using FastTrackTM-Yeast Transformation Kit (Geno Technology). The transformant was applied to the minimal agar medium in which 5-FOA was contained so as to give a final concentration of 1 mg/ml. This agar medium was cultured at 28°C for 3 days, then, a growable strain was obtained in the minimal agar medium containing 5-FOA, and named Saccharomyces cerevisiae X2180-S strain. The yeast Saccharomyces cerevisiae X2180 strain is available from American Type Culture Collection as a bank for cells, bacteria and genes.

This obtained vector pYES-TA01 was introduced into the yeast (Saccharomyces cerevisiae X2180-S strain). As the host, the other yeast can also be used providing it is a uracil auxotroph. For transformation, FastTrackTM-Yeast Transformation Kit (Geno Technology) was used.

(4) Production of T. aquaticus-derived heat-resistant DNA polymerase by S.cerevisiae

In the following experiment, the instruments and ultrapure water used were free of DNA. The resultant transformant was cultured at 28°C for 72 hours under vibration in 100 ml of an SD medium (0.67% Bacto yeast nitrogen base, 2% Galactose). These were centrifugally separated at 5000 rpm for 10 minutes and harvested, and suspended in a disrupting buffer solution (50 mM Tris-HCl pH7.5, 50 mM KCl), and bacterial bodies were disrupted using 0.5 mm glass beads, then, centrifugally separated at 12000 rpm for 30 minutes, to obtain the yeast disrupting solution supernatant and a precipitate as a cell extract. Next, the above-described cell extract was thermally treated at 70°C for 60 minutes, and after the thermal treatment, centrifugal separation was carried out at 5000 rpm for 30 minutes at 4°C and the supernatant was recovered. To this supernatant, polyethyleneimine (Sigma Aldrich) was added so as to give a final concentration of 0.1%, and the mixture was stirred vigorously for 1 minute, then, allowed to stand at room temperature for 5 minutes. Thereafter, the mixture was centrifugally separated at 8000 rpm at 4°C for 30 minutes and the supernatant was recovered, and this supernatant was allowed to pass through a filter of 0.45 mm.

(5) Purification of T. aquaticus-derived heat-resistant DNA polymerase

In the following experiment, the instruments and ultrapure water used were free of DNA, and the experiment was conducted in a low temperature cabinet. The recovered coarse extracted solution was subjected to HiTrap Q FF (GE) equilibrated with a buffer solution A (50 mM Tris-HCl pH7.5), and thereafter, a 5% buffer solution B (50 mM Tris-HCl pH7.5, 1 M NaCl) was allowed to flow through a column to elute foreign substances. Next, a 15% buffer solution B was allowed to flow through a column to elute a heat-resistant DNA polymerase. The eluted enzyme solution was subjected to HiTrap Heparin equilibrated with a 15% buffer solution B, the content of the buffer solution B was increased gradually up to 50%, and an enzyme eluted fraction was recovered. The buffer solution of this enzyme fraction was replaced by a preservation buffer solution (40 mM Tris-HCl pH8.0, 1M NaCl, 2 mM DTT, 0.2 mM EDTA), and further, a preservation solution (98% glycerol, 1 % Tween20, 1% NP-40) was added in an equal amount and the mixture was stirred, then, preserved as an enzyme solution at -20°C.

(6) Measurement of activity of T. aquaticus-derived heat-resistant DNA polymerase

First, a solution shown in Table 1 was prepared, and thermally insulated at 70°C for 5 minutes. Using this solution, a reaction solution was prepared as shown in Table 2, and the solution was reacted at 74°C for 5 minutes, and 50 μL of 10 mM EDTA was added to stop the reaction. After stopping of the reaction, 10 μL of SYBER Green I and 15 μL of sterile water were added and the mixture was allowed to stand at room temperature for 5 minutes, then, fluorescence thereof was measured (Em: 497 nm, Ex: 528 nm). Activity of the heat-resistant DNA polymerase was defined based on the unit number of Thunder Taq (Nippon Gene Co., Ltd.) as a standard.

[Table 1]

Table 1 Composition of reaction solution 1 for measurement of activity

| | |
|---|---|
| 1μg/μL M13mp18 DNA | 28μL |
| 10μM M13 primer | 2.4μL |
| 10mM Tris-HCl (pH8.8) | |
| 50mM KCl | |
| 0.08% Nonidet P-40 | |
| 2mM MgCl2 | |
| DW | Appropriate amount |
| Total | 140μL |

Table 2 Composition of reaction solution 2 for measurement of activity

| | |
|---|---|
| Solution of Table 14 | 2.4μL |
| 2mM dNTPs | 5μL |
| 10mM Tris-HCl (pH8.8) | |
| 50mM KCl | |
| 0.08% Nonidet P-40 | |
| 2mM MgCl2 | |
| Enzyme solution | 1μL |

(continued)

Table 2 Composition of reaction solution 2 for measurement of activity

| | |
|---|---|
| DW | Appropriate amount |
| Total | 25μL |

(Example 1) Bacterial identification by nested-PCR

In the present example, E. coli DNA was extracted from the culture solution of E. coli MG1655 using QIAamp DNA purification kit (QIAGEN), and after extraction, the DNA amount was measured by an absorption spectrometer and diluted with ultrapure water, and EC1 and EC3 shown in Table 3, out of the diluted solutions, were used as a template of the reaction. Rotor-Gene Q MDx 5plex HRM (QIAGEN) was used as a real time PCR apparatus. The instruments and ultrapure water used were free of DNA. The E. coli MG1655 strain is available from American Type Culture Collection as a bank for cells, bacteria and genes.

[Table 3]

Table 3 E. coli genome solution used in experiment

| Name | E. coli genome concentration (ng/mL) |
|---|---|
| EC1 | 10.0 |
| EC2 | 1.0 |
| EC3 | 0.1 |

In conducting nested-PCR, a reaction solution having a formulation shown in Table 4 was heated at 95°C for 5 minutes, then, a process including heating at 94°C for 10 seconds, at 65°C for 10 seconds and at 72°C for 30 seconds was repeated 40 times.

[Table 4]

Table 4 Reaction solution composition 1

| | |
|---|---|
| Template | 2.0 μL |
| 10X Thnuder Taq Buffer | 2.0 μL |
| 25mM MgCl2 | 1.6 μL |
| e-DNAP | 1.0 Unit |
| 2mM CleanAmp-dNTP | 2.0 μL |
| EvaGreen (Biotium) | 1.0 μL |
| 10μM forward primer | 0.6 μL |
| 10μM reverse primer | 0.6 μL |
| DW | Appropriate amount |
| Total | 20.0 μL |

As the primer, a forward primer SEQ ID No. 1 and a reverse primer SEQ ID No. 2 were used. After completion of PCR, the reaction solution was recovered, and diluted 500-fold with DNA-free ultrapure water. The reaction was carried out using this diluted solution as a template, with a formulation shown in Table 5.

[Table 5]

Table 5 Reaction solution composition 2

| | |
|---|---|
| Template | 2.0 μL |
| 10X Thnuder Taq Buffer | 2.0 μL |
| 25mM MgCl2 | 2.0 μL |
| e-DNAP | 1.0 Unit |
| 2mM CleanAmp-dNTP | 2.0 μL |
| EvaGreen (Biotium) | 1.0 μL |
| 10μM forward primer | 0.6 μL |
| 10μM reverse primer | 0.6 μL |
| DW | Appropriate amount |
| Total | 20.0 μL |

Regarding the method of PCR, the reaction solution was heated at 95°C for 5 minutes, then, a process including heating at 94°C for 10 seconds, at 60°C for 10 seconds and at 72°C for 10 seconds was repeated 35 times. For the primer, 7 sets including (1) a combination of a forward primer SEQ ID No. 1 and a reverse primer SEQ ID No. 16, (2) a combination of a forward primer SEQ ID No. 28 and a reverse primer SEQ ID No. 44, (3) a combination of a forward primer SEQ ID No. 51 and a reverse primer SEQ ID No. 52, (4) a combination of a forward primer SEQ ID No. 53 and a reverse primer SEQ ID No. 57, (5) a combination of a forward primer SEQ ID No. 62 and a reverse primer SEQ ID No. 70, (6) a combination of a forward primer SEQ ID No. 76 and a reverse primer SEQ ID No. 91 and (7) a combination of a forward primer SEQ ID No. 104 and a reverse primer SEQ ID No. 2 were adopted, amplification of DNA thereof was performed, then, the DNA dissociation curve thereof was made and the Tm values were measured. In creation of the DNA dissociation curve, the reaction solution was heated at 95°C for 10 seconds, then, incubated at 72°C for 90 seconds, and heated up to 95°C by 0.5°C stepwise. At each stage, the reaction solution was incubated for 2 seconds, and the data were obtained. The resultant Tm value was compared with the Tm value of E. coli previously obtained and the D value was determined, as described in Patent document 4. When the D value is 0.3 or less, they were judged to be homogeneous. This result is shown in Table 6 together with the result of Comparative Example 1.

(Comparative Example 1) (Direct-PCR) compared with Example 1 (nested-PCR)

The following comparative example is a replication experiment of Patent document 4. E. coli DNA was extracted from the culture solution of E. coli MG1655 using QIAamp DNA purification kit (QIAGEN), and after extraction, the DNA amount was measured by an absorption spectrometer and diluted with DNA free ultrapure water, and EC1 and EC3 shown in Table 3, out of the diluted solutions, were used as a template of the reaction. The reaction was conducted with a formulation shown in Table 5. The instruments and ultrapure water used were free of DNA. Regarding the method of PCR, the reaction solution was heated at 95°C for 5 minutes, then, a process including heating at 94°C for 10 seconds, at 60°C for 10 seconds and at 72°C for 20 seconds was repeated 35 times. For the primer, 7 sets including (1) a combination of a forward primer SEQ ID No. 1 and a reverse primer SEQ ID No. 16, (2) a combination of a forward primer SEQ ID No. 28 and a reverse primer SEQ ID No. 44, (3) a combination of a forward primer SEQ ID No. 51 and a reverse primer SEQ ID No. 52, (4) a combination of a forward primer SEQ ID No. 53 and a reverse primer SEQ ID No. 57, (5) a combination of a forward primer SEQ ID No. 62 and a reverse primer SEQ ID No. 70, (6) a combination of a forward primer SEQ ID No. 76 and a reverse primer SEQ ID No. 91 and (7) a combination of a forward primer SEQ ID No. 104 and a reverse primer SEQ ID No. 2 were adopted, amplification of DNA thereof was performed, then, the DNA dissociation curve thereof was made and the Tm values were measured. The resultant Tm value was compared with the Tm value of E. coli previously obtained and the D value was determined, according to Example 7 of Patent document 4. This result is shown in Table 6 together with the result of Example 1. When the D value is 0.3 or less, they were judged to be homogeneous.

[Table 6]

Table 6 Comparison between direct-PCR and nested-PCR

| Template | Comparative example 1 | Example 1 |
|---|---|---|
| EC1 | ○ | ○ |

(continued)

| Template | Comparative example 1 | Example 1 |
|---|---|---|
| EC3 | × | ○ |
| ○: Identification was successful<br>×: Identification was a failure | | |

(Example 2) Verification of primer (Electrophoresis with purified coli genome)

In the present example, EC3 shown in Table 3 was used as a template of the reaction. The instruments and ultrapure water used were free of DNA. For the primer combination, the reaction solution was so added as to give (1) respective combinations of a forward primer consisting of SEQ ID No. 1 and reverse primers consisting of SEQ ID No. 2 to 5, (2) respective combinations of forward primers consisting of SEQ ID No. 1 and 6 to 15 and reverse primers consisting of SEQ ID No. 16 to 27, (3) respective combinations of forward primers consisting of SEQ ID No. 28 to 43 and reverse primers consisting of SEQ ID No. 44 to 50, (4) respective combinations of forward primers consisting of SEQ ID No. 53 to 56 and reverse primers consisting of SEQ ID No. 57 to 61, (5) respective combinations of forward primers consisting of SEQ ID No. 62 to 69 and reverse primers consisting of SEQ ID No. 70 to 75, (6) respective combinations of forward primers consisting of SEQ ID No. 76 to 90 and reverse primers consisting of SEQ ID No. 91 to 103, (7) respective combinations of forward primers consisting of SEQ ID No. 104 to 113 and reverse primers consisting of SEQ ID No. 2 and 116 to 131, respective combinations of a forward primer consisting of SEQ ID No. 114 and reverse primers consisting of SEQ ID No. 2 and 5 and 125, respective combinations of a forward primer consisting of SEQ ID No. 115 and reverse primers consisting of SEQ ID No. 2 and 5 and 125, and PCR thereof was performed. The reaction was conducted with a formulation shown in Table 4 for the above-described combination (1), and with a formulation shown in Table 5 for the above-described combinations (2) to (7).

For (1) out of the above-described primer combinations, the reaction solution was heated at 95°C for 5 minutes, then, a process including heating at 94°C for 10 seconds, at 65°C for 10 seconds and at 72°C for 30 seconds was repeated 35 times. For (2) to (7) out of the above-described primer combinations, the reaction solution was heated at 95°C for 5 minutes, then, a process including heating at 94°C for 10 seconds, at 60°C for 10 seconds and at 72°C for 10 seconds was repeated 35 times. The solution after the reaction was electrophoresed on 2% agarose gel and stained with ethidium bromide, then, the amplification amount of the intended product and amplification of the non-specific product were verified. Here, the combination of a forward primer consisting of SEQ ID No. 1 and a reverse primer consisting of SEQ ID No. 2, the combination of a forward primer consisting of SEQ ID No. 1 and a reverse primer consisting of SEQ ID No. 16, the combination of a forward primer consisting of SEQ ID No. 28 and a reverse primer consisting of SEQ ID No. 44, the combination of a forward primer consisting of SEQ ID No. 53 and a reverse primer consisting of SEQ ID No. 57, the combination of a forward primer consisting of SEQ ID No. 62 and a reverse primer consisting of SEQ ID No. 70, the combination of a forward primer consisting of SEQ ID No. 76 and a reverse primer consisting of SEQ ID No. 91 and the combination of a forward primer consisting of SEQ ID No. 104 and a reverse primer consisting of SEQ ID No. 2 correspond to replication experiments of Patent document 4. The results are shown in Tables 7-1 to 7-7 together with the result of Comparative Example 2.

Evaluation in Table 7 was conducted according to the following criteria.

Amount of intended product:

◎ : amplification is particularly good
○: amplification is good
△: amplification is poor
x: amplification is particularly poor

Non-specific amplification product

o: non-specific product is not found
△: non-specific product is found
×: non-specific product is abundant

(Comparative Example 2) Comparative example for Example 2

The following comparative example is a replication experiment of Patent document 4. In the present comparative example, EC3 shown in Table 3 was used as a template of the reaction. The instruments and ultrapure water used were free of DNA. The reaction solution was so added as to give (1) a combination of a forward primer consisting of SEQ ID No. 1 and a reverse primer consisting of SEQ ID No. 2, (2) a combination of a forward primer consisting of SEQ ID No. 1 and a reverse primer consisting of SEQ ID No. 16, (3) a combination of a forward primer consisting of SEQ ID No. 28 and a reverse primer consisting of SEQ ID No. 44, (4) a combination of a forward primer consisting of SEQ ID No. 53 and a reverse primer consisting of SEQ ID No. 57, (5) a combination of a forward primer consisting of SEQ ID No. 62 and a reverse primer consisting of SEQ ID No. 70, (6) a combination of a forward primer consisting of SEQ ID No. 76 and a reverse primer consisting of SEQ ID No. 91 and (7) a combination of a forward primer consisting of SEQ ID No. 104 and a reverse primer consisting of SEQ ID No. 2, and PCR thereof was performed. The reaction was conducted with a formulation shown in Table 4 for the above-described combination (1), and with a formulation shown in Table 5 for the above-described combinations (2) to (7).

For (1) out of the above-described primer combinations, the reaction solution was heated at 95°C for 5 minutes, then, a process including heating at 94°C for 10 seconds, at 65°C for 10 seconds and at 72°C for 30 seconds was repeated 35 times. For (2) to (7) out of the above-described primer combinations, the reaction solution was heated at 95°C for 5 minutes, then, a process including heating at 94°C for 10 seconds, at 60°C for 10 seconds and at 72°C for 10 seconds was repeated 35 times. The solution after the reaction was electrophoresed on 2% agarose gel and stained with ethidium bromide, then, the amplification amount of the intended product and amplification of the non-specific product were verified. The results are shown in Tables 7-1 to 7-7 together with the result of Example 2.

[Table 7-1]

Table 7-1

| | | | | | |
|---|---|---|---|---|---|
| EC3 | Electrophoresis: Amount of intended product | | | | |
| | | SEQ ID No.2 | SEQ ID No. 3 | SEQ ID No. 4 | SEQ ID No. 5 |
| | SEQ ID No. 1 | ○ | ◎ | ○ | ○ |
| | | | | | |
| | Electrophoresis: Non specificityity | | | | |
| | | SEQ ID No. 2 | SEQ ID No. 3 | SEQID No. 4 | SEQ ID No. 5 |
| | SEQ ID No. 1 | ○ | ○ | ○ | ○ |

[Table 7-2]

Table 7-2

| | Electrophoresis: Amount of intended product | | | | | | | | | | | |
|---|---|---|---|---|---|---|---|---|---|---|---|---|
| | SEQ ID No.16 | SEQ ID No. 17 | SEQ ID No. 18 | SEQ ID No.19 | SEQ ID No.20 | SEQ ID No.21 | SEQ No. 22 | SEQ ID No.23 | SEQ ID No.24 | SEQ ID No.25 | SEQ ID No.26 | SEQ ID No.27 |
| SEQ ID No.1 | ○ | × | × | Δ | × | Δ | ◎ | × | Δ | × | × | × |
| SEQ ID No.6 | ◎ | × | × | ◎ | ◎ | Δ | × | Δ | Δ | Δ | ◎ | ◎ |
| SEQ ID No.7 | ◎ | ◎ | ◎ | ◎ | ◎ | ◎ | × | ○ | ○ | × | ◎ | ◎ |
| SEQ ID No.8 | ◎ | ◎ | ◎ | ◎ | ◎ | ◎ | Δ | ○ | Δ | Δ | Δ | Δ |
| SEQ ID No.9 | Δ | Δ | × | Δ | × | × | × | × | × | × | Δ | × |
| SEQ ID No.10 | Δ | × | Δ | × | Δ | Δ | × | × | × | × | × | × |
| SEQ ID No.11 | Δ | Δ | Δ | Δ | Δ | Δ | × | × | × | × | Δ | × |
| SEQ ID No.12 | × | ○ | ○ | ○ | ○ | ○ | × | Δ | Δ | × | ◎ | ○ |
| SEQ ID No.13 | ◎ | ◎ | ◎ | ◎ | ◎ | Δ | Δ | ◎ | ○ | × | ◎ | ◎ |
| SEQ ID No.14 | Δ | Δ | Δ | Δ | Δ | Δ | × | × | Δ | Δ | ○ | ○ |
| SEQ ID No.15 | ◎ | ◎ | ◎ | ◎ | ◎ | ◎ | × | ◎ | ◎ | ◎ | ◎ | ◎ |
| SEQ ID No. 11 | ○ | ○ | ○ | ○ | ○ | ○ | × | × | × | × | ○ | × |

(continued)

| | | | | | | | | | | | | | |
|---|---|---|---|---|---|---|---|---|---|---|---|---|---|
| | SEQ ID No.12 | × | ○ | ○ | ○ | ○ | ○ | × | ○ | ○ | × | ○ | ○ |
| | SEQ ID No.13 | ○ | ○ | ○ | ○ | ○ | ○ | ○ | ○ | ○ | × | ○ | ○ |
| | SEQ ID No.14 | ○ | ○ | ○ | ○ | ○ | ○ | × | × | ○ | ○ | ○ | ○ |
| | SEQ ID No.15 | ○ | ○ | ○ | ○ | ○ | ○ | × | ○ | ○ | ○ | ○ | ○ |
| EC3 | | | | | | | | | | | | | |
| | Electrophoresis : Non specificityity | | | | | | | | | | | | |
| | | SEQ ID No. 16 | SEQ ID No.17 | SEQ ID No.18 | SEQ ID No.19 | SEQ ID No.20 | SEQ ID No.21 | SEQ ID No.22 | SEQ ID No.23 | SEQ ID No.24 | SEQ ID No.25 | SEQ ID No.26 | SEQ ID No.27 |
| | SEQ ID No.1 | ○ | × | × | ○ | × | ○ | ○ | × | ○ | × | × | × |
| | SEQ ID No.6 | ○ | × | x | ○ | ○ | ○ | × | ○ | ○ | ○ | ○ | ○ |
| | SEQ ID No.7 | ○ | ○ | ○ | ○ | ○ | ○ | × | ○ | ○ | × | ○ | ○ |
| | SEQ ID No.8 | ○ | ○ | ○ | ○ | ○ | ○ | ○ | ○ | ○ | ○ | ○ | ○ |
| | SEQ ID No.9 | Δ | ○ | × | ○ | × | × | × | × | × | × | ○ | × |
| | SEQ ID No.10 | ○ | × | ○ | × | ○ | ○ | × | × | × | × | × | × |
| | SEQ ID No. 11 | ○ | ○ | ○ | ○ | ○ | ○ | × | × | × | × | ○ | × |
| | SEQ ID No.12 | × | ○ | ○ | ○ | ○ | ○ | × | ○ | ○ | × | ○ | ○ |
| | SEQ ID No.13 | ○ | ○ | ○ | ○ | ○ | ○ | ○ | ○ | ○ | × | ○ | ○ |

(continued)

| | | | | | | | | | | | | | |
|---|---|---|---|---|---|---|---|---|---|---|---|---|---|
| | SEQ ID No.14 | ○ | ○ | ○ | ○ | ○ | ○ | × | × | ○ | ○ | ○ | ○ |
| | SEQ ID No.15 | ○ | ○ | ○ | ○ | ○ | ○ | × | ○ | ○ | ○ | ○ | ○ |

[Table 7-3]

Table 7-3

| | | | | | | | | |
|---|---|---|---|---|---|---|---|---|
| | Electrophoresis: Amount of intended product | | | | | | | |
| | | SEQ ID No.44 | SEQ ID No.45 | SEQ ID No.46 | SEQ ID No.47 | SEQ ID No.48 | SEQ ID No.49 | SEQ ID No.50 |
| | SEQ ID No.28 | × | Δ | × | Δ | ○ | × | × |
| | SEQ ID No.29 | Δ | Δ | × | Δ | ○ | × | × |
| | SEQ ID No.30 | × | × | × | ○ | × | × | × |
| | SEQ ID No.31 | × | × | Δ | ○ | × | × | × |
| | SEQ ID No.32 | × | ○ | × | ○ | ○ | ○ | × |
| | SEQ ID No.33 | ○ | ◎ | × | ○ | ○ | × | × |
| | SEQ ID No.34 | × | ○ | × | ○ | × | × | × |
| | SEQ ID No.35 | Δ | × | × | × | ○ | ○ | × |
| | SEQ ID No.36 | × | ○ | × | × | ○ | ○ | × |
| | SEQ ID No.37 | × | ◎ | × | ○ | ○ | × | Δ |
| | SEQ ID No.38 | × | × | ○ | × | ○ | ○ | × |
| | SEQ ID No.39 | ◎ | × | ◎ | × | ○ | × | × |
| | SEQ ID No.40 | ○ | × | Δ | × | ○ | Δ | Δ |
| | SEQ ID No.41 | × | × | ○ | × | × | × | × |
| | SEQ ID No.42 | × | × | × | × | × | Δ | × |
| | SEQ ID No.43 | × | × | × | × | × | × | × |
| EC3 | | | | | | | | |
| | Electrophoresis : Non specificityity | | | | | | | |
| | | SEQ ID No.44 | SEQ ID No.45 | SEQ ID No.46 | SEQ ID No.47 | SEQ ID No.48 | SEQ ID No.49 | SEQ ID No.50 |
| | SEQ ID No.28 | × | ○ | × | ○ | O | × | × |

(continued)

| | | | | | | | | |
|---|---|---|---|---|---|---|---|---|
| | SEQ ID No.29 | O | ○ | × | ○ | ○ | × | × |
| | SEQ ID No.30 | × | × | × | ○ | × | × | × |
| | SEQ ID No.31 | × | × | ○ | ○ | × | × | × |
| | SEQ ID No.32 | × | ○ | × | ○ | ○ | ○ | × |
| | SEQ ID No.33 | O | Δ | × | ○ | ○ | × | × |
| | SEQ ID No.34 | × | ○ | × | ○ | × | × | × |
| | SEQ ID No.35 | ○ | × | × | × | ○ | ○ | × |
| | SEQ ID No.36 | × | ○ | × | × | ○ | ○ | × |
| | SEQ ID No.37 | × | ○ | × | ○ | ○ | × | ○ |
| | SEQ ID No.38 | × | × | ○ | × | ○ | ○ | × |
| | SEQ ID No.39 | ○ | × | ○ | × | ○ | × | × |
| | SEQ ID No.40 | O | × | ○ | × | ○ | ○ | ○ |
| | SEQ ID No.41 | × | × | ○ | × | × | × | × |
| | SEQ ID No.42 | × | × | × | × | × | ○ | × |
| | SEQ ID No.43 | × | × | × | × | × | × | × |

[Table 7-4]

Table 7-4

| | | | | | | | |
|---|---|---|---|---|---|---|---|
| EC3 | Electrophoresis : Amount of intended product | | | | | | |
| | | SEQ ID No. 57 | SEQ ID No. 58 | SEQ ID No. 59 | SEQ ID No. 60 | SEQ ID No. 61 | |
| | SEQ ID No.53 | × | Δ | ○ | × | ◎ | |
| | SEQ ID No.54 | × | × | Δ | × | Δ | |
| | SEQ ID No.55 | ○ | ○ | ○ | × | ○ | |
| | SEQ ID No.56 | ○ | ○ | ○ | × | ◎ | |
| | | | | | | | |
| | Electrophoresis : Non specificity | | | | | | |
| | | SEQ ID No.57 | SEQ ID No.58 | SEQ ID No. 59 | SEQ ID No. 60 | SEQ ID No. 61 | |
| | SEQ ID No.53 | × | ○ | ○ | × | ○ | |
| | SEQ ID No.54 | × | × | ○ | × | ○ | |
| | SEQ ID No.55 | ○ | ○ | ○ | × | ○ | |
| | SEQ ID No.56 | ○ | ○ | ○ | × | ○ | |
| | | | | | | | |
| EC3 | Electrophoresis : Amount of intended product | | | | | | |
| | | SEQ ID No.70 | SEQ ID No.71 | SEQ ID No. 72 | SEQ ID No. 73 | SEQ ID No. 74 | SEQID No. 75 |
| | SEQ ID No.62 | ○ | × | - | ○ | ○ | ○ |
| | SEQ ID No.63 | ◎ | × | Δ | Δ | ○ | × |
| | SEQ ID No.64 | ◎ | × | × | × | ○ | × |
| | SEQ ID No.65 | ◎ | ○ | Δ | ○ | × | × |
| | SEQ ID No.66 | Δ | × | × | × | × | Δ |
| | SEQ ID No.67 | Δ | × | × | ○ | × | × |
| | SEQ ID No.68 | ○ | × | × | × | × | × |
| | SEQ ID No.69 | ○ | × | × | × | × | × |
| | | | | | | | |
| | Electrophoresis : Non specificity | | | | | | |

(continued)

| | | SEQ ID No.70 | SEQ ID No.71 | SEQ ID No. 72 | SEQ ID No. 73 | SEQ ID No. 74 | SEQ ID No.75 |
|---|---|---|---|---|---|---|---|
| | SEQ ID No.62 | Δ | × | - | Δ | ○ | ○ |
| | SEQ ID No.63 | ○ | × | ○ | ○ | ○ | × |
| | SEQ ID No.64 | ○ | × | × | × | ○ | × |
| | SEQ ID No.65 | ○ | × | ○ | ○ | × | × |
| | SEQ ID No.66 | ○ | × | × | × | × | ○ |
| | SEQ ID No.67 | ○ | × | × | ○ | × | × |
| | SEQ ID No.68 | ○ | × | × | × | × | × |
| | SEQ ID No.69 | ○ | × | × | × | × | × |

[Table 7-5]

Table 7-5

| Electrophoresis : Amount of intended product | | | | | | | | | | | | | |
|---|---|---|---|---|---|---|---|---|---|---|---|---|---|
| | SEQ ID No.91 | SEQ ID No.92 | SEQ ID No.93 | SEQ ID No.94 | SEQ ID No.95 | SEQ ID No.96 | SEQ ID No.97 | SEQ ID No.98 | SEQ ID No.99 | SEQ ID No.100 | SEQ ID No.101 | SEQ ID No.102 | SEQ ID No.103 |
| SEQ ID No.76 | Δ | × | × | × | × | × | × | × | × | × | ◎ | ◎ | ◎ |
| SEQ ID No.77 | ○ | × | × | × | × | × | × | × | × | × | Δ | Δ | ○ |
| SEQ ID No.78 | ○ | × | × | × | × | × | × | × | × | × | ○ | ○ | ○ |
| SEQ ID No.79 | ○ | × | × | × | × | × | × | × | × | × | ○ | ○ | × |
| SEQ ID No.80 | ○ | × | × | × | × | × | × | × | × | × | ◎ | ◎ | ◎ |
| SEQ ID No.81 | ○ | × | × | × | × | × | × | × | × | × | ◎ | ◎ | ◎ |
| SEQ ID No.82 | ◎ | × | × | × | × | × | × | × | × | × | ◎ | ◎ | ○ |
| SEQ ID No.83 | ○ | × | × | × | × | × | × | × | × | × | ◎ | ◎ | × |
| SEQ ID No.84 | ○ | × | × | × | × | × | × | × | × | × | ◎ | ◎ | ◎ |
| SEQ ID No.85 | ○ | × | × | × | × | × | × | × | × | × | ◎ | ◎ | ○ |
| SEQ ID No.86 | ○ | × | × | × | × | × | × | × | × | × | ◎ | ○ | ○ |
| SEQ ID No.87 | ○ | × | × | × | × | × | × | × | × | × | ◎ | ◎ | ◎ |
| SEQ ID No.88 | ○ | × | × | × | × | × | × | × | × | × | ◎ | × | × |

(continued)

| | | | | | | | | | | | | | | |
|---|---|---|---|---|---|---|---|---|---|---|---|---|---|---|
| EC3 | SEQ ID No.89 | ◎ | × | × | × | × | × | × | × | × | × | × | ◎ | × |
| | SEQ ID No.90 | ○ | × | × | × | × | × | × | × | × | × | ○ | ○ | ○ |
| | | | | | | | | | | | | | | |
| | Electrophoresis : Non specificity | | | | | | | | | | | | | |
| | | SEQ ID No.91 | SEQ ID No.92 | SEQ ID No.93 | SEQ ID No.94 | SEQ ID No.95 | SEQ ID No.96 | SEQ ID No.97 | SEQ ID No.98 | SEQ ID No.99 | SEQ ID No.100 | SEQ ID No.101 | SEQ ID No.102 | SEQ ID No.103 |
| | SEQ ID No.76 | ○ | × | × | × | × | × | × | × | × | × | ○ | ○ | ○ |
| | SEQ ID No.77 | ○ | × | × | × | × | × | × | × | × | × | ○ | ○ | ○ |
| | SEQ ID No.78 | ○ | × | × | × | × | × | × | × | × | × | ○ | ○ | ○ |
| | SEQ ID No.79 | ○ | × | × | × | × | × | × | × | × | × | Δ | ○ | × |
| | SEQ ID No.80 | ○ | × | × | × | × | × | × | × | × | × | ○ | ○ | ○ |
| | SEQ ID No.81 | ○ | × | × | × | × | × | × | × | × | × | ○ | ○ | ○ |
| | SEQ ID No.82 | ○ | × | × | × | × | × | × | × | × | × | ○ | Δ | ○ |
| | SEQ ID No.83 | ○ | × | × | × | × | × | × | × | × | × | ○ | ○ | × |
| | SEQ ID No.84 | ○ | × | × | × | × | × | × | × | × | × | ○ | ○ | ○ |
| | SEQ ID No.85 | ○ | × | × | × | × | × | × | × | × | × | ○ | Δ | Δ |
| | SEQ ID No.86 | ○ | × | × | × | × | × | × | × | × | × | Δ | ○ | Δ |

(continued)

| | | | | | | | | | | | | | |
|---|---|---|---|---|---|---|---|---|---|---|---|---|---|
| | SEQ ID No.87 | ○ | × | × | × | × | × | × | × | × | × | ○ | ○ | Δ |
| | SEQ ID No.88 | ○ | × | × | × | × | × | × | × | × | × | ○ | × | × |
| | SEQ ID No.89 | × | × | × | × | × | × | × | × | × | × | × | ○ | × |
| | SEQ ID No.90 | ○ | × | × | × | × | × | × | × | × | × | ○ | ○ | ○ |

[Table 7-6]

Table 7-6

| EC3 | Electrophoresis: Amount of intended product | | | | | | | | |
|---|---|---|---|---|---|---|---|---|---|
| | SEQ ID No.2 | SEQ ID No.116 | SEQ ID No.117 | SEQ ID No.118 | SEQ ID No.119 | SEQ ID No.120 | SEQ ID No.121 | SEQ ID No.122 | SEQ ID No.123 |
| SEQ ID No.104 | ○ | △ | △ | ◎ | ◎ | ◎ | △ | ◎ | ◎ |
| SEQ ID No.105 | ○ | ○ | ○ | ◎ | ◎ | ◎ | ◎ | ◎ | ◎ |
| SEQ ID No.106 | × | △ | × | ○ | ○ | ○ | ○ | △ | ○ |
| SEQ ID No.107 | △ | × | × | × | △ | △ | △ | × | △ |
| SEQ ID No.108 | ○ | × | × | ○ | ○ | ○ | ○ | × | ○ |
| SEQ ID No.109 | ○ | △ | △ | ○ | ○ | ○ | ○ | ○ | ○ |
| SEQ ID No.110 | × | ○ | ○ | ○ | ○ | ○ | ○ | ○ | ○ |
| SEQ ID No.111 | ○ | △ | △ | ○ | ○ | ○ | ○ | ○ | ○ |
| SEQ ID No.112 | △ | △ | △ | ○ | ○ | ○ | ○ | ○ | ○ |
| SEQ ID No.113 | △ | △ | △ | × | ○ | ○ | ○ | ○ | ○ |
| SEQ ID No.114 | ○ | | | | | | | | |
| SEQ ID No.115 | ○ | | | | | | | | |

| EC3 | Electrophoresis: Amount of intended product | | | | | | | | |
|---|---|---|---|---|---|---|---|---|---|
| | SEQ ID No.124 | SEQ ID No.125 | SEQ ID No.5 | SEQ ID No.126 | SEQ ID No.127 | SEQ ID No.128 | SEQ ID No.129 | SEQ ID No.130 | SEQ ID No.131 |
| SEQ ID No.104 | ◎ | ◎ | ◎ | × | △ | ◎ | △ | △ | ◎ |
| SEQ ID No.105 | ◎ | ◎ | ◎ | × | × | × | × | × | × |
| SEQ ID No.106 | △ | ○ | ○ | × | △ | △ | ○ | ◎ | △ |
| SEQ ID No.107 | △ | △ | △ | × | △ | △ | △ | △ | × |
| SEQ ID No.108 | × | ○ | ○ | × | × | △ | × | × | × |
| SEQ ID No.109 | ○ | ○ | ○ | ◎ | ○ | ○ | ○ | ○ | ○ |
| SEQ ID No.110 | ○ | ○ | ○ | ○ | ○ | ○ | ○ | ○ | ○ |
| SEQ ID No.111 | ○ | ○ | ○ | ○ | ○ | ○ | ○ | ○ | ○ |
| SEQ ID No.112 | ○ | ○ | ○ | ○ | ○ | ○ | ○ | ○ | ○ |
| SEQ ID No.113 | ○ | ○ | ○ | ○ | ○ | ○ | ○ | ○ | ○ |
| SEQ ID No.114 | | ○ | ○ | | | | | | |
| SEQ ID No.115 | | × | ○ | | | | | | |

[Table 7-7]

Table 7-7

| | Electrophoresis | Non specific product | | | | | | | | |
|---|---|---|---|---|---|---|---|---|---|---|
| | | SEQ ID No.2 | SEQ ID No.116 | SEQ ID No.117 | SEQ ID No.118 | SEQ ID No.119 | SEQ ID No.120 | SEQ ID No.121 | SEQ ID No.122 | SEQ ID No.123 |
| EC3 | SEQ ID No.104 | ○ | ○ | ○ | ○ | ○ | ○ | △ | ○ | ○ |
| | SEQ ID No.105 | ○ | ○ | ○ | ○ | ○ | ○ | ○ | ○ | ○ |
| | SEQ ID No.106 | × | ○ | × | ○ | ○ | ○ | ○ | ○ | ○ |
| | SEQ ID No.107 | ○ | × | × | × | ○ | ○ | ○ | × | ○ |
| | SEQ ID No.108 | ○ | × | × | ○ | ○ | ○ | ○ | × | ○ |
| | SEQ ID No.109 | ○ | ○ | ○ | ○ | ○ | ○ | ○ | ○ | ○ |
| | SEQ ID No.110 | × | ○ | ○ | ○ | ○ | ○ | ○ | ○ | ○ |
| | SEQ ID No.111 | ○ | ○ | ○ | ○ | ○ | ○ | ○ | ○ | ○ |
| | SEQ ID No.112 | ○ | ○ | ○ | ○ | ○ | ○ | ○ | ○ | ○ |
| | SEQ ID No.113 | ○ | ○ | ○ | × | × | ○ | ○ | ○ | ○ |
| | SEQ ID No.114 | ○ | | | | | | | | |
| | SEQ ID No.115 | ○ | | | | | | | | |

| | Electrophoresis: | Non specific product | | | | | | | | |
|---|---|---|---|---|---|---|---|---|---|---|
| | | SEQ ID No.124 | SEQ ID No.125 | SEQ ID No.5 | SEQ ID No.126 | SEQ ID No.127 | SEQ ID No.128 | SEQ ID No.129 | SEQ ID No.130 | SEQ ID No.131 |
| EC3 | SEQ ID No.104 | ○ | ○ | ○ | × | ○ | ○ | ○ | ○ | ○ |
| | SEQ ID No.105 | ○ | ○ | ○ | × | × | × | × | × | × |
| | SEQ ID No.106 | ○ | ○ | ○ | × | ○ | ○ | ○ | ○ | ○ |
| | SEQ ID No.107 | ○ | ○ | ○ | × | ○ | ○ | ○ | ○ | × |
| | SEQ ID No.108 | × | ○ | ○ | × | × | ○ | × | × | × |
| | SEQ ID No.109 | ○ | ○ | ○ | ○ | ○ | ○ | ○ | ○ | ○ |
| | SEQ ID No.110 | ○ | ○ | ○ | ○ | ○ | ○ | ○ | ○ | ○ |
| | SEQ ID No.111 | ○ | ○ | ○ | ○ | ○ | ○ | ○ | ○ | ○ |
| | SEQ ID No.112 | ○ | ○ | ○ | ○ | ○ | ○ | ○ | ○ | ○ |
| | SEQ ID No.113 | ○ | × | ○ | ○ | ○ | ○ | ○ | ○ | ○ |
| | SEQ ID No.114 | | ○ | × | | | | | | |
| | SEQ ID No.115 | | × | ○ | | | | | | |

(Example 3) Verification of primer (PCR with purified coli genome and melting thereof)

In the present example, EC3 in Table 3 was used as a template. The instruments and ultrapure water used were free of DNA. The reaction solution was so added as to give (1) respective combinations of a forward primer consisting of SEQ ID No. 1 and reverse primers consisting of SEQ ID No. 2 to 5, (2) respective combinations of forward primers consisting of SEQ ID No. 1 and 6 to 15 and reverse primers consisting of SEQ ID No. 16 to 27, (3) respective combinations of forward primers consisting of SEQ ID No. 28 to 43 and reverse primers consisting of SEQ ID No. 44 to 50, (4) respective combinations of forward primers consisting of SEQ ID No. 53 to 56 and reverse primers consisting of SEQ ID No. 57 to 61, (5) respective combinations of forward primers consisting of SEQ ID No. 62 to 69 and reverse primers consisting of SEQ ID No. 70 to 75, (6) respective combinations of forward primers consisting of SEQ ID No. 76 to 90 and reverse primers consisting of SEQ ID No. 91 to 103, (7) respective combinations of forward primers consisting of SEQ ID No. 104 to 113 and reverse primers consisting of SEQ ID No. 2 and 116 to 131, respective combinations of a forward primer consisting of SEQ ID No. 114 and reverse primers consisting of SEQ ID No. 2 and 5 and 125 and respective combinations of a forward primer consisting of SEQ ID No. 115 and reverse primers consisting of SEQ ID No. 2 and 5 and 125, and PCR thereof was performed. The reaction was conducted with a formulation shown in Table 4 for the above-described combination (1), and with a formulation shown in Table 5 for the above-described combinations (2) to (7).

Rotor-Gene Q MDx 5plex HRM (QIAGEN) was used as a real time PCR apparatus, and in the program thereof, for (1) out of the above-described primer combinations, the reaction solution was heated at 95°C for 5 minutes, then, a process including heating at 94°C for 10 seconds, at 65°C for 10 seconds and at 72°C for 30 seconds was repeated 35 times, and for (2) to (7), the reaction solution was heated at 95°C for 5 minutes, then, a process including heating at 94°C for 10 seconds, at 60°C for 10 seconds and at 72°C for 10 seconds was repeated 35 times, then, the DNA dissociation curve of the amplification product was made. The reaction solution was heated at 95°C for 10 seconds, then, incubated at 72°C for 90 seconds, and heated up to 95°C by 0.5°C stepwise, in order to make the DNA dissociation curve. At each stage, the reaction solution was incubated for 2 seconds, and the data were obtained. The threshold cycle of the amplification curve and the dissociation curve were verified. Here, a combination of a forward primer consisting of SEQ ID No. 1 and a reverse primer consisting of SEQ ID No. 2, a combination of a forward primer consisting of SEQ ID No. 1 and a reverse primer consisting of SEQ ID No. 16, a combination of a forward primer consisting of SEQ ID No. 28 and a reverse primer consisting of SEQ ID No. 44, a combination of a forward primer consisting of SEQ ID No. 53 and a reverse primer consisting of SEQ ID No. 57, a combination of a forward primer consisting of SEQ ID No. 62 and a reverse primer consisting of SEQ ID No. 70, a combination of a forward primer consisting of SEQ ID No. 76 and a reverse primer consisting of SEQ ID No. 91 and a combination of a forward primer consisting of SEQ ID No. 104 and a reverse primer consisting of SEQ ID No. 2 correspond to replication experiments of Patent document 4. The results are shown in Tables 8-1 to 8-7 together with the result of Comparative Example 3.

Evaluation in Table 8 was carried out according to the following criteria.

Threshold cycle:

- ◎ : threshold cycle is fast (decreased by 2 cycles or more)
- ○: threshold cycle is somewhat fast (decreased by 0-1 cycle)
- Δ: threshold cycle is somewhat slow (increased by 1-2 cycles)
- ×: threshold cycle is slow (increased by 3 cycles or more)

Melting analysis

- ○: only one peak
- Δ: small peaks
- ×: several peaks
- -: no peak

(Comparative Example 3) Comparative example for Example 3

The following comparative example is a replication experiment of Patent document 4. In the present comparative example, EC3 shown in Table 3 was used as a template of the reaction. The instruments and ultrapure water used were free of DNA, and the operation was conducted in a clean bench. The reaction solution was so added as to give (1) a combination of a forward primer consisting of SEQ ID No. 1 and a reverse primer consisting of SEQ ID No. 2, (2) a combination of a forward primer consisting of SEQ ID No. 1 and a reverse primer consisting of SEQ ID No. 16, (3) a combination of a forward primer consisting of SEQ ID No. 28 and a reverse primer consisting of SEQ ID No. 44, (4) a combination of a forward primer consisting of SEQ ID No. 53 and a reverse primer consisting of SEQ ID No. 57, (5) a combination of a forward primer consisting of SEQ ID No. 62 and a reverse primer consisting of SEQ ID No. 70, (6) a combination of a forward primer consisting of SEQ ID No. 76 and a reverse primer consisting of SEQ ID No. 91 and (7) a combination of a forward primer consisting of SEQ ID No. 104 and a reverse primer consisting of SEQ ID No. 2, and PCR thereof was conducted. The reaction was conducted with a formulation shown in Table 4 for the above-described combination (1), and with a formulation shown in Table 5 for the above-described combinations (2) to (7).

Rotor-Gene Q MDx 5plex HRM (QIAGEN) was used as a real time PCR apparatus, and in the program thereof, for (1) out of the above-described primer combinations, the reaction solution was heated at 95°C for 5 minutes, then, a process including heating at 94°C for 10 seconds, at 65°C for 10 seconds and at 72°C for 30 seconds was repeated 35 times, and for (2) to (7), the reaction solution was heated at 95°C for 5 minutes, then, a process including heating at 94°C for 10 seconds, at 60°C for 10 seconds and at 72°C for 10 seconds was repeated 35 times, then, the DNA dissociation curve of the amplification product was made. In creation of the DNA dissociation curve, the reaction solution was heated at 95°C for 10 seconds, then, incubated at 72°C for 90 seconds, and heated up to 95°C by 0.5°C stepwise. At each stage, the reaction solution was incubated for 2 seconds, and the data were obtained. The threshold cycle of the amplification curve and the dissociation curve were verified. The results are shown in Tables 8-1 to 8-7 together with the result in Example 3.

[Table 8-1]

Table 8-1

| | | | | | |
|---|---|---|---|---|---|
| EC3 | PCR: Threshold cycle | | | | |
| | | SEQ ID No. 2 | SEQ ID No. 3 | SEQ ID No. 4 | SEQ ID No. 5 |
| | SEQ ID No. 1 | ○ | ○ | O | O |
| | | | | | |
| | PCR: Melting analysis | | | | |
| | | SEQ ID No. 2 | SEQ ID No. 3 | SEQ ID No. 4 | SEQ ID No. 5 |
| | SEQ ID No. 1 | × | ○ | ○ | |

[Table 8-2]

Table 8-2

| | PCR:Threshold cycle | | | | | | | | | | | | |
|---|---|---|---|---|---|---|---|---|---|---|---|---|---|
| | | SEQ ID No.16 | SEQ ID No. 17 | SEQ ID No.18 | SEQ ID No. 19 | SEQ ID No.20 | SEQ ID No.21 | SEQ ID No.22 | SEQ ID No.23 | SEQ ID No.24 | SEQ ID No.25 | SEQ ID No.26 | SEQ ID No.27 |
| | SEQ ID No.1 | ○ | - | - | - | - | - | Δ | - | ○ | - | - | - |
| | SEQ ID No.6 | ○ | - | - | Δ | Δ | ◎ | - | Δ | Δ | - | ◎ | ◎ |
| | SEQ ID No.7 | ○ | ○ | ○ | ◎ | ◎ | ○ | - | Δ | Δ | - | ○ | ○ |
| | SEQ ID No.8 | ◎ | ○ | Δ | ○ | ○ | ○ | - | ○ | ○ | - | ◎ | ◎ |
| | SEQ ID No.9 | ○ | - | - | - | - | - | - | - | - | - | - | - |
| | SEQ ID No.10 | - | - | - | - | ○ | × | - | - | - | - | - | - |
| | SEQ ID No. 11 | ◎ | ○ | × | - | - | - | - | - | - | - | Δ | - |
| | SEQ ID No.12 | - | ◎ | ◎ | ◎ | ◎ | ○ | Δ | Δ | Δ | × | ◎ | ◎ |
| | SEQ ID No.13 | ◎ | ◎ | ◎ | ◎ | ◎ | ◎ | - | ◎ | Δ | - | Δ | Δ |
| | SEQ ID No.14 | - | - | - | - | - | - | - | - | - | - | - | - |
| | SEQ ID No.15 | ◎ | ◎ | ◎ | ◎ | ◎ | ◎ | - | ◎ | ◎ | ◎ | ◎ | ◎ |
| EC3 | | | | | | | | | | | | | |
| | PCR: Melting analysis | | | | | | | | | | | | |
| | | SEQ ID No.16 | SEQ ID No.17 | SEQ ID No.18 | SEQ ID No.19 | SEQ ID No.20 | SEQ ID No.21 | SEQ ID No.22 | SEQ ID No.23 | SEQ ID No.24 | SEQ ID No.25 | SEQ ID No.26 | SEQ ID No.27 |

(continued)

| | | | | | | | | | | | | | |
|---|---|---|---|---|---|---|---|---|---|---|---|---|---|
| | SEQ ID No.1 | Δ | - | - | - | - | - | Δ | - | Δ | - | - | - |
| | SEQ ID No.6 | O | - | - | ○ | ○ | × | - | ○ | ○ | - | Δ | Δ |
| | SEQ ID No.7 | ○ | ○ | ○ | ○ | O | ○ | - | ○ | ○ | - | ○ | ○ |
| | SEQ ID No.8 | ○ | ○ | ○ | ○ | ○ | ○ | - | ○ | ○ | - | Δ | Δ |
| | SEQ ID No.9 | Δ | - | - | - | - | - | - | - | - | - | - | - |
| | SEQ ID No.10 | - | - | - | - | ○ | ○ | - | - | - | - | - | - |
| | SEQ ID No. 11 | Δ | Δ | ○ | - | - | - | - | - | - | - | ○ | - |
| | SEQ ID No.12 | - | ○ | ○ | ○ | ○ | ○ | ○ | ○ | ○ | ○ | ○ | ○ |
| | SEQ ID No.13 | ○ | ○ | ○ | ○ | ○ | Δ | - | ○ | ○ | - | ○ | ○ |
| | SEQ ID No.14 | - | - | - | - | - | - | - | - | - | - | - | - |
| | SEQ ID No.15 | ○ | ○ | ○ | ○ | ○ | Δ | - | ○ | ○ | ○ | ○ | ○ |

[Table 8-3]

Table 8-3

| | PCR:Threshold cycle | | | | | | | |
|---|---|---|---|---|---|---|---|---|
| | | SEQ ID No.44 | SEQ ID No.45 | SEQ ID SEQ ID No.46 No.47 | | SEQ ID No.48 | SEQ ID No.49 | SEQ ID No.50 |
| | SEQ ID No.28 | - | ○ | - | ○ | ○ | ○ | ○ |
| | SEQ ID No.29 | ○ | ○ | - | ○ | O | - | ○ |
| | SEQ ID No.30 | - | ◎ | - | ○ | ○ | ◎ | ◎ |
| | SEQ ID No.31 | - | - | - | ◎ | - | - | - |
| | SEQ ID No. 32 | - | ○ | - | ○ | ○ | ○ | - |
| | SEQ ID No.33 | ○ | ○ | - | ○ | ○ | ○ | ◎ |
| | SEQ ID No.34 | - | ◎ | - | ○ | ◎ | - | - |
| | SEQ ID No.35 | ◎ | - | - | - | ○ | ○ | - |
| | SEQ ID No. 36 | - | ○ | - | - | ○ | ◎ | - |
| | SEQ ID No.37 | - | ○ | - | ◎ | ○ | ○ | ◎ |
| | SEQ ID No. 38 | - | ○ | ○ | ○ | ○ | - | ○ |
| | SEQ ID No.39 | - | ○ | - | ○ | ○ | - | ○ |
| | SEQ ID No.40 | - | ○ | ○ | ○ | ○ | - | ○ |
| | SEQ ID No.41 | - | ○ | - | - | - | - | - |
| | SEQ ID No.42 | - | - | - | - | ○ | - | - |
| | SEQ ID No.43 | - | - | - | - | - | - | - |
| EC3 | | | | | | | | |
| | PCR: Melting analysis | | | | | | | |
| | | SEQ ID SEQ ID No.44 No.45 | | SEQ ID No.46 | SEQ ID No.47 | SEQ ID No.48 | SEQ ID No.49 | SEQ ID No.50 |
| | SEQ ID No.28 | - | Δ | - | Δ | Δ | Δ | Δ |

(continued)

| | | | | | | | | |
|---|---|---|---|---|---|---|---|---|
| | SEQ ID No.29 | Δ | ○ | - | ○ | Δ | - | Δ |
| | SEQ ID No.30 | - | × | - | ○ | × | × | × |
| | SEQ ID No.31 | - | - | - | - | - | - | - |
| | SEQ ID No.32 | - | Δ | - | Δ | ○ | Δ | - |
| | SEQ ID No.33 | ○ | × | - | × | × | × | × |
| | SEQ ID No.34 | - | Δ | - | Δ | Δ | - | - |
| | SEQ ID No.35 | Δ | - | - | - | ○ | Δ | - |
| | SEQ ID No.36 | - | ○ | - | - | ○ | ○ | - |
| | SEQ ID No.37 | - | ○ | - | ○ | ○ | Δ | Δ |
| | SEQ ID No.38 | - | Δ | Δ | Δ | × | - | Δ |
| | SEQ ID No.39 | - | ○ | - | ○ | Δ | - | × |
| | SEQ ID No.40 | - | Δ | ○ | ○ | × | - | Δ |
| | SEQ ID No.41 | - | ○ | - | - | - | - | - |
| | SEQ ID No.42 | - | - | - | - | ○ | - | - |
| | SEQ ID No.43 | - | - | - | - | - | - | - |

[Table 8-4]

Table 8-4

| | | | | | | | |
|---|---|---|---|---|---|---|---|
| EC3 | PCR:Threshold cycle | | | | | | |
| | | SEQ ID No. 57 | SEQ ID No. 58 | SEQ ID No. 59 | SEQ ID No. 60 | SEQ ID No. 61 | |
| | SEQ ID No. 53 | - | ○ | ○ | ○ | ○ | |
| | SEQ ID No. 54 | - | - | ○ | - | ○ | |
| | SEQ ID No. 55 | ○ | ○ | ○ | - | ○ | |
| | SEQ ID No. 56 | ○ | ○ | ○ | - | ○ | |
| | | | | | | | |
| | PCR: Melting analysis | | | | | | |
| | | SEQ ID No. 57 | SEQ ID No. 58 | SEQ ID No. 59 | SEQ ID No. 60 | SEQ ID No. 61 | |
| | SEQ ID No. 53 | - | ○ | ○ | Δ | ○ | |
| | SEQ ID No. 54 | - | - | ○ | - | ○ | |
| | SEQ ID No. 55 | ○ | ○ | ○ | - | ○ | |
| | SEQ ID No. 56 | ○ | ○ | ○ | - | ○ | |
| | | | | | | | |
| EC3 | PCR:Threshold cycle | | | | | | |
| | - | SEQ ID No. 70 | SEQ ID No. 71 | SEQ ID No. 72 | SEQ ID No. 73 | SEQ ID No. 74 | SEQ ID No. 75 |
| | SEQ ID No. 62 | - | - | | ○ | - | - |
| | SEQ ID No. 63 | ○ | ○ | - | ◎ | ◎ | ○ |
| | SEQ ID No. 64 | - | - | - | - | - | ○ |
| | SEQ ID No. 65 | ○ | ○ | - | ○ | ○ | - |
| | SEQ ID No. 66 | ○ | - | - | ○ | - | - |
| | SEQ ID No. 67 | ○ | ○ | - | ◎ | ○ | ○ |
| | SEQ ID No. 68 | ○ | ○ | ○ | ○ | ○ | ○ |
| | SEQ ID No. 69 | ○ | ○ | ○ | ○ | ○ | ○ |
| | | | | | | | |
| | PCR: Melting analysis | | | | | | |

(continued)

| | - | SEQ ID No. 70 | SEQ ID No. 71 | SEQ ID No. 72 | SEQ ID No. 73 | SEQ ID No. 74 | SEQ ID No. 75 |
|---|---|---|---|---|---|---|---|
| | SEQ ID No. 62 | - | - | | ○ | - | - |
| | SEQ ID No. 63 | ○ | ○ | - | Δ | × | ○ |
| | SEQ ID No. 64 | - | - | - | - | - | ○ |
| | SEQ ID No. 65 | ○ | Δ | - | ○ | ○ | - |
| | SEQ ID No. 66 | ○ | - | - | × | - | - |
| | SEQ ID No. 67 | Δ | ○ | - | Δ | Δ | ○ |
| | SEQ ID No. 68 | × | ○ | ○ | Δ | ○ | ○ |
| | SEQ ID No. 69 | Δ | ○ | ○ | × | ○ | Δ |

[Table 8-5]

Table 8-5

| | | | | | | | | | | | | | | |
|---|---|---|---|---|---|---|---|---|---|---|---|---|---|---|
| EC3 | PCR; Threshold cycle | | | | | | | | | | | | | |
| | ○ | SEQ ID No.91 | SEQ ID No.92 | SEQ ID No.93 | SEQ ID No.94 | SEQ ID No.95 | SEQ ID No.96 | SEQ ID No.97 | SEQ ID No.98 | SEQ ID No.99 | SEQ ID No.100 | SEQ ID No.101 | SEQ ID No.102 | SEQ ID No.103 |
| | SEQ ID No.76 | × | - | - | - | - | - | - | - | - | - | ○ | ○ | ○ |
| | SEQ ID No.77 | - | - | - | - | - | - | - | - | - | - | ○ | △ | △ |
| | SEQ ID No.78 | - | - | - | - | - | - | - | - | - | - | △ | × | × |
| | SEQ ID No.79 | × | - | - | - | - | - | × | - | - | - | △ | △ | ○ |
| | SEQ ID No.80 | × | - | - | - | - | × | × | × | × | × | ○ | ○ | ○ |
| | SEQ ID No.81 | × | - | - | - | - | - | - | - | - | - | ○ | ○ | ○ |
| | SEQ ID No.82 | △ | - | - | - | - | - | × | - | × | - | ○ | ○ | ○ |
| | SEQ ID No.83 | △ | - | - | - | - | - | - | - | - | - | ○ | ○ | ○ |
| | SEQ ID No.84 | ○ | - | - | - | - | - | - | - | - | - | ○ | ○ | ○ |
| | SEQ ID No.85 | △ | - | - | - | - | - | × | - | × | - | ○ | ○ | ○ |
| | SEQ ID No.86 | △ | - | - | - | - | - | × | - | - | - | ○ | ○ | ○ |
| | SEQ ID No.87 | △ | - | - | - | - | - | - | - | - | - | △ | ○ | ○ |
| | SEQ ID No.88 | ◎ | - | - | - | - | - | - | - | - | - | △ | - | - |
| | SEQ ID No.89 | ○ | - | - | - | - | - | - | - | - | - | ◎ | ○ | ○ |
| | SEQ ID No.90 | × | - | - | - | - | - | - | - | - | - | ○ | ○ | △ |
| | PCR: Melting analysis | | | | | | | | | | | | | |
| | ○ | SEQ ID No.91 | SEQ ID No.92 | SEQ ID No.93 | SEQ ID No.94 | SEQ ID No.95 | SEQ ID No.96 | SEQ ID No.97 | SEQ ID No.98 | SEQ ID No.99 | SEQ ID No.100 | SEQ ID No.101 | SEQ ID No.102 | SEQ ID No.103 |
| | SEQ ID No.76 | ○ | - | - | - | - | - | - | - | - | - | ○ | ○ | ○ |
| | SEQ ID No.77 | - | - | - | - | - | - | - | - | - | - | ○ | ○ | ○ |
| | SEQ ID No.78 | - | - | - | - | - | - | - | - | - | - | ○ | ○ | ○ |
| | SEQ ID No.79 | ○ | - | - | - | - | - | △ | - | - | - | ○ | ○ | △ |
| | SEQ ID No.80 | ○ | - | - | - | - | × | × | × | × | × | ○ | △ | ○ |
| | SEQ ID No.81 | ○ | - | - | - | - | - | - | - | - | - | ○ | ○ | ○ |
| | SEQ ID No.82 | ○ | - | - | - | - | - | △ | - | × | - | ○ | ○ | ○ |
| | SEQ ID No.83 | ○ | - | - | - | - | - | - | - | - | - | ○ | ○ | ○ |
| | SEQ ID No.84 | ○ | - | - | - | - | - | - | - | - | - | ○ | ○ | ○ |
| | SEQ ID No.85 | △ | - | - | - | - | - | × | - | × | - | ○ | ○ | ○ |
| | SEQ ID No.86 | ○ | - | - | - | - | - | △ | - | - | - | ○ | △ | ○ |
| | SEQ ID No.87 | ○ | - | - | - | - | - | - | - | - | - | ○ | △ | ○ |
| | SEQ ID No.88 | △ | - | - | - | - | - | - | - | - | - | ○ | - | - |
| | SEQ ID No.89 | ○ | - | - | - | - | - | - | - | - | - | △ | △ | ○ |
| | SEQ ID No.90 | ○ | - | - | - | - | - | - | - | - | - | △ | △ | ○ |

[Table 8-6]

Table 8-6

| | PCR;Threshold cycle | | | | | | | | | |
|---|---|---|---|---|---|---|---|---|---|---|
| | | SEQ ID No.2 | SEQ ID No.116 | SEQ ID No.117 | SEQ ID No.118 | SEQ ID No.119 | SEQ ID No.120 | SEQ ID No.121 | SEQ ID No.122 | SEQ ID No.123 |
| EC3 | SEQ ID No.104 | ○ | ○ | ○ | ○ | ○ | ○ | ○ | ○ | ◎ |
| | SEQ ID No.105 | - | ○ | ○ | ○ | ○ | ○ | ○ | ○ | ○ |
| | SEQ ID No.106 | ◎ | ○ | ○ | ○ | ○ | ○ | ○ | ○ | ○ |
| | SEQ ID No.107 | ○ | - | - | ○ | ○ | ○ | ○ | ○ | ○ |
| | SEQ ID No.108 | ○ | ○ | - | ○ | ○ | ○ | ○ | ○ | ○ |
| | SEQ ID No.109 | ○ | ○ | ○ | ○ | ○ | ○ | ○ | ○ | ○ |
| | SEQ ID No.110 | - | ○ | ○ | ○ | ○ | ○ | ○ | ○ | ○ |
| | SEQ ID No.111 | ○ | ○ | ○ | ○ | ○ | ○ | ○ | ○ | ○ |
| | SEQ ID No.112 | ○ | ○ | ○ | ○ | ○ | ○ | ○ | ○ | ○ |
| | SEQ ID No.113 | ○ | - | - | - | ○ | ○ | ○ | ○ | ○ |
| | SEQ ID No.114 | | | | | | | | | |
| | SEQ ID No.115 | | | | | | | | | |

| | PCR;Threshold cycle | | | | | | | | | |
|---|---|---|---|---|---|---|---|---|---|---|
| | | SEQ ID No.124 | SEQ ID No.125 | SEQ ID No.5 | SEQ ID No.126 | SEQ ID No.127 | SEQ ID No.128 | SEQ ID No.129 | SEQ ID No.130 | SEQ ID No.131 |
| EC3 | SEQ ID No.104 | ○ | ○ | ○ | - | - | ○ | ○ | ◎ | ○ |
| | SEQ ID No.105 | ○ | ○ | ○ | - | - | - | - | - | - |
| | SEQ ID No.106 | ○ | ○ | ○ | ○ | - | - | ○ | ○ | ○ |
| | SEQ ID No.107 | ○ | ○ | ○ | - | - | - | - | - | - |
| | SEQ ID No.108 | ○ | ○ | ○ | - | - | - | - | - | - |
| | SEQ ID No.109 | ○ | ○ | ○ | ○ | ○ | ○ | ○ | ○ | ○ |
| | SEQ ID No.110 | ○ | ○ | ○ | ○ | ○ | ○ | ○ | ○ | ○ |
| | SEQ ID No.111 | ○ | ○ | ○ | - | - | - | - | - | - |
| | SEQ ID No.112 | ○ | ○ | ○ | - | - | - | - | - | - |
| | SEQ ID No.113 | ○ | - | ○ | - | - | - | - | - | - |
| | SEQ ID No.114 | | | | | | | | | |
| | SEQ ID No.115 | | | | | | | | | |

[Table 8-7]

Table 8-7

| PCR: Melting analysis | | SEQ ID No.2 | SEQ ID No.116 | SEQ ID No.117 | SEQ ID No.118 | SEQ ID No.119 | SEQ ID No.120 | SEQ ID No.121 | SEQ ID No.122 | SEQ ID No.123 |
|---|---|---|---|---|---|---|---|---|---|---|
| EC3 | SEQ ID No.104 | ○ | △ | ○ | ○ | ○ | ○ | × | ○ | ○ |
| | SEQ ID No.105 | - | ○ | △ | ○ | ○ | ○ | ○ | △ | ○ |
| | SEQ ID No.106 | ○ | ○ | △ | ○ | ○ | ○ | ○ | ○ | ○ |
| | SEQ ID No.107 | ○ | - | - | × | ○ | ○ | ○ | ○ | ○ |
| | SEQ ID No.108 | ○ | △ | - | ○ | ○ | ○ | ○ | ○ | ○ |
| | SEQ ID No.109 | ○ | × | × | ○ | ○ | △ | △ | ○ | ○ |
| | SEQ ID No.110 | - | ○ | × | ○ | ○ | ○ | ○ | ○ | ○ |
| | SEQ ID No.111 | ○ | ○ | ○ | ○ | ○ | ○ | ○ | ○ | ○ |
| | SEQ ID No.112 | ○ | ○ | ○ | ○ | ○ | ○ | ○ | ○ | ○ |
| | SEQ ID No.113 | △ | - | - | - | △ | ○ | ○ | ○ | ○ |
| | SEQ ID No.114 | | | | | | | | | |
| | SEQ ID No.115 | | | | | | | | | |

| PCR: Melting analysis | | SEQ ID No.124 | SEQ ID No.125 | SEQ ID No.5 | SEQ ID No.126 | SEQ ID No.127 | SEQ ID No.128 | SEQ ID No.129 | SEQ ID No.130 | SEQ ID No.131 |
|---|---|---|---|---|---|---|---|---|---|---|
| EC3 | SEQ ID No.104 | ○ | ○ | ○ | - | - | ○ | ○ | × | ○ |
| | SEQ ID No.105 | ○ | ○ | ○ | - | - | - | - | - | - |
| | SEQ ID No.106 | ○ | ○ | ○ | ○ | - | - | ○ | ○ | ○ |
| | SEQ ID No.107 | ○ | ○ | ○ | - | - | - | - | - | - |
| | SEQ ID No.108 | ○ | ○ | ○ | - | - | - | - | - | - |
| | SEQ ID No.109 | ○ | ○ | ○ | ○ | ○ | ○ | ○ | ○ | ○ |
| | SEQ ID No.110 | ○ | ○ | ○ | ○ | ○ | ○ | ○ | ○ | ○ |
| | SEQ ID No.111 | ○ | ○ | ○ | - | - | - | - | - | - |
| | SEQ ID No.112 | ○ | ○ | ○ | - | - | - | - | - | - |
| | SEQ ID No.113 | ○ | - | ○ | - | - | - | - | - | - |
| | SEQ ID No.114 | | | | | | | | | |
| | SEQ ID No.115 | | | | | | | | | |

(Example 4) Verification of primer (Electrophoresis with simulated DNA sample)

In the present example, a solution HE prepared by adding E. coli genomic DNA to 100 μg/ml human genomic DNA (clonetech) to reach a final concentration of 10 pg/ml was used as a template. The instruments and ultrapure water used were free of DNA. With respect to the primer combination, the reaction solution was so added as to give (1) respective combinations of a forward primer consisting of SEQ ID No. 1 and reverse primers consisting of SEQ ID No. 2 to 5, (2) respective combinations of forward primers consisting of SEQ ID No. 1 and 6 to 15 and reverse primers consisting of SEQ ID No. 16 to 27, (3) respective combinations of a forward primer consisting of SEQ ID No. 28 and reverse primers consisting of SEQ ID No. 44 and 45 and 47, a combination of a forward primer consisting of SEQ ID No. 30 and a reverse primer consisting of SEQ ID No. 48, a combination of a forward primer consisting of SEQ ID No. 31 and a reverse primer consisting of SEQ ID No. 47, a combination of a forward primer consisting of SEQ ID No. 32 and a reverse primer consisting of SEQ ID No. 48, respective combinations of forward primers consisting of SEQ ID No. 33 to 43 and reverse primers consisting of SEQ ID No. 44 to 50, (4) respective combinations of a forward primer consisting of SEQ ID No. 53 and reverse primers consisting of SEQ ID No. 57 and 61, respective combinations of a forward primer consisting of SEQ ID No. 54 and reverse primers consisting of SEQ ID No. 59 and 61, respective combinations of a forward primer consisting of SEQ ID No. 55 reverse primers consisting of SEQ ID No. 57 to 59 and 61, a combination of a forward primer consisting of SEQ ID No. 56 and a reverse primer consisting of SEQ ID No. 61, (5) respective combinations of a forward primer consisting of SEQ ID No. 62 and reverse primers consisting of SEQ ID No. 70 and 71 and 73 to 75, respective combinations of forward primers consisting of SEQ ID No. 63 to 69 and reverse primers consisting of SEQ ID No. 70 to 75, (6) respective combinations of forward primers consisting of SEQ ID No. 76 to 90 and reverse primers consisting of SEQ ID No. 91 to 103 and (7) respective combinations of forward primers consisting of SEQ ID No. 104 to 113 and reverse primers consisting of SEQ ID No. 2 and 116 to 131, and PCR thereof was conducted. The reaction was conducted with a formulation shown in Table 4 for the above-described combination (1), and with a formulation shown in Table 5 for the above-described combinations (2) to (7).

For (1) out of the above-described primer combinations, the reaction solution was heated at 95°C for 5 minutes, then, a process including heating at 94°C for 10 seconds, at 65°C for 10 seconds and at 72°C for 30 seconds was repeated 35 times. For (2) to (7) out of the above-described primer combinations, the reaction solution was heated at 95°C for 5 minutes, then, a process including heating at 94°C for 10 seconds, at 60°C for 10 seconds and at 72°C for 10 seconds was repeated 35 times. The solution after the reaction was electrophoresed on 2% agarose gel and stained with ethidium bromide, then, the amplification amount of the intended product and amplification of the non-specific product were verified. Here, a combination of a forward primer consisting of SEQ ID No. 1 and a reverse primer consisting of SEQ ID No. 2, a combination of a forward primer consisting of SEQ ID No. 1 and a reverse primer consisting of SEQ ID No. 16, a combination of a forward primer consisting of SEQ ID No. 28 and a reverse primer consisting of SEQ ID No. 44, a combination of a forward primer consisting of SEQ ID No. 53 and a reverse primer consisting of SEQ ID No. 57, a combination of a forward primer consisting of SEQ ID No. 62 and a reverse primer consisting of SEQ ID No. 70, a combination of a forward primer consisting of SEQ ID No. 76 and a reverse primer consisting of SEQ ID No. 91 and a combination of a forward primer consisting of SEQ ID No. 104 and a reverse primer consisting of SEQ ID No. 2 correspond to replication experiments of Patent document 4. The results are shown in Tables 9-1 to 9-7 together with the result of Comparative Example 4.

Evaluation in Table 9 was carried out according to the following criteria.

Amount of intended product:

◎ : amplification is particularly good
○: amplification is good
Δ: amplification is poor
×: amplification is particularly poor

Non-specific product amplification

○: non-specific product is not found
Δ: non-specific product is found
×: non-specific product is abundant

(Comparative Example 4) Comparative example for Example 4

The following comparative example is a replication experiment of Patent document 4. In the present example, a solution HE prepared by adding E. coli genomic DNA to 100 μg/ml human genomic DNA (clonetech) to reach a final concentration of 10 pg/ml was used as a template. The instruments and ultrapure water used were free of DNA, and the operation was conducted in a clean bench. The reaction solution was so added as to give (1) a combination of a forward primer consisting of SEQ ID No. 1 and a reverse primer consisting of SEQ ID No. 2, (2) a combination of a forward primer consisting of SEQ ID No. 1 and a reverse primer consisting of SEQ ID No. 16, (3) a combination of a forward primer consisting of SEQ ID No. 28 and a reverse primer consisting of SEQ ID No. 44, (4) a combination of a forward primer consisting of SEQ ID No. 53 and a reverse primer consisting of SEQ ID No. 57, (5) a combination of a forward primer consisting of SEQ ID No. 62 and a reverse primer consisting of SEQ ID No. 70, (6) a combination of a forward primer consisting of SEQ ID No. 76 and a reverse primer consisting of SEQ ID No. 91 and (7) a combination of a forward primer consisting of SEQ ID No. 104 and a reverse primer consisting of SEQ ID No. 2, and PCR thereof was conducted. The reaction was conducted with a formulation shown in Table 4 for the above-described combination (1), and with a formulation shown in Table 5 for the above-described combinations (2) to (7).

For (1) out of the above-described primer combinations, the reaction solution was heated at 95°C for 5 minutes, then, a process including heating at 94°C for 10 seconds, at 65°C for 10 seconds and at 72°C for 30 seconds was repeated 35 times. For (2) to (7) out of the above-described primer combinations, the reaction solution was heated at 95°C for 5 minutes, then, a process including heating at 94°C for 10 seconds, at 60°C for 10 seconds and at 72°C for 10 seconds was repeated 35 times. The solution after the reaction was electrophoresed on 2% agarose gel and stained with ethidium bromide, then, the amplification amount of the intended product and amplification of the non-specific product were verified. The results are shown in Tables 9-1 to 9-7 together with the result of Example 4.

[Table 9-1]

Table 9-1

<table>
<tr><td rowspan="7">HE</td><td colspan="5">Electrophoresis : Amount of intended product</td></tr>
<tr><td></td><td>SEQ ID No. 2</td><td>SEQ ID No. 3</td><td>SEQ ID No. 4</td><td>I SEQ ID No. 5</td></tr>
<tr><td>SEQ ID No. 1</td><td>○</td><td>○</td><td>○</td><td>○</td></tr>
<tr><td colspan="5"></td></tr>
<tr><td colspan="5">Electrophoresis : Non specificity</td></tr>
<tr><td></td><td>SEQ ID No. 2</td><td>SEQ ID No. 3</td><td>SEQ ID No. 4</td><td>SEQ ID No. 5</td></tr>
<tr><td>SEQ ID No. 1</td><td>○</td><td>○</td><td>○</td><td>○</td></tr>
</table>

[Table 9-2]

Table 9-2

| | Electrophoresis : Amount of intended product | | | | | | | | | | | | |
|---|---|---|---|---|---|---|---|---|---|---|---|---|---|
| | | SEQ ID No.16 | SEQ ID ' No.17 | SEQ ID No.18 | SEQ ID No.19 | SEQ ID No.20 | SEQ ID No.21 | SEQ ID No.22 | SEQ ID No.23 | SEQ ID No.24 | SEQ ID No.25 | SEQ ID No.26 | SEQ ID No.27 |
| | SEQ ID No.1 | ○ | ○ | ○ | ○ | ○ | ○ | Δ | ○ | ○ | ○ | ○ | Δ |
| | SEQ ID No.6 | ○ | ○ | Δ | ○ | ○ | ○ | ○ | ○ | ○ | ○ | ○ | ○ |
| | SEQ ID No.7 | ○ | ○ | ○ | ○ | ○ | ○ | Δ | ○ | ○ | ○ | ○ | ○ |
| | SEQ ID No.8 | ○ | ○ | ○ | ○ | ○ | ○ | ○ | ○ | ○ | ○ | ○ | Δ |
| | SEQ ID No.9 | Δ | Δ | Δ | Δ | Δ | Δ | × | Δ | × | × | Δ | × |
| | SEQ ID No.10 | × | × | Δ | Δ | Δ | Δ | × | Δ | Δ | Δ | Δ | Δ |
| | SEQ ID No.11 | ○ | ○ | ○ | ○ | ○ | ○ | Δ | ○ | ○ | ○ | ○ | Δ |
| | SEQ ID No.12 | ◎ | ◎ | ◎ | ◎ | ◎ | ◎ | ◎ | ◎ | ◎ | ◎ | ◎ | ◎ |
| | SEQ ID No.13 | ◎ | ◎ | ◎ | ◎ | ◎ | ◎ | ◎ | ◎ | ◎ | ◎ | ◎ | ◎ |
| | SEQ ID No.14 | ○ | ○ | ○ | ○ | ○ | ○ | ○ | ○ | ○ | ○ | ○ | ○ |
| | SEQ ID No.15 | ◎ | ◎ | ◎ | ◎ | ◎ | ◎ | ◎ | ◎ | ◎ | ◎ | ◎ | ◎ |
| HE | | | | | | | | | | | | | |
| | Electrophoresis : Non specificity | | | | | | | | | | | | |
| | | SEQ ID No.16 | SEQ ID No.17 | SEQ ID No.18 | SEQ ID No.19 | SEQ ID No.20 | SEQ ID No.21 | SEQ ID No.22 | SEQ ID No.23 | SEQ ID No.24 | SEQ ID No.25 | SEQ ID No.26 | SEQ ID No.27 |

(continued)

| | | | | | | | | | | | | | |
|---|---|---|---|---|---|---|---|---|---|---|---|---|---|
| | SEQ ID No.1 | ○ | Δ | Δ | ○ | ○ | ○ | Δ | ○ | ○ | ○ | ○ | Δ |
| | SEQ ID No.6 | ○ | ○ | ○ | ○ | ○ | ○ | Δ | ○ | ○ | ○ | ○ | Δ |
| | SEQ ID No.7 | Δ | Δ | Δ | Δ | Δ | ○ | ○ | ○ | ○ | | | Δ |
| | SEQ ID No.8 | Δ | Δ | Δ | Δ | Δ | Δ | Δ | Δ | Δ | Δ | ○ | ○ |
| | SEQ ID No.9 | × | × | × | × | × | × | × | × | × | × | × | × |
| | SEQ ID No.10 | × | × | × | × | × | × | × | × | × | × | × | × |
| | SEQ ID No.11 | Δ | Δ | Δ | Δ | Δ | Δ | × | Δ | Δ | Δ | Δ | × |
| | SEQ ID No.12 | ○ | ○ | ○ | ○ | Δ | ○ | ○ | ○ | ○ | ○ | ○ | × |
| | SEQ ID No.13 | ○ | ○ | ○ | ○ | ○ | ○ | ○ | ○ | ○ | ○ | ○ | × |
| | SEQ ID No.14 | ○ | ○ | ○ | ○ | ○ | ○ | ○ | Δ | ○ | ○ | ○ | Δ |
| | SEQ ID No. 15 | ○ | ○ | ○ | Δ | ○ | ○ | ○ | ○ | ○ | ○ | Δ | Δ |

[Table 9-3]

Table 9-3

| | Electrophoresis : Amount of intended product | | | | | | | |
|---|---|---|---|---|---|---|---|---|
| | | SEQ ID No.44 | SEQ ID No.45 | SEQ ID No.46 | SEQ ID No.47 | SEQ ID No.48 | SEQ ID No.49 | SEQ ID No.50 |
| | SEQ ID No.28 | ○ | Δ | | ○ | | | |
| | SEQ ID No.29 | | | | | | | |
| | SEQ ID No.30 | | | | | ○ | | |
| | SEQ ID No.31 | | | | ○ | | | |
| | SEQ ID No.32 | | × | | | ○ | | |
| | SEQ ID No.33 | × | ○ | ○ | × | Δ | × | ◎ |
| | SEQ ID No.34 | × | × | ○ | Δ | ○ | ○ | ○ |
| | SEQ ID No.35 | Δ | ◎ | ○ | ○ | ○ | ○ | ○ |
| | SEQ ID No.36 | × | × | Δ | × | ○ | Δ | ○ |
| | SEQ ID No.37 | × | Δ | ○ | ○ | ○ | Δ | ○ |
| | SEQ ID No.38 | ○ | × | ○ | ○ | ○ | × | ○ |
| | SEQ ID No.39 | × | × | × | ○ | Δ | Δ | × |
| | SEQ ID No.40 | × | ○ | ○ | × | ○ | Δ | ○ |
| | SEQ ID No.41 | ○ | ○ | ○ | × | ○ | Δ | × |
| | SEQ ID No.42 | × | × | ○ | × | ○ | ○ | Δ |
| | SEQ ID No.43 | ○ | ○ | ○ | ◎ | ◎ | - | ◎ |
| HE | | | | | | | | |
| | Electrophoresis : Non specificity | | | | | | | |
| | | SEQ ID No.44 | SEQ ID No.45 | SEQ ID SEQ ID No.46 No.47 | | SEQ ID No.48 | SEQ ID No.49 | SEQ ID No.50 |
| | SEQ ID No.28 | ○ | × | | Δ | | | |

(continued)

| | | | | | | | | |
|---|---|---|---|---|---|---|---|---|
| | SEQ ID No.29 | | | | | | | |
| | SEQ ID No.30 | | | | | Δ | | |
| | SEQ ID No.31 | | | | × | | | |
| | SEQ ID No.32 | | × | | | Δ | | |
| | SEQ ID No.33 | × | ○ | Δ | × | ○ | × | Δ |
| | SEQ ID No.34 | × | × | Δ | × | ○ | ○ | ○ |
| | SEQ ID No.35 | × | ○ | × | × | ○ | ○ | ○ |
| | SEQ ID No.36 | × | × | × | × | × | × | ○ |
| | SEQ ID No.37 | × | × | ○ | ○ | ○ | × | ○ |
| | SEQ ID No.38 | Δ | × | ○ | ○ | ○ | × | ○ |
| | SEQ ID No.39 | × | × | × | ○ | × | × | × |
| | SEQ ID No.40 | × | Δ | ○ | × | Δ | × | × |
| | SEQ ID No.41 | ○ | ○ | ○ | × | ○ | × | × |
| | SEQ ID No.42 | × | × | × | × | Δ | Δ | × |
| | SEQ ID No.43 | ○ | × | ○ | ○ | ○ | - | ○ |

[Table 9-4]

Table 9-4

| | | | | | | | |
|---|---|---|---|---|---|---|---|
| HE | Electrophoresis : Amount of intended product | | | | | | |
| | | SEQ ID No. 57 | SEQ ID No. 58 | SEQ ID No. 59 | SEQ ID No. 60 | SEQ ID No. 61 | |
| | SEQ ID No. 53 | × | | | | ◎ | |
| | SEQ ID No. 54 | | | × | | ◎ | |
| | SEQ ID No. 55 | ◎ | ◎ | ◎ | | ◎ | |
| | SEQ ID No. 56 | | | | | × | |
| | | | | | | | |
| | Electrophoresis : Non specificity | | | | | | |
| | | SEQ ID No. 57 | SEQ ID No. 58 | SEQ ID No. 59 | SEQ ID . No. 60 | SEQ ID No. 61 | |
| | SEQ ID No. 53 | × | | | | ○ | |
| | SEQ ID No. 54 | | | × | | ○ | |
| | SEQ ID No. 55 | ○ | ○ | ○ | | ○ | |
| | SEQ ID No. 56 | | | | | × | |
| | | | | | | | |
| HE | Electrophoresis : Amount of intended product | | | | | | |
| | | SEQ ID No. 70 | SEQ ID No. 71 | SEQ ID No. 72 | SEQ ID No. 73 | SEQ ID No. 74 | SEQ ID No. 75 |
| | SEQ ID No. 62 | ○ | Δ | - | ○ | ○ | ○ |
| | SEQ ID No. 63 | ○ | ○ | ○ | ○ | ○ | ○ |
| | SEQ ID No. 64 | Δ | × | Δ | × | Δ | Δ |
| | SEQ ID No. 65 | ○ | ○ | ○ | ○ | ○ | ○ |
| | SEQ ID No. 66 | ○ | ○ | ○ | ○ | ○ | ○ |
| | SEQ ID No. 67 | ○ | ○ | ○ | ○ | ○ | ○ |
| | SEQ ID No. 68 | ○ | ○ | ○ | ○ | ○ | ○ |
| | SEQ ID No. 69 | ○ | Δ | ○ | ○ | ○ | ○ |
| | | | | | | | |
| | Electrophoresis : Non specificity | | | | | | |

(continued)

| | | SEQ ID No. 70 | SEQ ID No. 71 | SEQ ID No. 72 | SEQ ID No. 73 | SEQ ID No. 74 | SEQ ID No. 75 |
|---|---|---|---|---|---|---|---|
| | SEQ ID No. 62 | ○ | ○ | - | ○ | ○ | ○ |
| | SEQ ID No. 63 | Δ | ○ | ○ | Δ | ○ | Δ |
| | SEQ ID No. 64 | Δ | × | × | × | ○ | × |
| | SEQ ID No. 65 | Δ | ○ | ○ | Δ | ○ | ○ |
| | SEQ ID No. 66 | ○ | ○ | ○ | ○ | ○ | ○ |
| | SEQ ID No. 67 | ○ | ○ | ○ | ○ | ○ | ○ |
| | SEQ ID No. 68 | ○ | ○ | ○ | ○ | ○ | ○ |
| | SEQ ID No. 69 | ○ | Δ | ○ | ○ | ○ | ○ |

[Table 9-5]

Table 9-5

| Electrophoresis : Amount of intended product | | | | | | | | | | | | | |
|---|---|---|---|---|---|---|---|---|---|---|---|---|---|
| | SEQ ID No.91 | SEQ ID No.92 | SEQ ID No.93 | SEQ ID No.94 | SEQ ID No.95 | SEQ ID No.96 | SEQ ID No.97 | SEQ ID No.98 | SEQ ID No.99 | SEQ ID SEQ ID SEQ ID No.100 No. 101 No.102 | | | ID No. 103 |
| SEQ ID No.76 | × | × | × | × | × | ○ | × | × | × | × | × | × | × |
| SEQ ID No.77 | ○ | × | × | × | × | × | × | × | × | × | ○ | ○ | ○ |
| SEQ ID No.78 | ○ | × | × | × | × | × | × | × | × | × | ○ | ○ | ○ |
| SEQ ID No.79 | ○ | × | × | × | × | × | × | × | × | × | ○ | ○ | ○ |
| SEQ ID No.80 | ○ | × | × | × | × | × | × | × | × | ○ | ○ | ◎ | ◎ |
| SEQ ID No.81 | ◎ | × | Δ | × | × | × | × | × | × | × | ◎ | ◎ | ◎ |
| SEQ ID No.82 | ○ | × | × | × | × | ◎ | ○ | × | × | × | ◎ | ◎ | ◎ |
| SEQ ID No.83 | ○ | × | × | × | × | × | × | × | × | × | ◎ | ◎ | ○ |
| SEQ ID No.84 | Δ | × | × | × | × | × | × | × | × | × | ◎ | ◎ | ◎ |
| SEQ ID No.85 | × | × | × | × | × | × | × | × | × | × | ◎ | ◎ | ◎ |
| SEQ ID No-86 | ◎ | × | Δ | × | × | × | × | × | × | × | ◎ | ◎ | ◎ |
| SEQ ID No.87 | ◎ | × | × | × | × | × | × | × | × | × | ◎ | ◎ | ◎ |
| SEQ ID No.88 | ○ | × | × | × | × | × | × | × | × | × | ◎ | Δ | Δ |

(continued)

| | | | | | | | | | | | | | | |
|---|---|---|---|---|---|---|---|---|---|---|---|---|---|---|
| HE | SEQ ID No.89 | ○ | × | × | × | × | × | × | × | × | × | ◎ | ◎ | ◎ |
| | SEQ ID No.90 | ○ | × | × | × | × | × | × | × | × | × | ◎ | ◎ | ◎ |
| | | | | | | | | | | | | | | |
| | Electrophoresis : Non specificity | | | | | | | | | | | | | |
| | | SEQ ID No.91 | SEQ ID No.92 | SEQ ID No.93 | SEQ ID No.94 | SEQ ID No.95 | SEQ ID No.96 | SEQ ID No.97 | SEQ ID No.98 | SEQ ID No.99 | SEQ ID No.100 | SEQ ID No.101 | SEQ ID No.102 | SEQ ID No.103 |
| | SEQ ID No.76 | × | × | × | × | × | ○ | × | × | × | × | × | × | × |
| | SEQ ID No.77 | Δ | × | × | × | × | × | × | × | × | × | ○ | ○ | ○ |
| | SEQ ID No.78 | Δ | × | × | × | × | × | × | × | × | × | ○ | Δ | ○ |
| | SEQ ID No.79 | Δ | × | × | × | × | × | × | × | × | × | ○ | ○ | ○ |
| | SEQ ID No.80 | Δ | × | Δ | × | × | × | × | × | × | Δ | Δ | Δ | Δ |
| | SEQ ID No.81 | Δ | × | × | × | × | × | × | × | × | × | Δ | Δ | Δ |
| | SEQ ID No.82 | × | × | × | × | × | Δ | Δ | × | × | × | Δ | Δ | Δ |
| | SEQ ID No.83 | Δ | × | × | × | × | × | × | × | × | × | Δ | Δ | ○ |
| | SEQ ID No.84 | × | × | × | × | × | × | × | × | × | × | Δ | ○ | Δ |
| | SEQ ID No.85 | × | × | × | × | × | × | × | × | × | × | ○ | ○ | ○ |
| | SEQ ID No.86 | Δ | × | × | × | × | × | × | × | × | × | Δ | Δ | Δ |

(continued)

| | | | | | | | | | | | | | | |
|---|---|---|---|---|---|---|---|---|---|---|---|---|---|---|
| | SEQ ID No.87 | × | × | × | × | × | × | × | × | × | × | ○ | Δ | ○ |
| | SEQ ID No.88 | Δ | × | × | × | × | × | × | × | × | × | ○ | ○ | × |
| | SEQ ID No.89 | Δ | × | × | × | × | × | × | × | × | × | ○ | ○ | ○ |
| | SEQ ID No.90 | × | × | × | × | x | × | × | × | × | × | ○ | ○ | ○ |

[Table 9-6]

Table 9-6

| | Electrophoresis : Amount of intended product | | | | | | | | | |
|---|---|---|---|---|---|---|---|---|---|---|
| | | SEQ ID No.2 | SEQ ID No.116 | SEQ ID No.117 | SEQ ID No.118 | SEQ ID No.119 | SEQ ID No.120 | SEQ ID No.121 | SEQ ID No.122 | SEQ ID No.123 |
| HE | SEQ ID No.104 | ○ | × | ○ | ○ | ○ | ○ | ○ | ○ | ○ |
| | SEQ ID No.105 | ○ | ○ | ○ | ○ | ○ | ○ | ○ | ○ | ○ |
| | SEQ ID No.106 | △ | △ | △ | △ | △ | △ | △ | △ | ○ |
| | SEQ ID No.107 | ○ | ○ | ○ | ○ | ○ | ○ | ○ | ○ | ○ |
| | SEQ ID No.108 | ○ | ○ | △ | ○ | ○ | △ | △ | △ | ○ |
| | SEQ ID No.109 | ○ | ○ | ○ | ○ | ○ | ○ | ○ | ○ | ○ |
| | SEQ ID No.110 | ○ | ○ | ○ | ○ | ○ | ○ | ○ | ○ | ○ |
| | SEQ ID No.111 | ○ | ○ | ○ | ○ | ○ | ○ | ○ | ○ | ○ |
| | SEQ ID No.112 | ○ | ○ | ○ | ○ | ○ | ○ | ○ | ○ | ○ |
| | SEQ ID No.113 | ○ | ○ | △ | ○ | △ | △ | △ | △ | △ |
| | SEQ ID No.114 | | | | | | | | | |
| | SEQ ID No.115 | ○ | | | | | | | | |

| | Electrophoresis : Amount of intended product | | | | | | | | | |
|---|---|---|---|---|---|---|---|---|---|---|
| | | SEQ ID No.124 | SEQ ID No.125 | SEQ ID No.5 | SEQ ID No.126 | SEQ ID No.127 | SEQ ID No.128 | SEQ ID No.129 | SEQ ID No.130 | SEQ ID No.131 |
| HE | SEQ ID No.104 | ○ | ○ | ○ | ○ | ○ | ○ | ○ | ○ | ○ |
| | SEQ ID No.105 | ○ | ○ | ○ | ○ | ○ | ○ | ○ | ○ | ○ |
| | SEQ ID No.106 | ○ | ○ | ○ | ○ | ○ | ○ | ○ | ○ | ○ |
| | SEQ ID No.107 | ○ | ○ | ○ | ○ | ○ | ○ | ○ | ○ | ○ |
| | SEQ ID No.108 | △ | ○ | ○ | ○ | ○ | ○ | ○ | ○ | ○ |
| | SEQ ID No.109 | ○ | ○ | ○ | ○ | ○ | ○ | ○ | ○ | ○ |
| | SEQ ID No.110 | ○ | ○ | ○ | ○ | ○ | ○ | ○ | ○ | × |
| | SEQ ID No.111 | ○ | ○ | ○ | ○ | ○ | ○ | ○ | ○ | ○ |
| | SEQ ID No.112 | ○ | ○ | ○ | ○ | ○ | ○ | ○ | ○ | ○ |
| | SEQ ID No.113 | △ | ○ | ○ | ○ | ○ | ○ | ○ | ○ | ○ |
| | SEQ ID No.114 | | ○ | | | | | | | |
| | SEQ ID No.115 | | | ○ | | | | | | |

[Table 9-7]

Table 9-7

| Electrophoresis | | Non specific product SEQ ID No.2 | SEQ ID No.116 | SEQ ID No.117 | SEQ ID No.118 | SEQ ID No.119 | SEQ ID No.120 | SEQ ID No.121 | SEQ ID No.122 | SEQ ID No.123 |
|---|---|---|---|---|---|---|---|---|---|---|
| HE | SEQ ID No.104 | ○ | × | ○ | ○ | ○ | △ | ○ | ○ | ○ |
| | SEQ ID No.105 | ○ | △ | ○ | ○ | ○ | △ | △ | △ | △ |
| | SEQ ID No.106 | ○ | × | × | ○ | ○ | ○ | ○ | ○ | △ |
| | SEQ ID No.107 | ○ | ○ | ○ | ○ | ○ | △ | △ | △ | ○ |
| | SEQ ID No.108 | ○ | ○ | ○ | ○ | ○ | △ | ○ | ○ | ○ |
| | SEQ ID No.109 | ○ | ○ | × | × | × | × | ○ | ○ | ○ |
| | SEQ ID No.110 | ○ | × | × | × | × | × | ○ | × | ○ |
| | SEQ ID No.111 | ○ | × | × | × | × | × | × | × | ○ |
| | SEQ ID No.112 | ○ | × | × | × | × | × | × | ○ | ○ |
| | SEQ ID No.113 | ○ | × | × | ○ | × | × | × | × | × |
| | SEQ ID No.114 | | | | | | | | | |
| | SEQ ID No.115 | △ | | | | | | | | |

| Electrophoresis | | Non specific product SEQ ID No.124 | SEQ ID No.125 | SEQ ID No.5 | SEQ ID No.126 | SEQ ID No.127 | SEQ ID No.128 | SEQ ID No.129 | SEQ ID No.130 | SEQ ID No.131 |
|---|---|---|---|---|---|---|---|---|---|---|
| HE | SEQ ID No.104 | ○ | ○ | ○ | ○ | ○ | ○ | ○ | ○ | ○ |
| | SEQ ID No.105 | ○ | ○ | ○ | ○ | ○ | ○ | ○ | ○ | ○ |
| | SEQ ID No.106 | △ | ○ | ○ | ○ | ○ | ○ | ○ | ○ | △ |
| | SEQ ID No.107 | △ | ○ | ○ | ○ | ○ | ○ | ○ | ○ | ○ |
| | SEQ ID No.108 | ○ | ○ | ○ | ○ | ○ | ○ | △ | △ | ○ |
| | SEQ ID No.109 | ○ | ○ | ○ | ○ | ○ | ○ | ○ | ○ | ○ |
| | SEQ ID No.110 | ○ | ○ | ○ | ○ | ○ | ○ | ○ | ○ | × |
| | SEQ ID No.111 | × | ○ | ○ | △ | △ | △ | △ | △ | △ |
| | SEQ ID No.112 | ○ | ○ | ○ | △ | △ | △ | △ | △ | △ |
| | SEQ ID No.113 | × | ○ | ○ | △ | △ | △ | △ | △ | △ |
| | SEQ ID No.114 | | ○ | | | | | | | |
| | SEQ ID No.115 | | | ○ | | | | | | |

(Example 5) Verification of primer (PCR with simulated DNA sample and melting thereof)

In the present example, a solution HE prepared by adding E. coli genomic DNA to 100 μg /ml human genomic DNA (clonetech) to reach a final concentration of 10 pg/ml was used as a template. The instruments and ultrapure water used were free of DNA. With respect to the primer combination, the reaction solution was so added as to give (1) respective combinations of a forward primer consisting of SEQ ID No. 1 and reverse primers consisting of SEQ ID No. 2 to 5, (2) respective combinations of forward primers consisting of SEQ ID No. 1 and 6 to 15 and reverse primers consisting of SEQ ID No. 16 to 27, (3) respective combinations of a forward primer consisting of SEQ ID No. 28 and reverse primers consisting of SEQ ID No. 44 and 45 and 47, a combination of a forward primer consisting of SEQ ID No. 30 and a reverse primer consisting of SEQ ID No. 48, a combination of a forward primer consisting of SEQ ID No. 31 and a reverse primer consisting of SEQ ID No. 47, a combination of a forward primer consisting of SEQ ID No. 32 and a reverse primer consisting of SEQ ID No. 48, respective combinations of forward primers consisting of SEQ ID No. 33 to 43 and reverse primers consisting of SEQ ID No. 44 to 50, (4) respective combinations of a forward primer consisting of SEQ ID No. 53 and reverse primers consisting of SEQ ID No. 57 and 61, respective combinations of a forward primer consisting of SEQ ID No. 54 and reverse primers consisting of SEQ ID No. 59 and 61, respective combinations of a forward primer consisting of SEQ ID No. 55 and reverse primers consisting of SEQ ID No. 57 to 59 and 61, a combination of a forward primer consisting of SEQ ID No. 56 and a reverse primer consisting of SEQ ID No. 61, (5) respective combinations of a forward primer consisting of SEQ ID No. 62 and reverse primers consisting of SEQ ID No. 70 and 71 and 73 to 75, respective combinations of forward primers consisting of SEQ ID No. 63 to 69 and reverse primers consisting of SEQ ID No. 70 to 75, (6) respective combinations of forward primers consisting of SEQ ID No. 76 to 90 and reverse primers consisting of SEQ ID No. 91 to 103, (7) respective combinations of forward primers consisting of SEQ ID No. 104 to 113 and reverse primers consisting of SEQ ID No. 2 and 116 to 131, a combination of a forward primer consisting of SEQ ID No. 114 and a reverse primer consisting of SEQ ID No. 5 and respective combinations of a forward primer consisting of SEQ ID No. 115 and reverse primers consisting of SEQ ID No. 2 and 5, and PCR thereof was conducted. The reaction was conducted with a formulation shown in Table 4 for the above-described combination (1), and with a formulation shown in Table 5 for the above-described combinations (2) to (7).

Rotor-Gene Q MDx 5plex HRM (QIAGEN) was used as a real time PCR apparatus, and in the PCR program thereof, for (1) out of the above-described primer combinations, the reaction solution was heated at 95°C for 5 minutes, then, a process including heating at 94°C for 10 seconds, at 65°C for 10 seconds and at 72°C for 30 seconds was repeated 35 times, and for (2) to (7), the reaction solution was heated at 95°C for 5 minutes, then, a process including heating at 94°C for 10 seconds, at 60°C for 10 seconds and at 72°C for 10 seconds was repeated 35 times, then, the DNA dissociation curve of the amplification product was made. The reaction solution was heated at 95°C for 10 seconds, then, incubated at 72°C for 90 seconds, and heated up to 95°C by 0.5°C stepwise, in order to make the DNA dissociation curve. At each stage, the reaction solution was incubated for 2 seconds, and the data were obtained. The threshold cycle of the amplification curve and the dissociation curve were verified. Here, a combination of a forward primer consisting of SEQ ID No. 1 and a reverse primer consisting of SEQ ID No. 2, a combination of a forward primer consisting of SEQ ID No. 1 and a reverse primer consisting of SEQ ID No. 16, a combination of a forward primer consisting of SEQ ID No. 28 and a reverse primer consisting of SEQ ID No. 44, a combination of a forward primer consisting of SEQ ID No. 53 and a reverse primer consisting of SEQ ID No. 57, a combination of a forward primer consisting of SEQ ID No. 62 and a reverse primer consisting of SEQ ID No. 70, a combination of a forward primer consisting of SEQ ID No. 76 and a reverse primer consisting of SEQ ID No. 91 and a combination of a forward primer consisting of SEQ ID No. 104 and a reverse primer consisting of SEQ ID No. 2 correspond to replication experiments of Patent document 4. The results are shown in Tables 10-1 to 10-7 together with the result of Comparative Example 5.

Evaluation in Table 10 was carried out according to the following criteria.

Threshold cycle:

◎ : threshold cycle is fast (decreased by 2 cycles or more)
○: threshold cycle is somewhat fast (decreased by 0-1 cycle)
Δ: threshold cycle is somewhat slow (increased by 1-2 cycles)
×: threshold cycle is slow (increased by 3 cycles or more)

Melting analysis:

○: only one peak
Δ: small peaks
×: several peaks
-: no peak

(Comparative Example 5) Comparative example for Example 5

The following comparative example is a replication experiment of Patent document 4. In the present example, a solution HE prepared by adding E. coli genomic DNA to 100 μg /ml human genomic DNA (clonetech) to reach a final concentration of 10 pg/ml was used as a template. The instruments and ultrapure water used were free of DNA. The reaction solution was so added as to give (1) a combination of a forward primer consisting of SEQ ID No. 1 and a reverse primer consisting of SEQ ID No. 2, (2) a combination of a forward primer consisting of SEQ ID No. 1 and a reverse primer consisting of SEQ ID No. 16, (3) a combination of a forward primer consisting of SEQ ID No. 28 and a reverse primer consisting of SEQ ID No. 44, (4) a combination of a forward primer consisting of SEQ ID No. 53 and a reverse primer consisting of SEQ ID No. 57, (5) a combination of a forward primer consisting of SEQ ID No. 62 and a reverse primer consisting of SEQ ID No. 70, (6) a combination of a forward primer consisting of SEQ ID No. 76 and a reverse primer consisting of SEQ ID No. 91 and (7) a combination of a forward primer consisting of SEQ ID No. 104 and a reverse primer consisting of SEQ ID No. 2, and PCR thereof was conducted. The reaction was conducted with a formulation shown in Table 4 for the above-described combination (1), and with a formulation shown in Table 5 for the above-described combinations (2) to (7).

Rotor-Gene Q MDx 5plex HRM (QIAGEN) was used as a real time PCR apparatus, and in the PCR program thereof, for (1) out of the above-described primer combinations, the reaction solution was heated at 95°C for 5 minutes, then, a process including heating at 94°C for 10 seconds, at 65°C for 10 seconds and at 72°C for 30 seconds was repeated 35 times, and for (2) to (7), the reaction solution was heated at 95°C for 5 minutes, then, a process including heating at 94°C for 10 seconds, at 60°C for 10 seconds and at 72°C for 10 seconds was repeated 35 times, then, the DNA dissociation curve of the amplification product was made. The reaction solution was heated at 95°C for 10 seconds, then, incubated at 72°C for 90 seconds, and heated up to 95°C by 0.5°C stepwise, in order to make the DNA dissociation curve. At each stage, the reaction solution was incubated for 2 seconds, and the data were obtained. The threshold cycle of the amplification curve and the dissociation curve were verified. The results are shown in Tables 10-1 to 10-7 together with the result of Example 5.

[Table 10-1]

Table 10-1

| | | | | | |
|---|---|---|---|---|---|
| HE | PCR: Melting analysis | | | | |
| | | SEQ ID No. 2 | SEQ ID No. 3 | SEQ ID No. 4 | SEQ ID No. 5 |
| | SEQ ID No. 1 | ○ | ◎ | ◎ | ○ |
| | | | | | |
| | Electrophoresis : Non specificity | | | | |
| | | SEQ ID No. 2 | SEQ ID No. 3 | SEQ ID No. 4 | SEQ ID No. 5 |
| | SEQ ID No. 1 | ○ | ○ | ○ | ○ |

[Table 10-2]

Table 10-2

| | PCR:Threshold cycle | | | | | | | | | | | | |
|---|---|---|---|---|---|---|---|---|---|---|---|---|---|
| | ○ | SEQ ID No. 16 | SEQ ID No.17 | SEQ ID No. 18 | SEQ ID No.19 | SEQ ID No.20 | SEQ ID No.21 | SEQ ID No.22 | SEQ ID No.23 | SEQ ID' No.24 | SEQ ID No.25 | SEQ ID No.26 | SEQ ID No.27 |
| | SEQ ID No.1 | ○ | ○ | ○ | ○ | ○ | ○ | × | Δ | Δ | Δ | ○ | ◎ |
| | SEQ ID No.6 | ○ | ○ | ○ | ○ | ○ | ○ | - | Δ | Δ | Δ | ◎ | Δ |
| | SEQ ID No.7 | ○ | ○ | ○ | ○ | ○ | ○ | × | ○ | ○ | Δ | ○ | ◎ |
| | SEQ ID No.8 | ○ | ○ | ○ | ○ | ○ | ○ | - | ○ | Δ | Δ | ○ | ◎ |
| | SEQ ID No.9 | - | - | - | - | - | - | - | - | - | - | - | - |
| | SEQ ID No.10 | - | - | - | - | - | - | - | - | - | × | - | - |
| | SEQ ID No. 11 | × | - | - | × | × | × | - | × | × | - | × | - |
| | SEQ ID No.12 | Δ | Δ | Δ | Δ | Δ | Δ | × | Δ | Δ | × | Δ | ○ |
| | SEQ ID No.13 | ○ | ○ | ○ | Δ | ○ | ○ | × | ○ | Δ | Δ | ○ | ○ |
| | SEQ ID No. 14 | ○ | ○ | ○ | ○ | ○ | ○ | × | Δ | Δ | Δ | Δ | Δ |
| | SEQ ID No.15 | ◎ | ◎ | ○ | ○ | ○ | ○ | × | ○ | ○ | ○ | ○ | ◎ |
| HE | | | | | | | | | | | | | |
| | PCR: Melting analysis | | | | | | | | | | | | |
| | ○ | SEQ ID No.16 | SEQ ID No.17 | SEQ ID No.18 | SEQ ID No.19 | SEQ ID No.20 | SEQ ID No.21 | SEQ ID No.22 | SEQ ID No.23 | SEQ ID No.24 | SEQ ID No.25 | SEQ ID No.26 | SEQ ID No.27 |

(continued)

| | | | | | | | | | | | | | |
|---|---|---|---|---|---|---|---|---|---|---|---|---|---|
| | SEQ ID No.1 | ○ | ○ | ○ | ○ | ○ | ○ | ○ | ○ | ○ | ○ | ○ | ○ |
| | SEQ ID No.6 | | | | | | | - | ○ | ○ | ○ | ○ | ○ |
| | SEQ ID No.7 | ○ | ○ | ○ | ○ | ○ | ○ | ○ | ○ | ○ | ○ | ○ | ○ |
| | SEQ ID No.8 | ○ | Δ | ○ | ○ | ○ | ○ | - | ○ | ○ | ○ | ○ | Δ |
| | SEQ ID No.9 | - | - | - | - | - | - | - | - | - | - | - | - |
| | SEQ ID No.10 | - | - | - | - | - | - | - | - | - | Δ | - | - |
| | SEQ ID No.11 | × | - | - | ○ | ○ | Δ | - | ○ | ○ | - | Δ | - |
| | SEQ ID No.12 | ○ | ○ | ○ | ○ | ○ | ○ | ○ | ○ | ○ | ○ | ○ | Δ |
| | SEQ ID No.13 | ○ | ○ | ○ | ○ | ○ | ○ | ○ | ○ | ○ | ○ | ○ | ○ |
| | SEQ ID No.14 | ○ | ○ | ○ | ○ | ○ | ○ | ○ | ○ | ○ | ○ | ○ | ○ |
| | SEQ ID No.15 | ○ | ○ | ○ | ○ | ○ | ○ | ○ | ○ | ○ | ○ | ○ | ○ |

[Table 10-3]

Table 10-3

| | | | | | | | | |
|---|---|---|---|---|---|---|---|---|
| | PCR:Threshold cycle | | | | | | | |
| | ○ | SEQ ID No.44 | SEQ ID No.45 | SEQ ID No.46 | SEQ ID No.47 | SEQ ID No.48 | SEQ ID No.49 | SEQ ID No.50 |
| | SEQ ID No.28 | ○ | Δ | | ○ | | | |
| | SEQ ID No.29 | | | | | | | |
| | SEQ ID No.30 | | | | | ○ | | |
| | SEQ ID No.31 | | | | ◎ | | | |
| | SEQ ID No.32 | | ◎ | | | Δ | | |
| | SEQ ID No.33 | ◎ | - | - | - | - | - | ◎ |
| | SEQ ID No.34 | - | - | ○ | - | ○ | - | ○ |
| | SEQ ID No.35 | ◎ | - | - | - | ○ | - | ○ |
| | SEQ ID No.36 | ◎ | ○ | ○ | - | - | - | ○ |
| | SEQ ID No.37 | - | - | Δ | ○ | Δ | - | Δ |
| | SEQ ID No.38 | ◎ | - | Δ | Δ | ○ | - | ○ |
| | SEQ ID No.39 | - | - | × | × | × | - | Δ |
| | SEQ ID No.40 | - | - | - | - | ○ | - | Δ |
| | SEQ ID No.41 | ○ | ○ | ○ | ○ | ○ | - | Δ |
| | SEQ ID No.42 | ○ | ○ | Δ | Δ | ○ | - | Δ |
| | SEQ ID No.43 | ◎ | ○ | ◎ | Δ | Δ | | Δ |
| HE | | | | | | | | |
| | PCR: Melting analysis | | | | | | | |
| | ○ | SEQ ID No.44 | SEQ ID No.45 | SEQ ID No.46 | SEQ ID No.47 | SEQ ID No.48 | SEQ ID No.49 | SEQ ID No.50 |
| | SEQ 10 No.28 | ○ | Δ | | ○ | | | |

(continued)

| | | | | | | | | |
|---|---|---|---|---|---|---|---|---|
| | SEQ ID No.29 | | | | | | | |
| | SEQ ID No.30 | | | | | ○ | | |
| | SEQ ID No.31 | | | | × | | | |
| | SEQ ID No.32 | | × | | | ○ | | |
| | SEQ ID No.33 | × | - | - | - | - | - | ○ |
| | SEQ ID No.34 | - | - | ○ | - | Δ | - | ○ |
| | SEQ ID No.35 | × | - | - | - | × | - | ○ |
| | SEQ ID No.36 | × | × | ○ | - | - | - | ○ |
| | SEQ ID No.37 | - | - | ○ | × | Δ | - | ○ |
| | SEQ ID No.38 | ○ | - | Δ | Δ | ○ | - | ○ |
| | SEQ ID No.39 | - | - | Δ | × | Δ | - | ○ |
| | SEQ ID No.40 | - | - | - | - | ○ | - | Δ |
| | SEQ ID No.41 | ○ | × | ○ | × | ○ | - | ○ |
| | SEQ ID No.42 | ○ | × | ○ | Δ | × | - | ○ |
| | SEQ ID No.43 | Δ | ○ | Δ | ○ | ○ | | ○ |

[Table 10-4]

Table 10-4

| | PCR:Threshold cycle | | | | | | |
|---|---|---|---|---|---|---|---|
| | - | SEQ ID No.57 | SEQ ID No.58 | SEQ ID No.59 | SEQ ID No.60 | SEQ ID No.61 | |
| | SEQ ID No. 53 | - | | | | ◎ | |
| | SEQ ID No. 54 | | | - | | ◎ | |
| | SEQ ID No. 55 | ◎ | ◎ | ◎ | | ◎ | |
| | SEQ ID No. 56 | | | | | - | |

(continued)

| | | | | | | | |
|---|---|---|---|---|---|---|---|
| HE | | | | | | | |
| | PCR: Melting analysis | | | | | | |
| | - | SEQ ID No.57 | SEQ ID No.58 | SEQ ID No.59 | SEQ ID No.60 | SEQ ID No.61 | |
| | SEQ ID No. 53 | - | | | | ○ | |
| | SEQ ID No. 54 | | | - | | ○ | |
| | SEQ ID No. 55 | ○ | ○ | ○ | | ○ | |
| | SEQ ID No. 56 | | | | | - | |
| | | | | | | | |
| | PCR:Threshold cycle | | | | | | |
| | ○ | SEQ ID No.70 | SEQ ID No. 71 | SEQ ID No.72 | SEQ ID No.73 | SEQ ID No.74 | SEQ ID No.75 |
| | SEQ ID No. 62 | ○ | Δ | | ○ | ◎ | ○ |
| | SEQ ID No. 63 | ◎ | ◎ | ◎ | ◎ | ◎ | ○ |
| | SEQ ID No. 64 | ◎ | ◎ | ◎ | ◎ | ◎ | ○ |
| | SEQ ID No. 65 | ◎ | ○ | ◎ | ◎ | ◎ | ◎ |
| | SEQ ID No. 66 | ◎ | ○ | ◎ | ◎ | ○ | ○ |
| | SEQ ID No. 67 | ◎ | ○ | ○ | ◎ | ◎ | ◎ |
| | SEQ ID No. 68 | ○ | ○ | ○ | ○ | ○ | ◎ |
| | SEQ ID No. 69 | ◎ | ◎ | ○ | ◎ | ○ | ○ |
| HE | | | | | | | |
| | PCR: Melting analysis | | | | | | |
| | ○ | SEQ ID No. 70 | SEQ ID No.71 | SEQ ID No.72 | SEQ ID No.73 | SEQ ID No.74 | SEQ ID No.75 |
| | SEQ ID No. 62 | ○ | ○ | | ○ | ○ | ○ |
| | SEQ ID No. 63 | Δ | ○ | ○ | Δ | ○ | ○ |
| | SEQ ID No. 64 | × | ○ | × | Δ | Δ | × |
| | SEQ ID No. 65 | ○ | ○ | ○ | ○ | ○ | ○ |
| | SEQ ID No. 66 | ○ | ○ | ○ | ○ | ○ | ○ |

(continued)

| | | | | | | | |
|---|---|---|---|---|---|---|---|
| | SEQ ID No. 67 | ○ | ○ | ○ | ○ | ○ | ○ |
| | SEQ ID No. 68 | ○ | ○ | ○ | ○ | ○ | ○ |
| | SEQ ID No. 69 | ○ | ○ | ○ | ○ | ○ | ○ |

[Table 10-5]

Table 10-5

| O | PCR;Threshold cycle | | | | | | | | | | | | |
|---|---|---|---|---|---|---|---|---|---|---|---|---|---|
| | SEQ ID No.91 | SEQ ID No.92 | SEQ ID No.93 | SEQ ID No.94 | SEQ ID No.95 | SEQ ID No.96 | SEQ ID No.97 | SEQ ID No.98 | SEQ ID No.99 | SEQ ID No.100 | SEQ ID No.101 | SEQ ID No.102 | SEQ ID No 103 |
| SEQ ID No. 76 | - | - | △ | - | - | × | × | - | - | - | - | - | - |
| SEQ ID No. 77 | △ | × | × | - | - | × | × | × | × | × | ○ | △ | △ |
| SEQ ID No. 78 | × | - | × | - | - | - | - | × | × | - | △ | △ | △ |
| SEQ ID No. 79 | - | - | - | - | - | - | - | - | - | - | △ | △ | △ |
| SEQ ID No. 80 | △ | - | ○ | - | - | - | - | - | - | - | ○ | ○ | ○ |
| SEQ ID No. 81 | × | - | - | - | - | - | - | - | - | - | ○ | ○ | ○ |
| SEQ ID No. 82 | × | - | - | - | - | - | - | - | - | - | × | △ | × |
| SEQ ID No. 83 | - | - | - | - | - | - | - | - | - | - | △ | △ | △ |
| SEQ ID No. 84 | - | - | - | - | - | - | - | - | - | - | ○ | ○ | ○ |
| SEQ ID No. 85 | - | - | - | - | - | - | - | - | - | - | △ | △ | △ |
| SEQ ID No. 86 | △ | - | - | - | - | - | - | - | - | - | △ | △ | △ |
| SEQ ID No- 87 | △ | - | - | - | - | × | - | - | - | - | ○ | ○ | △ |
| SEQ ID No. 88 | △ | - | - | - | - | - | - | - | - | - | △ | ○ | - |

(continued)

| | | | | | | | | | | | | | | |
|---|---|---|---|---|---|---|---|---|---|---|---|---|---|---|
| HE | SEQ ID No. 89 | Δ | - | - | - | - | - | - | - | - | - | Δ | Δ | Δ |
| | SEQ ID No. 90 | × | × | - | - | - | × | - | × | × | × | × | × | × |
| | | | | | | | | | | | | | | |
| | P CR: Melting analysis | | | | | | | | | | | | | |
| | ○ | SEQ ID No.91 | SEQ ID No.92 | SEQ ID No.93 | SEQ ID No.94 | SEQ ID No.95 | SEQ ID No.96 | SEQ ID No.97 | SEQ ID No.98 | SEQ ID No.99 | SEQ ID No.100 | SEQ ID No.101 | SEQ ID No.102 | SEQ ID No.103 |
| | SEQ ID No. 76 | - | - | × | - | - | ○ | × | - | - | - | - | - | - |
| | SEQ ID No. 77 | ○ | × | × | - | - | Δ | × | × | × | × | ○ | ○ | ○ |
| | SEQ ID No. 78 | Δ | - | × | - | - | - | - | × | × | - | ○ | ○ | Δ |
| | SEQ ID No. 79 | - | - | - | - | - | - | - | - | - | - | ○ | ○ | ○ |
| | SEQ ID No. 80 | × | - | × | - | - | - | - | - | - | - | ○ | ○ | ○ |
| | SEQ ID No. 81 | × | - | - | - | - | - | - | - | - | - | ○ | Δ | ○ |
| | SEQ ID No. 82 | × | - | - | - | - | - | - | - | - | - | ○ | ○ | ○ |
| | S EQ ID No. 83 | - | - | - | - | - | - | - | - | - | - | ○ | ○ | ○ |
| | S EQ ID No. 84 | - | - | - | - | - | - | - | - | - | - | ○ | ○ | ○ |
| | S EQ ID No. 85 | - | - | - | - | - | - | - | - | - | - | ○ | ○ | Δ |
| | SEQ ID No. 86 | × | - | - | - | - | - | - | - | - | - | ○ | ○ | ○ |

(continued)

| | | | | | | | | | | | | | |
|---|---|---|---|---|---|---|---|---|---|---|---|---|---|
| | SEQ ID No. 87 | × | - | - | - | - | ○ | - | - | - | - | ○ | Δ | ○ |
| | SEQ ID No. 88 | × | - | - | - | - | - | - | - | - | - | ○ | Δ | - |
| | SEQ ID No. 89 | ○ | - | - | | - | - | - | - | - | - | ○ | ○ | ○ |
| | SEQ ID No. 90 | Δ | ○ | - | - | - | ○ | - | × | × | × | ○ | Δ | ○ |

Table 10-6

Table 10-6

| HE | PCR;Threshold cycle | | | | | | | | | |
|---|---|---|---|---|---|---|---|---|---|---|
| | ○ | SEQ ID No.2 | SEQ ID No.116 | SEQ ID No.117 | SEQ ID No.118 | SEQ ID No.119 | SEQ ID No.120 | SEQ ID No.121 | SEQ ID No.122 | SEQ ID No.123 |
| | SEQ ID No.104 | △ | - | × | ○ | ○ | ○ | ○ | ○ | ○ |
| | SEQ ID No.105 | △ | △ | × | ○ | ○ | ○ | ○ | ○ | △ |
| | SEQ ID No.106 | ○ | ○ | △ | ○ | ○ | ◎ | ◎ | ○ | ○ |
| | SEQ ID No.107 | × | × | × | △ | △ | ○ | ○ | △ | × |
| | SEQ ID No.108 | × | × | × | △ | △ | ○ | △ | △ | ○ |
| | SEQ ID No.109 | ○ | △ | × | ○ | ○ | ○ | ○ | ○ | △ |
| | SEQ ID No.110 | △ | △ | × | ○ | △ | ○ | ○ | △ | △ |
| | SEQ ID No.111 | ○ | △ | × | ○ | ○ | ◎ | ◎ | ◎ | ○ |
| | SEQ ID No.112 | ○ | ○ | △ | ○ | ◎ | ◎ | ◎ | ○ | ○ |
| | SEQ ID No.113 | ○ | △ | △ | ○ | ○ | ◎ | ◎ | ◎ | ◎ |
| | SEQ ID No.114 | | | | | | | | | |
| | SEQ ID No.115 | ○ | | | | | | | | |

| HE | PCR;Threshold cycle | | | | | | | | | |
|---|---|---|---|---|---|---|---|---|---|---|
| | ○ | SEQ ID No.124 | SEQ ID No.125 | SEQ ID No.5 | SEQ ID No.126 | SEQ ID No.127 | SEQ ID No.128 | SEQ ID No.129 | SEQ ID No.130 | SEQ ID No.131 |
| | SEQ ID No.104 | ○ | ○ | ○ | △ | ○ | △ | △ | △ | △ |
| | SEQ ID No.105 | ○ | ○ | ○ | △ | △ | △ | △ | △ | △ |
| | SEQ ID No.106 | ○ | ○ | ○ | ○ | ○ | ○ | ○ | ○ | ○ |
| | SEQ ID No.107 | △ | × | × | × | × | × | × | × | × |
| | SEQ ID No.108 | × | △ | × | × | × | × | × | × | × |
| | SEQ ID No.109 | ○ | △ | △ | × | × | × | × | × | × |
| | SEQ ID No.110 | ○ | △ | △ | ○ | ○ | ○ | ○ | ○ | - |
| | SEQ ID No.111 | ○ | ○ | ○ | ○ | ○ | ○ | ○ | ○ | ○ |
| | SEQ ID No.112 | ○ | ○ | ○ | △ | △ | △ | △ | △ | △ |
| | SEQ ID No.113 | ◎ | ○ | ○ | ○ | △ | △ | △ | ○ | ○ |
| | SEQ ID No.114 | | | ○ | | | | | | |
| | SEQ ID No.115 | | | ○ | | | | | | |

[Table 10-7]

[Table 10-7]

Table 10-7

| | PCR: Melting analysis | | | | | | | | | |
|---|---|---|---|---|---|---|---|---|---|---|
| | ○ | SEQ ID No.2 | SEQ ID No.116 | SEQ ID No.117 | SEQ ID No.118 | SEQ ID No.119 | SEQ ID No.120 | SEQ ID No.121 | SEQ ID No.122 | SEQ ID No.123 |
| HE | SEQ ID No.104 | ○ | - | ○ | ○ | ○ | ○ | ○ | ○ | ○ |
| | SEQ ID No.105 | ○ | ○ | ○ | ○ | ○ | ○ | ○ | ○ | ○ |
| | SEQ ID No.106 | ○ | ○ | ○ | ○ | ○ | ○ | ○ | ○ | ○ |
| | SEQ ID No.107 | ○ | ○ | ○ | ○ | ○ | △ | △ | ○ | ○ |
| | SEQ ID No.108 | ○ | ○ | ○ | ○ | ○ | △ | ○ | ○ | △ |
| | SEQ ID No.109 | ○ | ○ | ○ | ○ | ○ | ○ | ○ | ○ | ○ |
| | SEQ ID No.110 | ○ | ○ | ○ | ○ | ○ | ○ | ○ | ○ | ○ |
| | SEQ ID No.111 | ○ | ○ | ○ | ○ | △ | × | △ | × | ○ |
| | SEQ ID No.112 | ○ | ○ | × | ○ | ○ | ○ | ○ | ○ | ○ |
| | SEQ ID No.113 | ○ | ○ | × | ○ | △ | △ | △ | △ | ○ |
| | SEQ ID No.114 | | | | | | | | | |
| | SEQ ID No.115 | ○ | ○ | × | ○ | △ | △ | △ | △ | ○ |

| | PCR: Melting analysis | | | | | | | | | |
|---|---|---|---|---|---|---|---|---|---|---|
| | ○ | SEQ ID No.124 | SEQ ID No.125 | SEQ ID No.5 | SEQ ID No.126 | SEQ ID No.127 | SEQ ID No.128 | SEQ ID No.129 | SEQ ID No.130 | SEQ ID No.131 |
| HE | SEQ ID No.104 | ○ | ○ | ○ | ○ | ○ | ○ | ○ | ○ | ○ |
| | SEQ ID No.105 | ○ | ○ | ○ | ○ | ○ | ○ | ○ | ○ | ○ |
| | SEQ ID No.106 | ○ | ○ | ○ | ○ | ○ | ○ | ○ | ○ | ○ |
| | SEQ ID No.107 | ○ | ○ | ○ | ○ | ○ | ○ | ○ | ○ | ○ |
| | SEQ ID No.108 | ○ | ○ | ○ | ○ | ○ | ○ | ○ | ○ | ○ |
| | SEQ ID No.109 | ○ | ○ | ○ | ○ | ○ | ○ | ○ | ○ | ○ |
| | SEQ ID No.110 | ○ | ○ | ○ | ○ | ○ | ○ | ○ | ○ | - |
| | SEQ ID No.111 | ○ | ○ | ○ | ○ | ○ | ○ | ○ | ○ | ○ |
| | SEQ ID No.112 | ○ | ○ | ○ | ○ | ○ | ○ | ○ | ○ | ○ |
| | SEQ ID No.113 | △ | ○ | ○ | ○ | ○ | ○ | △ | △ | △ |
| | SEQ ID No.114 | | | ○ | | | | | | |
| | SEQ ID No.115 | △ | ○ | ○ | ○ | ○ | ○ | △ | △ | △ |

(Example 6) Comparison with Patent document 4 in direct-PCR

In the present example, E. coli DNA was extracted from the culture solution of E. coli MG1655 using QIAamp DNA purification kit (QIAGEN), and after extraction, the DNA amount was measured by an absorption spectrometer, and EC1 and EC2 solutions shown in Table 3 diluted with ultrapure water were used as a template of the reaction. The instruments and ultrapure water used were free of DNA. The reaction was carried out with a formulation shown in Table 5. Rotor-Gene Q MDx 5plex HRM (QIAGEN) was used as a real time PCR apparatus, and in the program thereof, the reaction solution was heated at 95°C for 5 minutes, then, a process including heating at 94°C for 10 seconds, at 60°C for 10 seconds and at 72°C for 10 seconds was repeated 35 times, then, the DNA dissociation curve of the amplification product was made. The reaction solution was heated at 95°C for 10 seconds, then, incubated at 72°C for 90 seconds, and heated up to 95°C by 0.5°C stepwise, in order to make the DNA dissociation curve. At each stage, the reaction solution was incubated for 2 seconds and the data were obtained, and the Tm value of the amplification product was measured. Regarding the primer, PCR using the following all seven sets was carried out:

(1) a combination of a forward primer SEQ ID No. 15 and a reverse primer SEQ ID No. 16,
(2) a combination of a forward primer SEQ ID No. 43 and a reverse primer SEQ ID No. 50,
(3) a combination of a forward primer SEQ ID No. 51 and a reverse primer SEQ ID No. 52,
(4) a combination of a forward primer SEQ ID No. 53 and a reverse primer SEQ ID No. 57,
(5) a combination of a forward primer SEQ ID No. 62 and a reverse primer SEQ ID No. 70,
(6) a combination of a forward primer SEQ ID No. 81 and a reverse primer SEQ ID No. 103 and
(7) a combination of a forward primer SEQ ID No. 115 and a reverse primer SEQ ID No. 5.

Then, the Tm value of the each resultant fragment was compared with the Tm value of E. coli previously obtained and the D value was determined, as described in Patent document 4. When the D value is 0.3 or less, they were judged to be homogeneous. This result is shown in Table 11 together with the result of Comparative Example 6.

(Comparative Example 6) Primer part in Patent document 4 in Example 6

The following comparative example is a replication experiment of Patent document 4. In the present comparative example, E. coli DNA was extracted from the culture solution of E. coli MG1655 using QIAamp DNA purification kit (QIAGEN), and after extraction, the DNA amount was measured by an absorption spectrometer, and EC1 and EC2 solutions shown in Table 3 diluted with ultrapure water were used as a template of the reaction. The reaction was carried out with a formulation shown in Table 5. The instruments and ultrapure water used were free of DNA. Rotor-Gene Q MDx 5plex HRM (QIAGEN) was used as a real time PCR apparatus, and in the program thereof, the reaction solution was heated at 95°C for 5 minutes, then, a process including heating at 94°C for 10 seconds, at 60°C for 10 seconds and at 72°C for 10 seconds was repeated 35 times, then, the DNA dissociation curve of the amplification product was made, and the Tm value of each amplification product was measured. Regarding the primer, PCR was carried out with the following all seven sets:

(1) a combination of a forward primer SEQ ID No. 1 and a reverse primer SEQ ID No. 16,
(2) a combination of a forward primer SEQ ID No. 28 and a reverse primer SEQ ID No. 44,
(3) a combination of a forward primer SEQ ID No. 51 and a reverse primer SEQ ID No. 52,
(4) a combination of a forward primer SEQ ID No. 53 and a reverse primer SEQ ID No. 57,
(5) a combination of a forward primer SEQ ID No. 62 and a reverse primer SEQ ID No. 70,
(6) a combination of a forward primer SEQ ID No. 76 and a reverse primer SEQ ID No. 91 and
(7) a combination of a forward primer SEQ ID No. 104 and a reverse primer SEQ ID No. 2.

Then, the Tm value of the each resultant fragment was compared with the Tm value of E. coli previously obtained and the D value was determined, as described in Patent document 4. This result is shown in Table 11 together with the result of Example 6. When the D value is 0.3 or less, they were judged to be homogeneous.

[Table 11]

Table 11 Comparison of Primers in direct-PCR

| Template | Comparative Example 6 | Example 6 |
|---|---|---|
| EC1 | ○ | ○ |
| EC2 | X | × |

○: Identification was successful.
×: Identification was a failure.

(Example 7) Detection sensitivity with simulated sample and comparison of nested PCR with primer in Patent document 4

In the present example, an E. coli suspension was added to 2 ml of whole blood of a healthy individual as shown in Table 12 to prepare a solution. Experiments were all conducted in clinical laboratory center of Toyama University Hospital (2630 Sugitani, Toyama city, Toyama prefecture, 930-0194, Japan).

[Table 12]

Table 12 Concentration in model blood sample

| Name | *E. coli* concentration (CFU/ml) |
|---|---|
| B1 | 2560 |
| B2 | 50 |

The solution prepared by adding an E. coli suspension to whole blood was centrifugally separated at 100 g for 5 minutes and the plasma fraction was recovered, then, the supernatant thereof was further centrifugally separated at 13000 g for 5 minutes, and the precipitate was recovered. DNA solutions B1 and B2 extracted from this precipitate using QIAamp DNA purification kit (QIAGEN) were used as a template and PCR was carried out. The instruments and ultrapure water used were free of DNA. Rotor-Gene Q MDx 5plex HRM (QIAGEN) was used as a real time PCR apparatus, and in the PCR program thereof, first, the reaction solution was heated at 95°C for 5 minutes, then, a process including heating at 94°C for 10 seconds, at 65°C for 10 seconds and at 72°C for 30 seconds was repeated 40 times. For the primer, a combination of a forward primer SEQ ID No. 1 and a reverse primer SEQ ID No. 5 was adopted and the reaction was conducted with a formulation shown in Table 4. Next, this reaction product was diluted 500-fold with DNA-free ultrapure water, and further, this was used as a template and heated at 95°C for 5 minutes with a formulation shown in Table 5, then, a process including heating at 94°C for 10 seconds, at 60°C for 10 seconds and at 72°C for 10 seconds was repeated 30 times, then, the DNA dissociation curve of the amplification product was made. As the primer, the following all seven sets were used:

(1) a combination of a forward primer SEQ ID No. 15 and a reverse primer SEQ ID No. 16,
(2) a combination of a forward primer SEQ ID No. 43 and a reverse primer SEQ ID No. 50,
(3) a combination of a forward primer SEQ ID No. 51 and a reverse primer SEQ ID No. 52,
(4) a combination of a forward primer SEQ ID No. 53 and a reverse primer SEQ ID No. 57,
(5) a combination of a forward primer SEQ ID No. 62 and a reverse primer SEQ ID No. 70,
(6) a combination of a forward primer SEQ ID No. 81 and SEQ ID No. 103 and
(7) a combination of a forward primer SEQ ID No. 115 and a reverse primer SEQ ID No. 5.

The resultant Tm value was compared with the Tm value of E. coli previously obtained and verified, as described in Patent document 4. The reaction solution was heated at 95°C for 10 seconds, then, incubated at 72°C for 90 seconds, and heated up to 95°C by 0.5°C stepwise, in order to make the DNA dissociation curve. At each stage, the reaction solution was incubated for 2 seconds and the data were obtained, and the Tm value of the amplification product was measured. The results are shown in Table 13 together with the result of Comparative Example 7. When the D value is 0.3 or less, they were judged to be homogeneous.

(Comparative Example 7) Primer part in Patent document 4 in Example 7

The following comparative example is a replication experiment of Patent document 4. In the present comparative example, an E. coli suspension was added to 2 ml of whole blood of a healthy individual as shown in Table 12 to prepare a solution. Experiments were all conducted in clinical laboratory center of Toyama University Hospital. The solution prepared by adding an E. coli suspension to whole blood was centrifugally separated at 100 g for 5 minutes and the plasma fraction was recovered, then, the supernatant thereof was further centrifugally separated at 13000 g for 5 minutes, and the precipitate was recovered. A DNA solution extracted from this precipitate using QIAamp DNA purification kit (QIAGEN) was used as a template and PCR was carried out. The instruments and ultrapure water used were free of DNA. Rotor-Gene Q MDx 5plex HRM (QIAGEN) was used as a real time PCR apparatus, and in the PCR program thereof, first, the reaction solution was heated at 95°C for 5 minutes, then, a process including heating at 94°C for 10 seconds, at 65°C for 10 seconds and at 72°C for 30 seconds was repeated 40 times. For the primer, a combination of a forward primer SEQ ID No. 1 and a reverse primer SEQ ID No. 2 was adopted and the reaction was conducted with a formulation shown in Table 1 . Next, this reaction product was diluted 500-fold with DNA-free ultrapure water, and this was used as a template and heated at 95°C for 5 minutes with a formulation shown in Table 2, then, a process including heating at 94°C for 10 seconds, at 60°C for 10 seconds and at 72°C for 10 seconds was repeated 30 times, then, the DNA dissociation curve of the amplification product was made, and the Tm value of each amplification product was measured. Regarding the primer, the following all seven sets were used:

(1) a combination of a forward primer SEQ ID No. 1 and a reverse primer SEQ ID No. 16,
(2) a combination of a forward primer SEQ ID No. 28 and a reverse primer SEQ ID No. 44,
(3) a combination of a forward primer SEQ ID No. 51 and a reverse primer SEQ ID No. 52,
(4) a combination of a forward primer SEQ ID No. 53 and a reverse primer SEQ ID No. 57,
(5) a combination of a forward primer SEQ ID No. 62 and a reverse primer SEQ ID No. 70,
(6) a combination of a forward primer SEQ ID No. 76 and a reverse primer SEQ ID No. 91 and
(7) a combination of a forward primer SEQ ID No. 104 and a reverse primer SEQ ID No. 2.

The resultant Tm value was compared with the Tm value of E. coli previously obtained and verified, as described in Patent document 4. The results are shown in Table 13 together with the result of Example 7. When the D value is 0.3 or less, they were judged to be homogeneous.

[Table 13]

Table 13 Investigation of identification sensitivity using model blood sample

| Template | Comparative example 7 | Example 7 |
|---|---|---|
| B1 | ○ | ○ |
| B2 | × | ○ |

○ : Identification was successful.
×: Identification was a failure.

(Example 8) Verification of primer with multiple bacterial species (electrophoresis)

In the present example, genomes extracted from cultured bacteria of Bacteroides flagilis, Campylobacter jejuni, Clostridium perfringes, Acinetobacter calcoaceticus, Klebsiella pneumoniae, Staphylococcus aureus, Enterococcus faecalis, Bacillus cereus, Pseudomonas aeruginosa, Corynebacterium xerosis and Enterobacter cloacae preserved in Toyama University (clinical laboratory center of Toyama University Hospital) using QIAamp DNA purification kit (QIAGEN) were used as a template of the reaction. The reaction was carried out with a formulation shown in Table 5. The instruments and ultrapure water used were free of DNA. In the PCR program, the reaction solution was heated at 95°C for 5 minutes, then, a process including heating at 94°C for 10 seconds, at 60°C for 10 seconds and at 72°C for 10 seconds was repeated 35 times. PCR was carried out for the following primer combination:

(1) a combination of SEQ ID No. 13 and SEQ ID No. 17,
(2) a combination of SEQ ID No. 34 and SEQ ID No. 50,
(3) a combination of SEQ ID No. 43 and SEQ ID No. 50,
(4) a combination of SEQ ID No. 54 and SEQ ID No. 61,
(5) a combination of SEQ ID No. 55 and SEQ ID No. 58,
(6) a combination of SEQ ID No. 55 and SEQ ID No. 61,
(7) a combination of SEQ ID No. 68 and SEQ ID No. 72,
(8) a combination of SEQ ID No. 77 and SEQ ID No. 103,
(9) a combination of SEQ ID No. 81 and SEQ ID No. 103,
(10) a combination of SEQ ID No. 114 and SEQ ID No. 125 and
(11) a combination of SEQ ID No. 115 and SEQ ID No. 5.

Then, the presence or absence of amplification of the intended fragment was verified. The results are shown in Tables 14-1 to 14-5 together with the result of Comparative Example 8.

Bacterial strains of Bacteroides flagilis, Campylobacter jejuni, Clostridium perfringes, Acinetobacter calcoaceticus, Klebsiella pneumoniae, Staphylococcus aureus, Enterococcus faecalis, Bacillus cereus, Pseudomonas aeruginosa, Corynebacterium xerosis and Enterobacter cloacae are available from RIKEN BioResource Center and Japan Culture Collection of Microorganisms.

Evaluation in Table 14 was carried out according to the following criteria.

Amount of intended product:

◎ : amplification is particularly good
○: amplification is good
△: amplification is poor
×: amplification is particularly poor

Amplification of non-specific product

○: non-specific product is not found
△: non-specific product is found
×: non-specific product is abundant

Threshold cycle:

◎ : threshold cycle is fast (decreased by 2 cycles or more)
○: threshold cycle is somewhat fast (decreased by 0-1 cycle)
△: threshold cycle is somewhat slow (increased by 1-2 cycles)
×: threshold cycle is slow (increased by 3 cycles or more)

Melting analysis:

○: only one peak
△: small peaks
×: several peaks
-: no peak

(Comparative Example 8) Verification of primer with multiple bacterial species (electrophoresis)

The following comparative example is a replication experiment of Patent document 4. In the present comparative example, genomes extracted from cultured bacteria of Bacteroides flagilis, Campylobacter jejuni, Clostridium perfringes, Acinetobacter calcoaceticus, Klebsiella pneumoniae, Staphylococcus aureus, Enterococcus faecalis, Bacillus cereus, Pseudomonas aeruginosa, Corynebacterium xerosis and Enterobacter cloacae preserved in Toyama University (clinical laboratory center of Toyama University Hospital) using QIAamp DNA purification kit (QIAGEN) were used as a template of the reaction. The reaction was carried out with a formulation shown in Table 5. The instruments and ultrapure water used were free of DNA. In the PCR program, the reaction solution was heated at 95°C for 5 minutes, then, a process including heating at 94°C for 10 seconds, at 60°C for 10 seconds and at 72°C for 10 seconds was repeated 35 times.

PCR was carried out for the following primers:

(1) a combination of SEQ ID No. 1 and SEQ ID No. 16,
(2) a combination of SEQ ID No. 28 and SEQ ID No. 44,
(3) a combination of SEQ ID No. 51 and SEQ ID No. 52,
(4) a combination of SEQ ID No. 53 and SEQ ID No. 57,
(5) a combination of SEQ ID No. 62 and SEQ ID No. 70,
(6) a combination of SEQ ID No. 76 and SEQ ID No. 91 and
(7) a combination of SEQ ID No. 104 and SEQ ID No. 2.

Then, the presence or absence of amplification of the intended fragment was verified. The results are shown in Table 14 together with the result of Example 8.

(Example 9) Verification of primer with multiple bacterial species (PCR)

In the present example, genomes extracted from cultured bacteria of Bacteroides flagilis, Campylobacter jejuni, Clostridium perfringes, Acinetobacter calcoaceticus, Klebsiella pneumoniae, Staphylococcus aureus, Enterococcus faecalis, Bacillus cereus, Pseudomonas aeruginosa, Corynebacterium xerosis and Enterobacter cloacae preserved in Toyama University (clinical laboratory center of Toyama University Hospital) using QIAamp DNA purification kit (QIAGEN) were used as a template of the reaction. The reaction was carried out with a formulation shown in Table 5. The instruments and ultrapure water used were free of DNA. Rotor-Gene Q MDx 5plex HRM (QIAGEN) was used as a real time PCR apparatus, and in the program thereof, the reaction solution was heated at 95°C for 5 minutes, then, a process including heating at 94°C for 10 seconds, at 60°C for 10 seconds and at 72°C for 10 seconds was repeated 35 times, then, the DNA dissociation curve of the amplification product was made. The reaction solution was heated at 95°C for 10 seconds, then, incubated at 72°C for 90 seconds, and heated up to 95°C by 0.5°C stepwise, in order to make the DNA dissociation curve.

PCR was carried out for the following primers:

(1) a combination of SEQ ID No. 13 and SEQ ID No. 17,
(2) a combination of SEQ ID No. 34 and SEQ ID No. 50,
(3) a combination of SEQ ID No. 43 and SEQ ID No. 50,
(4) a combination of SEQ ID No. 54 and SEQ ID No. 61,
(5) a combination of SEQ ID No. 55 and SEQ ID No. 58,
(6) a combination of SEQ ID No. 55 and SEQ ID No. 61,
(7) a combination of SEQ ID No. 68 and SEQ ID No. 72,
(8) a combination of SEQ ID No. 77 and SEQ ID No. 103,
(9) a combination of SEQ ID No. 81 and SEQ ID No. 103,
(10) a combination of SEQ ID No. 114 and SEQ ID No. 125 and
(11) a combination of SEQ ID No. 115 and SEQ ID No. 5.

Then, the DNA dissociation curve of the amplification product was made. The reaction solution was heated at 95°C for 10 seconds, then, incubated at 72°C for 90 seconds, and heated up to 95°C by 0.5°C stepwise, in order to make the DNA dissociation curve. At each stage, the reaction solution was incubated for 2 seconds and the data were obtained, and the Tm value of the amplification product was measured. Based on the results, the threshold cycle of the amplification curve and the dissociation curve were verified. The results are shown in Table 14 together with the result of Comparative Example 5.

(Comparative Example 9) Verification of primer with multiple bacterial species. (PCR)

The following comparative example is a replication experiment of Patent document 4. In the present comparative example, genomes extracted from cultured bacteria of Bacteroides flagilis, Campylobacter jejuni, Clostridium perfringes, Acinetobacter calcoaceticus, Klebsiella pneumoniae, Staphylococcus aureus, Enterococcus faecalis, Bacillus cereus, Pseudomonas aeruginosa, Corynebacterium xerosis and Enterobacter cloacae preserved in Toyama University (clinical laboratory center of Toyama University Hospital) using QIAamp DNA purification kit (QIAGEN) were used as a template of the reaction. The reaction was carried out with a formulation shown in Table 5. The instruments and ultrapure water used were free of DNA. Rotor-Gene Q MDx 5plex HRM (QIAGEN) was used as a real time PCR apparatus, and in the program, the reaction solution was heated at 95°C for 5 minutes, then, a process including heating at 94°C for 10 seconds, at 60°C for 10 seconds and at 72°C for 10 seconds was repeated 35 times.

For the primer, a combination of SEQ ID No. 1 and SEQ ID No. 16, a combination of SEQ ID No. 28 and SEQ ID No. 44, a combination of SEQ ID No. 51 and SEQ ID No. 52, a combination of SEQ ID No. 53 and SEQ ID No. 57, a combination of SEQ ID No. 62 and SEQ ID No. 70, a combination of SEQ ID No. 76 and SEQ ID No. 91 and a combination of SEQ ID No. 104 and SEQ ID No. 2 were used for PCR, and thereafter, the DNA dissociation curve of the amplification product was made. The reaction solution was heated at 95°C for 10 seconds, then, incubated at 72°C for 90 seconds, and heated up to 95°C by 0.5°C stepwise, in order to make the DNA dissociation curve. At each stage, the reaction solution was incubated for 2 seconds and the data were obtained, and the Tm value of the amplification product was measured. Based on the results, the threshold cycle of the amplification curve and the dissociation curve were verified. The results are shown in Table 14 together with the result of Example 9.

[Table 14-1]

Table 14-1

| | | *Bacteroides flagilis* | | | | *Campylobacter jejuni* | | | | *Clostridium perfringes* | | | |
|---|---|---|---|---|---|---|---|---|---|---|---|---|---|
| | | Electrophoresis | | PCR | | Electrophoresis | | PCR | | Electrophoresis | | PCR | |
| Forward | Reverce | Amplifi -cation | Non- specifi- city | Threshold cycle | Melting Analysis | Amplifi -cation | Non- specificity | Threshold cycle | Melting Analysis | Amplifi -cation | Non- specificity | Threshold cycle | Melting Analysis |
| SEQ ID No.1 | SEQ ID No. 16 | Δ | Δ | ○ | ○ | Δ | Δ | ○ | Δ | - | - | - | - |
| SEQ ID No.13 | SEQ ID No. 17 | Δ | Δ | x | ○ | Δ | Δ | ○ | ○ | ○ | ○ | ◎ | ○ |
| SEQ ID No.28 | SEQ ID No, 44 | Δ | Δ | ○ | x | Δ | Δ | ○ | ○ | Δ | Δ | ○ | ○ |
| SEQ ID No.34 | SEQ ID No, 50 | ○ | ○ | ○ | x | ○ | ○ | ○ | ○ | - | - | - | - |
| SEQ ID No.43 | SEQ ID No. 50 | ○ | ○ | ○ | x | Δ | Δ | ○ | ○ | Δ | Δ | ○ | ○ |
| SEQ ID No.51 | SEQ ID No. 52 | Δ | Δ | ○ | ○ | Δ | Δ | ○ | ○ | Δ | Δ | ○ | ○ |
| SEQ ID No.53 | SEQ ID No. 57 | x | - | - | - | Δ | Δ | ○ | ○ | - | - | - | - |
| SEQ ID No.54 | SEQ ID No. 61 | ○ | Δ | Δ | ○ | Δ | x | x | Δ | - | - | - | - |
| SEQ ID No.55 | SEQ ID No. 58 | ○ | ○ | ◎ | ○ | ○ | Δ | ○ | ○ | ○ | ○ | ○ | ○ |
| SEQ ID No.55 | SEQ ID No, 61 | ○ | Δ | ◎ | ○ | - | - | - | - | ○ | ○ | ○ | ○ |
| SEQ ID No.62 | SEQ ID No. 70 | Δ | Δ | ○ | ○ | Δ | Δ | ○ | ○ | Δ | Δ | ○ | ○ |
| SEQ ID No.68 | SEQ ID No 72 | x | x | x | Δ | Δ | Δ | ○ | ○ | Δ | Δ | ○ | ○ |
| SEQ ID No.76 | SEQ ID No. 91 | x | x | ○ | Δ | Δ | Δ | ○ | ○ | Δ | Δ | ○ | ○ |

(continued)

| | | *Bacteroides flagilis* | | | | *Campylobacter jejuni* | | | | *Clostridium perfringes* | | | |
|---|---|---|---|---|---|---|---|---|---|---|---|---|---|
| | | Electrophoresis | | PCR | | Electrophoresis | | PCR | | Electrophoresis | | PCR | |
| Forward | Reverce | Amplifi -cation | Non- specifi- city | Threshold cycle | Melting Analysis | Amplifi -cation | Non- specificity | Threshold cycle | Melting Analysis | Amplifi -cation | Non- specificity | Threshold cycle | Melting Analysis |
| SEQ ID No.77 | SEQ ID No. 103 | ○ | ○ | ◎ | ○ | ○ | ○ | ◎ | ○ | ○ | ○ | ○ | ○ |
| SEQ ID No.81 | SEQ ID No. 103 | ○ | Δ | ◎ | ○ | ○ | ○ | ◎ | ○ | ○ | ○ | ○ | ○ |
| SEQ ID No.1 04 | SEQ ID No. 2 | Δ | Δ | ○ | ○ | Δ | Δ | ○ | ○ | Δ | Δ | ○ | ○ |
| SEQ ID No.114 | SEQ ID No. 125 | Δ | Δ | ○ | ○ | Δ | Δ | ○ | ○ | Δ | Δ | ○ | ○ |
| SEQ ID No.115 | SEQ ID No. 5 | ○ | ○ | ○ | ○ | Δ | Δ | ○ | ○ | Δ | Δ | ○ | ○ |

[Table 14-2]

Table 14-2

| Forward | Reverce | *Acinetobacter calcoaceticus* Electrophoresis Amplifi-cation | *Acinetobacter calcoaceticus* Electrophoresis Non-specifi-city | *Acinetobacter calcoaceticus* PCR Threshold cycle | *Acinetobacter calcoaceticus* PCR Melting Analysis | *Klebsiella pneumoniae* Electrophoresis Ampfifi-cation | *Klebsiella pneumoniae* Electrophoresis Non-specifi-city | *Klebsiella pneumoniae* PCR Threshold cycle | *Klebsiella pneumoniae* PCR Melting Analysis | *Staphylococcus* aureus Electrophoresis Amplifi-cation | *Staphylococcus* aureus Electrophoresis Non-specifi-city | *Staphylococcus* aureus PCR Threshold cycle | *Staphylococcus* aureus PCR Melting Analysis |
|---|---|---|---|---|---|---|---|---|---|---|---|---|---|
| SEQ ID No.1 | SEQ ID No. 16 | Δ | Δ | ○ | ○ | - | - | - | - | - | - | - | - |
| SEQ ID No.13 | SEQ ID No. 17 | Δ | Δ | ○ | ○ | ○ | ○ | ○ | ○ | ○ | Δ | ○ | ○ |
| SEQ ID No.28 | SEQ ID No. 44 | Δ | Δ | ○ | ○ | Δ | Δ | ○ | ○ | Δ | Δ | ○ | ○ |
| SEQ ID No.34 | SEQ ID No. 50 | ○ | Δ | x | ○ | Δ | Δ | ○ | ○ | - | - | | |
| SEQ ID No.43 | SEQ ID No. 50 | Δ | Δ | ○ | ○ | - | - | - | - | Δ | Δ | ◎ | ○ |
| SEQ ID No.51 | SEQ ID No. 52 | Δ | Δ | ○ | ○ | Δ | Δ | ○ | ○ | Δ | Δ | ○ | ○ |
| SEQ ID No.53 | SEQ ID No. 57 | Δ | Δ | ○ | ○ | Δ | Δ | ○ | ○ | Δ | Δ | ○ | ○ |
| SEQ ID No. 54 | SEQ ID No. 61 | Δ | Δ | Δ | ○ | ○ | ○ | ◎ | ○ | ○ | ○ | Δ | ○ |
| SEQ ID No.55 | SEQ ID No. 58 | Δ | Δ | x | ○ | Δ | Δ | ◎ | ○ | Δ | Δ | ◎ | ○ |
| SEQ ID No.55 | SEQ ID No. 61 | Δ | Δ | Δ | ○ | ○ | ○ | ◎ | ○ | ○ | ○ | ◎ | ○ |
| SEQ ID No.62 | SEQ ID No. 70 | Δ | Δ | ○ | ○ | - | - | - | - | Δ | Δ | ○ | ○ |
| SEQ ID No.68 | SEQ ID No. 72 | Δ | Δ | ○ | ○ | ○ | ○ | ◎ | ○ | Δ | ○ | ○ | ○ |
| SEQ ID No.76 | SEQ ID No. 91 | - | - | - | - | - | - | - | - | ○ | ○ | ○ | ○ |

(continued)

| | | *Acinetobacter calcoaceticus* | | | | *Klebsiella pneumoniae* | | | | *Staphylococcus* aureus | | | |
|---|---|---|---|---|---|---|---|---|---|---|---|---|---|
| | | Electrophoresis | | PCR | | Electrophoresis | | PCR | | Electrophoresis | | PCR | |
| Forward | Reverce | Amplifi -cation | Non- specifi- city | Threshold cycle | Melting Analysis | Ampfifi -cation | Non- specificity | Threshold cycle | Melting Analysis | Amplifi -cation | Non- specificity | Threshold cycle | Melting Analysis |
| SEQ ID No.77 | SEQ ID No. 103 | Δ | Δ | ◎ | ○ | ○ | ○ | ◎ | ○ | Δ | Δ | ○ | ○ |
| SEQ ID No.81 | SEQ ID No. 103 | ○ | ○ | ◎ | ○ | ○ | ○ | ◎ | ○ | ○ | Δ | ○ | ○ |
| SEQ ID No.104 | SEQ ID No. 2 | Δ | Δ | ○ | ○ | Δ | Δ | ○ | ○ | Δ | Δ | ○ | ○ |
| SEQ ID No. 114 | SEQ ID No. 125 | Δ | Δ | ○ | ○ | Δ | Δ | ○ | ○ | Δ | Δ | ○ | ○ |
| SEQ ID No.115 | SEQ ID No. 5 | Δ | Δ | ○ | ○ | Δ | Δ | ○ | ○ | Δ | Δ | ○ | ○ |

[Table 14-3]

Table 14-3

| Forward | Reverce | *Enterococcus faecalis* Electrophoresis Amplifi -cation | Non- specificity | PCR Threshold cycle | Melting Analysis |
|---|---|---|---|---|---|
| SEQ ID No.1 | SEQ ID No.16 | Δ | Δ | ○ | ○ |
| SEQ ID No.13 | SEQ ID No.17 | ○ | ○ | Δ | ○ |
| SEQ ID No.28 | SEQ ID No.44 | Δ | Δ | ○ | ○ |
| SEQ ID No.34 | SEQ ID No.50 | Δ | Δ | Δ | ○ |
| SEQ ID No.43 | SEQ ID No.50 | Δ | Δ | ○ | ○ |
| SEQ ID No.51 | SEQ ID No.52 | Δ | Δ | ○ | ○ |
| SEQ ID No.53 | SEQ ID No.57 | Δ | Δ | ○ | ○ |
| SEQ ID No.54 | SEQ ID No.61 | Δ | Δ | ○ | ○ |
| SEQ ID No.55 | SEQ ID No.58 | Δ | Δ | ○ | ○ |
| SEQ ID No.55 | SEQ ID No.61 | Δ | Δ | ○ | ○ |
| SEQ ID No.62 | SEQ ID No.70 | Δ | Δ | ○ | ○ |
| SEQ ID No.68 | SEQ ID No.72 | Δ | Δ | ○ | ○ |
| SEQ ID No.76 | SEQ ID No.91 | Δ | Δ | ○ | ○ |
| SEQ ID No.77 | SEQ ID No.103 | Δ | Δ | ○ | ○ |
| SEQ ID No.81 | SEQ ID No.103 | Δ | Δ | ○ | ○ |
| SEQ ID No.104 | SEQ ID No.2 | Δ | × | ○ | ○ |
| SEQ ID No.114 | SEQ ID No.125 | ○ | ○ | ○ | ○ |
| SEQ ID No.115 | SEQ ID No.5 | ○ | ○ | ○ | ○ |

[Table 14-4]

Table 14-4

| Forward | Reverce | *Bacillus cereus* Electrophoresis Amplifi -cation | *Bacillus cereus* Electrophoresis No-specificity | *Bacillus cereus* PCR Threshold cycle | *Bacillus cereus* PCR Melting Analysis | *Streptococcus agalactiae* Electrophoresis Amplifi -cation | *Streptococcus agalactiae* Electrophoresis Non-specificity | *Streptococcus agalactiae* PCR Threshold cycle | *Streptococcus agalactiae* PCR Melting Analysis | *Pseudomonas aeruginosa* Electrophoresis Amplifi -cation | *Pseudomonas aeruginosa* Electrophoresis Non-specificity | *Pseudomonas aeruginosa* PCR Threshold cycle | *Pseudomonas aeruginosa* PCR Melting Analysis |
|---|---|---|---|---|---|---|---|---|---|---|---|---|---|
| SEQ ID No.1 | SEQ ID No.16 | Δ | Δ | ○ | ○ | Δ | Δ | ○ | ○ | x | x | ○ | ○ |
| SEQ ID No. 13 | SEQ ID No.17 | Δ | Δ | Δ | ○ | Δ | Δ | x | ○ | ○ | ○ | Δ | ○ |
| SEQ ID No.28 | SEQ ID No.44 | Δ | Δ | ○ | ○ | Δ | Δ | ○ | ○ | Δ | Δ | ○ | ○ |
| SEQ ID No.34 | SEQ ID No.50 | Δ | Δ | ○ | ○ | Δ | Δ | ○ | ○ | Δ | Δ | ○ | ○ |
| SEQ ID No.43 | SEQ ID No.50 | Δ | Δ | ○ | ○ | Δ | Δ | ○ | ○ | Δ | Δ | ○ | ○ |
| SEQ ID No.51 | SEQ ID No.52 | Δ | Δ | ○ | ○ | Δ | Δ | ○ | ○ | Δ | Δ | ○ | ○ |
| SEQ ID No.53 | SEQ ID No.57 | Δ | Δ | ○ | ○ | Δ | Δ | ○ | ○ | Δ | Δ | ○ | ○ |
| SEQ ID No.54 | SEQ ID No.61 | Δ | Δ | ○ | ○ | Δ | Δ | ○ | ○ | Δ | Δ | ○ | ○ |
| SEQ ID No.55 | SEQ ID No.58 | Δ | Δ | ○ | ○ | Δ | Δ | ○ | ○ | - | - | | |
| SEQ ID No.55 | SEQ ID No.61 | Δ | Δ | ○ | ○ | Δ | Δ | ○ | ○ | Δ | Δ | ○ | ○ |
| SEQ ID No.62 | SEQ ID No.70 | - | - | - | - | Δ | Δ | ○ | ○ | Δ | Δ | ○ | ○ |
| SEQ ID NO. 58 | SEQ ID No.72 | ○ | ○ | ◎ | ○ | Δ | Δ | Δ | ○ | Δ | Δ | Δ | ○ |
| SEQ ID No.76 | SEQ ID No.91 | Δ | Δ | ○ | ○ | Δ | Δ | ○ | ○ | Δ | Δ | ○ | ○ |

(continued)

| | | | | | | | | | | | | | |
|---|---|---|---|---|---|---|---|---|---|---|---|---|---|
| SEQ ID NO. 77 | SEQ ID No.103 | Δ | Δ | ◎ | ○ | Δ | Δ | ○ | ○ | ○ | ○ | ◎ | ○ |
| SEQ ID No.81 | SEQ ID No. 103 | Δ | Δ | ◎ | ○ | Δ | Δ | Δ | ○ | ○ | ○ | ◎ | ○ |
| SEQ ID No. 104 | SEQ ID No.2 | Δ | Δ | ○ | ○ | Δ | Δ | ○ | ○ | x | x | ○ | ○ |
| SEQ ID No. 114 | SEQ ID No.125 | ○ | ○ | Δ | ○ | ○ | ○ | ○ | ○ | ○ | ○ | ○ | ○ |
| SEQfDNo.-! 15 | SEQ ID No.5 | ○ | ○ | Δ | ○ | ○ | ○ | ○ | ○ | Δ | Δ | ○ | ○ |

[Table 14-5]

Table 14-5

| Forward | Reverce | *Corynebacterium xerosis* Electrophoresis Amplifi -cation | *Corynebacterium xerosis* Electrophoresis Non- specifi-city | *Corynebacterium xerosis* PCR Threshold cycle | *Corynebacterium xerosis* PCR Melting Analysis | *Enterobacter cloacae* Electrophoresis Amplifi -cation | *Enterobacter cloacae* Electrophoresis Non- specifi-city | *Enterobacter cloacae* PCR Threshold cycle | *Enterobacter cloacae* PCR Melting Analysis | *Escherichia coli* Electrophoresis Amplifi -cation | *Escherichia coli* Electrophoresis Non- specifi-city | *Escherichia coli* PCR Threshold cycle | *Escherichia coli* PCR Melting Analysis |
|---|---|---|---|---|---|---|---|---|---|---|---|---|---|
| SEQ ID No.1 | SEQ ID No. 16 | Δ | Δ | ○ | ○ | Δ | Δ | ○ | ○ | Δ | Δ | ○ | ○ |
| SEQ ID No.13 | SEQ ID No. 17 | Δ | Δ | Δ | ○ | Δ | Δ | ○ | ○ | Δ | Δ | ○ | ○ |
| SEQ ID No.28 | SEQ ID No. 44 | Δ | Δ | ○ | ○ | Δ | Δ | ○ | ○ | Δ | Δ | ○ | ○ |
| SEQ ID No.34 | SEQ ID No. 50 | ○ | ○ | × | ○ | Δ | Δ | ◎ | ○ | Δ | Δ | Δ | ○ |
| SEQ ID No.43 | SEQ ID No. 50 | ○ | ○ | ○ | ○ | Δ | Δ | ◎ | ○ | Δ | Δ | ○ | ○ |
| SEQ ID No.51 | SEQ ID No. 52 | Δ | Δ | ○ | ○ | Δ | Δ | ○ | ○ | Δ | Δ | ○ | 0 |
| SEQ ID No.53 | SEQ ID No. 57 | Δ | Δ | ○ | ○ | Δ | × | ○ | ○ | Δ | Δ | ○ | ○ |
| SEQ ID No.54 | SEQ ID No. 61 | ○ | ○ | ◎ | ○ | ○ | ○ | Δ | ○ | ○ | ○ | ◎ | ○ |
| SEQ ID No.55 | SEQ ID No. 58 | Δ | Δ | ◎ | ○ | ○ | ○ | Δ | ○ | ○ | ○ | ○ | ○ |
| SEQ ID No.55 | SEQ ID No. 61 | ○ | ○ | ◎ | ○ | ○ | ○ | Δ | ○ | - | - | - | - |
| SEQ ID No.62 | SEQ ID No. 70 | Δ | Δ | ○ | ○ | Δ | Δ | ○ | Δ | Δ | Δ | ○ | ○ |
| SEQ ID No.68 | SEQ ID No. 72 | Δ | Δ | ○ | ○ | Δ | Δ | ○ | ○ | Δ | Δ | ○ | ○ |
| SEQ ID No.76 | SEQ ID No. 91 | Δ | Δ | ○ | ○ | Δ | Δ | ○ | Δ | Δ | Δ | ○ | ○ |

(continued)

| Forward | Reverce | *Corynebacterium xerosis* | | | | *Enterobacter cloacae* | | | | *Escherichia coli* | | | |
|---|---|---|---|---|---|---|---|---|---|---|---|---|---|
| | | Electrophoresis | | PCR | | Electrophoresis | | PCR | | Electrophoresis | | PCR | |
| | | Amplifi -cation | Non- specificity | Threshold cycle | Melting Analysis | Amplifi -cation | Non- specificity | Threshold cycle | Melting Analysis | Amplifi -cation | Non- specificity | Threshold cycle | Melting Analysis |
| SEQ ID No.77 | SEQ ID No. 103 | ○ | ○ | × | ○ | Δ | Δ | ○ | Δ | Δ | Δ | ○ | ○ |
| SEQ ID No.81 | SEQ ID No. 103 | ○ | ○ | × | 0 | Δ | Δ | ○ | Δ | Δ | Δ | ○ | ○ |
| SEQ ID No.104 | SEQ ID No. 2 | Δ | Δ | ○ | ○ | Δ | Δ | ○ | ○ | Δ | Δ | ○ | ○ |
| SEQ ID No.114 | SEQ ID No. 125 | Δ | Δ | ○ | ○ | Δ | Δ | ○ | ○ | ○ | ○ | ○ | ○ |
| SEQ ID No.115 | SEQ ID No. 5 | Δ | Δ | ○ | ○ | Δ | Δ | ○ | ○ | ○ | ○ | ○ | ○ |

(Example 10)Verification with simulated blood sample of other bacterial species than E. coli

In the present example, cultured bacteria of Bacillus cereus, Pseudomonas aeruginosa, Enterobacter aerogenes and Streptococcus agalctiae preserved in Toyama University (clinical laboratory center of Toyama University Hospital) were added to 2 ml of whole blood from a healthy individual so as to give concentrations shown in Table 15 to prepare solutions, being prepared along with the efforts of Toyama University Hospital. Experiments were all conducted in clinical laboratory center of Toyama University Hospital. The solution prepared by adding a bacterium suspension to whole blood was centrifugally separated at 100 g for 5 minutes and the plasma fraction was recovered, then, the supernatant thereof was further centrifugally separated at 13000 g for 5 minutes, and the precipitate was recovered. A DNA solution extracted from this precipitate using QIAamp DNA purification kit (QIAGEN) was used as a template and PCR was carried out in clinical laboratory center of Toyama University Hospital. The instruments and ultrapure water used were free of DNA. Rotor-Gene Q MDx 5plex HRM (QIAGEN) was used as a real time PCR apparatus, and in the PCR program thereof, first, the reaction solution was heated at 95°C for 5 minutes, then, a process including heating at 94°C for 10 seconds, at 65°C for 10 seconds and at 72°C for 30 seconds was repeated 40 times. For the primer, a combination of a forward primer SEQ ID No. 1 and a reverse primer SEQ ID No. 5 was adopted. The reaction was conducted with a formulation shown in Table 4. This reaction product was diluted 500-fold with DNA-free ultrapure water, and further, this was used as a template and heated at 95°C for 5 minutes with a formulation shown in Table 5, then, a process including heating at 94°C for 10 seconds, at 60°C for 10 seconds and at 72°C for 10 seconds was repeated 30 times, then, the DNA dissociation curve of the amplification product was made. The reaction solution was heated at 95°C for 10 seconds, then, incubated at 72°C for 90 seconds, and heated up to 95°C by 0.5°C stepwise, in order to make the DNA dissociation curve. At each stage, the reaction solution was incubated for 2 seconds and the data were obtained, and the Tm value of the amplification product was measured.

As the primer, the following seven sets were used:

a combination of a forward primer SEQ ID No. 15 and a reverse primer SEQ ID No. 16,
a combination of a forward primer SEQ ID No. 43 and a reverse primer SEQ ID No. 50,
a combination of a forward primer SEQ ID No. 51 and a reverse primer SEQ ID No. 52,
a combination of a forward primer SEQ ID No. 53 and a reverse primer SEQ ID No. 57,
a combination of a forward primer SEQ ID No. 62 and a reverse primer SEQ ID No. 70,
a combination of a forward primer SEQ ID No. 81 and SEQ ID No. 103 and
a combination of a forward primer SEQ ID No. 115 and a reverse primer SEQ ID No. 5.

Then, the resultant Tm value was compared with the Tm values of various bacterial species previously obtained and verified, as described in Patent document 4.
The results are shown in Table 15.

[Table 15]

Table 15 Concentrations of model blood samples prepared by using bacteria other than E, coli and results

| Name | Species | CFU/ml | Identification |
|---|---|---|---|
| Ak19Ba | Bacillus cereus | 5040 | ○ |
| AK24St | Streptococcus agalactiae | 548 | ○ |
| AK24Ps | Pseudomonas aeruginosa | 530 | ○ |
| AEa24 | Enterobacter aerogenes | 262 | ○ |

| Name | Species | CFU/ml | Identification |
|---|---|---|---|
| PA-Bc | Bacillus cereus | 5040 | ○ |
| PA-Sa | Streptococcus agalactiae | 548 | ○ |
| PA-Ps | Pseudomonas aeruginosa | 530 | ○ |
| PA-Ea | Enterobacter aerogenes | 262 | ○ |

(Example 11)Verification with clinical sample of Hospital of Toyama University

In the present example, a DNA solution extracted from a blood sample of a patient suspected of sepsis in Toyama University Hospital was used as a template, and experiments were all conducted in a laboratory of the examination department of Toyama University Hospital. The instruments and ultrapure water used were free of DNA. Rotor-Gene Q MDx 5plex HRM was used as a real time PCR apparatus, and in the PCR program thereof, first, the reaction solution was heated at 95°C for 5 minutes, then, a process including heating at 94°C for 10 seconds, at 65°C for 10 seconds and at 72°C for 30 seconds was repeated 40 times. For the primer, a combination of a forward primer SEQ ID No. 1 and a reverse primer SEQ ID No. 5 was adopted. The reaction was conducted with a formulation shown in Table 4. This reaction product was diluted 500-fold with DNA-free ultrapure water, and further, this was used as a template and heated at 95°C for 5 minutes with a formulation shown in Table 5, then, a process including heating at 94°C for 10 seconds, at 60°C for 10 seconds and at 72°C for 10 seconds was repeated 30 times, then, the DNA dissociation curve of the amplification product was made. The reaction solution was heated at 95°C for 10 seconds, then, incubated at 72°C for 90 seconds, and heated up to 95°C by 0.5°C stepwise, in order to make the DNA dissociation curve. At each stage, the reaction solution was incubated for 2 seconds and the data were obtained, and the Tm value of the amplification product was measured. As the primer, the following seven sets were used:

a combination of a forward primer SEQ ID No. 15 and a reverse primer SEQ ID No. 16,
a combination of a forward primer SEQ ID No. 43 and a reverse primer SEQ ID No. 50,
a combination of a forward primer SEQ ID No. 51 and a reverse primer SEQ ID No. 52,
a combination of a forward primer SEQ ID No. 53 and a reverse primer SEQ ID No. 57,
a combination of a forward primer SEQ ID No. 62 and a reverse primer SEQ ID No. 70,
a combination of a forward primer SEQ ID No. 81 and SEQ ID No. 1 03 and
a combination of a forward primer SEQ ID No. 115 and a reverse primer SEQ ID No. 5

Then, the resultant Tm value was compared with data in the data base containing input Tm values of various bacterial species previously obtained and verified, as described in Patent document 4. The results are shown in Table 16.

[Table 16]

Table 16 Identification results of clinical samples in Toyama University

| | Results of Example 11 | Blood culture method | Consistency of results |
|---|---|---|---|
| CS-1 | *Bacteroides vulgatus* | *Bacteroides vulgatus* | ○ |
| CS-2 | *Klebsiella pneumoniae* | *Klebsiella pneumoniae, Corynebacterium sp.* | ○ |
| CS-3 | *Staphylococcus haemolyticus or aureus* | *Staphylococcus haemolyticus* | ○ |
| CS-4 | *Citrobacter freundii* | *Citrobacter freundii* | ○ |
| CS-5 | *Bacillus cereus* | *Bacillus cereus* | ○ |
| CS-6 | *Staphylococcus captis or epidermidis,* | *Staphylococcus epidermidis* | ○ |
| CS-7 | *Staphylococcus aureus* | *Staphylococcus aureus* | ○ |
| CS-8 | *Klebsiella pneumoniae* | *Klebsiella pneumoniae* | ○ |
| CS-9 | *Escherichia coli* | *Escherichia coli* | ○ |
| CS-10 | *Enterobacter cloacae* | *Enterobacter cloacae* | ○ |

(continued)

| | Results of Example 11 | Blood culture method | Consistency of results |
|---|---|---|---|
| CS-11 | *Escherichia coli* | *Escherichia coli* | ○ |

(Example 12)Regarding hot start PCR

In the present example, a DNA solution (HE) was used as a template, and an amplification reaction was conducted using CleanAmp dNTPs (Sigma-Aldrich) as dNTP for hot start and using dNTP conventionally used, and the results were compared. The reaction was conducted with a formulation shown in Tables 4 and 17.

[Table 17]

Table 17 Composition of Reaction solution 3

| | |
|---|---|
| Template | 2.0 μL |
| 10X Thnuder Taq Buffer | 2.0 μL |
| 25mM MgCl2 | 1.6 μL |
| e-DNAP | 1.0 Unit |
| 2mM dNTP | 2.0 μL |
| EvaGreen (Biotium) | 1.0 μL |
| 10μM forward primer | 0.6 μL |
| 10μM reverse primer | 0.6 μL |
| DW | Appropriate amount |
| Total | 20.0 μL |

The instruments and ultrapure water used were free of DNA. As the template, EC3 shown in Table 3 was used. For the primer, a combination of a forward primer SEQ ID No. 1 and a reverse primer SEQ ID No. 2 was adopted, and in the PCR program, first, the reaction solution was heated at 95°C for 5 minutes, then, a process including heating at 94°C for 10 seconds, at 65°C for 10 seconds and at 72°C for 30 seconds was repeated 35 times. The solution after the reaction was electrophoresed on 2% agarose gel and stained with ethidium bromide, then, the amplification of the intended product and amplification of the non-specific product were verified.

The results are shown in Fig. 2.

(Example 13)DNA removal treatment (ultrafiltration, E. coli genome addition experiment)

The instruments and ultrapure water used were free of DNA. Five hundred microliter of sterile water was added into Amicon UFC50508 (Millipore) and centrifugal separation was performed at 5000 g for 5 minutes, and, then, the filtrate and the solution remaining in the filter were removed. Next, 500 μL of 1 N hydrochloric acid was supplied and centrifugal separation was performed at 5000 g for 5 minutes, and, then, the filtrate and the solution remaining in the filter were removed. Finally, 500 μL of sterile water was supplied and centrifugal separation was performed at 5000 g for 5 minutes, and, then, the filtrate and the solution remaining in the filter were removed. Such an operation was repeated twice. Next, 100 μL of a mixed solution composed of components obtained after removal of the template and EvaGreen from a formulation of the reaction solution shown in Table 4 was prepared, and further, 4 pg of E. coli DNA was added. The resultant solution was put into Amicon UFC50508 after the above operations and centrifugal separation was performed at 5000 g for 5 minutes. Then, the filtrate was recovered. EvaGreen and sterile water were added to the resultant filtrate and PCR was conducted. Rotor-Gene Q MDx 5plex HRM (QIAGEN) was used as a PCR apparatus, and in the PCR program thereof, first, the reaction solution was heated at 95°C for 5 minutes, then, a process including heating at 94°C for 10 seconds, at 65°C for 10 seconds and at 72°C for 30 seconds was repeated 35 times. For the primer, a combination of SEQ ID No. 1 and 2 was adopted. The product of this reaction solution was diluted 500-fold, and further, PCR was conducted with a formulation shown in Table 5 using this diluted product as a template. The reaction solution was heated at 95°C for 5 minutes, then, a process including heating at 94°C for 10 seconds, at 60°C for 10 seconds and at 72°C for 10 seconds was repeated 30 times, then, the DNA dissociation curve of the amplification product was made, and the Tm value of each amplification product was measured. PCR was carried out with the following seven sets:

a combination of a forward primer SEQ ID No. 1 and a reverse primer SEQ ID No. 16,
a combination of a forward primer SEQ ID No. 28 and a reverse primer SEQ ID No. 44,
a combination of a forward primer SEQ ID No. 51 and a reverse primer SEQ ID No. 52,
a combination of a forward primer SEQ ID No. 53 and a reverse primer SEQ ID No. 57,
a combination of a forward primer SEQ ID No. 62 and a reverse primer SEQ ID No. 70,
a combination of a forward primer SEQ ID No. 76 and a reverse primer SEQ ID No. 91 and
a combination of a forward primer SEQ ID No. 104 and a reverse primer SEQ ID No. 2.

Then, the presence or absence of amplification of each DNA sample was verified. The results are shown in Fig. 3 together with the result of Comparative Example 10.

(Comparative Example 10) DNA removal treatment (ultrafiltration, negative control of E. coli genome addition experiment)

Five hundred microliter of sterile water was added into Amicon UFC50508 (Millipore) and centrifugal separation was performed at 5000 g for 5 minutes, and, then, the filtrate and the solution remaining in the filter were removed. Next, 500 μL of 1 N hydrochloric acid was supplied and centrifugal separation was performed at 5000 g for 5 minutes, then remove the filtrate and the solution remaining in the filter. Finally, 500 μL of sterile water was supplied and centrifugal separation was performed at 5000 g for 5 minutes, and, then, the filtrate and the solution remaining in the filter were removed. Such an operation was repeated twice. Next, 100 μL of a mixed solution composed of components obtained after removal of the template and EvaGreen from a formulation of the reaction solution shown in Table 4 was prepared. The resultant solution was put into Amicon UFC50508 after the above operations and centrifugal separation was performed at 5000 g for 5 minutes. Then, the filtrate was recovered. EvaGreen and sterile water were added to the resultant filtrate and PCR was conducted. Rotor-Gene Q MDx 5plex HRM (QIAGEN) was used as a PCR apparatus, and in the PCR program thereof, first, the reaction solution was heated at 95°C for 5 minutes, then, a process including heating at 94°C for 10 seconds, at 65°C for 10 seconds and at 72°C for 30 seconds was repeated 35 times. For the primer, a combination of SEQ ID No. 1 and 2 was adopted. The product of this reaction solution was diluted 500-fold, and further, PCR was conducted with a formulation shown in Table 5 using this diluted product as a template. The reaction solution was heated at 95°C for 5 minutes, then, a process including heating at 94°C for 10 seconds, at 60°C for 10 seconds and at 72°C for 10 seconds was repeated 30 times, then, the DNA dissociation curve of the amplification product was made, and the Tm value of each amplification product was measured. PCR was carried out with the following seven sets:

a combination of a forward primer SEQ ID No. 1 and a reverse primer SEQ ID No. 16,
a combination of a forward primer SEQ ID No. 28 and a reverse primer SEQ ID No. 44,
a combination of a forward primer SEQ ID No. 51 and a reverse primer SEQ ID No. 52,
a combination of a forward primer SEQ ID No. 53 and a reverse primer SEQ ID No. 57,
a combination of a forward primer SEQ ID No. 62 and a reverse primer SEQ ID No. 70,
a combination of a forward primer SEQ ID No. 76 and a reverse primer SEQ ID No. 91 and
a combination of a forward primer SEQ ID No. 104 and a reverse primer SEQ ID No. 2.

Then, the presence or absence of amplification of each DNA sample was verified. The results are shown in Fig. 3 together with the result of Example 13.

(Example 14)DNA removal treatment (Purification of contaminated reagent, direct-PCR)

The instruments and ultrapure water used were free of DNA. Five hundred microliter of sterile water was added into Amicon UFC50508 (Millipore) and centrifugal separation was performed at 5000 g for 5 minutes, and, then, the filtrate and the solution remaining in the filter were removed. . Next, 500 μL of 1 N hydrochloric acid was supplied and centrifugal separation was performed at 5000 g for 5 minutes, and, then, the filtrate and the solution remaining in the filter were removed. Finally, 500 μL of sterile water was supplied and centrifugal separation was performed at 5000 g for 5 minutes, and, then, the filtrate and the solution remaining in the filter were removed. Such an operation was repeated twice. Next, 100 μL of a mixed solution composed of components obtained after removal of the template and EvaGreen from a formulation of the reaction solution shown in Table 4 was prepared. The resultant solution was put into Amicon UFC50508 after the above operations and centrifugal separation was performed at 5000 g for 5 minutes. Then, the filtrate was recovered. EvaGreen and sterile water were added to the resultant filtrate and PCR was conducted. Rotor-Gene Q MDx 5plex HRM (QIAGEN) was used as a PCR apparatus, and in the program of PCR, first, the reaction solution was heated at 95°C for 5 minutes, then, a process including heating at 94°C for 10 seconds, at 65°C for 10 seconds and at 72°C for 30 seconds was repeated 35 times. For the primer, a combination of SEQ ID No. 1 and 2 was adopted. The product of this reaction solution was diluted 500-fold, and further, PCR was conducted with a formulation shown in Table 5 using this diluted product as a template. Rotor-Gene Q MDx 5plex HRM (QIAGEN) was used as a PCR apparatus, and the reaction solution was heated at 95°C for 5 minutes, then, a process including heating at 94°C for 10 seconds, at 60°C for 10 seconds and at 72°C for 10 seconds was repeated 26 times, then, the DNA dissociation curve of the amplification product was made, and the Tm value of each amplification product was measured. PCR was carried out with the following seven sets:

(1) a combination of a forward primer SEQ ID No. 15 and a reverse primer SEQ ID No. 16,
(2) a combination of a forward primer SEQ ID No. 43 and a reverse primer SEQ ID No. 50,
(3) a combination of a forward primer SEQ ID No. 51 and a reverse primer SEQ ID No. 52,
(4) a combination of a forward primer SEQ ID No. 53 and a reverse primer SEQ ID No. 57,
(5) a combination of a forward primer SEQ ID No. 62 and a reverse primer SEQ ID No. 70,
(6) a combination of a forward primer SEQ ID No. 81 and a reverse primer SEQ ID No. 103 and
(7) a combination of a forward primer SEQ ID No. 115 and a reverse primer SEQ ID No. 5.

Then, the presence or absence of amplification of the intended fragment was verified. The results are shown in Fig. 4 together with the result of Comparative Example 11.

(Comparative Example 11) DNA removal treatment (Purification of contaminated reagent, direct-PCR)

The instruments and ultrapure water used were free of DNA. A solution having a reaction formulation shown in Table 4 was prepared, and PCR was conducted. Rotor-Gene Q MDx 5plex HRM (QIAGEN) was used as a PCR apparatus, and in the PCR program thereof, first, the reaction solution was heated at 95°C for 5 minutes, then, a process including heating at 94°C for 10 seconds, at 65°C for 10 seconds and at 72°C for 30 seconds was repeated 35 times. For the primer, a combination of SEQ ID No. 1 and 2 was adopted. The product of this reaction solution was diluted 500-fold, and further, PCR was conducted with a formulation shown in Table 5 using this diluted product as a template. Rotor-Gene Q MDx 5plex HRM (QIAGEN) was used as a PCR apparatus, and the reaction solution was heated at 95°C for 5 minutes, then, a process including heating at 94°C for 10 seconds, at 60°C for 10 seconds and at 72°C for 10 seconds was repeated 26 times, then, the DNA dissociation curve of the amplification product was made, and the Tm value of each amplification product was measured. PCR was carried out with the following seven sets:

(1) a combination of a forward primer SEQ ID No. 15 and a reverse primer SEQ ID No. 16,
(2) a combination of a forward primer SEQ ID No. 43 and a reverse primer SEQ ID No. 50,
(3) a combination of a forward primer SEQ ID No. 51 and a reverse primer SEQ ID No. 52,
(4) a combination of a forward primer SEQ ID No. 53 and a reverse primer SEQ ID No. 57,
(5) a combination of a forward primer SEQ ID No. 62 and a reverse primer SEQ ID No. 70,
(6) a combination of a forward primer SEQ ID No. 81 and a reverse primer SEQ ID No. 103 and
(7) a combination of a forward primer SEQ ID No. 115 and a reverse primer SEQ ID No. 5.

Then, the presence or absence of amplification of the intended fragment was verified. The results are shown in Fig. 4 together with the result of Example 14.

(Example 15)DNA removal treatment (gamma ray sterilization, E. coli DNA is treated with gamma ray)

The instruments and ultrapure water used were free of DNA. A solution was prepared by adding 500 fg of E. coli DNA to 100 $\mu$L of sterile water, and this was irradiated with a gamma ray of 10 kGy and 25 kGy. This solution was used as a template and PCR was conducted under the following conditions. The reaction was carried out with a formulation shown in Table 1. Rotor-Gene Q MDx 5plex HRM (QIAGEN) was used as a PCR apparatus, and in the program of PCR, first, the reaction solution was heated at 95°C for 5 minutes, then, a process including heating at 94°C for 10 seconds, at 65°C for 10 seconds and at 72°C for 30 seconds was repeated 40 times, then, the DNA dissociation curve was made. For the primer, a combination of SEQ ID No. 1 and 2 was adopted. The product of this reaction solution was diluted 500-fold, and further, this was used as a template and heated at 95°C for 5 minutes, then, a process including heating at 94°C for 10 seconds, at 60°C for 10 seconds and at 72°C for 10 seconds was repeated 30 times, then, the DNA dissociation curve of the amplification product was made. PCR was carried out with the following seven sets:

(1) a combination of a forward primer SEQ ID No. 1 and a reverse primer SEQ ID No. 16,

(2) a combination of a forward primer SEQ ID No. 28 and a reverse primer SEQ ID No. 44,
(3) a combination of a forward primer SEQ ID No. 51 and a reverse primer SEQ ID No. 52,
(4) a combination of a forward primer SEQ ID No. 53 and a reverse primer SEQ ID No. 57,
(5) a combination of a forward primer SEQ ID No. 62 and a reverse primer SEQ ID No. 70,
(6) a combination of a forward primer SEQ ID No. 76 and a reverse primer SEQ ID No. 91 and
(7) a combination of a forward primer SEQ ID No. 104 and a reverse primer SEQ ID No. 2..

Then, the presence or absence of amplification of each DNA sample was verified. The results are shown in Table 18.

[Table 18]

Table 18 gamma ray sterilization

| | Amplification of target product |
|---|---|
| No treatment | ○ |
| 10kGy | Δ |
| 25kGy | × |

○ : Amplification was observed.
Δ : Amplification was slightly observed.
×: No amplification was observed.

(Example 16)DNA removal treatment (EMA)

The instruments and ultrapure water used were free of DNA, and the operation was conducted in a clean bench. A solution was prepared by omitting the template, the primers and EvaGreen from the composition of the reaction liquid formulation of Table 5, but adding 25 ng of E, coli genomic DNA. Next, Ethidium Monoazide Bromid (Molecular Probe) was added to the resultant solution so that the final concentration of Ethidium Monoazide Bromid was 20 μM to provide a solution containing Ethidium Monoazide Bromid, while the above resultant solution without addition of Ethidium Monoazide Bromid was provided as the negative control. These solutions were placed under an LED bulb of 570 Im and irradiated with light for 15 minutes. Thereafter, primers and EvaGreen were added, and each mixture was heated at 95°C for 10 minutes, then, a process including heating at 95°C for 10 seconds, at 60°C for 30 seconds and at 72°C was repeated 60 times, then, the DNA dissociation curve of the amplification product was made. The presence or absence of amplification of the intended product was verified. For the primer, a forward primer SEQ ID No. 51 and a reverse primer SEQ ID No. 52 were adopted. The results are shown in Fig. 5.

(Example 17)Identification of bacterial species by four Tm values

A blind test was conducted by preparing the unidentified solution having no bacterial names of 4 bacterial species. Escherichia coli, Clostridium difficile, Klebsiella pneumoniae and Enterobacter cloacae were named as Bacterium No. 1 to No. 4, respectively and DNA was extracted from each bacterium to try to identify them. PCR reactions were carried out in the same manner as Example 11. The identification was made only on the basis of a combination of four Tm values among them. In view of the measurement error of Rotor-Gene Q, those in which Dist < 0.2 or less were regarded as homogeneous.

[Table 19]

Table 19 Discrimination of bacterial species by four Tm values

| Sample name | Name of bacterial species | Dist. |
|---|---|---|
| Bacterium No. 1 | E. coli | ≦0.18 |
| Bacterium No. 2 | C. difficile | ≦0.15 |

(continued)

| Sample name | Name of bacterial species | Dist. |
|---|---|---|
| Bacterium No. 3 | K. pneumoniae | ≦0.19 |
| Bacterium No. 4 | E. cloacae | ≦0.19 |

## Claims

1. A primer set for polymerase chain reaction (PCR) for amplification of bacterial DNA, which comprises one primer pair which consists of a combination of a forward primer consisting of a sequence of SEQ ID: No. 43 and a reverse primer consisting of any one sequence of SEQ ID: No. 44, 46 to 48 and 50;

2. A primer set according to claim 1, which further comprises at least one primer pair of seven primer pairs obtained by selecting one primer pair from each group of the S1 and S3 to S7 groups:

Group S1:

1-1A: a primer pair, which consists of a combination of a forward primer consisting of a sequence of SEQ ID: No. 1 and a reverse primer consisting of any one sequence of SEQ ID: No. 22 and 27;
1-2A: a primer pair, which consists of a combination of a forward primer consisting of a sequence of SEQ ID: No. 10 and a reverse primer consisting of any one sequence of SEQ ID: No. 20 and 21;
1-3A: a primer pair, which consists of a combination of a forward primer consisting of a sequence of SEQ ID: No. 11 and a reverse primer consisting of any one sequence of SEQ ID: No. 16, 18 and 26;
1-4; a primer pair, which consists of a combination of a forward primer consisting of a sequence of SEQ ID: No. 12 and a reverse primer consisting of any one sequence of SEQ ID: No. 16 to 27;
1-5: a primer pair, which consists of a combination of a forward primer consisting of a sequence of SEQ ID: No. 13 and a reverse primer consisting of any one sequence of SEQ ID: No. 16 to 27;
1-8A: a primer pair, which consists of a combination of a forward primer consisting of a sequence of SEQ ID: No. 6 and a reverse primer consisting of any one sequence of SEQ ID: No. 16, 19 to 21, 23, 24, 26 and 27;
1-9A: a primer pair, which consists of a combination of a forward primer consisting of a sequence of SEQ ID: No. 7 and a reverse primer consisting of any one sequence of SEQ ID: No. 16 to 21, 23, 24, 26 and 27; or
1-10A: a primer pair, which consists of a combination of a forward primer consisting of a sequence of SEQ ID: No. 8 and a reverse primer consisting of any one sequence of SEQ ID: No. 16 to 21, 23, 24, 26 and 27;
Group S3:
a primer pair, which consists of a combination of a forward primer consisting of a sequence of SEQ ID: No. 51 and a reverse primer consisting of a sequence of SEQ ID: No. 52;

Group S4:

4-1: a primer pair, which consists of a combination of a forward primer consisting of a sequence of SEQ ID: No. 53 and a reverse primer consisting of any one sequence of SEQ ID: No. 57 to 61;
4-2: a primer pair, which consists of a combination of a forward primer consisting of a sequence of SEQ ID: No. 54 and a reverse primer consisting of any one sequence of SEQ ID: No. 59 and 61;
4-3: a primer pair, which consists of a combination of a forward primer consisting of a sequence of SEQ ID: No. 55 and a reverse primer consisting of any one sequence of SEQ ID: No. 57 to 59 and 61; or
4-4: a primer pair, which consists of a combination of a forward primer consisting of a sequence of SEQ ID: No. 56 and a reverse primer consisting of any one sequence of SEQ ID: No. 57 to 59 and 61;

Group S5:

5-1A: a primer pair, which consists of a combination of a forward primer consisting of a sequence of SEQ ID: No. 62 and a reverse primer consisting of any one sequence of SEQ ID: No. 70, 73 to 75;
5-2: a primer pair, which consists of a combination of a forward primer consisting of a sequence of SEQ ID: No. 63 and a reverse primer consisting of any one sequence of SEQ ID: No. 70 to 75;
5-3: a primer pair, which consists of a combination of a forward primer consisting of a sequence of SEQ ID: No. 64 and a reverse primer consisting of any one sequence of SEQ ID: No. 70 to 75;
5-4: a primer pair, which consists of a combination of a forward primer consisting of a sequence of SEQ ID: No. 65 and a reverse primer consisting of any one sequence of SEQ ID: No. 70 to 75;
5-5A: a primer pair, which consists of a combination of a forward primer consisting of a sequence of SEQ ID: No. 66 and a reverse primer consisting of any one sequence of SEQ ID: No. 70, 72, 73 and 75;
5-6A: a primer pair, which consists of a combination of a forward primer consisting of a sequence of SEQ ID: No. 67 and a reverse primer consisting of any one sequence of SEQ ID: No. 70, 71 and 73 to 75;
5-7: a primer pair, which consists of a combination of a forward primer consisting of a sequence of SEQ ID: No. 68 and a reverse primer consisting of any one sequence of SEQ ID: No. 70 to 75; or

5-8: a primer pair, which consists of a combination of a forward primer consisting of a sequence of SEQ ID: No. 69 and a reverse primer consisting of any one sequence of SEQ ID: No. 70 to 75;

Group S6:

6-6: a primer pair, which consists of a combination of a forward primer consisting of a sequence of SEQ ID: No. 81 and a reverse primer consisting of any one sequence of SEQ ID: No. 91, 93 and 101 to 103;
6-1A: a primer pair, which consists of a combination of a forward primer consisting of a sequence of SEQ ID: No. 76 and a reverse primer consisting of any one sequence of SEQ ID: No. 93, 96 and 101 to 103;
6-2: a primer pair, which consists of a combination of a forward primer consisting of a sequence of SEQ ID: No. 77 and a reverse primer consisting of any one sequence of SEQ ID: No. 91, 96 and 101 to 103;
6-3: a primer pair, which consists of a combination of a forward primer consisting of a sequence of SEQ ID: No. 78 and a reverse primer consisting of any one sequence of SEQ ID: No. 91 and 101 to 103;
6-4A: a primer pair, which consists of a combination of a forward primer consisting of a sequence of SEQ ID: No. 79 and a reverse primer consisting of any one sequence of SEQ ID: No. 91 and 101 to 103;
6-5: a primer pair, which consists of a combination of a forward primer consisting of a sequence of SEQ ID: No. 80 and a reverse primer consisting of any one sequence of SEQ ID: No. 91, 93 and 100 to 103;
6-7: a primer pair, which consists of a combination of a forward primer consisting of a sequence of SEQ ID: No. 82 and a reverse primer consisting of any one sequence of SEQ ID: No. 91, 96, 97 and 101 to 103;
6-8: a primer pair, which consists of a combination of a forward primer consisting of a sequence of SEQ ID: No. 83 and a reverse primer consisting of any one sequence of SEQ ID: No. 91, 101 to 103;
6-9: a primer pair, which consists of a combination of a forward primer consisting of a sequence of SEQ ID: No. 84 and a reverse primer consisting of any one sequence of SEQ ID: No. 91 and 101 to 103;
6-10: a primer pair, which consists of a combination of a forward primer consisting of a sequence of SEQ ID: No. 85 and a reverse primer consisting of any one sequence of SEQ ID: No. 91 and 101 to 103;
6-11A: a primer pair, which consists of a combination of a forward primer consisting of a sequence of SEQ ID: No. 86 and a reverse primer consisting of any one sequence of SEQ ID: No. 91, 93 and 101 to 103;
6-12: a primer pair, which consists of a combination of a forward primer consisting of a sequence of SEQ ID: No. 87 and a reverse primer consisting of any one sequence of SEQ ID: Nos. 91, 96 and 101 to 103;
6-13: a primer pair, which consists of a combination of a forward primer consisting of a sequence of SEQ ID: No. 88 and a revers primer consisting of any one sequence of SEQ ID: No. 91 and 101 to 103;
6-14: a primer pair, which consists of a combination of a forward primer consisting of a sequence of SEQ ID: No. 89 and a reverse primer consisting of any one sequence of SEQ ID: No. 91 and 101 to 103; or
6-15: a primer pair, which consists of a combination of a forward primer consisting of a sequence of SEQ ID: No. 90 and a reverse primer consisting of any one sequence of SEQ ID: No. 91, 92, 96 and 101 to 103;

Group S7:

7-12A: a primer pair, which consists of a combination of a forward primer consisting of a sequence of SEQ ID: No. 115 and a reverse primer consisting of any one sequence of SEQ ID: No. 2 and 5;
7-1A: a primer pair, which consists of a combination of a forward primer consisting of a sequence of SEQ ID: No. 104 and a reverse primer consisting of any one sequence of SEQ ID: No. 5 and 118 to 125, 127, 128, 130 and 131;
7-2A: a primer pair, which consists of a combination of a forward primer consisting of a sequence of SEQ ID: No. 105 and a reverse primer consisting of any one sequence of SEQ ID: No. 5 and 118 to 125;
7-3A: a primer pair, which consists of a combination of a forward primer consisting of a sequence of SEQ ID: No. 106 and a reverse primer consisting of any one sequence of SEQ ID: No. 2, 5 and 116 and 118 to 131;
7-4A: a primer pair, which consists of a combination of a forward primer consisting of a sequence of SEQ ID: No. 107 and a reverse primer consisting of any one sequence of SEQ ID: No. 120 and 121;
7-5A: a primer pair, which consists of a combination of a forward primer consisting of a sequence of SEQ ID: No. 108 and a reverse primer consisting of any one sequence of SEQ ID: No. 120 and 123;
7-6A: a primer pair, which consists of a combination of a forward primer consisting of a sequence of SEQ ID: No. 109 and a reverse primer consisting of any one sequence of SEQ ID: No. 2, 118 to 122, 124 and 126;
7-7A: a primer pair, which consists of a combination of a forward primer consisting of a sequence of SEQ ID: No. 110 and a reverse primer consisting of any one sequence of SEQ ID: No. 118, 120, 121, 124 and 126 to 130;
7-8A: a primer pair, which consists of a combination of a forward primer consisting of a sequence of SEQ ID: No. 111 and a reverse primer consisting of any one sequence of SEQ ID: No. 2, 5 and 118 to 131;

7-9A: a primer pair, which consists of a combination of a forward primer consisting of a sequence of SEQ ID: No. 112 and a reverse primer consisting of any one sequence of SEQ ID: No. 2, 5 and 116, and 118 to 125;
7-10A: a primer pair, which consists of a combination of a forward primer consisting of a sequence of SEQ ID: No. 113 and a reverse primer consisting of any one sequence of SEQ ID: No. 2, 5, 118 to 126, 130 and 131; or
7-11A: a primer pair, which consists of a combination of a forward primer consisting of a sequence of SEQ ID: No. 114 and a reverse primer consisting of a sequence of SEQ ID: No. 5;

**3.** A kit for detection of a bacterial species, which comprises one primer pair which consists of a combination of a forward primer consisting of a sequence of SEQ ID: No. 43 and a reverse primer consisting of any one sequence of SEQ ID: No. 44 to 48 and 50.

**4.** A kit according to claim 3, which further comprises at least one primer pair of seven primer pairs obtained by selecting one primer pair from each group of the K1 and K3 to K7 groups:

Group K1:

1-1: a primer pair, which consists of a combination of a forward primer consisting of a sequence of SEQ ID: No. 1 and a reverse primer consisting of any one sequence of SEQ ID: No. 16 to 27;
1-2: a primer pair, which consists of a combination of a forward primer consisting of a sequence of SEQ ID: No. 10 and a reverse primer consisting of any one sequence of SEQ ID: No. 16, 18, 20 and 21;
1-3: a primer pair, which consists of a combination of a forward primer consisting of a sequence of SEQ ID: No. 11 and a reverse primer consisting of any one sequence of SEQ ID: No. 16 to 21 and 23 to 26;
1-4: a primer pair, which consists of a combination of a forward primer consisting of a sequence of SEQ ID: No. 12 and a reverse primer consisting of any one sequence of SEQ ID: No. 16 to 27;
1-5: a primer pair, which consists of a combination of a forward primer consisting of a sequence of SEQ ID: No. 13 and a reverse primer consisting of any one sequence of SEQ ID: No. 16 to 27;
1-6: a primer pair, which consists of a combination of a forward primer consisting of a sequence of SEQ ID: No. 14 and a reverse primer consisting of any one sequence of SEQ ID: No. 16 to 27;
1-8: a primer pair, which consists of a combination of a forward primer consisting of a sequence of SEQ ID: No. 6 and a reverse primer consisting of any one sequence of SEQ ID: No. 16 to 27;
1-9: a primer pair, which consists of a combination of a forward primer consisting of a sequence of SEQ ID: No. 7 and a reverse primer consisting of any one sequence of SEQ ID: No. 16 to 27;
1-10: a primer pair, which consists of a combination of a forward primer consisting of a sequence of SEQ ID: No. 8 and a reverse primer consisting of any one sequence of SEQ ID: No. 16 to 27; or
1-11: a primer pair, which consists of a combination of a forward primer consisting of a sequence of SEQ ID: No. 9 and a reverse primer consisting of any one sequence of SEQ ID: No. 16, 17, 19 and 26;

Group K3:
a primer pair, which consists of a combination of a forward primer consisting of a sequence of SEQ ID: No. 51 and a reverse primer consisting of a sequence of SEQ ID: No. 52;
Group K4:

4-1: a primer pair, which consists of a combination of a forward primer consisting of a sequence of SEQ ID: No. 53 and a reverse primer consisting of any one sequence of SEQ ID: No. 57 to 61;
4-2: a primer pair, which consists of a combination of a forward primer consisting of a sequence of SEQ ID: No. 54 and a reverse primer consisting of any one sequence of SEQ ID: No. 59 and 61;
4-3: a primer pair, which consists of a combination of a forward primer consisting of a sequence of SEQ ID: No. 55 and a reverse primer consisting of any one sequence of SEQ ID: No. 57 to 59 and 61; or
4-4: a primer pair, which consists of a combination of a forward primer consisting of a sequence of SEQ ID: No. 56 and a reverse primer consisting of any one sequence of SEQ ID: No. 57 to 59 and 61;

Group K5:

5-1: a primer pair, which consists of a combination of a forward primer consisting of a sequence of SEQ ID: No. 62 and a reverse primer consisting of any one sequence of SEQ ID: No. 70, 71 and 73 to 75;
5-2: a primer pair, which consists of a combination of a forward primer consisting of a sequence of SEQ ID: No. 63 and a reverse primer consisting of any one sequence of SEQ ID: No. 70 to 75;

5-3: a primer pair, which consists of a combination of a forward primer consisting of a sequence of SEQ ID: No. 64 and a reverse primer consisting of any one sequence of SEQ ID: No. 70 to 75;
5-4: a primer pair, which consists of a combination of a forward primer consisting of a sequence of SEQ ID: No. 65 and a reverse primer consisting of any one sequence of SEQ ID: No. 70 to 75;
5-5: a primer pair, which consists of a combination of a forward primer consisting of a sequence of SEQ ID: No. 66 and a reverse primer consisting of any one sequence of SEQ ID: No. 70 to 75;
5-6: a primer pair, which consists of a combination of a forward primer consisting of a sequence of SEQ ID: No. 67 and a reverse primer consisting of any one sequence of SEQ ID: No. 70 to 75;
5-7: a primer pair, which consists of a combination of a forward primer consisting of a sequence of SEQ ID: No. 68 and a reverse primer consisting of any one sequence of SEQ ID: No. 70 to 75; or
5-8: a primer pair, which consists of a combination of a forward primer consisting of a sequence of SEQ ID: No. 69 and a reverse primer consisting of any one sequence of SEQ ID: No. 70 to 75;

Group K6:

6-6: a primer pair, which consists of a combination of a forward primer consisting of a sequence of SEQ ID: No. 81 and a reverse primer consisting of any one sequence of SEQ ID: No. 91, 93 and 101 to 103;
6-1: a primer pair, which consists of a combination of a forward primer consisting of a sequence of SEQ ID: No. 76 and a reverse primer consisting of any one sequence of SEQ ID: No. 91, 93, 96 and 101 to 103;
6-2: a primer pair, which consists of a combination of a forward primer consisting of a sequence of SEQ ID: No. 77 and a reverse primer consisting of any one sequence of SEQ ID: No. 91, 96 and 101 to 103;
6-3: a primer pair, which consists of a combination of a forward primer consisting of a sequence of SEQ ID: No. 78 and a reverse primer consisting of any one sequence of SEQ ID: No. 91 and 101 to 103;
6-4: a primer pair, which consists of a combination of a forward primer consisting of a sequence of SEQ ID: No. 79 and a reverse primer consisting of any one sequence of SEQ ID: No. 91, 97 and 101 to 103;
6-5: a primer pair, which consists of a combination of a forward primer consisting of a sequence of SEQ ID: No. 80 and a reverse primer consisting of any one sequence of SEQ ID: No. 91, 93 and 100 to 103;
6-7: a primer pair, which consists of a combination of a forward primer consisting of a sequence of SEQ ID: No. 82 and a reverse primer consisting of any one sequence of SEQ ID: No. 91, 96, 97 and 101 to 103;
6-8: a primer pair, which consists of a combination of a forward primer consisting of a sequence of SEQ ID: No. 83 and a reverse primer consisting of any one sequence of SEQ ID: No. 91, 101 to 103;
6-9: a primer pair, which consists of a combination of a forward primer consisting of a sequence of SEQ ID: No. 84 and a reverse primer consisting of any one sequence of SEQ ID: No. 91 and 101 to 103;
6-10: a primer pair, which consists of a combination of a forward primer consisting of a sequence of SEQ ID: No. 85 and a reverse primer consisting of any one sequence of SEQ ID: No. 91 and 101 to 103;
6-11: a primer pair, which consists of a combination of a forward primer consisting of a sequence of SEQ ID: No. 86 and a reverse primer consisting of any one sequence of SEQ ID: No. 91, 93, 97 and 101 to 103;
6-12: a primer pair, which consists of a combination of a forward primer consisting of a sequence of SEQ ID: No. 87 and a reverse primer consisting of any one sequence of SEQ ID: No. 91, 96 and 101 to 103;
6-13: a primer pair, which consists of a combination of a forward primer consisting of a sequence of SEQ ID: No. 88 and a reverse primer consisting of any one sequence of SEQ ID: No. 91 and 101 to 103;
6-14: a primer pair, which consists of a combination of a forward primer consisting of a sequence of SEQ ID: No. 89 and a reverse primer consisting of any one sequence of SEQ ID: No. 91 and 101 to 103; or
6-15: a primer pair, which consists of a combination of a forward primer consisting of a sequence of SEQ ID: No. 90 and a reverse primer consisting of any one sequence of SEQ ID: No. 91, 92, 96 and 101 to 103;

Group K7:

7-12: a primer pair, which consists of a combination of a forward primer consisting of a sequence of SEQ ID: No. 115 and a reverse primer consisting of any one sequence of SEQ ID: No. 2, 5, 116, 118, 123 and 125 to 128;
7-1: a primer pair, which consists of a combination of a forward primer consisting of a sequence of SEQ ID: No. 104 and a reverse primer consisting of any one sequence of SEQ ID: No. 2, 5 and 116 to 131;
7-2: a primer pair, which consists of a combination of a forward primer consisting of a sequence of SEQ ID: No. 105 and a reverse primer consisting of any one sequence of SEQ ID: No. 2, 5 and 116 to 131;
7-3: a primer pair, which consists of a combination of a forward primer consisting of a sequence of SEQ ID: No. 106 and a reverse primer consisting of any one sequence of SEQ ID: No. 2, 5 and 116 to 131;
7-4: a primer pair, which consists of a combination of a forward primer consisting of a sequence of SEQ ID: No. 107 and a reverse primer consisting of any one sequence of SEQ ID: No. 2, 5 and 116 to 131;
7-5: a primer pair, which consists of a combination of a forward primer consisting of a sequence of SEQ ID: No. 108 and a reverse primer consisting of any one sequence of SEQ ID: No. 2, 5 and 116 to 131;
7-6: a primer pair, which consists of a combination of a forward primer consisting of a sequence of SEQ ID: No. 109 and a reverse primer consisting of any one sequence of SEQ ID: No. 2, 5 and 116 to 131;
7-7: a primer pair, which consists of a combination of a forward primer consisting of a sequence of SEQ ID: No. 110 and a reverse primer consisting of any one sequence of SEQ ID: No. 2, 5 and 116 to 131;
7-8: a primer pair, which consists of a combination of a forward primer consisting of a sequence of SEQ ID: No. 111 and a reverse primer consisting of any one sequence of SEQ ID: No. 2, 5 and 116 to 131;
7-9: a primer pair, which consists of a combination of a forward primer consisting of a sequence of SEQ ID: No. 112 and a reverse primer consisting of any one sequence of SEQ ID: No. 2, 5 and 116 to 131;
7-10: a primer pair, which consists of a combination of a forward primer consisting of a sequence of SEQ ID: No. 113 and a reverse primer consisting of any one sequence of SEQ ID: No. 2, 5 and 116 to 131; or
7-11: a primer pair, which consists of a combination of a forward primer consisting of a sequence of SEQ ID: No. 114 and a reverse primer consisting of any one sequence of SEQ ID: No. 2, 5 and 125;

**5.** A primer set according to claim 2 or a kit according to claim 4, which comprises at least one primer pair of the following seven primer pairs 1) to 7):

1) a primer pair, which consists of a combination of a forward primer consisting of a sequence of SEQ ID: No. 15 and a reverse primer consisting of a sequence of SEQ ID: No. 16;
2) a primer pair, which consists of a combination of a forward primer consisting of a sequence of SEQ ID: No. 43 and a reverse primer consisting of a sequence of SEQ ID: No. 50;
3) a primer pair, which consists of a combination of a forward primer consisting of a sequence of SEQ ID: No. 81 and a reverse primer consisting of a sequence of SEQ ID: No. 103;
4) a primer pair, which consists of a combination of a forward primer consisting of a sequence of SEQ ID: No. 115 and a reverse primer consisting of a sequence of SEQ ID: No. 5;
5) a primer pair, which consists of a combination of a forward primer consisting of a sequence of SEQ ID: No. 51 and a reverse primer consisting of a sequence of SEQ ID: No. 52;
6) a primer pair, which consists of a combination of a forward primer consisting of a sequence of SEQ ID: No. 53 and a reverse primer consisting of a sequence of SEQ ID: No. 57; and
7) a primer pair, which consists of a combination of a forward primer consisting of a sequence of SEQ ID: No. 62 and a reverse primer consisting of a sequence of SEQ ID: No. 70.

**6.** A primer set or a kit according to claim 5, comprising any one of the primer pairs 1) to 7).

**7.** A primer set or a kit according to claim 6, comprising any one of the following combinations A to G:

(A) when the primer set comprises primer pair 1), the primer set further comprises at least 3 primer pairs selected from 2) to 7);
(B) when the primer set comprises primer pair 2), the primer set further comprises at least 3 primer pairs selected from 1) and 3) to 7);
(C) when the primer set comprises primer pair 3), the primer set further comprises at least 3 primer pairs selected from 1), 2) and 4) to 7);
(D) when the primer set comprises primer pair 4), the primer set further comprises at least 3 primer pairs selected from 1) to 3) and 5) to 7);
(E) when the primer set comprises primer pair 5), the primer set further comprises at least 3 primer pairs selected from 1) to 4) and 6) to 7);
(F) when the primer set comprises primer pair 6), the primer set further comprises at least 3 primer pairs selected from 1) to 5) and 7);
(G) when the primer set comprises primer pair 7), the primer set further comprises at least 3 primer pairs selected from 1) to 6).

**8.** A primer set according to any one of claims 1, 2 or 5 to 7 for detection and/or identification of a bacterial species.

**9.** A kit according to any one of claims 3 to 7 for identification of a bacterial species.

**10.** A primer set according to any one of claims 2, or 5 to 8 or a kit according to any one of claims 4, 5 to 7, or 9, wherein the primer set or kit comprises at least 4 primer pairs of the seven primer pairs.

**11.** A kit according to any one of claims 3 to 7, or 9, which further comprises an additional primer pair of a forward primer consisting of a sequence of SEQ ID: No. 1 and a reverse primer consisting of any one sequence of SEQ ID: No. 2 to 5, such as wherein the additional primer pair consists of a forward primer consisting of a sequence of SEQ ID No. 1 and a reverse primer consisting of a sequence of SEQ ID No. 5.

**12.** A kit according to any one of claims 3 to 7, or 9, which further comprises an enzyme for PCR.

**13.** A kit according to claim 12, wherein

(i) the primers and the enzyme for PCR have, independently or as a whole, a contamination amount of bacterial nucleic acid of 10 fg or less than 10 fg; or
(ii) which further comprises at least one of a pH buffer, dNTP, $MgCl_2$ and a vessel for measurement, and preferably the pH buffer, dNTP, $MgCl_2$ and the vessel for measurement have, independently or as a whole, a contamination amount of bacterial nucleic acid of 10 fg or less than 10 fg, and more preferably the primers, the enzyme for PCR, the pH buffer, dNTP, $MgCl_2$ and the vessel for measurement are individually packaged and the contamination amount of bacterial nucleic acid in each of the packages is 10 fg or less than 10 fg.

**14.** A kit according to claim 12 or 13, part (i), which further comprises fluorochrome, and preferably the primer, the enzyme for PCR, the pH buffer, dNTP, the fluorochrome, $MgCl_2$ and the vessel for measurement have, independently or as a whole, a contamination amount of bacterial nucleic acid of 10 fg or less than 10 fg, and more preferably the primer, the enzyme for PCR, the pH buffer, dNTP, the fluorochrome, $MgCl_2$ and the vessel for measurement are individually packaged and the contamination amount of bacterial nucleic acid in each of the packages is 10 fg or less than 10 fg.

**15.** A kit according to claims 13 or 14, wherein dNTP is chemically modified for hot start PCR.

**16.** A kit according to any one of claims 12 to 15, wherein the enzyme for PCR is a Taq DNA Polymerase having an amount of bacterial DNA of less than 10 fg/μl.

**17.** A method of detection and/or identification of a bacterial species as a target in a sample, comprises

a step of conducting PCR using bacterial genome DNA prepared from the sample, primers to obtain amplification product comprising a target gene specific to the bacterial species as a target, a thermostable DNA polymerase, and
a step of detecting and/or identifying the bacterial species as a target in the sample by detecting the target gene in the amplification product by PCR, or analyzing the amplification product by PCR,
wherein, as the primers, the primer set according to any one of claims 1, 2, and 5 to 8, or the kit according to any one of claims 3 to 7, and 9 to 16 is used.

**18.** A method of identification of a bacterial species according to claim 17, wherein a kit according to any one of claims 3 to 7, 9 to 16 is used.

**Patentansprüche**

**1.** Primer-Satz für eine Polymerasekettenreaktion (PCR) zur Amplifikation von bakterieller DNA, der ein Primer-Paar umfasst, bestehend aus einer Kombination von einem Vorwärts-Primer, der aus einer Sequenz aus SEQ ID NO: 43 besteht, und einem Rückwärts-Primer, der aus einer beliebigen der Sequenzen aus SEQ ID NO: 44, 46 bis 48 und 50 besteht.

**2.** Primer-Satz nach Anspruch 1, der ferner zumindest ein Primer-Paar aus sieben Primer-Paaren umfasst, das durch Auswählen eines Primer-Paars aus jeder Gruppe der Gruppen S1 und S3 bis S7 erhalten wird:

Gruppe S1:

1-1A: Primer-Paar, bestehend aus einer Kombination von einem Vorwärts-Primer, der aus einer Sequenz aus SEQ ID NO: 1 besteht, und einem Rückwärts-Primer, der aus einer beliebigen der Sequenzen aus SEQ ID NO: 22 und 27 besteht;

1-2A: Primer-Paar, bestehend aus einer Kombination von einem Vorwärts-Primer, der aus einer Sequenz aus SEQ ID NO: 10 besteht, und einem Rückwärts-Primer, der aus einer beliebigen der Sequenzen aus SEQ ID NO: 20 und 21 besteht;

1-3A: Primer-Paar, bestehend aus einer Kombination von einem Vorwärts-Primer, der aus einer Sequenz aus SEQ ID NO: 11 besteht, und einem Rückwärts-Primer, der aus einer beliebigen der Sequenzen aus SEQ ID NO: 16, 18 und 26 besteht;

1-4: Primer-Paar, bestehend aus einer Kombination von einem Vorwärts-Primer, der aus einer Sequenz aus SEQ ID NO: 12 besteht, und einem Rückwärts-Primer, der aus einer beliebigen der Sequenzen aus SEQ ID NO: 16 bis 27 besteht;

1-5: Primer-Paar, bestehend aus einer Kombination von einem Vorwärts-Primer, der aus einer Sequenz aus SEQ ID NO: 13 besteht, und einem Rückwärts-Primer, der aus einer beliebigen der Sequenzen aus SEQ ID NO: 16 bis 27 besteht;

1-8A: Primer-Paar, bestehend aus einer Kombination von einem Vorwärts-Primer, der aus einer Sequenz aus SEQ ID NO: 6 besteht, und einem Rückwärts-Primer, der aus einer beliebigen der Sequenzen aus SEQ ID NO: 16, 19 bis 21, 23, 24, 26 und 27 besteht;

1-9A: Primer-Paar, bestehend aus einer Kombination von einem Vorwärts-Primer, der aus einer Sequenz aus SEQ ID NO: 7 besteht, und einem Rückwärts-Primer, der aus einer beliebigen der Sequenzen aus SEQ ID NO: 16 bis 21, 23, 24, 26 und 27 besteht; oder

1-10A: Primer-Paar, bestehend aus einer Kombination von einem Vorwärts-Primer, der aus einer Sequenz aus SEQ ID NO: 8 besteht, und einem Rückwärts-Primer, der aus einer beliebigen der Sequenzen aus SEQ ID NO: 16 bis 21, 23, 24, 26 und 27 besteht;

Gruppe S3:

Primer-Paar, bestehend aus einer Kombination von einem Vorwärts-Primer, der aus einer Sequenz aus SEQ ID NO: 51 besteht, und einem Rückwärts-Primer, der aus einer Sequenz aus SEQ ID NO: 52 besteht;

Gruppe S4:

4-1: Primer-Paar, bestehend aus einer Kombination von einem Vorwärts-Primer, der aus einer Sequenz aus SEQ ID NO: 53 besteht, und einem Rückwärts-Primer, der aus einer beliebigen der Sequenzen aus SEQ ID NO: 57 bis 61 besteht;

4-2: Primer-Paar, bestehend aus einer Kombination von einem Vorwärts-Primer, der aus einer Sequenz aus SEQ ID NO: 54 besteht, und einem Rückwärts-Primer, der aus einer beliebigen der Sequenzen aus SEQ ID NO: 59 und 61 besteht;

4-3: Primer-Paar, bestehend aus einer Kombination von einem Vorwärts-Primer, der aus einer Sequenz aus SEQ ID NO: 55 besteht, und einem Rückwärts-Primer, der aus einer beliebigen der Sequenzen aus SEQ ID NO: 57 bis 59 und 61 besteht; oder

4-4: Primer-Paar, bestehend aus einer Kombination von einem Vorwärts-Primer, der aus einer Sequenz aus SEQ ID NO: 56 besteht, und einem Rückwärts-Primer, der aus einer beliebigen der Sequenzen aus SEQ ID NO: 57 bis 59 und 61 besteht;

Gruppe S5:

5-1A: Primer-Paar, bestehend aus einer Kombination von einem Vorwärts-Primer, der aus einer Sequenz aus SEQ ID NO: 62 besteht, und einem Rückwärts-Primer, der aus einer beliebigen der Sequenzen aus SEQ ID NO: 70, 73 bis 75 besteht;

5-2: Primer-Paar, bestehend aus einer Kombination von einem Vorwärts-Primer, der aus einer Sequenz aus SEQ ID NO: 63 besteht, und einem Rückwärts-Primer, der aus einer beliebigen der Sequenzen aus SEQ ID NO: 70 bis 75 besteht;

5-3: Primer-Paar, bestehend aus einer Kombination von einem Vorwärts-Primer, der aus einer Sequenz aus SEQ ID NO: 64 besteht, und einem Rückwärts-Primer, der aus einer beliebigen der Sequenzen aus SEQ ID NO: 70 bis 75 besteht;

5-4: Primer-Paar, bestehend aus einer Kombination von einem Vorwärts-Primer, der aus einer Sequenz aus SEQ ID NO: 65 besteht, und einem Rückwärts-Primer, der aus einer beliebigen der Sequenzen aus SEQ ID NO: 70 bis 75 besteht;

5-5A: Primer-Paar, bestehend aus einer Kombination von einem Vorwärts-Primer, der aus einer Sequenz aus SEQ ID NO: 66 besteht, und einem Rückwärts-Primer, der aus einer beliebigen der Sequenzen aus SEQ ID NO: 70, 72, 73 und 75 besteht;

5-6A: Primer-Paar, bestehend aus einer Kombination von einem Vorwärts-Primer, der aus einer Sequenz aus SEQ ID NO: 67 besteht, und einem Rückwärts-Primer, der aus einer beliebigen der Sequenzen aus SEQ ID NO: 70, 71 und 73 bis 75 besteht;

5-7: Primer-Paar, bestehend aus einer Kombination von einem Vorwärts-Primer, der aus einer Sequenz aus SEQ ID NO: 68 besteht, und einem Rückwärts-Primer, der aus einer beliebigen der Sequenzen aus SEQ ID NO: 70 bis 75 besteht; oder

5-8: Primer-Paar, bestehend aus einer Kombination von einem Vorwärts-Primer, der aus einer Sequenz aus SEQ ID NO: 69 besteht, und einem Rückwärts-Primer, der aus einer beliebigen der Sequenzen aus SEQ ID NO: 70 bis 75 besteht;

Gruppe S6:

6-6: Primer-Paar, bestehend aus einer Kombination von einem Vorwärts-Primer, der aus einer Sequenz aus SEQ ID NO: 81 besteht, und einem Rückwärts-Primer, der aus einer beliebigen der Sequenzen aus SEQ ID NO: 91, 93 und 101 bis 103 besteht;

6-1A: Primer-Paar, bestehend aus einer Kombination von einem Vorwärts-Primer, der aus einer Sequenz aus SEQ ID NO: 76 besteht, und einem Rückwärts-Primer, der aus einer beliebigen der Sequenzen aus SEQ ID NO: 93, 96 und 101 bis 103 besteht;

6-2: Primer-Paar, bestehend aus einer Kombination von einem Vorwärts-Primer, der aus einer Sequenz aus SEQ ID NO: 77 besteht, und einem Rückwärts-Primer, der aus einer beliebigen der Sequenzen aus SEQ ID NO: 91, 96 und 101 bis 103 besteht;

6-3: Primer-Paar, bestehend aus einer Kombination von einem Vorwärts-Primer, der aus einer Sequenz aus SEQ ID NO: 78 besteht, und einem Rückwärts-Primer, der aus einer beliebigen der Sequenzen aus SEQ ID NO: 91 und 101 bis 103 besteht;

6-4A: Primer-Paar, bestehend aus einer Kombination von einem Vorwärts-Primer, der aus einer Sequenz aus SEQ ID NO: 79 besteht, und einem Rückwärts-Primer, der aus einer beliebigen der Sequenzen aus SEQ ID NO: 91 und 101 bis 103 besteht;

6-5: Primer-Paar, bestehend aus einer Kombination von einem Vorwärts-Primer, der aus einer Sequenz aus SEQ ID NO: 80 besteht, und einem Rückwärts-Primer, der aus einer beliebigen der Sequenzen aus SEQ ID NO: 91, 93 und 100 bis 103 besteht;

6-7: Primer-Paar, bestehend aus einer Kombination von einem Vorwärts-Primer, der aus einer Sequenz aus SEQ ID NO: 82 besteht, und einem Rückwärts-Primer, der aus einer beliebigen der Sequenzen aus SEQ ID NO: 91, 96, 97 und 101 bis 103 besteht;

6-8: Primer-Paar, bestehend aus einer Kombination von einem Vorwärts-Primer, der aus einer Sequenz aus SEQ ID NO: 83 besteht, und einem Rückwärts-Primer, der aus einer beliebigen der Sequenzen aus SEQ ID NO: 91, 101 bis 103 besteht;

6-9: Primer-Paar, bestehend aus einer Kombination von einem Vorwärts-Primer, der aus einer Sequenz aus SEQ ID NO: 84 besteht, und einem Rückwärts-Primer, der aus einer beliebigen der Sequenzen aus SEQ ID NO: 91 und 101 bis 103 besteht;

6-10: Primer-Paar, bestehend aus einer Kombination von einem Vorwärts-Primer, der aus einer Sequenz aus SEQ ID NO: 85 besteht, und einem Rückwärts-Primer, der aus einer beliebigen der Sequenzen aus SEQ ID NO: 91 und 101 bis 103 besteht;

6-11A: Primer-Paar, bestehend aus einer Kombination von einem Vorwärts-Primer, der aus einer Sequenz aus SEQ ID NO: 86 besteht, und einem Rückwärts-Primer, der aus einer beliebigen der Sequenzen aus SEQ ID NO: 91, 93 und 101 bis 103 besteht;

6-12: Primer-Paar, bestehend aus einer Kombination von einem Vorwärts-Primer, der aus einer Sequenz aus SEQ ID NO: 87 besteht, und einem Rückwärts-Primer, der aus einer beliebigen der Sequenzen aus SEQ ID NO: 91, 96 und 101 bis 103 besteht;

6-13: Primer-Paar, bestehend aus einer Kombination von einem Vorwärts-Primer, der aus einer Sequenz aus SEQ ID NO: 88 besteht, und einem Rückwärts-Primer, der aus einer beliebigen der Sequenzen aus SEQ ID NO: 91 und 101 bis 103 besteht;

6-14: Primer-Paar, bestehend aus einer Kombination von einem Vorwärts-Primer, der aus einer Sequenz aus SEQ ID NO: 89 besteht, und einem Rückwärts-Primer, der aus einer beliebigen der Sequenzen aus SEQ ID NO: 91 und 101 bis 103 besteht; oder

6-15: Primer-Paar, bestehend aus einer Kombination von einem Vorwärts-Primer, der aus einer Sequenz aus SEQ ID NO: 90 besteht, und einem Rückwärts-Primer, der aus einer beliebigen der Sequenzen aus SEQ ID NO: 91, 92, 96 und 101 bis 103 besteht;

Gruppe S7:

7-12A: Primer-Paar, bestehend aus einer Kombination von einem Vorwärts-Primer, der aus einer Sequenz aus SEQ ID NO: 115 besteht, und einem Rückwärts-Primer, der aus einer beliebigen der Sequenzen aus SEQ ID NO: 2 und 5 besteht;
7-1A: Primer-Paar, bestehend aus einer Kombination von einem Vorwärts-Primer, der aus einer Sequenz aus SEQ ID NO: 104 besteht, und einem Rückwärts-Primer, der aus einer beliebigen der Sequenzen aus SEQ ID NO: 5 und 118 bis 125, 127, 128, 130 und 131 besteht;
7-2A: Primer-Paar, bestehend aus einer Kombination von einem Vorwärts-Primer, der aus einer Sequenz aus SEQ ID NO: 105 besteht, und einem Rückwärts-Primer, der aus einer beliebigen der Sequenzen aus SEQ ID NO: 5 und 118 bis 125 besteht;
7-3A: Primer-Paar, bestehend aus einer Kombination von einem Vorwärts-Primer, der aus einer Sequenz aus SEQ ID NO: 106 besteht, und einem Rückwärts-Primer, der aus einer beliebigen der Sequenzen aus SEQ ID NO: 2, 5 und 116 und 118 bis 131 besteht;
7-4A: Primer-Paar, bestehend aus einer Kombination von einem Vorwärts-Primer, der aus einer Sequenz aus SEQ ID NO: 107 besteht, und einem Rückwärts-Primer, der aus einer beliebigen der Sequenzen aus SEQ ID NO: 120 und 121 besteht;
7-5A: Primer-Paar, bestehend aus einer Kombination von einem Vorwärts-Primer, der aus einer Sequenz aus SEQ ID NO: 108 besteht, und einem Rückwärts-Primer, der aus einer beliebigen der Sequenzen aus SEQ ID NO: 120 und 123 besteht;
7-6A: Primer-Paar, bestehend aus einer Kombination von einem Vorwärts-Primer, der aus einer Sequenz aus SEQ ID NO: 109 besteht, und einem Rückwärts-Primer, der aus einer beliebigen der Sequenzen aus SEQ ID NO: 2, 118 bis 122, 124 und 126 besteht;
7-7A: Primer-Paar, bestehend aus einer Kombination von einem Vorwärts-Primer, der aus einer Sequenz aus SEQ ID NO: 110 besteht, und einem Rückwärts-Primer, der aus einer beliebigen der Sequenzen aus SEQ ID NO: 118, 120, 121, 124 und 126 bis 130 besteht;
7-8A: Primer-Paar, bestehend aus einer Kombination von einem Vorwärts-Primer, der aus einer Sequenz aus SEQ ID NO: 111 besteht, und einem Rückwärts-Primer, der aus einer beliebigen der Sequenzen aus SEQ ID NO: 2, 5 und 118 bis 131 besteht;
7-9A: Primer-Paar, bestehend aus einer Kombination von einem Vorwärts-Primer, der aus einer Sequenz aus SEQ ID NO: 112 besteht, und einem Rückwärts-Primer, der aus einer beliebigen der Sequenzen aus SEQ ID NO: 2, 5 und 116 und 118 bis 125 besteht;
7-10A: Primer-Paar, bestehend aus einer Kombination von einem Vorwärts-Primer, der aus einer Sequenz aus SEQ ID NO: 113 besteht, und einem Rückwärts-Primer, der aus einer beliebigen der Sequenzen aus SEQ ID NO: 2, 5, 118 bis 126, 130 und 131 besteht; oder
7-11A: Primer-Paar, bestehend aus einer Kombination von einem Vorwärts-Primer, der aus einer Sequenz aus SEQ ID NO: 114 besteht, und einem Rückwärts-Primer, der aus einer Sequenz aus SEQ ID NO: 5 besteht.

**3.** Set zur Detektion einer Bakterienspezies, das ein Primer-Paar umfasst, bestehend aus einer Kombination von einem Vorwärts-Primer, der aus einer Sequenz aus SEQ ID NO: 43 besteht, und einem Rückwärts-Primer, der aus einer beliebigen der Sequenzen aus SEQ ID NO: 44 bis 48 und 50 besteht.

**4.** Set nach Anspruch 3, das außerdem zumindest ein Primer-Paar von sieben Primer-Paaren umfasst, das durch Auswählen eines Primer-Paars aus jeder der Gruppen K1 und K3 bis K7 erhalten wird:

Gruppe K1:

1-1: Primer-Paar, bestehend aus einer Kombination von einem Vorwärts-Primer, der aus einer Sequenz aus SEQ ID NO: 1 besteht, und einem Rückwärts-Primer, der aus einer beliebigen der Sequenzen aus SEQ ID NO: 16 bis 27 besteht;
1-2: Primer-Paar, bestehend aus einer Kombination von einem Vorwärts-Primer, der aus einer Sequenz aus SEQ ID NO: 10 besteht, und einem Rückwärts-Primer, der aus einer beliebigen der Sequenzen aus SEQ ID NO: 16, 18, 20 und 21 besteht;
1-3: Primer-Paar, bestehend aus einer Kombination von einem Vorwärts-Primer, der aus einer Sequenz aus SEQ ID NO: 11 besteht, und einem Rückwärts-Primer, der aus einer beliebigen der Sequenzen aus SEQ ID NO: 16 bis 21 und 23 bis 26 besteht;
1-4: Primer-Paar, bestehend aus einer Kombination von einem Vorwärts-Primer, der aus einer Sequenz aus SEQ ID NO: 12 besteht, und einem Rückwärts-Primer, der aus einer beliebigen der Sequenzen aus SEQ ID NO: 16 bis 27 besteht;

1-5: Primer-Paar, bestehend aus einer Kombination von einem Vorwärts-Primer, der aus einer Sequenz aus SEQ ID NO: 13 besteht, und einem Rückwärts-Primer, der aus einer beliebigen der Sequenzen aus SEQ ID NO: 16 bis 27 besteht;

1-6: Primer-Paar, bestehend aus einer Kombination von einem Vorwärts-Primer, der aus einer Sequenz aus SEQ ID NO: 14 besteht, und einem Rückwärts-Primer, der aus einer beliebigen der Sequenzen aus SEQ ID NO: 16 bis 27 besteht;

1-8: Primer-Paar, bestehend aus einer Kombination von einem Vorwärts-Primer, der aus einer Sequenz aus SEQ ID NO: 6 besteht, und einem Rückwärts-Primer, der aus einer beliebigen der Sequenzen aus SEQ ID NO: 16 bis 27 besteht;

1-9: Primer-Paar, bestehend aus einer Kombination von einem Vorwärts-Primer, der aus einer Sequenz aus SEQ ID NO: 7 besteht, und einem Rückwärts-Primer, der aus einer beliebigen der Sequenzen aus SEQ ID NO: 16 bis 27 besteht;

1-10: Primer-Paar, bestehend aus einer Kombination von einem Vorwärts-Primer, der aus einer Sequenz aus SEQ ID NO: 8 besteht, und einem Rückwärts-Primer, der aus einer beliebigen der Sequenzen aus SEQ ID NO: 16 bis 27 besteht; oder

1-11: Primer-Paar, bestehend aus einer Kombination von einem Vorwärts-Primer, der aus einer Sequenz aus SEQ ID NO: 9 besteht, und einem Rückwärts-Primer, der aus einer beliebigen der Sequenzen aus SEQ ID NO: 16, 17, 19 und 26 besteht;

Gruppe K3:
Primer-Paar, bestehend aus einer Kombination von einem Vorwärts-Primer, der aus einer Sequenz aus SEQ ID NO: 51 besteht, und einem Rückwärts-Primer, der aus einer Sequenz aus SEQ ID NO: 52 besteht;
Gruppe K4:

4-1: Primer-Paar, bestehend aus einer Kombination von einem Vorwärts-Primer, der aus einer Sequenz aus SEQ ID NO: 53 besteht, und einem Rückwärts-Primer, der aus einer beliebigen der Sequenzen aus SEQ ID NO: 57 bis 61 besteht;

4-2: Primer-Paar, bestehend aus einer Kombination von einem Vorwärts-Primer, der aus einer Sequenz aus SEQ ID NO: 54 besteht, und einem Rückwärts-Primer, der aus einer beliebigen der Sequenzen aus SEQ ID NO: 59 und 61 besteht;

4-3: Primer-Paar, bestehend aus einer Kombination von einem Vorwärts-Primer, der aus einer Sequenz aus SEQ ID NO: 55 besteht, und einem Rückwärts-Primer, der aus einer beliebigen der Sequenzen aus SEQ ID NO: 57 bis 59 und 61 besteht; oder

4-4: Primer-Paar, bestehend aus einer Kombination von einem Vorwärts-Primer, der aus einer Sequenz aus SEQ ID NO: 56 besteht, und einem Rückwärts-Primer, der aus einer beliebigen der Sequenzen aus SEQ ID NO: 57 bis 59 und 61 besteht;

Gruppe K5:

5-1: Primer-Paar, bestehend aus einer Kombination von einem Vorwärts-Primer, der aus einer Sequenz aus SEQ ID NO: 62 besteht, und einem Rückwärts-Primer, der aus einer beliebigen der Sequenzen aus SEQ ID NO: 70, 71 und 73 bis 75 besteht;

5-2: Primer-Paar, bestehend aus einer Kombination von einem Vorwärts-Primer, der aus einer Sequenz aus SEQ ID NO: 63 besteht, und einem Rückwärts-Primer, der aus einer beliebigen der Sequenzen aus SEQ ID NO: 70 bis 75 besteht;

5-3: Primer-Paar, bestehend aus einer Kombination von einem Vorwärts-Primer, der aus einer Sequenz aus SEQ ID NO: 64 besteht, und einem Rückwärts-Primer, der aus einer beliebigen der Sequenzen aus SEQ ID NO: 70 bis 75 besteht;

5-4: Primer-Paar, bestehend aus einer Kombination von einem Vorwärts-Primer, der aus einer Sequenz aus SEQ ID NO: 65 besteht, und einem Rückwärts-Primer, der aus einer beliebigen der Sequenzen aus SEQ ID NO: 70 bis 75 besteht;

5-5: Primer-Paar, bestehend aus einer Kombination von einem Vorwärts-Primer, der aus einer Sequenz aus SEQ ID NO: 66 besteht, und einem Rückwärts-Primer, der aus einer beliebigen der Sequenzen aus SEQ ID NO: 70 bis 75 besteht;

5-6: Primer-Paar, bestehend aus einer Kombination von einem Vorwärts-Primer, der aus einer Sequenz aus SEQ ID NO: 67 besteht, und einem Rückwärts-Primer, der aus einer beliebigen der Sequenzen aus SEQ ID NO: 70 bis 75 besteht;

5-7: Primer-Paar, bestehend aus einer Kombination von einem Vorwärts-Primer, der aus einer Sequenz aus SEQ ID NO: 68 besteht, und einem Rückwärts-Primer, der aus einer beliebigen der Sequenzen aus SEQ ID NO: 70 bis 75 besteht; oder

5-8: Primer-Paar, bestehend aus einer Kombination von einem Vorwärts-Primer, der aus einer Sequenz aus SEQ ID NO: 69 besteht, und einem Rückwärts-Primer, der aus einer beliebigen der Sequenzen aus SEQ ID NO: 70 bis 75 besteht;

Gruppe K6:

6-6: Primer-Paar, bestehend aus einer Kombination von einem Vorwärts-Primer, der aus einer Sequenz aus SEQ ID NO: 81 besteht, und einem Rückwärts-Primer, der aus einer beliebigen der Sequenzen aus SEQ ID NO: 91, 93 und 101 bis 103 besteht;

6-1: Primer-Paar, bestehend aus einer Kombination von einem Vorwärts-Primer, der aus einer Sequenz aus SEQ ID NO: 76 besteht, und einem Rückwärts-Primer, der aus einer beliebigen der Sequenzen aus SEQ ID NO: 91, 93, 96 und 101 bis 103 besteht;

6-2: Primer-Paar, bestehend aus einer Kombination von einem Vorwärts-Primer, der aus einer Sequenz aus SEQ ID NO: 77 besteht, und einem Rückwärts-Primer, der aus einer beliebigen der Sequenzen aus SEQ ID NO: 91, 96 und 101 bis 103 besteht;

6-3: Primer-Paar, bestehend aus einer Kombination von einem Vorwärts-Primer, der aus einer Sequenz aus SEQ ID NO: 78 besteht, und einem Rückwärts-Primer, der aus einer beliebigen der Sequenzen aus SEQ ID NO: 91 und 101 bis 103 besteht;

6-4: Primer-Paar, bestehend aus einer Kombination von einem Vorwärts-Primer, der aus einer Sequenz aus SEQ ID NO: 79 besteht, und einem Rückwärts-Primer, der aus einer beliebigen der Sequenzen aus SEQ ID NO: 91, 97 und 101 bis 103 besteht;

6-5: Primer-Paar, bestehend aus einer Kombination von einem Vorwärts-Primer, der aus einer Sequenz aus SEQ ID NO: 80 besteht, und einem Rückwärts-Primer, der aus einer beliebigen der Sequenzen aus SEQ ID NO: 91, 93 und 101 bis 103 besteht;

6-7: Primer-Paar, bestehend aus einer Kombination von einem Vorwärts-Primer, der aus einer Sequenz aus SEQ ID NO: 82 besteht, und einem Rückwärts-Primer, der aus einer beliebigen der Sequenzen aus SEQ ID NO: 91, 96, 97 und 101 bis 103 besteht;

6-8: Primer-Paar, bestehend aus einer Kombination von einem Vorwärts-Primer, der aus einer Sequenz aus SEQ ID NO: 83 besteht, und einem Rückwärts-Primer, der aus einer beliebigen der Sequenzen aus SEQ ID NO: 91, 101 bis 103 besteht;

6-9: Primer-Paar, bestehend aus einer Kombination von einem Vorwärts-Primer, der aus einer Sequenz aus SEQ ID NO: 84 besteht, und einem Rückwärts-Primer, der aus einer beliebigen der Sequenzen aus SEQ ID NO: 91 und 101 bis 103 besteht;

6-10: Primer-Paar, bestehend aus einer Kombination von einem Vorwärts-Primer, der aus einer Sequenz aus SEQ ID NO: 85 besteht, und einem Rückwärts-Primer, der aus einer beliebigen der Sequenzen aus SEQ ID NO: 91 und 101 bis 103 besteht;

6-11: Primer-Paar, bestehend aus einer Kombination von einem Vorwärts-Primer, der aus einer Sequenz aus SEQ ID NO: 86 besteht, und einem Rückwärts-Primer, der aus einer beliebigen der Sequenzen aus SEQ ID NO: 91, 93, 97 und 101 bis 103 besteht;

6-12: Primer-Paar, bestehend aus einer Kombination von einem Vorwärts-Primer, der aus einer Sequenz aus SEQ ID NO: 87 besteht, und einem Rückwärts-Primer, der aus einer beliebigen der Sequenzen aus SEQ ID NO: 91, 96 und 101 bis 103 besteht;

6-13: Primer-Paar, bestehend aus einer Kombination von einem Vorwärts-Primer, der aus einer Sequenz aus SEQ ID NO: 88 besteht, und einem Rückwärts-Primer, der aus einer beliebigen der Sequenzen aus SEQ ID NO: 91 und 101 bis 103 besteht;

6-14: Primer-Paar, bestehend aus einer Kombination von einem Vorwärts-Primer, der aus einer Sequenz aus SEQ ID NO: 89 besteht, und einem Rückwärts-Primer, der aus einer beliebigen der Sequenzen aus SEQ ID NO: 91 und 101 bis 103 besteht; oder

6-15: Primer-Paar, bestehend aus einer Kombination von einem Vorwärts-Primer, der aus einer Sequenz aus SEQ ID NO: 90 besteht, und einem Rückwärts-Primer, der aus einer beliebigen der Sequenzen aus SEQ ID NO: 91, 92, 96 und 101 bis 103 besteht;

Gruppe K7:

7-12: Primer-Paar, bestehend aus einer Kombination von einem Vorwärts-Primer, der aus einer Sequenz aus SEQ ID NO: 115 besteht, und einem Rückwärts-Primer, der aus einer beliebigen der Sequenzen aus SEQ ID NO: 2, 5, 116, 118, 123 und 125 bis 128 besteht;
7-1: Primer-Paar, bestehend aus einer Kombination von einem Vorwärts-Primer, der aus einer Sequenz aus SEQ ID NO: 104 besteht, und einem Rückwärts-Primer, der aus einer beliebigen der Sequenzen aus SEQ ID NO: 2, 5 und 116 bis 131 besteht;
7-2: Primer-Paar, bestehend aus einer Kombination von einem Vorwärts-Primer, der aus einer Sequenz aus SEQ ID NO: 105 besteht, und einem Rückwärts-Primer, der aus einer beliebigen der Sequenzen aus SEQ ID NO: 2, 5 und 116 bis 131 besteht;
7-3: Primer-Paar, bestehend aus einer Kombination von einem Vorwärts-Primer, der aus einer Sequenz aus SEQ ID NO: 106 besteht, und einem Rückwärts-Primer, der aus einer beliebigen der Sequenzen aus SEQ ID NO: 2, 5 und 116 bis 131 besteht;
7-4: Primer-Paar, bestehend aus einer Kombination von einem Vorwärts-Primer, der aus einer Sequenz aus SEQ ID NO: 107 besteht, und einem Rückwärts-Primer, der aus einer beliebigen der Sequenzen aus SEQ ID NO: 2, 5 und 116 bis 131 besteht;
7-5: Primer-Paar, bestehend aus einer Kombination von einem Vorwärts-Primer, der aus einer Sequenz aus SEQ ID NO: 108 besteht, und einem Rückwärts-Primer, der aus einer beliebigen der Sequenzen aus SEQ ID NO: 2, 5 und 116 bis 131 besteht;
7-6: Primer-Paar, bestehend aus einer Kombination von einem Vorwärts-Primer, der aus einer Sequenz aus SEQ ID NO: 109 besteht, und einem Rückwärts-Primer, der aus einer beliebigen der Sequenzen aus SEQ ID NO: 2, 5 und 116 bis 131 besteht;
7-7: Primer-Paar, bestehend aus einer Kombination von einem Vorwärts-Primer, der aus einer Sequenz aus SEQ ID NO: 110 besteht, und einem Rückwärts-Primer, der aus einer beliebigen der Sequenzen aus SEQ ID NO: 2, 5 und 116 bis 131 besteht;
7-8: Primer-Paar, bestehend aus einer Kombination von einem Vorwärts-Primer, der aus einer Sequenz aus SEQ ID NO: 111 besteht, und einem Rückwärts-Primer, der aus einer beliebigen der Sequenzen aus SEQ ID NO: 2, 5 und 116 bis 131 besteht;
7-9: Primer-Paar, bestehend aus einer Kombination von einem Vorwärts-Primer, der aus einer Sequenz aus SEQ ID NO: 112 besteht, und einem Rückwärts-Primer, der aus einer beliebigen der Sequenzen aus SEQ ID NO: 2, 5 und 116 bis 131 besteht;
7-10: Primer-Paar, bestehend aus einer Kombination von einem Vorwärts-Primer, der aus einer Sequenz aus SEQ ID NO: 113 besteht, und einem Rückwärts-Primer, der aus einer beliebigen der Sequenzen aus SEQ ID NO: 2, 5 und 116 bis 131 besteht; oder
7-11: Primer-Paar, bestehend aus einer Kombination von einem Vorwärts-Primer, der aus einer Sequenz aus SEQ ID NO: 114 besteht, und einem Rückwärts-Primer, der aus einer beliebigen der Sequenzen aus SEQ ID NO: 2, 5 und 125 besteht.

**5.** Primer-Satz nach Anspruch 2 oder Set nach Anspruch 4, das zumindest ein Primer-Paar der nachstehenden sieben Primer-Paare 1) bis 7) umfasst:

1) Primer-Paar, bestehend aus einer Kombination von einem Vorwärts-Primer, der aus einer Sequenz aus SEQ ID NO: 15 besteht, und einem Rückwärts-Primer, der aus einer Sequenz aus SEQ ID NO: 16 besteht;
2) Primer-Paar, bestehend aus einer Kombination von einem Vorwärts-Primer, der aus einer Sequenz aus SEQ ID NO: 43 besteht, und einem Rückwärts-Primer, der aus einer Sequenz aus SEQ ID NO: 50 besteht;
3) Primer-Paar, bestehend aus einer Kombination von einem Vorwärts-Primer, der aus einer Sequenz aus SEQ ID NO: 81 besteht, und einem Rückwärts-Primer, der aus einer Sequenz aus SEQ ID NO: 103 besteht;
4) Primer-Paar, bestehend aus einer Kombination von einem Vorwärts-Primer, der aus einer Sequenz aus SEQ ID NO: 115 besteht, und einem Rückwärts-Primer, der aus einer Sequenz aus SEQ ID NO: 5 besteht;
5) Primer-Paar, bestehend aus einer Kombination von einem Vorwärts-Primer, der aus einer Sequenz aus SEQ ID NO: 51 besteht, und einem Rückwärts-Primer, der aus einer Sequenz aus SEQ ID NO: 52 besteht;
6) Primer-Paar, bestehend aus einer Kombination von einem Vorwärts-Primer, der aus einer Sequenz aus SEQ ID NO: 53 besteht, und einem Rückwärts-Primer, der aus einer Sequenz aus SEQ ID NO: 57 besteht; und
7) Primer-Paar, bestehend aus einer Kombination von einem Vorwärts-Primer, der aus einer Sequenz aus SEQ ID NO: 62 besteht, und einem Rückwärts-Primer, der aus einer Sequenz aus SEQ ID NO: 70 besteht.

**6.** Primer-Satz oder Set nach Anspruch 5, umfassend ein beliebiges von den Primer-Paaren 1) bis 7).

**7.** Primer-Satz oder Set nach Anspruch 6, umfassend eine beliebige der nachstehenden Kombinationen A bis G:

(A) wenn der Primer-Satz Primer-Paar 1) umfasst, umfasst der Primer-Satz außerdem zumindest 3 Primer-Paare ausgewählt aus 2) bis 7);
(B) wenn der Primer-Satz Primer-Paar 2) umfasst, umfasst der Primer-Satz außerdem zumindest 3 Primer-Paare ausgewählt aus 1) und 3) bis 7);
(C) wenn der Primer-Satz Primer-Paar 3) umfasst, umfasst der Primer-Satz außerdem zumindest 3 Primer-Paare ausgewählt aus 1), 2) und 4) bis 7);
(D) wenn der Primer-Satz Primer-Paar 4) umfasst, umfasst der Primer-Satz außerdem zumindest 3 Primer-Paare ausgewählt aus 1) bis 3) und 5) bis 7);
(E) wenn der Primer-Satz Primer-Paar 5) umfasst, umfasst der Primer-Satz außerdem zumindest 3 Primer-Paare ausgewählt aus 1) bis 4) und 6) bis 7);
(F) wenn der Primer-Satz Primer-Paar 6) umfasst, umfasst der Primer-Satz außerdem zumindest 3 Primer-Paare ausgewählt aus 1) bis 5) und 7);
(G) wenn der Primer-Satz Primer-Paar 7) umfasst, umfasst der Primer-Satz außerdem zumindest 3 Primer-Paare ausgewählt aus 1) bis 6).

**8.** Primer-Satz nach einem der Ansprüche 1, 2 oder 5 bis 7 zur Detektion und/oder Identifikation einer Bakterienspezies.

**9.** Set nach einem der Ansprüche 3 bis 7 zur Identifikation einer Bakterienspezies.

**10.** Primer-Satz nach einem der Ansprüche 2 oder 5 bis 8 oder Set nach einem der Ansprüche 4, 5 bis 7 oder 9, wobei der Primer-Satz oder das Set zumindest 4 Primer-Paare aus den sieben Primer-Paaren umfasst.

**11.** Set nach einem der Ansprüche 3 bis 7 oder 9, das außerdem ein zusätzliches Primer-Paar aus einem Vorwärts-Primer bestehend aus einer Sequenz aus SEQ ID NO: 1 und einem Rückwärts-Primer bestehend aus einer beliebigen Sequenz aus SEQ ID NO: 2 bis 5 umfasst, wobei das zusätzliche Primer-Paar beispielsweise aus einem Vorwärts-Primer bestehend aus einer Sequenz aus SEQ ID NO: 1 und einem Rückwärts-Primer bestehend aus einer Sequenz aus SEQ ID NO: 5 besteht.

**12.** Set nach einem der Ansprüche 3 bis 7 oder 9, das außerdem ein Enzym für PCR umfasst.

**13.** Set nach Anspruch 12, wobei

(i) die Primer und das Enzym für PCR unabhängig voneinander oder insgesamt eine Verunreinigungsmenge an bakterieller Nucleinsäure von 10 fg oder weniger als 10 fg aufweisen; oder
(ii) das außerdem zumindest einen pH-Puffer, dNTP, $MgCl_2$ und ein Messgefäß umfasst, wobei vorzugsweise der pH-Puffer, dNTP, $MgCl_2$ und das Messgefäß unabhängig voneinander oder insgesamt eine Verunreinigungsmenge an bakterieller Nucleinsäure von 10 fg oder weniger als 10 fg aufweisen und wobei noch bevorzugter die Primer, das Enzym für PCR, der pH-Puffer, dNTP, $MgCl_2$ und das Messgefäß einzeln verpackt sind und die Verunreinigungsmenge an bakterieller Nucleinsäure in jeder der Verpackungen 10 fg oder weniger als 10 fg beträgt.

**14.** Set nach Anspruch 12 oder 13, Teil (i), das außerdem Fluorochrom umfasst und wobei vorzugsweise der Primer, das Enzym für PCR, der pH-Puffer, dNTP, das Fluorochrom, $MgCl_2$ und das Messgefäß unabhängig voneinander oder insgesamt eine Verunreinigungsmenge an bakterieller Nucleinsäure von 10 fg oder weniger als 10 fg aufweisen und wobei noch bevorzugter das Enzym für PCR, der pH-Puffer, dNTP, das Fluorochrom, $MgCl_2$ und das Messgefäß einzeln verpackt sind und die Verunreinigungsmenge an bakterieller Nucleinsäure in jeder der Verpackungen 10 fg oder weniger als 10 fg beträgt.

**15.** Set nach Anspruch 13 oder 14, wobei dNTP für Heißstart-PCR chemisch modifiziert ist.

**16.** Set nach einem der Ansprüche 12 bis 15, wobei das Enzym für PCR eine Taq-DNA-Polymerase ist, die eine Menge an bakterieller DNA von weniger als 10 fg/μl aufweist.

**17.** Verfahren zur Detektion und/oder Identifikation einer Bakterienspezies als Ziel in einer Probe, wobei das Verfahren Folgendes umfasst:

einen Schritt des Durchführens einer PCR unter Verwendung von Bakteriengenom-DNA, die aus der Probe hergestellt wurde, Primern, um ein Amplifikationsprodukt zu erhalten, das ein Ziel-Gen umfasst, das spezifisch für die Bakterienspezies als Ziel ist, einer thermostabilen DNA-Polymerase und
einen Schritt des Detektierens und/oder Identifizierens der Bakterienspezies als Ziel in der Probe durch Detektion des Ziel-Gens in dem Amplifikationsprodukt mittels PCR oder durch Analysieren des Amplifikationsprodukts mittels PCR,
wobei als Primer ein Primer-Set nach einem der Ansprüche 1, 2 und 5 bis 8 oder ein Set nach einem der Ansprüche 3 bis 7 und 9 bis 16 verwendet wird.

**18.** Verfahren zur Identifikation einer Bakterienspezies nach Anspruch 17, wobei ein Set nach einem der Ansprüche 3 bis 7, 9 bis 16 verwendet wird.

## Revendications

**1.** Ensemble d'amorces pour une réaction en chaîne par polymérase (PCR) pour l'amplification d'ADN bactérien, qui comprend une paire d'amorces, qui consiste en une combinaison d'une amorce sens consistant en une séquence de SEQ ID NO: 43 et d'une amorce antisens consistant en une quelconque séquence de SEQ ID NO: 44, 46 à 48 et 50.

**2.** Ensemble d'amorces selon la revendication 1, qui comprend en outre au moins une paire d'amorces de sept paires d'amorces obtenues en sélectionnant une paire d'amorces dans chaque groupe des groupes S1 et S3 à S7 :

Groupe S1 :

1-1 A : une paire d'amorces, qui consiste en une combinaison d'une amorce sens consistant en une séquence de SEQ ID NO: 1 et d'une amorce antisens consistant en une quelconque séquence de SEQ ID NO: 22 et 27 ;
1-2A : une paire d'amorces, qui consiste en une combinaison d'une amorce sens consistant en une séquence de SEQ ID NO: 10 et d'une amorce antisens consistant en une quelconque séquence de SEQ ID NO: 20 et 21 ;
1-3A : une paire d'amorces, qui consiste en une combinaison d'une amorce sens consistant en une séquence de SEQ ID NO: 11 et d'une amorce antisens consistant en une quelconque séquence de SEQ ID NO: 16, 18 et 26 ;
1-4 : une paire d'amorces, qui consiste en une combinaison d'une amorce sens consistant en une séquence de SEQ ID NO: 12 et d'une amorce antisens consistant en une quelconque séquence de SEQ ID NO: 16 à 27 ;
1-5 : une paire d'amorces, qui consiste en une combinaison d'une amorce sens consistant en une séquence de SEQ ID NO: 13 et d'une amorce antisens consistant en une quelconque séquence de SEQ ID NO: 16 à 27 ;
1-8A : une paire d'amorces, qui consiste en une combinaison d'une amorce sens consistant en une séquence de SEQ ID NO: 6 et d'une amorce antisens consistant en une quelconque séquence de SEQ ID NO: 16, 19 à 21, 23, 24, 26 et 27 ;
1-9A : une paire d'amorces, qui consiste en une combinaison d'une amorce sens consistant en une séquence de SEQ ID NO: 7 et d'une amorce antisens consistant en une quelconque séquence de SEQ ID NO: 16 à 21, 23, 24, 26 et 27 ; ou
1-10A : une paire d'amorces, qui consiste en une combinaison d'une amorce sens consistant en une séquence de SEQ ID NO: 8 et d'une amorce antisens consistant en une quelconque séquence de SEQ ID NO: 16 à 21, 23, 24, 26 et 27 ;

Groupe S3 :
une paire d'amorces, qui consiste en une combinaison d'une amorce sens consistant en une séquence de SEQ ID NO: 51 et d'une amorce antisens consistant en une séquence de SEQ ID NO: 52 ;
Groupe S4 :

4-1 : une paire d'amorces, qui consiste en une combinaison d'une amorce sens consistant en une séquence de SEQ ID NO: 53 et d'une amorce antisens consistant en une quelconque séquence de SEQ ID NO: 57 à 61 ;
4-2 : une paire d'amorces, qui consiste en une combinaison d'une amorce sens consistant en une séquence de SEQ ID NO: 54 et d'une amorce antisens consistant en une quelconque séquence de SEQ ID NO: 59 et 61 ;
4-3 : une paire d'amorces, qui consiste en une combinaison d'une amorce sens consistant en une séquence de SEQ ID NO: 55 et d'une amorce antisens consistant en une quelconque séquence de SEQ ID NO: 57 à 59 et 61 ; ou

4-4 : une paire d'amorces, qui consiste en une combinaison d'une amorce sens consistant en une séquence de SEQ ID NO: 56 et d'une amorce antisens consistant en une quelconque séquence de SEQ ID NO: 57 à 59 et 61 ;

Groupe S5 :

5-1A : une paire d'amorces, qui consiste en une combinaison d'une amorce sens consistant en une séquence de SEQ ID NO: 62 et d'une amorce antisens consistant en une quelconque séquence de SEQ ID NO: 70, 73 à 75 ;
5-2 : une paire d'amorces, qui consiste en une combinaison d'une amorce sens consistant en une séquence de SEQ ID NO: 63 et d'une amorce antisens consistant en une quelconque séquence de SEQ ID NO: 70 à 75 ;
5-3 : une paire d'amorces, qui consiste en une combinaison d'une amorce sens consistant en une séquence de SEQ ID NO: 64 et d'une amorce antisens consistant en une quelconque séquence de SEQ ID NO: 70 à 75 ;
5-4 : une paire d'amorces, qui consiste en une combinaison d'une amorce sens consistant en une séquence de SEQ ID NO: 65 et d'une amorce antisens consistant en une quelconque séquence de SEQ ID NO: 70 à 75 ;
5-5A : une paire d'amorces, qui consiste en une combinaison d'une amorce sens consistant en une séquence de SEQ ID NO: 66 et d'une amorce antisens consistant en une quelconque séquence de SEQ ID NO: 70, 72, 73 et 75 ;
5-6A : une paire d'amorces, qui consiste en une combinaison d'une amorce sens consistant en une séquence de SEQ ID NO: 67 et d'une amorce antisens consistant en une quelconque séquence de SEQ ID NO: 70, 71 et 73 à 75 ;
5-7 : une paire d'amorces, qui consiste en une combinaison d'une amorce sens consistant en une séquence de SEQ ID NO: 68 et d'une amorce antisens consistant en une quelconque séquence de SEQ ID NO: 70 à 75 ; ou
5-8 : une paire d'amorces, qui consiste en une combinaison d'une amorce sens consistant en une séquence de SEQ ID NO: 69 et d'une amorce antisens consistant en une quelconque séquence de SEQ ID NO: 70 à 75 ;

Groupe S6 :

6-6 : une paire d'amorces, qui consiste en une combinaison d'une amorce sens consistant en une séquence de SEQ ID NO: 81 et d'une amorce antisens consistant en une quelconque séquence de SEQ ID NO: 91, 93 et 101 à 103 ;
6-1A : une paire d'amorces, qui consiste en une combinaison d'une amorce sens consistant en une séquence de SEQ ID NO: 76 et d'une amorce antisens consistant en une quelconque séquence de SEQ ID NO: 93, 96 et 101 à 103 ;
6-2 : une paire d'amorces, qui consiste en une combinaison d'une amorce sens consistant en une séquence de SEQ ID NO: 77 et d'une amorce antisens consistant en une quelconque séquence de SEQ ID NO: 91, 96 et 101 à 103 ;
6-3 : une paire d'amorces, qui consiste en une combinaison d'une amorce sens consistant en une séquence de SEQ ID NO: 78 et d'une amorce antisens consistant en une quelconque séquence de SEQ ID NO: 91 et 101 à 103 ;
6-4A : une paire d'amorces, qui consiste en une combinaison d'une amorce sens consistant en une séquence de SEQ ID NO: 79 et d'une amorce antisens consistant en une quelconque séquence de SEQ ID NO: 91 et 101 à 103 ;
6-5 : une paire d'amorces, qui consiste en une combinaison d'une amorce sens consistant en une séquence de SEQ ID NO: 80 et d'une amorce antisens consistant en une quelconque séquence de SEQ ID NO: 91, 93 et 100 à 103 ;
6-7 : une paire d'amorces, qui consiste en une combinaison d'une amorce sens consistant en une séquence de SEQ ID NO: 82 et d'une amorce antisens consistant en une quelconque séquence de SEQ ID NO: 91, 96, 97 et 101 à 103 ;
6-8 : une paire d'amorces, qui consiste en une combinaison d'une amorce sens consistant en une séquence de SEQ ID NO: 83 et d'une amorce antisens consistant en une quelconque séquence de SEQ ID NO: 91, 101 à 103 ;
6-9 : une paire d'amorces, qui consiste en une combinaison d'une amorce sens consistant en une séquence de SEQ ID NO: 84 et d'une amorce antisens consistant en une quelconque séquence de SEQ ID NO: 91 et 101 à 103 ;
6-10 : une paire d'amorces, qui consiste en une combinaison d'une amorce sens consistant en une séquence de SEQ ID NO: 85 et d'une amorce antisens consistant en une quelconque séquence de SEQ ID NO: 91 et 101 à 103 ;
6-11A : une paire d'amorces, qui consiste en une combinaison d'une amorce sens consistant en une séquence de SEQ ID NO: 86 et d'une amorce antisens consistant en une quelconque séquence de SEQ ID NO: 91, 93 et 101 à 103 ;
6-12 : une paire d'amorces, qui consiste en une combinaison d'une amorce sens consistant en une séquence de SEQ ID NO: 87 et d'une amorce antisens consistant en une quelconque séquence de SEQ ID NO: 91, 96 et 101 à 103 ;
6-13 : une paire d'amorces, qui consiste en une combinaison d'une amorce sens consistant en une séquence de SEQ ID NO: 88 et d'une amorce antisens consistant en une quelconque séquence de SEQ ID NO: 91 et 101 à 103 ;
6-14 : une paire d'amorces, qui consiste en une combinaison d'une amorce sens consistant en une séquence de SEQ ID NO: 89 et d'une amorce antisens consistant en une quelconque séquence de SEQ ID NO: 91 et 101 à 103 ; ou
6-15 : une paire d'amorces, qui consiste en une combinaison d'une amorce sens consistant en une séquence de SEQ ID NO: 90 et d'une amorce antisens consistant en une quelconque séquence de SEQ ID NO: 91, 92, 96 et 101 à 103 ;

Groupe S7 :

7-12A : une paire d'amorces, qui consiste en une combinaison d'une amorce sens consistant en une séquence de SEQ ID NO: 115 et d'une amorce antisens consistant en une quelconque séquence de SEQ ID NO: 2 et 5 ;
7-1A : une paire d'amorces, qui consiste en une combinaison d'une amorce sens consistant en une séquence de SEQ ID NO: 104 et d'une amorce antisens consistant en une quelconque séquence de SEQ ID NO: 5 et 118 à 125, 127, 128, 130 et 131 ;
7-2A : une paire d'amorces, qui consiste en une combinaison d'une amorce sens consistant en une séquence de SEQ ID NO: 105 et d'une amorce antisens consistant en une quelconque séquence de SEQ ID NO: 5 et 118 à 125 ;
7-3A : une paire d'amorces, qui consiste en une combinaison d'une amorce sens consistant en une séquence de SEQ ID NO: 106 et d'une amorce antisens consistant en une quelconque séquence de SEQ ID NO: 2, 5 et 116 et 118 à 131 ;
7-4A : une paire d'amorces, qui consiste en une combinaison d'une amorce sens consistant en une séquence de SEQ ID NO: 107 et d'une amorce antisens consistant en une quelconque séquence de SEQ ID NO: 120 et 121 ;
7-5A : une paire d'amorces, qui consiste en une combinaison d'une amorce sens consistant en une séquence de SEQ ID NO: 108 et d'une amorce antisens consistant en une quelconque séquence de SEQ ID NO: 120 et 123 ;
7-6A : une paire d'amorces, qui consiste en une combinaison d'une amorce sens consistant en une séquence de SEQ ID NO: 109 et d'une amorce antisens consistant en une quelconque séquence de SEQ ID NO: 2, 118 à 122, 124 et 126 ;
7-7A : une paire d'amorces, qui consiste en une combinaison d'une amorce sens consistant en une séquence de SEQ ID NO: 110 et d'une amorce antisens consistant en une quelconque séquence de SEQ ID NO: 118, 120, 121, 124 et 126 à 130 ;
7-8A : une paire d'amorces, qui consiste en une combinaison d'une amorce sens consistant en une séquence de SEQ ID NO: 111 et d'une amorce antisens consistant en une quelconque séquence de SEQ ID NO: 2, 5 et 118 à 131 ;
7-9A : une paire d'amorces, qui consiste en une combinaison d'une amorce sens consistant en une séquence de SEQ ID NO: 112 et d'une amorce antisens consistant en une quelconque séquence de SEQ ID NO: 2, 5 et 116, et 118 à 125 ;
7-10A : une paire d'amorces, qui consiste en une combinaison d'une amorce sens consistant en une séquence de SEQ ID NO: 113 et d'une amorce antisens consistant en une quelconque séquence de SEQ ID NO: 2, 5, 118 à 126, 130 et 131 ; ou
7-11A : une paire d'amorces, qui consiste en une combinaison d'une amorce sens consistant en une séquence de SEQ ID NO: 114 et d'une amorce antisens consistant en une séquence de SEQ ID NO: 5.

3. Trousse pour la détection d'une espèce bactérienne, qui comprend une paire d'amorces, qui consiste en une combinaison d'une amorce sens consistant en une séquence de SEQ ID NO: 43 et d'une amorce antisens consistant en une quelconque séquence de SEQ ID NO: 44 à 48 et 50.

**4.** Trousse selon la revendication 3, qui comprend en outre au moins une paire d'amorce de sept paires d'amorces obtenues en sélectionnant une paire d'amorces dans chaque groupe des groupes K1 et K3 à K7 :

Groupe K1 :

1-1 : une paire d'amorces, qui consiste en une combinaison d'une amorce sens consistant en une séquence de SEQ ID NO: 1 et d'une amorce antisens consistant en une quelconque séquence de SEQ ID NO: 16 à 27 ;
1-2 : une paire d'amorces, qui consiste en une combinaison d'une amorce sens consistant en une séquence de SEQ ID NO: 10 et d'une amorce antisens consistant en une quelconque séquence de SEQ ID NO: 16, 18, 20 et 21 ;
1-3 : une paire d'amorces, qui consiste en une combinaison d'une amorce sens consistant en une séquence de SEQ ID NO: 11 et d'une amorce antisens consistant en une quelconque séquence de SEQ ID NO: 16 à 21 et 23 à 26 ;
1-4 : une paire d'amorces, qui consiste en une combinaison d'une amorce sens consistant en une séquence de SEQ ID NO: 12 et d'une amorce antisens consistant en une quelconque séquence de SEQ ID NO: 16 à 27 ;
1-5 : une paire d'amorces, qui consiste en une combinaison d'une amorce sens consistant en une séquence de SEQ ID NO: 13 et d'une amorce antisens consistant en une quelconque séquence de SEQ ID NO: 16 à 27 ;
1-6 : une paire d'amorces, qui consiste en une combinaison d'une amorce sens consistant en une séquence de SEQ ID NO: 14 et d'une amorce antisens consistant en une quelconque séquence de SEQ ID NO: 16 à 27 ;
1-8 : une paire d'amorces, qui consiste en une combinaison d'une amorce sens consistant en une séquence de SEQ ID NO: 6 et d'une amorce antisens consistant en une quelconque séquence de SEQ ID NO: 16 à 27 ;
1-9 : une paire d'amorces, qui consiste en une combinaison d'une amorce sens consistant en une séquence de SEQ ID NO: 7 et d'une amorce antisens consistant en une quelconque séquence de SEQ ID NO: 16 à 27 ;
1-10 : une paire d'amorces, qui consiste en une combinaison d'une amorce sens consistant en une séquence de SEQ ID NO: 8 et d'une amorce antisens consistant en une quelconque séquence de SEQ ID NO: 16 à 27 ; ou
1-11 : une paire d'amorces, qui consiste en une combinaison d'une amorce sens consistant en une séquence de SEQ ID NO: 9 et d'une amorce antisens consistant en une quelconque séquence de SEQ ID NO: 16, 17, 19 et 26 ;

Groupe K3 :

une paire d'amorces, qui consiste en une combinaison d'une amorce sens consistant en une séquence de SEQ ID NO: 51 et d'une amorce antisens consistant en une séquence de SEQ ID NO: 52 ;

Groupe K4 :

4-1 : une paire d'amorces, qui consiste en une combinaison d'une amorce sens consistant en une séquence de SEQ ID NO: 53 et d'une amorce antisens consistant en une quelconque séquence de SEQ ID NO: 57 à 61 ;
4-2 : une paire d'amorces, qui consiste en une combinaison d'une amorce sens consistant en une séquence de SEQ ID NO: 54 et d'une amorce antisens consistant en une quelconque séquence de SEQ ID NO: 59 et 61 ;
4-3 : une paire d'amorces, qui consiste en une combinaison d'une amorce sens consistant en une séquence de SEQ ID NO: 55 et d'une amorce antisens consistant en une quelconque séquence de SEQ ID NO: 57 à 59 et 61 ; ou
4-4 : une paire d'amorces, qui consiste en une combinaison d'une amorce sens consistant en une séquence de SEQ ID NO: 56 et d'une amorce antisens consistant en une quelconque séquence de SEQ ID NO: 57 à 59 et 61 ;

Groupe K5 :

5-1 : une paire d'amorces, qui consiste en une combinaison d'une amorce sens consistant en une séquence de SEQ ID NO: 62 et d'une amorce antisens consistant en une quelconque séquence de SEQ ID NO: 70, 71 et 73 à 75 ;
5-2 : une paire d'amorces, qui consiste en une combinaison d'une amorce sens consistant en une séquence de SEQ ID NO: 63 et d'une amorce antisens consistant en une quelconque séquence de SEQ ID NO: 70 à 75 ;
5-3 : une paire d'amorces, qui consiste en une combinaison d'une amorce sens consistant en une séquence de SEQ ID NO: 64 et d'une amorce antisens consistant en une quelconque séquence de SEQ ID NO: 70 à 75 ;

5-4 : une paire d'amorces, qui consiste en une combinaison d'une amorce sens consistant en une séquence de SEQ ID NO: 65 et d'une amorce antisens consistant en une quelconque séquence de SEQ ID NO: 70 à 75 ;
5-5 : une paire d'amorces, qui consiste en une combinaison d'une amorce sens consistant en une séquence de SEQ ID NO: 66 et d'une amorce antisens consistant en une quelconque séquence de SEQ ID NO: 70 à 75 ;
5-6 : une paire d'amorces, qui consiste en une combinaison d'une amorce sens consistant en une séquence de SEQ ID NO: 67 et d'une amorce antisens consistant en une quelconque séquence de SEQ ID NO: 70 à 75 ;
5-7 : une paire d'amorces, qui consiste en une combinaison d'une amorce sens consistant en une séquence de SEQ ID NO: 68 et d'une amorce antisens consistant en une quelconque séquence de SEQ ID NO: 70 à 75 ; ou
5-8 : une paire d'amorces, qui consiste en une combinaison d'une amorce sens consistant en une séquence de SEQ ID NO: 69 et d'une amorce antisens consistant en une quelconque séquence de SEQ ID NO: 70 à 75 ;

Groupe K6 :

6-6 : une paire d'amorces, qui consiste en une combinaison d'une amorce sens consistant en une séquence de SEQ ID NO: 81 et d'une amorce antisens consistant en une quelconque séquence de SEQ ID NO: 91, 93 et 101 à 103 ;
6-1 : une paire d'amorces, qui consiste en une combinaison d'une amorce sens consistant en une séquence de SEQ ID NO: 76 et d'une amorce antisens consistant en une quelconque séquence de SEQ ID NO: 91, 93, 96 et 101 à 103 ;
6-2 : une paire d'amorces, qui consiste en une combinaison d'une amorce sens consistant en une séquence de SEQ ID NO: 77 et d'une amorce antisens consistant en une quelconque séquence de SEQ ID NO: 91, 96 et 101 à 103 ;
6-3 : une paire d'amorces, qui consiste en une combinaison d'une amorce sens consistant en une séquence de SEQ ID NO: 78 et d'une amorce antisens consistant en une quelconque séquence de SEQ ID NO: 91 et 101 à 103 ;
6-4 : une paire d'amorces, qui consiste en une combinaison d'une amorce sens consistant en une séquence de SEQ ID NO: 79 et d'une amorce antisens consistant en une quelconque séquence de SEQ ID NO: 91, 97 et 101 à 103 ;
6-5 : une paire d'amorces, qui consiste en une combinaison d'une amorce sens consistant en une séquence de SEQ ID NO: 80 et d'une amorce antisens consistant en une quelconque séquence de SEQ ID NO: 91, 93 et 100 à 103 ;
6-7 : une paire d'amorces, qui consiste en une combinaison d'une amorce sens consistant en une séquence de SEQ ID NO: 82 et d'une amorce antisens consistant en une quelconque séquence de SEQ ID NO: 91, 96, 97 et 101 à 103 ;
6-8 : une paire d'amorces, qui consiste en une combinaison d'une amorce sens consistant en une séquence de SEQ ID NO: 83 et d'une amorce antisens consistant en une quelconque séquence de SEQ ID NO: 91, 101 à 103 ;
6-9 : une paire d'amorces, qui consiste en une combinaison d'une amorce sens consistant en une séquence de SEQ ID NO: 84 et d'une amorce antisens consistant en une quelconque séquence de SEQ ID NO: 91 et 101 à 103 ;
6-10 : une paire d'amorces, qui consiste en une combinaison d'une amorce sens consistant en une séquence de SEQ ID NO: 85 et d'une amorce antisens consistant en une quelconque séquence de SEQ ID NO: 91 et 101 à 103 ;
6-11 : une paire d'amorces, qui consiste en une combinaison d'une amorce sens consistant en une séquence de SEQ ID NO: 86 et d'une amorce antisens consistant en une quelconque séquence de SEQ ID NO: 91, 93, 97 et 101 à 103 ;
6-12 : une paire d'amorces, qui consiste en une combinaison d'une amorce sens consistant en une séquence de SEQ ID NO: 87 et d'une amorce antisens consistant en une quelconque séquence de SEQ ID NO: 91, 96 et 101 à 103 ;
6-13 : une paire d'amorces, qui consiste en une combinaison d'une amorce sens consistant en une séquence de SEQ ID NO: 88 et d'une amorce antisens consistant en une quelconque séquence de SEQ ID NO: 91 et 101 à 103 ;
6-14 : une paire d'amorces, qui consiste en une combinaison d'une amorce sens consistant en une séquence de SEQ ID NO: 89 et d'une amorce antisens consistant en une quelconque séquence de SEQ ID NO: 91 et 101 à 103 ; ou
6-15 : une paire d'amorces, qui consiste en une combinaison d'une amorce sens consistant en une séquence de SEQ ID NO: 90 et d'une amorce antisens consistant en une quelconque séquence de SEQ ID NO: 91, 92, 96 et 101 à 103 ;

Groupe K7 :

7-12 : une paire d'amorces, qui consiste en une combinaison d'une amorce sens consistant en une séquence de SEQ ID NO: 115 et d'une amorce antisens consistant en une quelconque séquence de SEQ ID NO: 2, 5, 116, 118, 123 et 125 à 128 ;
7-1 : une paire d'amorces, qui consiste en une combinaison d'une amorce sens consistant en une séquence de SEQ ID NO: 104 et d'une amorce antisens consistant en une quelconque séquence de SEQ ID NO: 2, 5 et 116 à 131 ;
7-2 : une paire d'amorces, qui consiste en une combinaison d'une amorce sens consistant en une séquence de SEQ ID NO: 105 et d'une amorce antisens consistant en une quelconque séquence de SEQ ID NO: 2, 5 et 116 à 131 ;
7-3 : une paire d'amorces, qui consiste en une combinaison d'une amorce sens consistant en une séquence de SEQ ID NO: 106 et d'une amorce antisens consistant en une quelconque séquence de SEQ ID NO: 2, 5 et 116 à 131 ;
7-4 : une paire d'amorces, qui consiste en une combinaison d'une amorce sens consistant en une séquence de SEQ ID NO: 107 et d'une amorce antisens consistant en une quelconque séquence de SEQ ID NO: 2, 5 et 116 à 131 ;
7-5 : une paire d'amorces, qui consiste en une combinaison d'une amorce sens consistant en une séquence de SEQ ID NO: 108 et d'une amorce antisens consistant en une quelconque séquence de SEQ ID NO: 2, 5 et 116 à 131 ;
7-6 : une paire d'amorces, qui consiste en une combinaison d'une amorce sens consistant en une séquence de SEQ ID NO: 109 et d'une amorce antisens consistant en une quelconque séquence de SEQ ID NO: 2, 5 et 116 à 131 ;
7-7 : une paire d'amorces, qui consiste en une combinaison d'une amorce sens consistant en une séquence de SEQ ID NO: 110 et d'une amorce antisens consistant en une quelconque séquence de SEQ ID NO: 2, 5 et 116 à 131 ;
7-8 : une paire d'amorces, qui consiste en une combinaison d'une amorce sens consistant en une séquence de SEQ ID NO: 111 et d'une amorce antisens consistant en une quelconque séquence de SEQ ID NO: 2, 5 et 116 à 131 ;
7-9 : une paire d'amorces, qui consiste en une combinaison d'une amorce sens consistant en une séquence de SEQ ID NO: 112 et d'une amorce antisens consistant en une quelconque séquence de SEQ ID NO: 2, 5 et 116 à 131 ;
7-10 : une paire d'amorces, qui consiste en une combinaison d'une amorce sens consistant en une séquence de SEQ ID NO: 113 et d'une amorce antisens consistant en une quelconque séquence de SEQ ID NO: 2, 5 et 116 à 131 ; ou
7-11 : une paire d'amorces, qui consiste en une combinaison d'une amorce sens consistant en une séquence de SEQ ID NO: 114 et d'une amorce antisens consistant en une quelconque séquence de SEQ ID NO: 2, 5 et 125.

**5.** Ensemble d'amorces selon la revendication 2 ou trousse selon la revendication 4, qui comprend au moins une paire d'amorces des sept paires d'amorces 1) à 7) suivantes :

1) une paire d'amorces, qui consiste en une combinaison d'une amorce sens consistant en une séquence de SEQ ID NO: 15 et d'une amorce antisens consistant en une séquence de SEQ ID NO: 16 ;
2) une paire d'amorces, qui consiste en une combinaison d'une amorce sens consistant en une séquence de SEQ ID NO: 43 et d'une amorce antisens consistant en une séquence de SEQ ID NO: 50 ;
3) une paire d'amorces, qui consiste en une combinaison d'une amorce sens consistant en une séquence de SEQ ID NO: 81 et d'une amorce antisens consistant en une séquence de SEQ ID NO: 103 ;
4) une paire d'amorces, qui consiste en une combinaison d'une amorce sens consistant en une séquence de SEQ ID NO: 115 et d'une amorce antisens consistant en une séquence de SEQ ID NO: 5 ;
5) une paire d'amorces, qui consiste en une combinaison d'une amorce sens consistant en une séquence de SEQ ID NO: 51 et d'une amorce antisens consistant en une séquence de SEQ ID NO: 52 ;
6) une paire d'amorces, qui consiste en une combinaison d'une amorce sens consistant en une séquence de SEQ ID NO: 53 et d'une amorce antisens consistant en une séquence de SEQ ID NO: 57 ; et
7) une paire d'amorces, qui consiste en une combinaison d'une amorce sens consistant en une séquence de SEQ ID NO: 62 et d'une amorce antisens consistant en une séquence de SEQ ID NO: 70.

**6.** Ensemble d'amorces ou trousse selon la revendication 5, comprenant l'une quelconque des paires d'amorces 1) à 7).

**7.** Ensemble d'amorces ou trousse selon la revendication 6, comprenant l'une quelconque des combinaisons A à G suivantes :

(A) lorsque l'ensemble d'amorces comprend la paire d'amorces 1), l'ensemble d'amorces comprend en outre au moins 3 paires d'amorces sélectionnées parmi 2) à 7) ;
(B) lorsque l'ensemble d'amorces comprend la paire d'amorces 2), l'ensemble d'amorces comprend en outre au moins 3 paires d'amorces sélectionnées parmi 1) et 3) à 7) ;
(C) lorsque l'ensemble d'amorces comprend la paire d'amorces 3), l'ensemble d'amorces comprend en outre au moins 3 paires d'amorces sélectionnées parmi 1), 2) et 4) à 7) ;
(D) lorsque l'ensemble d'amorces comprend la paire d'amorces 4), l'ensemble d'amorces comprend en outre au moins 3 paires d'amorces sélectionnées parmi 1) à 3) et 5) à 7) ;
(E) lorsque l'ensemble d'amorces comprend la paire d'amorces 5), l'ensemble d'amorces comprend en outre au moins 3 paires d'amorces sélectionnées parmi 1) à 4) et 6) à 7) ;
(F) lorsque l'ensemble d'amorces comprend la paire d'amorces 6), l'ensemble d'amorces comprend en outre au moins 3 paires d'amorces sélectionnées parmi 1) à 5) et 7) ;
(G) lorsque l'ensemble d'amorces comprend la paire d'amorces 7), l'ensemble d'amorces comprend en outre au moins 3 paires d'amorces sélectionnées parmi 1) à 6).

**8.** Ensemble d'amorces selon l'une quelconque des revendications 1, 2 ou 5 à 7 pour la détection et/ou l'identification d'une espèce bactérienne.

**9.** Trousse selon l'une quelconque des revendications 3 à 7 pour l'identification d'une espèce bactérienne.

**10.** Ensemble d'amorces selon l'une quelconque des revendications 2, ou 5 à 8, ou trousse selon l'une quelconque des revendications 4, 5 à 7, ou 9, où l'ensemble d'amorces ou la trousse comprend au moins 4 paires d'amorces des sept paires d'amorces.

**11.** Trousse selon l'une quelconque des revendications 3 à 7, ou 9, qui comprend en outre une paire d'amorces supplémentaire d'une amorce sens consistant en une séquence de SEQ ID NO: 1 et d'une amorce antisens consistant en une quelconque séquence de SEQ ID NO: 2 à 5, par exemple dans laquelle la paire d'amorces supplémentaire consiste en une amorce sens consistant en une séquence de SEQ ID NO: 1 et une amorce antisens consistant en séquence de SEQ ID NO: 5.

**12.** Trousse selon l'une quelconque des revendications 3 à 7, ou 9, qui comprend en outre une enzyme pour une PCR.

**13.** Trousse selon la revendication 12, dans laquelle

(i) les amorces et l'enzyme pour la PCR présentent, indépendamment ou ensemble, une quantité de contamination d'acide nucléique bactérien de 10 fg ou de moins de 10 fg ; ou
(ii) qui comprend en outre au moins un tampon du pH, un dNTP, du $MgCl_2$ et un récipient pour la mesure, et de préférence le tampon du pH, le dNTP, le $MgCl_2$ et le récipient pour la mesure présentent, indépendamment ou ensemble, une quantité de contamination d'acide nucléique bactérien de 10 fg ou de moins de 10 fg, et de manière davantage préférée les amorces, l'enzyme pour la PCR, le tampon du pH, le dNTP, le $MgCl_2$ et le récipient pour la mesure sont conditionnés individuellement et la quantité de contamination d'acide nucléique bactérien dans chacun des conditionnements est de 10 fg ou de moins de 10 fg.

**14.** Trousse selon la revendication 12 ou 13, partie (i), qui comprend en outre un fluorochrome, et de préférence l'amorce, l'enzyme pour la PCR, le tampon du pH, le dNTP, le fluorochrome, le $MgCl_2$ et le récipient pour la mesure présentent, indépendamment ou ensemble, une quantité de contamination d'acide nucléique bactérien de 10 fg ou de moins de 10 fg, et de manière davantage préférée l'amorce, l'enzyme pour la PCR, le tampon du pH, le dNTP, le fluorochrome, le $MgCl_2$ et le récipient pour la mesure sont conditionnés individuellement et la quantité de contamination d'acide nucléique bactérien dans chacun des conditionnements est de 10 fg ou de moins de 10 fg.

**15.** Trousse selon les revendications 13 ou 14, dans laquelle le dNTP est chimiquement modifié pour une PCR à démarrage à chaud (Hot Start).

16. Trousse selon l'une quelconque des revendications 12 à 15, dans laquelle l'enzyme pour la PCR est une Taq ADN polymérase présentant une quantité d'ADN bactérien inférieure à 10 fg/μl.

17. Procédé de détection et/ou d'identification d'une espèce bactérienne en tant que cible dans un échantillon, qui comprend

une étape consistant à réaliser une PCR en utilisant de l'ADN de génome bactérien préparé à partir de l'échantillon, des amorces pour obtenir un produit d'amplification comprenant un gène cible spécifique à l'espèce bactérienne en tant que cible, une ADN polymérase thermostable, et
une étape consistant à détecter et/ou identifier l'espèce bactérienne en tant que cible dans l'échantillon en détectant le gène cible dans le produit d'amplification par PCR, ou en analysant le produit d'amplification par PCR,
dans lequel, en tant qu'amorces, l'ensemble d'amorces selon l'une quelconque des revendications 1, 2, et 5 à 8, ou la trousse selon l'une quelconque des revendications 3 à 7, et 9 à 16, est utilisé(e).

18. Procédé d'identification d'une espèce bactérienne selon la revendication 17, dans lequel une trousse selon l'une quelconque des revendications 3 à 7, 9 à 16, est utilisée.

[Fig. 1A]

Results of electrophoresis of amplification products using each primer

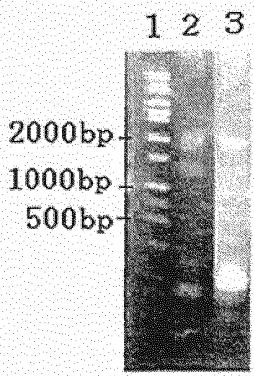


Lane 1: Marker

Lane 2: Amplification result with the primers of the prior application (SEQ ID Nos. 76 and 91)

Lane3: Amplification result with the primers of the present application (SEQ ID Nos. 90 and 103)

[Fig. 1B]

PCR amplification curves using each primer

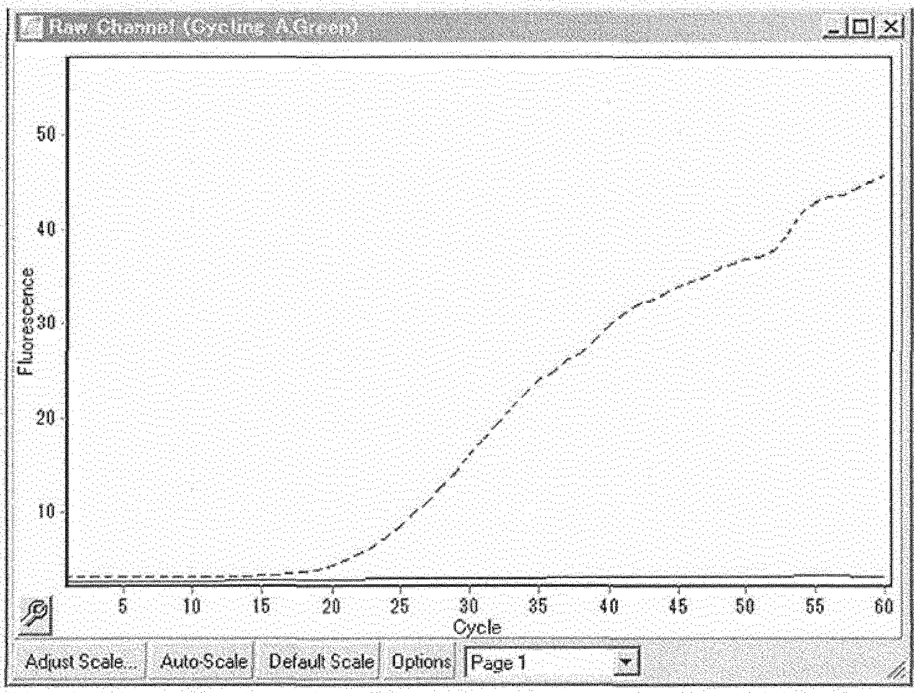


Solid line: Amplification result with the primers of the prior application (SEQ ID Nos. 76 and 91)

Dotted line: Amplification result with the primers of the present application (SEQ ID Nos. 90 and 103)

[Fig. 1C]

Results of PCR melting analysis using each primer

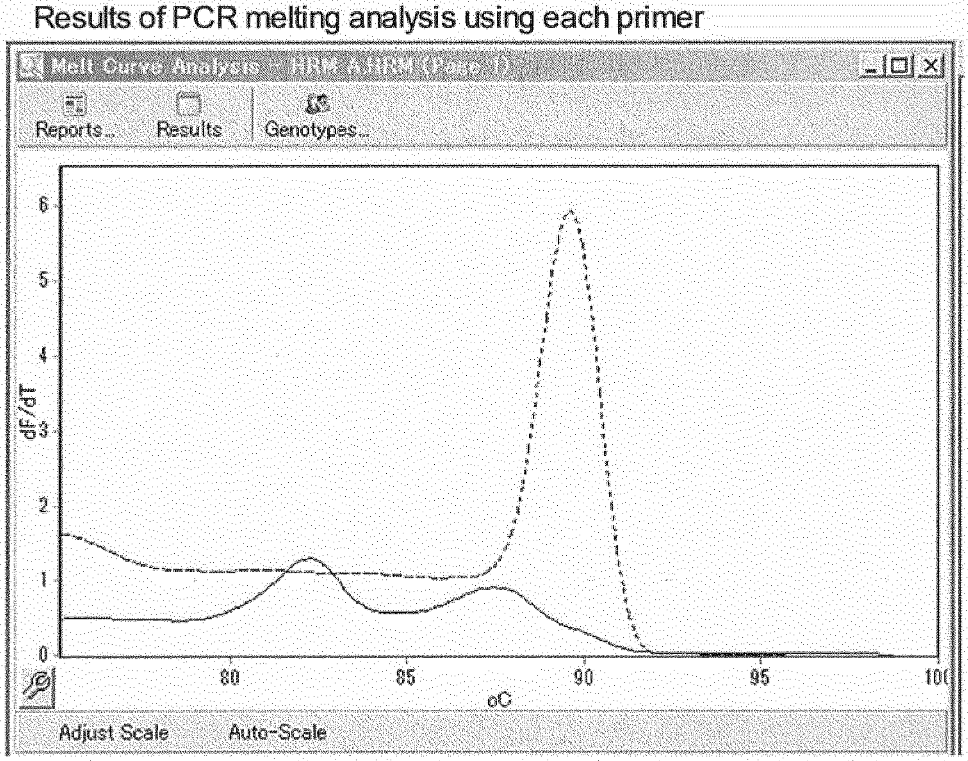


Solid line: Amplification result with the primers of the prior application (SEQ ID Nos. 76 and 91)

Dotted line: Amplification result with the primers of the present application (SEQ ID Nos. 90 and 103)

[Fig. 2]

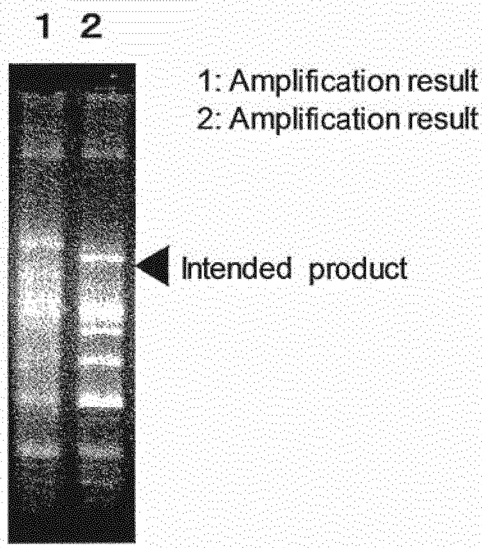


1: Amplification result with Composition 1 (Table 3)
2: Amplification result with Composition 2 (Table 1)

[Fig. 3]

Effects of ultrafiltration treatment for DNA removal

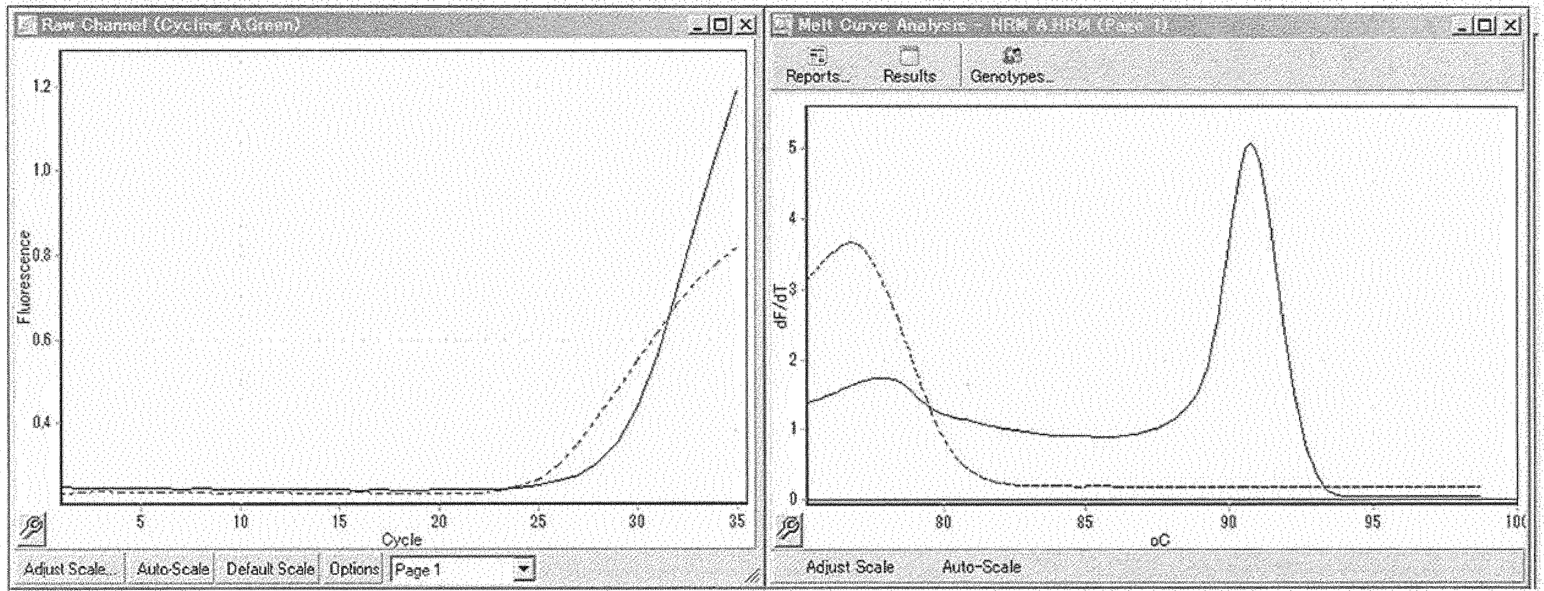


Solid line: Reagent with addition of genome, but without Amicon treatment
Dotted line: Reagent with addition of genome after Amicon treatment

[Fig. 4]

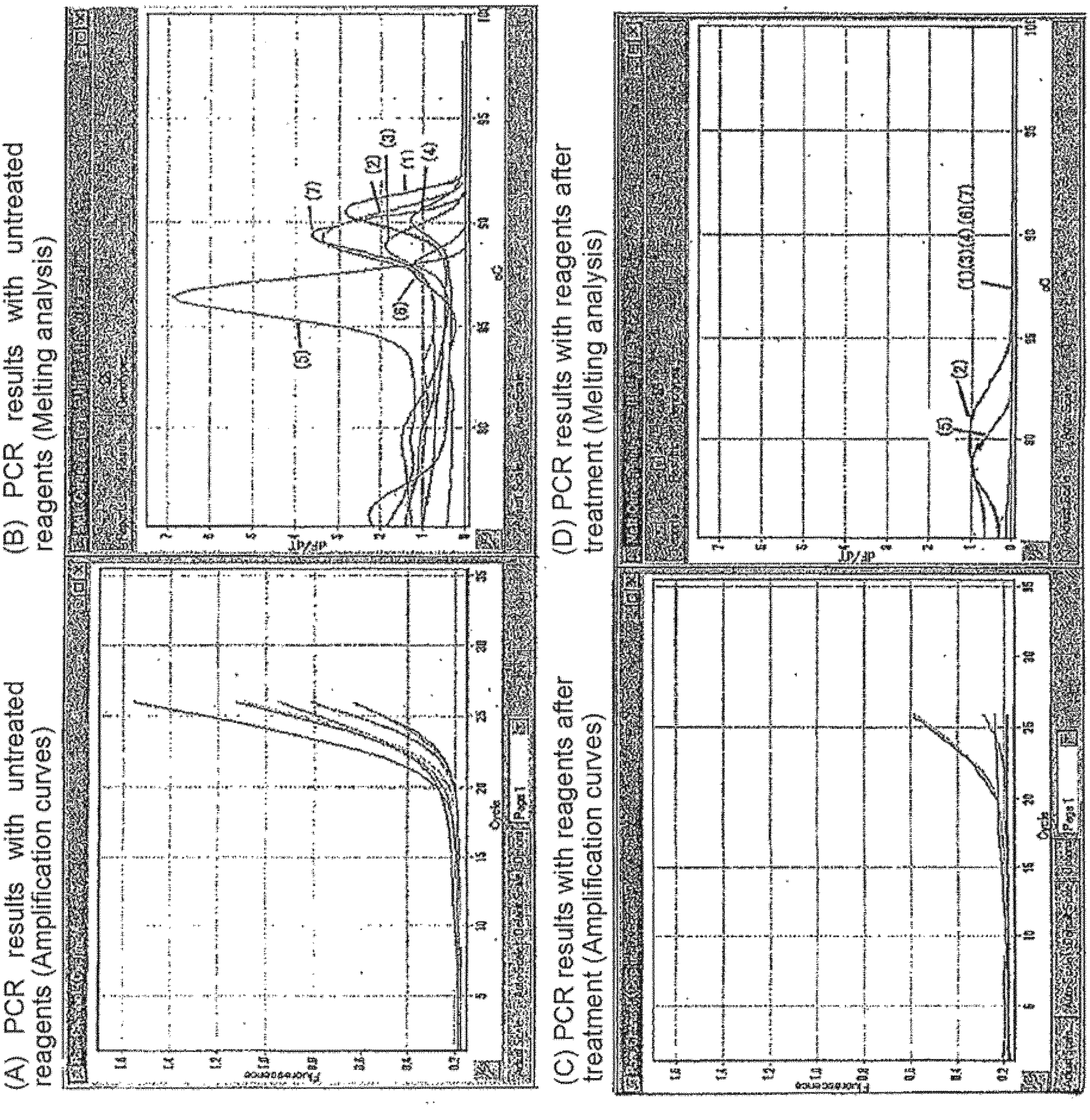


(A) PCR results with untreated reagents (Amplification curves)

(B) PCR results with untreated reagents (Melting analysis)

(C) PCR results with reagents after treatment (Amplification curves)

(D) PCR results with reagents after treatment (Melting analysis)

[Fig. 5]

(A) Amplification curves with EMA treated / untreated reagents

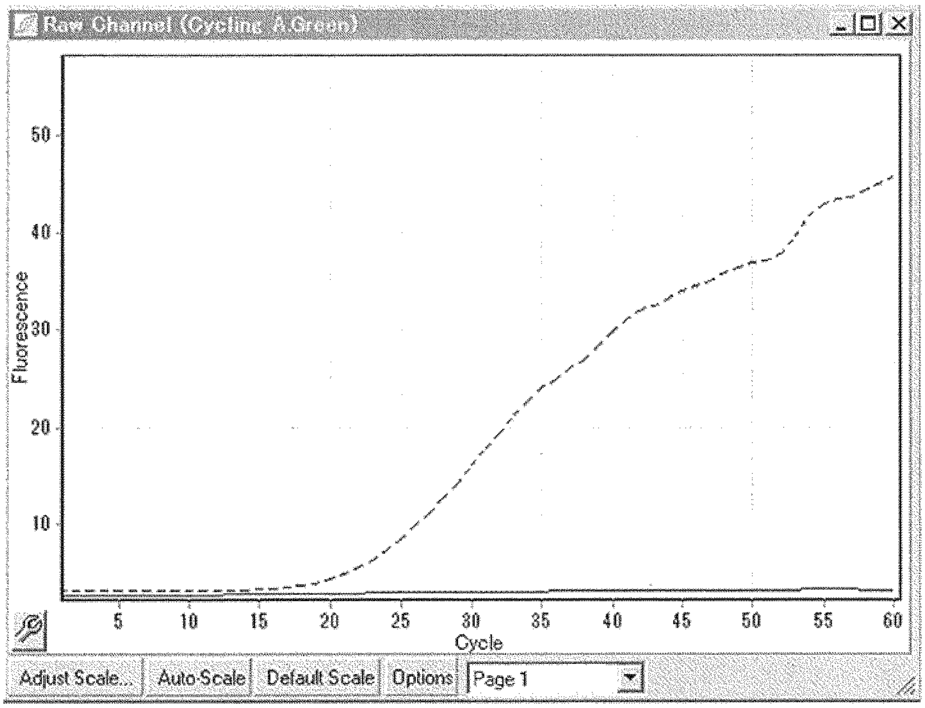


(B) Melting analysis with EMA treated / untreated reagents

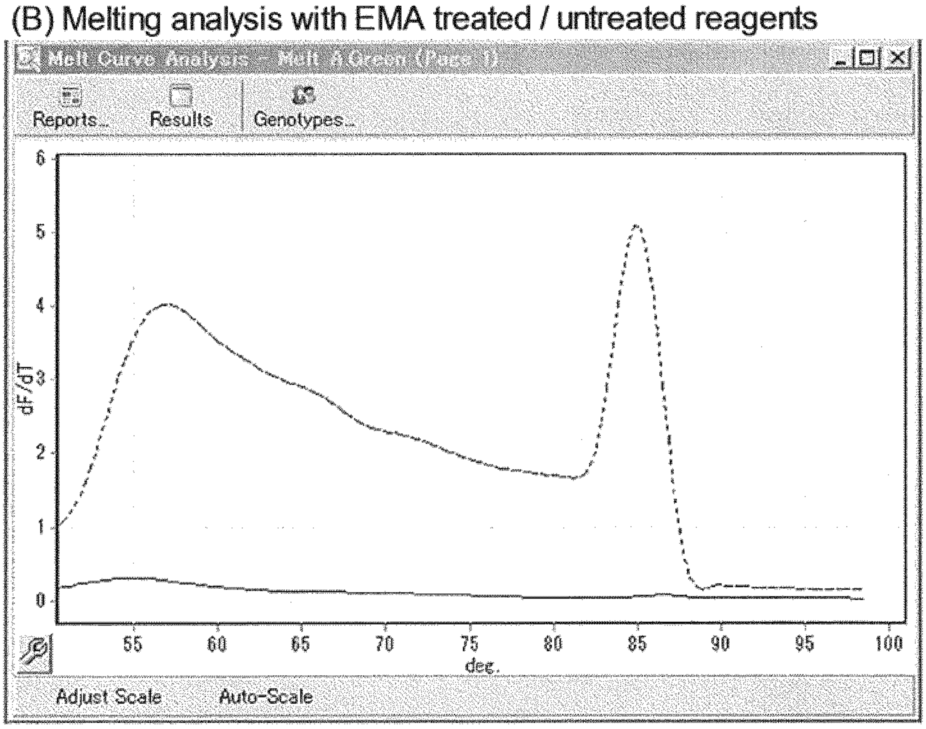


Solid line: Reagent with addition of genome, but without EMA treatment

Dotted line: Reagent with addition of genome after EMA treatment

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 6090799 A
- WO 2007097323 A
- JP 2006180886 A
- WO 2010082640 A
- JP H0690799 A
- JP 2000004847 A
- JP 10276776 A
- JP H10276776 A
- US 20070281308 A
- EP 007437718 A
- EP 14853073
- JP 2014076864 W
- JP 2013210606 A


### Non-patent literature cited in the description


- *Journal of Analytical Bio-Science,* 2005, vol. 28 (5), 400-40
- *Journal of Clinical Microbiology,* 2009, 2252-2255
- *Journal of Clinical Microbiology,* 2010, 3410-3413
- *Journal of Clinical Microbiology,* 2010, 258-267
- *Journal of Analytical Bio-Science,* 2005, vol. 28 (5), 400-40

SEQUENCE LISTING

<110> Mitsui chemicals, inc.

<120> PCR Primer Set for Bacterial DNA Amplification, Kit for Detecting and/or Identifying Bacterial Species, and Method for Detecting and/or Identifying Bacterial Species

<130> 007437718

<140> EP
<141> 2014-10-7

<150> 14853073.6
<151> 2014-10-7

<150> PCT/JP2014/076864
<151> 2014-10-7

<150> JP 2013-210606
<151> 2013-10-7

<160> 137

<170> PatentIn version 3.1

<210> 1
<211> 20
<212> DNA
<213> Artificial

<220>
<223> Oligonucleotide to act as a primer

<400> 1
agagtttgat catggctcag 20

<210> 2
<211> 17
<212> DNA
<213> Artificial

<220>
<223> Oligonucleotide to act as a primer

<400> 2
ccgggaacgt attcacc 17

<210> 3
<211> 27

<212> DNA
<213> Artificial

<220>
<223> Oligonucleotide to act as a primer

<400> 3
tacggctacc ttgttacgac ttcaccc 27

<210> 4
<211> 25
<212> DNA
<213> Artificial

<220>
<223> Oligonucleotide to act as a primer

<400> 4
gctaccttgt tacgacttca cccca 25

<210> 5
<211> 18
<212> DNA
<213> Artificial

<220>
<223> Oligonucleotide to act as a primer

<400> 5
agacccggga acgtattc 18

<210> 6
<211> 21
<212> DNA
<213> Artificial

<220>
<223> Oligonucleotide to act as a primer

<400> 6
gcctaataca tgcaagtcga g 21

<210> 7
<211> 14
<212> DNA
<213> Artificial

<220>
<223> Oligonucleotide to act as a primer

<400> 7
gatgaacgct ggcg 14

<210> 8
<211> 13
<212> DNA
<213> Artificial

<220>
<223> Oligonucleotide to act as a primer

<400> 8
gaacgctggc ggc 13

<210> 9
<211> 12
<212> DNA
<213> Artificial

<220>
<223> Oligonucleotide to act as a primer

<400> 9
gctggcggcg tg 12

<210> 10
<211> 12
<212> DNA
<213> Artificial

<220>
<223> Oligonucleotide to act as a primer

<400> 10
ctggcggcgt gc 12

<210> 11
<211> 16
<212> DNA
<213> Artificial

<220>
<223> Oligonucleotide to act as a primer

<400> 11
gcggcgtgcc taatac 16

<210> 12
<211> 19
<212> DNA
<213> Artificial

<220>
<223> Oligonucleotide to act as a primer

<400> 12
cgtgcctaat acatgcaag 19

<210> 13
<211> 21
<212> DNA
<213> Artificial

<220>
<223> Oligonucleotide to act as a primer

<400> 13
ctaatacatg caagtcgagc g 21

<210> 14
<211> 15
<212> DNA
<213> Artificial

<220>
<223> Oligonucleotide to act as a primer

<400> 14
catgcaagtc gagcg 15

<210> 15
<211> 23
<212> DNA
<213> Artificial

<220>
<223> Oligonucleotide to act as a primer

<400> 15
gcaggcttaa cacatgcaag tcg 23

<210> 16
<211> 18
<212> DNA
<213> Artificial

<220>
<223> Oligonucleotide to act as a primer

<400> 16
cgtaggagtc tggaccgt 18

<210> 17
<211> 20
<212> DNA
<213> Artificial

<220>
<223> Oligonucleotide to act as a primer

<400> 17
gagtctggac cgtgtctcag 20

<210> 18
<211> 19
<212> DNA
<213> Artificial

<220>
<223> Oligonucleotide to act as a primer

<400> 18
ggagtctgga ccgtgtctc 19

<210> 19
<211> 17
<212> DNA
<213> Artificial

<220>
<223> Oligonucleotide to act as a primer

<400> 19
ggagtctgga ccgtgtc 17

<210> 20
<211> 18
<212> DNA
<213> Artificial

<220>
<223> Oligonucleotide to act as a primer

<400> 20
gtaggagtct ggaccgtg 18

<210> 21
<211> 17
<212> DNA
<213> Artificial

<220>
<223> Oligonucleotide to act as a primer

<400> 21
cgtaggagtc tggaccg 17

<210> 22
<211> 19
<212> DNA
<213> Artificial

<220>
<223> Oligonucleotide to act as a primer

<400> 22
ctcccgtagg agtctggac 19

<210> 23
<211> 18
<212> DNA
<213> Artificial

<220>
<223> Oligonucleotide to act as a primer

<400> 23
gcctcccgta ggagtctg 18

<210> 24
<211> 18
<212> DNA

<213> Artificial

<220>
<223> Oligonucleotide to act as a primer

<400> 24
ctgcctcccg taggagtc 18

<210> 25
<211> 16
<212> DNA
<213> Artificial

<220>
<223> Oligonucleotide to act as a primer

<400> 25
ctgcctcccg taggag 16

<210> 26
<211> 19
<212> DNA
<213> Artificial

<220>
<223> Oligonucleotide to act as a primer

<400> 26
ctactgctgc ctcccgtag 19

<210> 27
<211> 16
<212> DNA
<213> Artificial

<220>
<223> Oligonucleotide to act as a primer

<400> 27
ccctactgct gcctcc 16

<210> 28
<211> 17
<212> DNA
<213> Artificial

<220>
<223> Oligonucleotide to act as a primer

<400> 28
gactcctacg ggaggca 17

<210> 29
<211> 17
<212> DNA
<213> Artificial

<220>

<223> Oligonucleotide to act as a primer

<400> 29
ccagactcct acgggag 17

<210> 30
<211> 16
<212> DNA
<213> Artificial

<220>
<223> Oligonucleotide to act as a primer

<400> 30
ctcctacggg aggcag 16

<210> 31
<211> 16
<212> DNA
<213> Artificial

<220>
<223> Oligonucleotide to act as a primer

<400> 31
ctacgggagg cagcag 16

<210> 32
<211> 16
<212> DNA
<213> Artificial

<220>
<223> Oligonucleotide to act as a primer

<400> 32
ggaggcagca gtaggg 16

<210> 33
<211> 17
<212> DNA
<213> Artificial

<220>
<223> Oligonucleotide to act as a primer

<400> 33
gccacactgg gactgag 17

<210> 34
<211> 19
<212> DNA
<213> Artificial

<220>
<223> Oligonucleotide to act as a primer

<400> 34

cacactggga ctgagacac 19

<210> 35
<211> 18
<212> DNA
<213> Artificial

<220>
<223> Oligonucleotide to act as a primer

<400> 35
cactgggact gagacacg 18

<210> 36
<211> 17
<212> DNA
<213> Artificial

<220>
<223> Oligonucleotide to act as a primer

<400> 36
ctgggactga gacacgg 17

<210> 37
<211> 17
<212> DNA
<213> Artificial

<220>
<223> Oligonucleotide to act as a primer

<400> 37
gggactgaga cacggtc 17

<210> 38
<211> 20
<212> DNA
<213> Artificial

<220>
<223> Oligonucleotide to act as a primer

<400> 38
gactgagaca cggtccagac 20

<210> 39
<211> 20
<212> DNA
<213> Artificial

<220>
<223> Oligonucleotide to act as a primer

<400> 39
ctgagacacg gtccagactc 20

<210> 40

<211> 19
<212> DNA
<213> Artificial

<220>
<223> Oligonucleotide to act as a primer

<400> 40
gagacacggt ccagactcc 19

<210> 41
<211> 20
<212> DNA
<213> Artificial

<220>
<223> Oligonucleotide to act as a primer

<400> 41
gacacggtcc agactcctac 20

<210> 42
<211> 18
<212> DNA
<213> Artificial

<220>
<223> Oligonucleotide to act as a primer

<400> 42
cacggtccag actcctac 18

<210> 43
<211> 19
<212> DNA
<213> Artificial

<220>
<223> Oligonucleotide to act as a primer

<400> 43
gtccagactc ctacgggag 19

<210> 44
<211> 17
<212> DNA
<213> Artificial

<220>
<223> Oligonucleotide to act as a primer

<400> 44
tattaccgcg gctgctg 17

<210> 45
<211> 13
<212> DNA
<213> Artificial

<220>
<223> Oligonucleotide to act as a primer

<400> 45
ggctgctggc acg 13

<210> 46
<211> 13
<212> DNA
<213> Artificial

<220>
<223> Oligonucleotide to act as a primer

<400> 46
gtattaccgc ggc 13

<210> 47
<211> 16
<212> DNA
<213> Artificial

<220>
<223> Oligonucleotide to act as a primer

<400> 47
ggcacgtagt tagccg 16

<210> 48
<211> 19
<212> DNA
<213> Artificial

<220>
<223> Oligonucleotide to act as a primer

<400> 48
gctgctggca cgtagttag 19

<210> 49
<211> 15
<212> DNA
<213> Artificial

<220>
<223> Oligonucleotide to act as a primer

<400> 49
ctgctggcac gtagt 15

<210> 50
<211> 17
<212> DNA
<213> Artificial

<220>
<223> Oligonucleotide to act as a primer

<400> 50
cctacgtatt accgcgg 17

<210> 51
<211> 16
<212> DNA
<213> Artificial

<220>
<223> Oligonucleotide to act as a primer

<400> 51
agcagccgcg gtaata 16

<210> 52
<211> 23
<212> DNA
<213> Artificial

<220>
<223> Oligonucleotide to act as a primer

<400> 52
ggactaccag ggtatctaat cct 23

<210> 53
<211> 23
<212> DNA
<213> Artificial

<220>
<223> Oligonucleotide to act as a primer

<400> 53
aacaggatta gataccctgg tag 23

<210> 54
<211> 22
<212> DNA
<213> Artificial

<220>
<223> Oligonucleotide to act as a primer

<400> 54
caaacaggat tagataccct gg 22

<210> 55
<211> 19
<212> DNA
<213> Artificial

<220>
<223> Oligonucleotide to act as a primer

<400> 55
gataccctgg tagtccacg 19

<210> 56
<211> 22
<212> DNA
<213> Artificial

<220>
<223> Oligonucleotide to act as a primer

<400> 56
ggattagata ccctggtagt cc 22

<210> 57
<211> 21
<212> DNA
<213> Artificial

<220>
<223> Oligonucleotide to act as a primer

<400> 57
aattaaacca catgctccac c 21

<210> 58
<211> 20
<212> DNA
<213> Artificial

<220>
<223> Oligonucleotide to act as a primer

<400> 58
cttcgaatta aaccacatgc 20

<210> 59
<211> 19
<212> DNA
<213> Artificial

<220>
<223> Oligonucleotide to act as a primer

<400> 59
cgttgcttcg aattaaacc 19

<210> 60
<211> 17
<212> DNA
<213> Artificial

<220>
<223> Oligonucleotide to act as a primer

<400> 60
gttcttcgcg ttgcttc 17

<210> 61
<211> 19
<212> DNA

<213> Artificial

<220>
<223> Oligonucleotide to act as a primer

<400> 61
ggtaaggttc ttcgcgttg 19

<210> 62
<211> 21
<212> DNA
<213> Artificial

<220>
<223> Oligonucleotide to act as a primer

<400> 62
tggtttaatt cgatgcaacg c 21

<210> 63
<211> 15
<212> DNA
<213> Artificial

<220>
<223> Oligonucleotide to act as a primer

<400> 63
gcacaagcgg tggag 15

<210> 64
<211> 15
<212> DNA
<213> Artificial

<220>
<223> Oligonucleotide to act as a primer

<400> 64
gcggtggagc atgtg 15

<210> 65
<211> 21
<212> DNA
<213> Artificial

<220>
<223> Oligonucleotide to act as a primer

<400> 65
gtggagcatg tggtttaatt c 21

<210> 66
<211> 20
<212> DNA
<213> Artificial

<220>

<223> Oligonucleotide to act as a primer

<400> 66
gcatgtggtt taattcgaag 20

<210> 67
<211> 19
<212> DNA
<213> Artificial

<220>
<223> Oligonucleotide to act as a primer

<400> 67
gtttaattcg aagcaacgc 19

<210> 68
<211> 17
<212> DNA
<213> Artificial

<220>
<223> Oligonucleotide to act as a primer

<400> 68
gaagcaacgc gaagaac 17

<210> 69
<211> 19
<212> DNA
<213> Artificial

<220>
<223> Oligonucleotide to act as a primer

<400> 69
caacgcgaag aaccttacc 19

<210> 70
<211> 17
<212> DNA
<213> Artificial

<220>
<223> Oligonucleotide to act as a primer

<400> 70
gagctgacga cagccat 17

<210> 71
<211> 17
<212> DNA
<213> Artificial

<220>
<223> Oligonucleotide to act as a primer

<400> 71

ctgacgacaa ccatgca 17

<210> 72
<211> 19
<212> DNA
<213> Artificial

<220>
<223> Oligonucleotide to act as a primer

<400> 72
gacacgagct gacgacaac 19

<210> 73
<211> 17
<212> DNA
<213> Artificial

<220>
<223> Oligonucleotide to act as a primer

<400> 73
tcacgacacg agctgac 17

<210> 74
<211> 17
<212> DNA
<213> Artificial

<220>
<223> Oligonucleotide to act as a primer

<400> 74
cgacacgagc tgacgac 17

<210> 75
<211> 18
<212> DNA
<213> Artificial

<220>
<223> Oligonucleotide to act as a primer

<400> 75
cgagctgacg acaaccat 18

<210> 76
<211> 16
<212> DNA
<213> Artificial

<220>
<223> Oligonucleotide to act as a primer

<400> 76
ttgggttaag tcccgc 16

<210> 77

<211> 18
<212> DNA
<213> Artificial

<220>
<223> Oligonucleotide to act as a primer

<400> 77
atgttgggtt aagtcccg 18

<210> 78
<211> 17
<212> DNA
<213> Artificial

<220>
<223> Oligonucleotide to act as a primer

<400> 78
gggttaagtc ccgcaac 17

<210> 79
<211> 14
<212> DNA
<213> Artificial

<220>
<223> Oligonucleotide to act as a primer

<400> 79
gtcccgcaac gagc 14

<210> 80
<211> 14
<212> DNA
<213> Artificial

<220>
<223> Oligonucleotide to act as a primer

<400> 80
caacgagcgc aacc 14

<210> 81
<211> 18
<212> DNA
<213> Artificial

<220>
<223> Oligonucleotide to act as a primer

<400> 81
gttaagtccc gcaacgag 18

<210> 82
<211> 13
<212> DNA
<213> Artificial

<220>
<223> Oligonucleotide to act as a primer

<400> 82
cccgcaacga gcg 13

<210> 83
<211> 14
<212> DNA
<213> Artificial

<220>
<223> Oligonucleotide to act as a primer

<400> 83
gcaacgagcg caac 14

<210> 84
<211> 16
<212> DNA
<213> Artificial

<220>
<223> Oligonucleotide to act as a primer

<400> 84
aacgagcgca accctt 16

<210> 85
<211> 13
<212> DNA
<213> Artificial

<220>
<223> Oligonucleotide to act as a primer

<400> 85
cgcaacgagc gca 13

<210> 86
<211> 16
<212> DNA
<213> Artificial

<220>
<223> Oligonucleotide to act as a primer

<400> 86
aagtcccgca acgagc 16

<210> 87
<211> 17
<212> DNA
<213> Artificial

<220>
<223> Oligonucleotide to act as a primer

<400> 87
atggctgtcg tcagctc 17

<210> 88
<211> 17
<212> DNA
<213> Artificial

<220>
<223> Oligonucleotide to act as a primer

<400> 88
gctgtcgtca gctcgtg 17

<210> 89
<211> 16
<212> DNA
<213> Artificial

<220>
<223> Oligonucleotide to act as a primer

<400> 89
ggctgtcgtc agctcg 16

<210> 90
<211> 15
<212> DNA
<213> Artificial

<220>
<223> Oligonucleotide to act as a primer

<400> 90
gcatggctgt cgtcg 15

<210> 91
<211> 16
<212> DNA
<213> Artificial

<220>
<223> Oligonucleotide to act as a primer

<400> 91
cgtcatcccc accttc 16

<210> 92
<211> 19
<212> DNA
<213> Artificial

<220>
<223> Oligonucleotide to act as a primer

<400> 92
gggatgacgt caaatcatc 19

<210> 93
<211> 17
<212> DNA
<213> Artificial

<220>
<223> Oligonucleotide to act as a primer

<400> 93
gaggaaggtg gggatga 17

<210> 94
<211> 19
<212> DNA
<213> Artificial

<220>
<223> Oligonucleotide to act as a primer

<400> 94
atgacgtcaa atcatcatg 19

<210> 95
<211> 18
<212> DNA
<213> Artificial

<220>
<223> Oligonucleotide to act as a primer

<400> 95
tggggatgac gtcaaatc 18

<210> 96
<211> 17
<212> DNA
<213> Artificial

<220>
<223> Oligonucleotide to act as a primer

<400> 96
aaggtgggga tgacgtc 17

<210> 97
<211> 16
<212> DNA
<213> Artificial

<220>
<223> Oligonucleotide to act as a primer

<400> 97
gaaggtgggg atgacg 16

<210> 98
<211> 16
<212> DNA

<213> Artificial

<220>
<223> Oligonucleotide to act as a primer

<400> 98
ggaggaaggt ggggat 16

<210> 99
<211> 16
<212> DNA
<213> Artificial

<220>
<223> Oligonucleotide to act as a primer

<400> 99
ggaaggtggg gatgac 16

<210> 100
<211> 16
<212> DNA
<213> Artificial

<220>
<223> Oligonucleotide to act as a primer

<400> 100
gaggaaggtg gggatg 16

<210> 101
<211> 20
<212> DNA
<213> Artificial

<220>
<223> Oligonucleotide to act as a primer

<400> 101
attgtagcac gtgtgtagcc 20

<210> 102
<211> 18
<212> DNA
<213> Artificial

<220>
<223> Oligonucleotide to act as a primer

<400> 102
gtagcacgtg tgtagccc 18

<210> 103
<211> 20
<212> DNA
<213> Artificial

<220>

<223> Oligonucleotide to act as a primer

<400> 103
ccattgtagc acgtgtgtag 20

<210> 104
<211> 20
<212> DNA
<213> Artificial

<220>
<223> Oligonucleotide to act as a primer

<400> 104
ggctacacac gtgctacaat 20

<210> 105
<211> 19
<212> DNA
<213> Artificial

<220>
<223> Oligonucleotide to act as a primer

<400> 105
gggatgacgt caaatcatc 19

<210> 106
<211> 17
<212> DNA
<213> Artificial

<220>
<223> Oligonucleotide to act as a primer

<400> 106
gaggaaggtg gggatga 17

<210> 107
<211> 19
<212> DNA
<213> Artificial

<220>
<223> Oligonucleotide to act as a primer

<400> 107
atgacgtcaa atcatcatg 19

<210> 108
<211> 18
<212> DNA
<213> Artificial

<220>
<223> Oligonucleotide to act as a primer

<400> 108

tggggatgac gtcaaatc 18

<210> 109
<211> 17
<212> DNA
<213> Artificial

<220>
<223> Oligonucleotide to act as a primer

<400> 109
aaggtgggga tgacgtc 17

<210> 110
<211> 16
<212> DNA
<213> Artificial

<220>
<223> Oligonucleotide to act as a primer

<400> 110
gaaggtgggg atgacg 16

<210> 111
<211> 16
<212> DNA
<213> Artificial

<220>
<223> Oligonucleotide to act as a primer

<400> 111
ggaggaaggt ggggat 16

<210> 112
<211> 16
<212> DNA
<213> Artificial

<220>
<223> Oligonucleotide to act as a primer

<400> 112
ggaaggtggg gatgac 16

<210> 113
<211> 16
<212> DNA
<213> Artificial

<220>
<223> Oligonucleotide to act as a primer

<400> 113
gaggaaggtg gggatg 16

<210> 114

<211> 18
<212> DNA
<213> Artificial

<220>
<223> Oligonucleotide to act as a primer

<400> 114
gggctacaca cgtgctac 18

<210> 115
<211> 22
<212> DNA
<213> Artificial

<220>
<223> Oligonucleotide to act as a primer

<400> 115
ggctacacac gtgctacaat gg 22

<210> 116
<211> 18
<212> DNA
<213> Artificial

<220>
<223> Oligonucleotide to act as a primer

<400> 116
gggcggtgtg tacaagac 18

<210> 117
<211> 18
<212> DNA
<213> Artificial

<220>
<223> Oligonucleotide to act as a primer

<400> 117
cgggcggtgt gtacaaga 18

<210> 118
<211> 19
<212> DNA
<213> Artificial

<220>
<223> Oligonucleotide to act as a primer

<400> 118
gtgacgggcg gtgtgtaca 19

<210> 119
<211> 18
<212> DNA
<213> Artificial

<220>
<223> Oligonucleotide to act as a primer

<400> 119
gtgacgggcg gtgtgtac 18

<210> 120
<211> 18
<212> DNA
<213> Artificial

<220>
<223> Oligonucleotide to act as a primer

<400> 120
gtgtgacggg cggtgtgt 18

<210> 121
<211> 17
<212> DNA
<213> Artificial

<220>
<223> Oligonucleotide to act as a primer

<400> 121
gtgtgacggg cggtgtg 17

<210> 122
<211> 17
<212> DNA
<213> Artificial

<220>
<223> Oligonucleotide to act as a primer

<400> 122
tggtgtgacg ggcggtg 17

<210> 123
<211> 16
<212> DNA
<213> Artificial

<220>
<223> Oligonucleotide to act as a primer

<400> 123
tggtgtgacg ggcggt 16

<210> 124
<211> 15
<212> DNA
<213> Artificial

<220>
<223> Oligonucleotide to act as a primer

<400> 124
tggtgtgacg ggcgg 15

<210> 125
<211> 17
<212> DNA
<213> Artificial

<220>
<223> Oligonucleotide to act as a primer

<400> 125
gacccgggaa cgtattc 17

<210> 126
<211> 16
<212> DNA
<213> Artificial

<220>
<223> Oligonucleotide to act as a primer

<400> 126
acaagacccg ggaacg 16

<210> 127
<211> 17
<212> DNA
<213> Artificial

<220>
<223> Oligonucleotide to act as a primer

<400> 127
tacaagaccc gggaacg 17

<210> 128
<211> 17
<212> DNA
<213> Artificial

<220>
<223> Oligonucleotide to act as a primer

<400> 128
gtacaagacc cgggaac 17

<210> 129
<211> 17
<212> DNA
<213> Artificial

<220>
<223> Oligonucleotide to act as a primer

<400> 129
gtgtgtacaa gacccgg 17

<210> 130
<211> 17
<212> DNA
<213> Artificial

<220>
<223> Oligonucleotide to act as a primer

<400> 130
ggtgtgtaca agacccg 17

<210> 131
<211> 17
<212> DNA
<213> Artificial

<220>
<223> Oligonucleotide to act as a primer

<400> 131
gcggtgtgta caagacc 17

<210> 132
<211> 2522
<212> DNA
<213> Artificial

<220>
<223> Oligonucleotide to act as a primer

<400> 132

```
aagcttacgt atacaacatg agaggtatgc ttccattgtt cgaacctaaa ggtagagtat     60
tgttggttga tggtcatcat ctagcttaca gaactttcca cgctctaaaa ggtttaacaa    120
catcaagagg tgaacctgtt caagctgtat acggttttgc taagtcttta ctaaaagcat    180
tgaaggaaga cggtgacgcc gttattgttg ttttcgatgc taaggcacca agttttagac    240
atgaagcata cggtggttat aaggctggaa gagcaccaac tcctgaagac ttccctagac    300
aattggcact aatcaaggaa ctagtcgact tactaggtct tgcaagatta gaagtcccag    360
gttatgaggc agatgatgta ctagcctctt tagcaaagaa ggcagaaaag gagggttatg    420
aagttagaat tttaaccgct gataaggact tatatcaatt gctatctgat aggattcatg    480
tgttacaccc tgaaggttat ttgataactc cagcttggtt atgggagaag tacggtttga    540
ggccagacca atgggccgat tatagagctt taaccggcga cgagtcagac aatcttccag    600
gtgttaaagg aattggcgaa aagactgcta ggaagttgtt ggaagagtgg ggctccttgg    660
aggccttact taaaaatttg gacaggctaa aaccagcaat cagggaaaag atactagctc    720
acatggatga tcttaaattg tcttgggact tagccaaggt cagaactgat ttgcctttag    780
aggtcgactt cgctaagaga agggaacctg atagggaaag gttaagagcc ttcttggaaa    840
gacttgagtt tggatcatta ttgcatgaat ttggtttatt agaatcccct aaggccttgg    900
aagaagcacc atggccacct ccagaaggtg cctttgtagg cttcgtctta agcaggaaag    960
aaccaatgtg ggcagactta ttggctctag ctgctgccag aggaggaaga gtgcatagag   1020
```

```
ccccagaacc atataaagcc ttgagagact tgaaggaagc aagaggtttg ttagctaaag   1080
atttgagcgt attagccttg agggaaggtt taggactacc accaggtgac gacccaatgt   1140
tgcttgctta tttgcttgat ccatcaaaca caacacctga aggagtagct agaaggtatg   1200
gtggagaatg gactgaagag gctggagaga gagccgctct atctgagaga ttgtttgcta   1260
atttgtgggg tagacttgaa ggtgaggaaa gattgttgtg gctatacagg gaagtagaaa   1320
ggccattatc tgcagtattg gctcatatgg aggccacagg cgttagatta gatgttgctt   1380
acttaagagc tttgtcattg gaagtcgccg aagaaattgc aagacttgaa gctgaggtgt   1440
tcagacttgc cggtcatcca ttcaatctta atagtagaga ccagctagaa agagtgttat   1500
tcgacgagct tggattacca gcaatcggaa agacagaaaa gactggtaaa aggtctacaa   1560
gtgccgccgt tttggaagca ttgagggagg cccatccaat tgttgaaaag atattgcagt   1620
atagagaatt gacaaaatta aaatcaactt atatcgatcc acttccagac ttaatccatc   1680
caaggacagg cagattacac accaggttta accagaccgc aactgctaca ggcagattat   1740
catcttcaga tcctaactta caaaacattc ctgtaaggac tccactaggt cagagaatta   1800
gaagagcttt tatcgctgag gaaggctggt tgcttgtggc tttagattat agtcaaattg   1860
agttaagggt cttggctcac ttgtctggtg acgaaaatct tatcagagtt tttcaggaag   1920
gtagggatat acatacagag accgcctcat ggatgtttgg tgttccaagg gaggccgtcg   1980
atccactaat gaggagagca gccaaaacta ttaactttgg agtattgtat ggtatgagtg   2040
ctcacagatt atcccaagag ttggccatcc cttacgagga agcacaggct tttatagaaa   2100
ggtatttcca gtcttttcct aaggttagag catggattga aaagacacta gaggaaggta   2160
ggaggagggg ttacgtggag accttattcg gaagaaggag atacgttcca gacttagagg   2220
ctagagtgaa atcagttaga gaagccgcag agagaatggc attcaatatg ccagtacaag   2280
gcactgccgc agatttgatg aaactagcca tggttaagct atttccaaga ttggaagaaa   2340
tgggagctag aatgctatta caagttcatg atgaacttgt tttagaggct cctaaagaaa   2400
gggctgaagc agtggccagg ttagctaaag aagtaatgga gggcgtttac ccattggcag   2460
ttcctttaga ggtcgaagtg ggtataggtg aagactggct atctgcaaag gaataagaat   2520
tc                                                                  2522
```

<210> 133
<211> 832
<212> PRT
<213> Thermus aquaticus

<400> 133

```
Met Arg Gly Met Leu Pro Leu Phe Glu Pro Lys Gly Arg Val Leu Leu
1               5                   10                  15
```

```
Val Asp Gly His His Leu Ala Tyr Arg Thr Phe His Ala Leu Lys Gly
            20                  25                  30

Leu Thr Thr Ser Arg Gly Glu Pro Val Gln Ala Val Tyr Gly Phe Ala
        35                  40                  45

Lys Ser Leu Leu Lys Ala Leu Lys Glu Asp Gly Asp Ala Val Ile Val
    50                  55                  60

Val Phe Asp Ala Lys Ala Pro Ser Phe Arg His Glu Ala Tyr Gly Gly
65                  70                  75                  80

Tyr Lys Ala Gly Arg Ala Pro Thr Pro Glu Asp Phe Pro Arg Gln Leu
                85                  90                  95

Ala Leu Ile Lys Glu Leu Val Asp Leu Leu Gly Leu Ala Arg Leu Glu
            100                 105                 110

Val Pro Gly Tyr Glu Ala Asp Asp Val Leu Ala Ser Leu Ala Lys Lys
        115                 120                 125

Ala Glu Lys Glu Gly Tyr Glu Val Arg Ile Leu Thr Ala Asp Lys Asp
    130                 135                 140

Leu Tyr Gln Leu Leu Ser Asp Arg Ile His Val Leu His Pro Glu Gly
145                 150                 155                 160

Tyr Leu Ile Thr Pro Ala Trp Leu Trp Glu Lys Tyr Gly Leu Arg Pro
                165                 170                 175

Asp Gln Trp Ala Asp Tyr Arg Ala Leu Thr Gly Asp Glu Ser Asp Asn
            180                 185                 190

Leu Pro Gly Val Lys Gly Ile Gly Glu Lys Thr Ala Arg Lys Leu Leu
        195                 200                 205

Glu Glu Trp Gly Ser Leu Glu Ala Leu Leu Lys Asn Leu Asp Arg Leu
    210                 215                 220

Lys Pro Ala Ile Arg Glu Lys Ile Leu Ala His Met Asp Asp Leu Lys
225                 230                 235                 240

Leu Ser Trp Asp Leu Ala Lys Val Arg Thr Asp Leu Pro Leu Glu Val
                245                 250                 255

Asp Phe Ala Lys Arg Arg Glu Pro Asp Arg Glu Arg Leu Arg Ala Phe
```

```
            260                 265                 270

Leu Glu Arg Leu Glu Phe Gly Ser Leu Leu His Glu Phe Gly Leu Leu
        275                 280                 285

Glu Ser Pro Lys Ala Leu Glu Glu Ala Pro Trp Pro Pro Pro Glu Gly
    290                 295                 300

Ala Phe Val Gly Phe Val Leu Ser Arg Lys Glu Pro Met Trp Ala Asp
305                 310                 315                 320

Leu Leu Ala Leu Ala Ala Ala Arg Gly Gly Arg Val His Arg Ala Pro
                325                 330                 335

Glu Pro Tyr Lys Ala Leu Arg Asp Leu Lys Glu Ala Arg Gly Leu Leu
            340                 345                 350

Ala Lys Asp Leu Ser Val Leu Ala Leu Arg Glu Gly Leu Gly Leu Pro
        355                 360                 365

Pro Gly Asp Asp Pro Met Leu Leu Ala Tyr Leu Leu Asp Pro Ser Asn
    370                 375                 380

Thr Thr Pro Glu Gly Val Ala Arg Arg Tyr Gly Gly Glu Trp Thr Glu
385                 390                 395                 400

Glu Ala Gly Glu Arg Ala Ala Leu Ser Glu Arg Leu Phe Ala Asn Leu
                405                 410                 415

Trp Gly Arg Leu Glu Gly Glu Glu Arg Leu Leu Trp Leu Tyr Arg Glu
            420                 425                 430

Val Glu Arg Pro Leu Ser Ala Val Leu Ala His Met Glu Ala Thr Gly
        435                 440                 445

Val Arg Leu Asp Val Ala Tyr Leu Arg Ala Leu Ser Leu Glu Val Ala
    450                 455                 460

Glu Glu Ile Ala Arg Leu Glu Ala Glu Val Phe Arg Leu Ala Gly His
465                 470                 475                 480

Pro Phe Asn Leu Asn Ser Arg Asp Gln Leu Glu Arg Val Leu Phe Asp
                485                 490                 495

Glu Leu Gly Leu Pro Ala Ile Gly Lys Thr Glu Lys Thr Gly Lys Arg
            500                 505                 510
```

```
Ser Thr Ser Ala Ala Val Leu Glu Ala Leu Arg Glu Ala His Pro Ile
        515                 520                 525

Val Glu Lys Ile Leu Gln Tyr Arg Glu Leu Thr Lys Leu Lys Ser Thr
    530                 535                 540

Tyr Ile Asp Pro Leu Pro Asp Leu Ile His Pro Arg Thr Gly Arg Leu
545                 550                 555                 560

His Thr Arg Phe Asn Gln Thr Ala Thr Ala Thr Gly Arg Leu Ser Ser
                565                 570                 575

Ser Asp Pro Asn Leu Gln Asn Ile Pro Val Arg Thr Pro Leu Gly Gln
            580                 585                 590

Arg Ile Arg Arg Ala Phe Ile Ala Glu Glu Gly Trp Leu Leu Val Ala
        595                 600                 605

Leu Asp Tyr Ser Gln Ile Glu Leu Arg Val Leu Ala His Leu Ser Gly
    610                 615                 620

Asp Glu Asn Leu Ile Arg Val Phe Gln Glu Gly Arg Asp Ile His Thr
625                 630                 635                 640

Glu Thr Ala Ser Trp Met Phe Gly Val Pro Arg Glu Ala Val Asp Pro
                645                 650                 655

Leu Met Arg Arg Ala Ala Lys Thr Ile Asn Phe Gly Val Leu Tyr Gly
            660                 665                 670

Met Ser Ala His Arg Leu Ser Gln Glu Leu Ala Ile Pro Tyr Glu Glu
        675                 680                 685

Ala Gln Ala Phe Ile Glu Arg Tyr Phe Gln Ser Phe Pro Lys Val Arg
    690                 695                 700

Ala Trp Ile Glu Lys Thr Leu Glu Glu Gly Arg Arg Arg Gly Tyr Val
705                 710                 715                 720

Glu Thr Leu Phe Gly Arg Arg Arg Tyr Val Pro Asp Leu Glu Ala Arg
                725                 730                 735

Val Lys Ser Val Arg Glu Ala Ala Glu Arg Met Ala Phe Asn Met Pro
            740                 745                 750

Val Gln Gly Thr Ala Ala Asp Leu Met Lys Leu Ala Met Val Lys Leu
        755                 760                 765
```

```
Phe Pro Arg Leu Glu Glu Met Gly Ala Arg Met Leu Leu Gln Val His
    770                 775                 780

Asp Glu Leu Val Leu Glu Ala Pro Lys Glu Arg Ala Glu Ala Val Ala
785                 790                 795                 800

Arg Leu Ala Lys Glu Val Met Glu Gly Val Tyr Pro Leu Ala Val Pro
                805                 810                 815

Leu Glu Val Glu Val Gly Ile Gly Glu Asp Trp Leu Ser Ala Lys Glu
            820                 825                 830
```

<210> 134
<211> 19
<212> DNA
<213> Artificial

<220>
<223> Oligonucleotide to act as a primer

<400> 134
gtagaggact cagatattg 19

<210> 135
<211> 30
<212> DNA
<213> Artificial

<220>
<223> Oligonucleotide to act as a primer

<400> 135
gttttgaatt cgatttatct tcgtttcctg 30

<210> 136
<211> 36
<212> DNA
<213> Artificial

<220>
<223> Oligonucleotide to act as a primer

<400> 136
gataaatcga attcaaaact gtattataag taaatg 36

<210> 137
<211> 19
<212> DNA
<213> Artificial

<220>
<223> Oligonucleotide to act as a primer

<400> 137
catgctaaaa atgggctac 19